(19) Europäisches Patentamt
European Patent Office
Office européen des brevets

(11) EP 2 808 324 A1

(12) **EUROPEAN PATENT APPLICATION**
published in accordance with Art. 153(4) EPC

(43) Date of publication:
**03.12.2014 Bulletin 2014/49**

(21) Application number: **13740939.7**

(22) Date of filing: **24.01.2013**

(51) Int Cl.:
***C07D 403/04*** *(2006.01)* ***A61K 31/41*** *(2006.01)*
***A61K 31/422*** *(2006.01)* ***A61K 31/4245*** *(2006.01)*
***A61K 31/454*** *(2006.01)* ***A61K 31/496*** *(2006.01)*
***A61P 35/00*** *(2006.01)* ***C07D 413/04*** *(2006.01)*
***C07D 413/14*** *(2006.01)*

(86) International application number:
**PCT/JP2013/051497**

(87) International publication number:
**WO 2013/111831 (01.08.2013 Gazette 2013/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2012 JP 2012012561**

(71) Applicant: **Kabushiki Kaisha Yakult Honsha**
**Tokyo 105-8660 (JP)**

(72) Inventors:
- **IKEDA, Takashi**
**Tokyo 105-8660 (JP)**
- **ONO, Masahiro**
**Tokyo 105-8660 (JP)**
- **UENO, Satoshi**
**Tokyo 105-8660 (JP)**
- **YAMAZAKI, Ryuta**
**Tokyo 105-8660 (JP)**
- **YAEGASHI, Takashi**
**Tokyo 105-8660 (JP)**
- **MATSUZAKI, Takeshi**
**Tokyo 105-8660 (JP)**

(74) Representative: **Blodig, Wolfgang**
**Wächtershäuser & Hartz**
**Patentanwaltspartnerschaft mbB**
**Weinstrasse 8**
**80333 München (DE)**

# (54) PYRROLE COMPOUND

(57) Provided is a highly safe and effective compound represented by the following general formula (1) or a salt thereof which acts on tubulin and has an anti-cancer effect, wherein Ar represents an aryl group or a heteroaryl group; $Z^1$, $Z^2$, $Z^3$, and $Z^4$ each independently represent CH, a nitrogen atom, an oxygen atom, or a sulfur atom; $R^1$ represents a hydrogen atom, a halogen atom, an alkyl group, or the like; $R^2$ and $R^3$ are the same or different and each represent a hydrogen atom, a halogen atom, a carboxyl group, or the like; n units of $R^4$ are the same or different and each represent a hydrogen atom, an alkyl group, or the like; and n represents a number of 0 to 4.

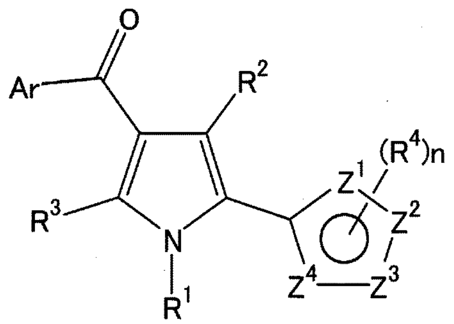

(1)



Printed by Jouve, 75001 PARIS (FR)

## Description

[Technical Field]

The present invention relates to a pyrrole compound which has an excellent tubulin polymerization inhibitory effect and is useful as a pharmaceutical drug such as an anticancer agent, and to a pharmaceutical drug comprising the same.

[Background Art]

Tubulin is a major protein constituting the microtubule which plays an important role in cell division, and is also important as a target of an anticancer agent. Vinca alkaloids or taxanes are anticancer agents targeting tubulin. The vinca alkaloids promote the dissociation of the microtubule, while the taxanes stabilize the microtubule to inhibit cell division. Many other anticancer agents targeting the tubulin have been developed (Non Patent Documents 1 to 3).

[Citation List]

[Non Patent Document]

[Non Patent Document 1] Expert. Opin. Investig. Drugs (2008) 17 (5), 707-722
[Non Patent Document 2] Oncogene (2003) 22, 7280-7295
[Non Patent Document 3] Cancer Investigation, 23: 264-273, 2005

[Summary of the Invention]

[Problems to be Solved by the Invention]

These existing drugs acting on tubulin, however, have an insufficient anticancer effect and also insufficiently render the anticancer effect distinct from their effects on normal cells. In addition, problems such as the emergence of drug resistance have been pointed out.

Thus, an object of the present invention is to provide a highly safe and effective compound which acts on tubulin and has anticancer activity.

[Means for Solving the Problems]

Accordingly, the present inventors have synthesized a large number of compounds and studied their binding activity against tubulin, effects on tubulin polymerization, anticancer activity, drug resistance, and the like. As a result, the present inventors have found that a pyrrole compound represented by the general formula (1) shown below (hereinafter, also referred to as "pyrrole compound (1)") or a salt thereof acts on tubulin, has excellent cytotoxic activity against tumor cells, and exhibits cytotoxic activity even against drug-resistant lines. On the basis of these findings, the present invention has been completed.

Specifically, the present invention relates to the following [1] to [24]:

[1] A compound represented by the general formula (1) or a salt thereof:

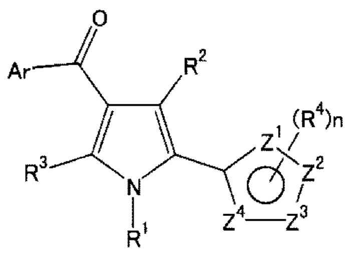

(1)

wherein Ar represents an optionally substituted aryl group or an optionally substituted heteroaryl group;
$Z^1$, $Z^2$, $Z^3$, and $Z^4$ each independently represent C, CH, a nitrogen atom, an oxygen atom, or a sulfur atom;

$R^1$ represents a hydrogen atom, a halogen atom, an optionally substituted alkyl group, an optionally substituted cycloalkyl group, an optionally substituted alkylcarbonyl group, an optionally substituted alkylsulfonyl group, an optionally substituted cycloalkylsulfonyl group, an optionally substituted aryl group, an optionally substituted aralkyl group, or an optionally substituted arylcarbonyl group;
$R^2$ and $R^3$ are the same or different and each represent a hydrogen atom, a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, an optionally substituted arylcarbonyl group, or an optionally substituted dithianyl group;
n units of $R^4$ are the same or different and each represent a hydrogen atom, an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group; and
n represents a number of 0 to 4.
[2] The compound according to [1] or a salt thereof, wherein in the general formula (1), $R^2$ is a halogen atom, a formyl group, or an optionally substituted alkyl group having 1 to 8 carbon atoms.
[3] The compound according to [1] or [2] or a salt thereof, wherein in the general formula (1), at least one of $Z^1$, $Z^2$, $Z^3$, and $Z^4$ is a nitrogen atom.
[4] The compound according to any one of [1] to [3] or a salt thereof, wherein in the general formula (1), the ring constituted by $Z^1$, $Z^2$, $Z^3$, and $Z^4$ has a structure represented by any of the following formulas (2a) to (2e) :

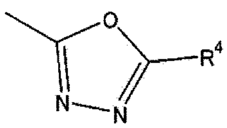
(2a)

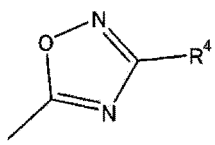
(2b)

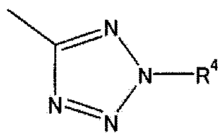
(2c)

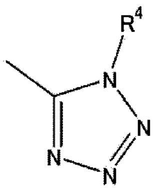
(2d)

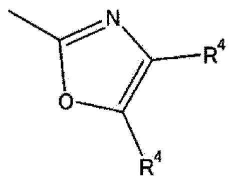
(2e)

wherein $R^4$ is as defined above.
[5] The compound according to any one of [1] to [4] or a salt thereof, wherein Ar is an optionally substituted aryl group having 6 to 14 carbon atoms or an optionally substituted 5- to 10-membered heteroaryl group.
[6] The compound according to any one of [1] to [5] or a salt thereof, wherein Ar is an optionally substituted aryl group having 6 to 14 carbon atoms.
[7] A compound selected from the following or a salt thereof:

(3-bromophenyl)(2,4-dimethyl-5-(1H-tetrazol-5-yl)-1H-pyrrol-3-yl)methanone [compound No. 96]
(3-bromophenyl)(5-(2-ethyl-2H-tetrazol-5-yl)-2,4-dimethyl-1H-pyrrol-3-yl)methanone [compound No. 97]
(3-bromophenyl)(5-(1-ethyl-1H-tetrazol-5-yl)-2,4-dimethyl-1H-pyrrol-3-yl)methanone [compound No. 98]
(3-bromophenyl)(2,4-dimethyl-5-(2-(2,2,2-trifluoroethyl)-2H-tetrazol-5-yl)-1H-pyrrol-3-yl)methanone [compound No. 99]
(3-bromophenyl)(2,4-dimethyl-5-(1-(2,2,2-trifluoroethyl)-1H-tetrazol-5-yl)-1H-pyrrol-3-yl)methanone [compound No. 100]
(3-bromophenyl)(5-(2-(2-(dimethylamino)ethyl)-2H-tetrazol-5-yl)-2,4-dimethyl-1H-pyrrol-3-yl)methanone [compound No. 101]
(3-bromophenyl)(5-(2-(2-hydroxyethyl)-2H-tetrazol-5-yl)-2,4-dimethyl-1H-pyrrol-3-yl)methanone [compound No. 102]

(5-((2-(2-aminoethyl)-2H-tetrazol-5-yl)-2,4-dimethyl-1H-pyrrol-3-yl)(3-bromophenyl)methanone [compound No. 103]
2-((5-(4-(3-bromobenzoyl)-3,5-dimethyl-1H-pyrrol-2-yl)-2H-tetrazol-2-yl)ethyl acetate [compound No. 104]
(2,4-dimethyl-5-(1H-tetrazol-5-yl)-1H-pyrrol-3-yl)(3-methoxyphenyl)methanone [compound No. 105]
(3-methoxyphenyl)(5-(2-ethyl-2H-tetrazol-5-yl)-2,4-dimethyl-1H-pyrrol-3-yl)methanone [compound No. 106]
(5-((2-ethyl-2H-tetrazol-5-yl)-2,4-dimethyl-1H-pyrrol-3-yl)(3-methoxyphenyl)methanone [compound No. 107]
(3-bromo-4-methoxyphenyl)(2,4-dimethyl-5-(1H-tetrazol-5-yl)-1H-pyrrol-3-yl)methanone [compound No. 108]
(3-bromo-4-methoxyphenyl)(5-(2-ethyl-2H-tetrazol-5-yl)-2,4-dimethyl-1H-pyrrol-3-yl)methanone [compound No. 109]
(3-bromo-4-methoxyphenyl)(5-(2-(2-hydroxyethyl)-2H-tetrazol-5-yl)-2,4-dimethyl-1H-pyrrol-3-yl)methanone [compound No. 110]
2-((5-(4-(3-bromo-4-methoxybenzoyl)-3,5-dimethyl-1H-pyrrol-2-yl)-2H-tetrazol-2-yl)ethyl acetate [compound No. 111]
(3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone [compound No. 112]
(3-cyclopropylphenyl)(2,4-dimethyl-5-(1H-tetrazol-5-yl)-1H-pyrrol-3-yl)methanone [compound No. 113]
2-((5-(4-(3-cyclopropylbenzoyl)-3,5-dimethyl-1H-pyrrol-2-yl)ethyl acetate [compound No. 114]
(3-cyclopropylphenyl)(5-(2-(2-hydroxyethyl)-2H-tetrazol-5-yl)-2,4-dimethyl-1H-pyrrol-3-yl)methanone [compound No. 115]
2-((5-(4-(3-bromobenzoyl)-5-formyl-3-methyl-1H-pyrrol-2-yl)-2H-tetrazol-2-yl)ethyl acetate [compound No. 116]
3-((3-bromobenzoyl)-5-(2-(2-hydroxyethyl)-2H-tetrazol-5-yl)-4-methyl-1H-pyrrole-2-carboaldehyde [compound No. 117]
2-((5-(4-(3-bromobenzoyl)-5-(difluoromethyl)-3-methyl-1H-pyrrol-2-yl)-2H-tetrazol-2-yl)ethyl acetate [compound No. 118]
(3-bromophenyl)(2-(difluoromethyl)-5-(2-(2-hydroxyethyl)-2H-tetrazol-5-yl)-4-methyl-1H-pyrrol-3-yl)methanone [compound No. 119]
2-((5-(4-(3-bromobenzoyl)-5-(hydroxymethyl)-3-methyl-1H-pyrrol-2-yl)-2H-tetrazol-2-yl)ethyl acetate [compound No. 120]
(3-bromophenyl)(5-(2-(2-hydroxyethyl)-2H-tetrazol-5-yl)-2-(hydroxymethyl)-4-methyl-1H-pyrrol-3-yl)methanone [compound No. 121]
2-((5-(4-(3-bromobenzoyl)-5-(1-hydroxyethyl)-3-methyl-1H-pyrrol-2-yl)-2H-tetrazol-2-yl)ethyl acetate [compound No. 122]
(3-bromophenyl)(2-(1-hydroxyethyl)-5-(2-(2-hydroxyethyl)-2H-tetrazol-5-yl)-4-methyl-1H-pyrrol-3-yl)methanone [compound No. 123]
2-((5-(4-(3-bromo-4-methoxybenzoyl)-5-formyl-3-methyl-1H-pyrrol-2-yl)-2H-tetrazol-2-yl)ethyl acetate [compound No. 124]
3-((3-bromo-4-methoxybenzoyl)-5-(2-(2-hydroxyethyl)-2H-tetrazol-5-yl)-4-methyl-1H-pyrrole-2-carboaldehyde [compound No. 125]
2-((5-(4-(3-bromo-4-methoxybenzoyl)-5-(difluoromethyl)-3-methyl-1H-pyrrol-2-yl)-2H-tetrazol-2-yl)ethyl acetate [compound No. 126]
(3-bromo-4-methoxyphenyl)(2-(difluoromethyl)-5-(2-(2-hydroxyethyl)-2H-tetrazol-5-yl)-4-methyl-1H-pyrrol-3-yl)methanone [compound No. 127]
2-((5-(4-(3-bromo-4-methoxybenzoyl)-5-(hydroxymethyl)-3-methyl-1H-pyrrol-2-yl)-2H-tetrazol-2-yl)ethyl acetate [compound No. 128]
(3-bromo-4-methoxyphenyl)(5-(2-(2-hydroxyethyl)-2H-tetrazol-5-yl)-2-(hydroxymethyl)-4-methyl-1H-pyrrol-3-yl)methanone [compound No. 129]
2-((5-(4-(3-bromo-4-methoxybenzoyl)-5-(1-hydroxyethyl)-3-methyl-1H-pyrrol-2-yl)-2H-tetrazol-2-yl)ethyl acetate [compound No. 130]
(3-bromo-4-methoxyphenyl)(2-(1-hydroxyethyl)-5-(2-(2-hydroxyethyl)-2H-tetrazol-5-yl)-4-methyl-1H-pyrrol-3-yl)methanone [compound No. 131]
2-((5-(4-(3-bromobenzoyl)-5-cyano-3-methyl-1H-pyrrol-2-yl)-2H-tetrazol-2-yl)ethyl acetate [compound No. 132]
3-((3-bromobenzoyl)-5-(2-(2-hydroxyethyl)-2H-tetrazol-5-yl)-4-methyl-1H-pyrrole-2-carbonitrile [compound No. 133] 5-(2-(2-acetoxyethyl)-2H-tetrazol-5-yl)-3-(3-bromobenzoyl)-4-methyl-1H-pyrrole-2-carboxylic acid [compound No. 134]
3-((3-bromobenzoyl)-5-(2-(2-hydroxyethyl)-2H-tetrazol-5-yl)-4-methyl-1H-pyrrole-2-carboxylic acid [compound No. 135]
methyl 5-(2-(2-acetoxyethyl)-2H-tetrazol-5-yl)-3-(3-bromobenzoyl)-4-methyl-1H-pyrrole-2-carboxylate [compound No. 136]

methyl 3-(3-bromobenzoyl)-5-(2-(2-hydroxyethyl)-2H-tetrazol-5-yl)-4-methyl-1H-pyrrole-2-carboxylate [compound No. 137]
2-((5-(4-(3-bromo-4-methoxybenzoyl)-5-cyano-3-methyl-1H-pyrrol-2-yl)-2H-tetrazol-2-yl)ethyl acetate [compound No. 138]
3-((3-bromo-4-methoxybenzoyl)-5-(2-(2-hydroxyethyl)-2H-tetrazol-5-yl)-4-methyl-1H-pyrrole-2-carbonitrile [compound No. 139]
5-((2-(2-acetoxyethyl)-2H-tetrazol-5-yl)-3-(3-bromo-4-methoxybenzoyl)-4-methyl-1H-pyrrole-2-carboxylic acid [compound No. 140]
3-((3-bromo-4-methoxybenzoyl)-5-(2-(2-hydroxyethyl)-2H-tetrazol-5-yl)-4-methyl-1H-pyrrole-2-carboxylic acid [compound No. 141]
3-((3-bromobenzoyl)-5-(2-(2-hydroxyethyl)-2H-tetrazol-5-yl)-N,4-dimethyl-1H-pyrrole-2-carboxamide [compound No. 142]
3-((3-bromobenzoyl)-5-(2-(2-hydroxyethyl)-2H-tetrazol-5-yl)-N,N,4-trimethyl-1H-pyrrole-2-carboxamide [compound No. 143]
N-(2-aminoethyl)-3-(3-bromobenzoyl)-5-(2-(2-hydroxyethyl)-2H-tetrazol-5-yl)-4-methyl-1H-pyrrole-2-carboxamide [compound No. 144]
3-((3-bromobenzoyl)-N-ethyl-5-(2-(2-hydroxyethyl)-2H-tetrazol-5-yl)-4-methyl-1H-pyrrole-2-carboxamide [compound No. 145]
3-((3-bromobenzoyl)-5-(2-(2-hydroxyethyl)-2H-tetrazol-5-yl)-N-isopropyl-4-methyl-1H-pyrrole-2-carboxamide [compound No. 146]
(3-((3-bromobenzoyl)-5-(2-(2-hydroxyethyl)-2H-tetrazol-5-yl)-4-methyl-1H-pyrrol-2-yl)(4-methylpiperazin-1-yl)methanone [compound No. 147]
(3-((3-bromobenzoyl)-5-(2-(2-hydroxyethyl)-2H-tetrazol-5-yl)-4-methyl-1H-pyrrol-2-yl)(piperazin-1-yl)methanone [compound No. 148]
3-((3-bromo-4-methoxybenzoyl)-5-(2-(2-hydroxyethyl)-2H-tetrazol-5-yl)-N,4-dimethyl-1H-pyrrole-2-carboxamide [compound No. 149]
3-((3-bromo-4-methoxybenzoyl)-5-(2-(2-hydroxyethyl)-2H-tetrazol-5-yl)-4-methyl-1H-pyrrole-2-carboxamide [compound No. 150]
2-((5-(4-(3-bromobenzoyl)-3,5-dimethyl-1H-pyrrol-2-yl)-2H-tetrazol-2-yl)ethyl 2-((tert-butoxycarbonyl)amino)acetate [compound No. 151]
2-((5-(4-(3-bromobenzoyl)-3,5-dimethyl-1H-pyrrol-2-yl)-2H-tetrazol-2-yl)ethyl 2-aminoacetate [compound No. 152]
2-((5-(4-(3-bromobenzoyl)-5-formyl-3-methyl-1H-pyrrol-2-yl)-2H-tetrazol-2-yl)ethyl 2-((tert-butoxycarbonyl)amino)acetate [compound No. 153]
2-((5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate [compound No. 154]
2-((5-(4-(3-bromobenzoyl)-5-(hydroxymethyl)-3-methyl-1H-pyrrol-2-yl)-2H-tetrazol-2-yl)ethyl 2-aminoacetate [compound No. 155]
methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate [compound No. 156]
methyl 5-(2-(2-(2-aminoacetoxy)ethyl)-2H-tetrazol-5-yl)-3-(3-bromobenzoyl)-4-methyl-1H-pyrrole-2-carboxylate [compound No. 157]
2-((5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate [compound No. 158]
2-((5-(4-(3-bromobenzoyl)-3-methyl-5-(methylcarbamoyl)-1H-pyrrol-2-yl)-2H-tetrazol-2-yl)ethyl 2-aminoacetate [compound No. 159]
2-((5-(3,5-dimethyl-4-(3-vinylbenzoyl)-1H-pyrrol-2-yl)-2H-tetrazol-2-yl)ethyl acetate [compound No. 160]
2-((5-(4-(3-formylbenzoyl)-3,5-dimethyl-1H-pyrrol-2-yl)-2H-tetrazol-2-yl)ethyl acetate [compound No. 161]
2-((5-(4-(3-(hydroxymethyl)benzoyl)-3,5-dimethyl-1H-pyrrol-2-yl)-2H-tetrazol-2-yl)ethyl acetate [compound No. 162]
(5-((2-(2-hydroxyethyl)-2H-tetrazol-5-yl)-2,4-dimethyl-1H-pyrrol-3-yl)(3-(hydroxymethyl)phenyl)methanone [compound No. 163]
(5-((2-ethyl-2H-tetrazol-5-yl)-2,4-dimethyl-1H-pyrrol-3-yl)(3-hydroxyphenyl)methanone [compound No. 164]
(3-((2-aminoethoxy)phenyl)(5-(2-ethyl-2H-tetrazol-5-yl)-2,4-dimethyl-1H-pyrrol-3-yl)methanone [compound No. 165]
(3-bromo-4-hydroxyphenyl)(5-(2-ethyl-2H-tetrazol-5-yl)-2,4-dimethyl-1H-pyrrol-3-yl)methanone [compound No. 166]
(4-((2-aminoethoxy)-3-bromophenyl)(5-(2-ethyl-2H-tetrazol-5-yl)-2,4-dimethyl-1H-pyrrol-3-yl)methanone

[compound No. 167]
(4-((2-aminoethoxy)-3-bromophenyl)(5-(2-(2-hydroxyethyl)-2H-tetrazol-5-yl)-2,4-dimethyl-1H-pyrrol-3-yl)methanone [compound No. 168]
(E)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 169]
(Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 170]
2-((5-(4-(3-bromobenzoyl)-5-(chloromethyl)-3-methyl-1H-pyrrol-2-yl)-2H-tetrazol-2-yl)ethyl acetate [compound No. 171]
2-((5-(4-(3-bromo-4-methoxybenzoyl)-5-(chloromethyl)-3-methyl-1H-pyrrol-2-yl)-2H-tetrazol-2-yl)ethyl acetate [compound No. 172]
(3-bromophenyl)(5-(5-ethyl-1,3,4-oxadiazol-2-yl)-2,4-dimethyl-1H-pyrrol-3-yl)methanone [compound No. 173]
(3-bromophenyl)(5-(5-(2-chloroethyl)-1,3,4-oxadiazol-2-yl)-2,4-dimethyl-1H-pyrrol-3-yl)methanone [compound No. 174]
(3-bromophenyl)(2,4-dimethyl-5-(5-phenyl-1,3,4-oxadiazol-2-yl)-1H-pyrrol-3-yl)methanone [compound No. 175]
(5-((5-benzyl-1,3,4-oxadiazol-2-yl)-2,4-dimethyl-1H-pyrrol-3-yl)(3-bromophenyl)methanone [compound No. 176]
(3-bromophenyl)(5-(5-cyclopropyl-1,3,4-oxadiazol-2-yl)-2,4-dimethyl-1H-pyrrol-3-yl)methanone [compound No. 177]
(3-bromophenyl)(5-(5-isopropyl-1,3,4-oxadiazol-2-yl)-2,4-dimethyl-1H-pyrrol-3-yl)methanone [compound No. 178]
(3-bromophenyl)(5-(5-cyclobutyl-1,3,4-oxadiazol-2-yl)-2,4-dimethyl-1H-pyrrol-3-yl)methanone [compound No. 179]
(3-bromophenyl)(5-(5-cyclopentyl-1,3,4-oxadiazol-2-yl)-2,4-dimethyl-1H-pyrrol-3-yl)methanone [compound No. 180]
(3-bromophenyl)(5-(5-cyclohexyl-1,3,4-oxadiazol-2-yl)-2,4-dimethyl-1H-pyrrol-3-yl)methanone [compound No. 181]
methyl 3-(5-(4-(3-bromobenzoyl)-3,5-dimethyl-1H-pyrrol-2-yl)-1,3,4-oxadiazol-2-yl)propionate [compound No. 182]
ethyl 2-(5-(4-(3-bromobenzoyl)-3,5-dimethyl-1H-pyrrol-2-yl)-1,3,4-oxadiazol-2-yl)acetate [compound No. 183]
(3-bromophenyl)(5-(5-tert-butyl)-1,3,4-oxadiazol-2-yl)-2,4-dimethyl-1H-pyrrol-3-yl)methanone [compound No. 184]
(3-bromophenyl)(2,4-dimethyl-5-(5-methyl-1,3,4-oxadiazol-2-yl)-1H-pyrrol-3-yl)methanone [compound No. 185]
(3-bromophenyl)(5-(5-(difluoromethyl)-1,3,4-oxadiazol-2-yl)-2,4-dimethyl-1H-pyrrol-3-yl)methanone [compound No. 186]
(3-bromophenyl)(2,4-dimethyl-5-(5-(2,2,2-trifluoroethyl)-1,3,4-oxadiazol-2-yl)-1H-pyrrol-3-yl)methanone [compound No. 187]
(3-bromophenyl)(5-(5-(1-hydroxycyclopropyl)-1,3,4-oxadiazol-2-yl)-2,4-dimethyl-1H-pyrrol-3-yl)methanone [compound No. 188]
(3-bromophenyl)(2,4-dimethyl-5-(5-(1-methylcyclopropyl)-1,3,4-oxadiazol-2-yl)-1H-pyrrol-3-yl)methanone [compound No. 189]
(3-bromophenyl)(2,4-dimethyl-5-(5-(2-methylcyclopropyl)-1,3,4-oxadiazol-2-yl)-1H-pyrrol-3-yl)methanone [compound No. 190]
(3-bromophenyl)(5-(5-(2,2-dimethylcyclopropyl)-1,3,4-oxadiazol-2-yl)-2,4-dimethyl-1H-pyrrol-3-yl)methanone [compound No. 191]
(3-bromophenyl)(2,4-dimethyl-5-(5-(2,2,3,3-tetramethylcyclopropyl)-1,3,4-oxadiazol-2-yl)-1H-pyrrol-3-yl)methanone [compound No. 192]
(5-((5-ethyl-1,3,4-oxadiazol-2-yl)-2,4-dimethyl-1H-pyrrol-3-yl)(3-methoxyphenyl)methanone [compound No. 193]
(5-((5-cyclopropyl-1,3,4-oxadiazol-2-yl)-2,4-dimethyl-1H-pyrrol-3-yl)(3-methoxyphenyl)methanone [compound No. 194]
(5-((5-ethyl-1,3,4-oxadiazol-2-yl)-2,4-dimethyl-1H-pyrrol-3-yl)(3-nitrophenyl)methanone [compound No. 195]
(5-((5-cyclopropyl-1,3,4-oxadiazol-2-yl)-2,4-dimethyl-1H-pyrrol-3-yl)(3-nitrophenyl)methanone [compound No. 196]
(3-bromo-4-methoxyphenyl)(5-(5-ethyl-1,3,4-oxadiazol-2-yl)-2,4-dimethyl-1H-pyrrol-3-yl)methanone [compound No. 197]

(3-bromo-4-methoxyphenyl)(5-(5-cyclopropyl-1,3,4-oxadiazol-2-yl)-2,4-dimethyl-1H-pyrrol-3-yl)methanone [compound No. 198]
(5-((5-cyclopropyl-1,3,4-oxadiazol-2-yl)-2,4-dimethyl-1H-pyrrol-3-yl)(3-(hydroxymethyl)phenyl)methanone [compound No. 199]
(3-bromophenyl)(5-(5-cyclopropyl-1,3,4-oxadiazol-2-yl)-2-(1,3-dithian-2-yl)-4-methyl-1H-pyrrol-3-yl)methanone [compound No. 200]
(5-bromo-2-methoxyphenyl)(5-(5-(chloromethyl)-1,3,4-oxadiazol-2-yl)-2,4-dimethyl-1H-pyrrol-3-yl)methanone [compound No. 201]
(5-((4-(5-bromo-2-methoxybenzoyl)-3,5-dimethyl-1H-pyrrol-2-yl)-1,3,4-oxadiazol-2-yl)methyl acetate [compound No. 202]
(5-bromo-2-hydroxyphenyl)(5-(5-(hydroxymethyl)-1,3,4-oxadiazol-2-yl)-2,4-dimethyl-1H-pyrrol-3-yl)methanone [compound No. 203]
2-((5-(4-(3-bromobenzoyl)-3,5-dimethyl-1H-pyrrol-2-yl)-1,3,4-oxadiazol-2-yl)ethyl acetate [compound No. 204]
(3-bromophenyl)(5-(5-(2-hydroxyethyl)-1,3,4-oxadiazol-2-yl)-2,4-dimethyl-1H-pyrrol-3-yl)methanone [compound No. 205]
(3-bromophenyl)(2,4-dimethyl-5-(5-vinyl-1,3,4-oxadiazol-2-yl)-1H-pyrrol-3-yl)methanone [compound No. 206]
(3-bromophenyl)(5-(5-(chloromethyl)-1,3,4-oxadiazol-2-yl)-2,4-dimethyl-1H-pyrrol-3-yl)methanone [compound No. 207]
(3-bromophenyl)(2,4-dimethyl-5-(5-((4-methylpiperazin-1-yl)methyl)-1,3,4-oxadiazol-2-yl)-1H-pyrrol-3-yl)methanone [compound No. 208]
(3-bromophenyl)(5-(5-((dimethylamino)methyl)-1,3,4-oxadiazol-2-yl)-2,4-dimethyl-1H-pyrrol-3-yl)methanone [compound No. 209]
(5-((4-(3-bromobenzoyl)-3,5-dimethyl-1H-pyrrol-2-yl)-1,3,4-oxadiazol-2-yl)methyl acetate [compound No. 210]
(3-bromophenyl)(5-(5-(hydroxymethyl)-1,3,4-oxadiazol-2-yl)-2,4-dimethyl-1H-pyrrol-3-yl)methanone [compound No. 211]
S-((5-(4-(3-bromobenzoyl)-3,5-dimethyl-1H-pyrrol-2-yl)-1,3,4-oxadiazol-2-yl)methyl)ethanethioate [compound No. 212]
2-(((5-(4-(3-bromobenzoyl)-3,5-dimethyl-1H-pyrrol-2-yl)-1,3,4-oxadiazol-2-yl)methyl)isoindole-1,3-dione [compound No. 213]
(5-((5-(aminomethyl)-1,3,4-oxadiazol-2-yl)-2,4-dimethyl-1H-pyrrol-3-yl)(3-bromophenyl)methanone [compound No. 214]
N-((5-(4-(3-bromobenzoyl)-3,5-dimethyl-1H-pyrrol-2-yl)-1,3,4-oxadiazol-2-yl)methyl)acetamide [compound No. 215]
methyl((5-(4-(3-bromobenzoyl)-3,5-dimethyl-1H-pyrrol-2-yl)-1,3,4-oxadiazol-2-yl)methyl)carbamate [compound No. 216]
N-((5-(4-(3-bromobenzoyl)-3,5-dimethyl-1H-pyrrol-2-yl)-1,3,4-oxadiazol-2-yl)methyl)methanesulfonamide [compound No. 217]
1-(((5-(4-(3-bromobenzoyl)-3,5-dimethyl-1H-pyrrol-2-yl)-1,3,4-oxadiazol-2-yl)methyl)-3-ethylurea [compound No. 218]
1-(((5-(4-(3-bromobenzoyl)-3,5-dimethyl-1H-pyrrol-2-yl)-1,3,4-oxadiazol-2-yl)methyl)-3-ethylthiourea [compound No. 219]
3-(((5-(4-(3-bromobenzoyl)-3,5-dimethyl-1H-pyrrol-2-yl)-1,3,4-oxadiazol-2-yl)methyl)-1,1-dimethylthiourea [compound No. 220]
2-((((5-(4-(3-bromobenzoyl)-3,5-dimethyl-1H-pyrrol-2-yl)-1,3,4-oxadiazol-2-yl)methyl)amino)-2-oxoethyl acetate [compound No. 221]
N-((5-(4-(3-bromobenzoyl)-3,5-dimethyl-1H-pyrrol-2-yl)-1,3,4-oxadiazol-2-yl)methyl)-2-hydroxyacetamide [compound No. 222]
(3-bromophenyl)(5-(5-ethyl-1,3,4-oxadiazol-2-yl)-1,2,4-trimethyl-1H-pyrrol-3-yl)methanone [compound No. 223]
1-((3-(3-bromobenzoyl)-5-(5-ethyl-1,3,4-oxadiazol-2-yl)-2,4-dimethyl-1H-pyrrol-1-yl)ethanone [compound No. 224]
(3-bromophenyl)(5-(5-ethyl-1,3,4-oxadiazol-2-yl)-2,4-dimethyl-1-(methylsulfonyl)-1H-pyrrol-3-yl)methanone [compound No. 225]
2-((3-(3-bromobenzoyl)-5-(5-ethyl-1,3,4-oxadiazol-2-yl)-2,4-dimethyl-1H-pyrrol-1-yl)-2-oxoethyl acetate [compound No. 226]
(3-bromophenyl)(1-(cyclopropylsulfonyl)-5-(5-ethyl-1,3,4-oxadiazol-2-yl)-2,4-dimethyl-1H-pyrrol-3-yl)methanone [compound No. 227]
(3-bromophenyl)(1-(difluoromethyl)-5-(5-ethyl-1,3,4-oxadiazol-2-yl)-2,4-dimethyl-1H-pyrrol-3-yl)methanone

[compound No. 228]
(3-bromophenyl)(5-(5-ethyl-1,3,4-oxadiazol-2-yl)-2,4-dimethyl-1-(2,2,2-trifluoroethyl)-1H-pyrrol-3-yl)methanone [compound No. 229]
(3-bromophenyl)(1-cyclopropyl-5-(5-ethyl-1,3,4-oxadiazol-2-yl)-2,4-dimethyl-1H-pyrrol-3-yl)methanone [compound No. 230]
tert-butyl(2-(3-(3-bromobenzoyl)-5-(5-ethyl-1,3,4-oxadiazol-2-yl)-2,4-dimethyl-1H-pyrrol-1-yl)ethyl)carbamate [compound No. 231]
(3-bromophenyl)(1-ethyl-2,4-dimethyl-5-(5-ethyl-1,3,4-oxadiazol-2yl)-1H-pyrrol-3-yl)methanone [compound No. 232]
(3-bromophenyl)(1-isopropyl-2,4-dimethyl-5-(5-ethyl-1,3,4-oxadiazol-2-yl)-1H-pyrrol-3-yl)methanone [compound No. 233]
(1-((2-aminoethyl)-5-(5-ethyl-1,3,4-oxadiazol-2-yl)-2,4-dimethyl-1H-pyrrol-3-yl)(3-bromophenyl)methanone [compound No. 234]
2-((3-(3-bromobenzoyl)-2,4-dimethyl-5-(5-ethyl-1,3,4-oxadiazol-2-yl)-1H-pyrrol-1-yl)ethyl acetate [compound No. 235]
(3-bromophenyl)(1-(2-hydroxyethyl)-2,4-dimethyl-5-(5-ethyl-1,3,4-oxadiazol-2-yl)-1H-pyrrol-3-yl)methanone [compound No. 236]
(1-benzoyl-5-(5-ethyl-1,3,4-oxadiazol-2-yl)-2,4-dimethyl-1H-pyrrol-3-yl)(3-bromophenyl)methanone [compound No. 237]
tert-butyl(2-(4-bromo-2-(5-(5-(hydroxymethyl)-1,3,4-oxadiazol-2-yl)-2,4-dimethyl-1H-pyrrole-3-carbonyl)phenoxy)ethyl)carbamate [compound No. 238]
(2-((2-aminoethoxy)-5-bromophenyl)(5-(5-(hydroxymethyl)-1,3,4-oxadiazol-2-yl)-2,4-dimethyl-1H-pyrrol-3-yl)methanone [compound No. 239]
(5-bromo-2-methoxyphenyl)(5-(5-cyclopropyl-1,3,4-oxadiazol-2-yl)-2,4-dimethyl-1H-pyrrol-3-yl)methanone [compound No. 240]
(5-bromo-2-hydroxyphenyl)(5-(5-cyclopropyl-1,3,4-oxadiazol-2-yl)-2,4-dimethyl-1H-pyrrol-3-yl)methanone [compound No. 241]
tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate [compound No. 242]
tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate [compound No. 243]
(2-((2-aminoethoxy)-5-bromophenyl)(5-(5-cyclopropyl-1,3,4-oxadiazol-2-yl)-2,4-dimethyl-1H-pyrrol-3-yl)methanone [compound No. 244]
(2-((2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone [compound No. 245]
(5-((5-ethyl-1,3,4-oxadiazol-2-yl)-2,4-dimethyl-1H-pyrrol-3-yl)(3-hydroxyphenyl)methanone [compound No. 246]
tert-butyl(2-(3-(5-(5-ethyl-1,3,4-oxadiazol-2-yl)-2,4-dimethyl-1H-pyrrole-3-carbonyl)phenoxy)ethyl)carbamate [compound No. 247]
(3-((2-aminoethoxy)phenyl)(5-(5-ethyl-1,3,4-oxadiazol-2-yl)-2,4-dimethyl-1H-pyrrol-3-yl)methanone [compound No. 248]
(3-bromo-4-hydroxyphenyl)(5-(5-ethyl-1,3,4-oxadiazol-2-yl)-2,4-dimethyl-1H-pyrrol-3-yl)methanone [compound No. 249]
tert-butyl(2-(2-bromo-4-(5-(5-ethyl-1,3,4-oxadiazol-2-yl)-2,4-dimethyl-1H-pyrrole-3-carbonyl)phenoxy)ethyl)carbamate [compound No. 250]
(4-((2-aminoethoxy)-3-bromophenyl)(5-(5-ethyl-1,3,4-oxadiazol-2-yl)-2,4-dimethyl-1H-pyrrol-3-yl)methanone [compound No. 251]
(5-((5-cyclopropyl-1,3,4-oxadiazol-2-yl)-2,4-dimethyl-1H-pyrrol-3-yl)(3-hydroxyphenyl)methanone [compound No. 252]
(3-((2-aminoethoxy)phenyl)(5-(5-cyclopropyl-1,3,4-oxadiazol-2-yl)-2,4-dimethyl-1H-pyrrol-3-yl)methanone [compound No. 253]
(3-bromo-4-hydroxyphenyl)(5-(5-cyclopropyl-1,3,4-oxadiazol-2-yl)-2,4-dimethyl-1H-pyrrol-3-yl)methanone [compound No. 254]
tert-butyl(2-(2-bromo-4-(5-(5-cyclopropyl-1,3,4-oxadiazol-2-yl)-2,4-dimethyl-1H-pyrrole-3-carbonyl)phenoxy)ethyl)carbamate [compound No. 255]
(4-((2-aminoethoxy)-3-bromophenyl)(5-(5-cyclopropyl-1,3,4-oxadiazol-2-yl)-2,4-dimethyl-1H-pyrrol-3-yl)methanone [compound No. 256]
(3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone [compound No. 257]
3-((5-bromo-2-methoxybenzoyl)-5-(5-cyclopropyl-1,3,4-oxadiazol-2-yl)-4-methyl-1H-pyrrole-2-carboaldehyde [compound No. 258]
(5-bromo-2-methoxyphenyl)(5-(5-cyclopropyl-1,3,4-oxadiazol-2-yl)-2-(hydroxymethyl)-4-methyl-1H-pyrrol-3-yl)methanone [compound No. 259]
(3-bromophenyl)(5-(5-cyclopropyl-1,3,4-oxadiazol-2-yl)-4-methyl-2-vinyl-1H-pyrrol-3-yl)methanone [compound No. 260]
3-((3-bromobenzoyl)-5-(5-cyclopropyl-1,3,4-oxadiazol-2-yl)-4-methyl-1H-pyrrole-2-carboaldehyde [compound No. 261]
(3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone [compound No. 262]
3-((3-bromo-4-methoxybenzoyl)-5-(5-cyclopropyl-1,3,4-oxadiazol-2-yl)-4-methyl-1H-pyrrole-2-carboaldehyde [compound No. 263]
(3-bromophenyl)(5-(5-cyclopropyl-1,3,4-oxadiazol-2-yl)-2-(hydroxymethyl)-4-methyl-1H-pyrrol-3-yl)methanone [compound No. 264]
(3-bromophenyl)(5-(5-cyclopropyl-1,3,4-oxadiazol-2-yl)-2-(difluoromethyl)-4-methyl-1H-pyrrol-3-yl)methanone [compound No. 265]
(3-bromo-4-methoxyphenyl)(5-(5-cyclopropyl-1,3,4-oxadiazol-2-yl)-2-(hydroxymethyl)-4-methyl-1H-pyrrol-3-yl)methanone [compound No. 266]
(3-bromo-4-methoxyphenyl)(5-(5-cyclopropyl-1,3,4-oxadiazol-2-yl)-2-(difluoromethyl)-4-methyl-1H-pyrrol-3-yl)methanone [compound No. 267]
(3-bromo-4-methoxyphenyl)(5-(5-cyclopropyl-1,3,4-oxadiazol-2-yl)-2-(1-hydroxyethyl)-4-methyl-1H-pyrrol-3-yl)methanone [compound No. 268]
(3-((3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate [compound No. 269]
(3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone [compound No. 270]
(3-bromophenyl)(2,4-dimethyl-5-(3-methyl-1,2,4-oxadiazol-5-yl)-1H-pyrrol-3-yl)methanone [compound No. 271]
(3-bromophenyl)(5-(5-ethyloxazol-2-yl)-2,4-dimethyl-1H-pyrrol-3-yl)methanone [compound No. 272]
(5-bromo-2-methoxyphenyl)(5-(5-(1-bromocyclopropyl)-1,3,4-oxadiazol-2-yl)-2,4-dimethyl-1H-pyrrol-3-yl)methanone [compound No. 273]
(5-bromo-2-methoxyphenyl)(1-bromo-5-(5-(1-bromocyclopropyl)-1,3,4-oxadiazol-2-yl)-2,4-dimethyl-1H-pyrrol-3-yl)methanone [compound No. 274]
(3-bromophenyl)(2,4-dimethyl-5-(1,3,4-oxadiazol-2-yl)-1H-pyrrol-3-yl)methanone [compound No. 275]
(5-((5-(2-aminoethyl)-1,3,4-oxadiazol-2-yl)-2,4-dimethyl-1H-pyrrol-3-yl)(3-bromophenyl)methanone [compound No. 276]
2-((2-(5-(4-(3-bromobenzoyl)-3,5-dimethyl-1H-pyrrol-2-yl)-1,3,4-oxadiazol-2-yl)ethyl)isoindoline-1,3-dione [compound No. 277]
3-((3-bromobenzoyl)-5-(2-(2-(1,3-dioxoisoindolin-2-yl)ethyl)-2H-tetrazol-5-yl)-4-methyl-1H-pyrrole-2-carboaldehyde [compound No. 278]
2-((2-(5-(4-(3-bromobenzoyl)-5-(hydroxymethyl)-3-methyl-1H-pyrrol-2-yl)-2H-tetrazol-2-yl)ethyl)isoindoline-1,3-dione [compound No. 279]
(5-((2-(2-aminoethyl)-2H-tetrazol-5-yl)-2-(hydroxymethyl)-4-methyl-1H-pyrrol-3-yl)(3-bromophenyl)methanone [compound No. 280]
3-((3-bromobenzoyl)-5-(2-(2-(1,3-dioxoisoindolin-2-yl)ethyl)-2H-tetrazol-5-yl)-N,4-dimethyl-1H-pyrrole-2-carboxamide [compound No. 281]
5-((2-(2-aminoethyl)-2H-tetrazol-5-yl)-3-(3-bromobenzoyl)-N,4-dimethyl-1H-pyrrole-2-carboxamide [compound No. 282]
3-((3-bromobenzoyl)-5-(2-(2-(1,3-dioxoisoindolin-2-yl)ethyl)-2H-tetrazol-5-yl)-4-methyl-1H-pyrrole-2-carbonitrile [compound No. 283]
5-((2-(2-aminoethyl)-2H-tetrazol-5-yl)-3-(3-bromobenzoyl)-4-methyl-1H-pyrrole-2-carbonitrile [compound No. 284]
(3-bromophenyl)(5-(2-(2-hydroxyethyl)-2H-tetrazol-5-yl)-2-(2-hydroxypropan-2-yl)-4-methyl-1H-pyrrol-3-yl)methanone [compound No. 285]
(4-bromo-2-methyl-5-(1H-tetrazol-5-yl)-1H-pyrrol-3-yl)(3-bromophenyl)methanone [compound No. 286]
2-((5-(3-bromo-4-(3-bromobenzoyl)-5-methyl-1H-pyrrol-2-yl)-2H-tetrazol-2-yl)ethyl acetate [compound No. 287]

(4-bromo-5-(2-(2-hydroxyethyl)-2H-tetrazol-5-yl)-2-methyl-1H-pyrrol-3-yl)(3-bromophenyl)methanone [compound No. 288]
tert-butyl(2-(5-(3-bromo-4-(3-bromobenzoyl)-5-methyl-1H-pyrrol-2-yl)-2H-tetrazol-2-yl)ethyl)carbamate [compound No. 289]
(5-((2-(2-aminoethyl)-2H-tetrazol-5-yl)-4-bromo-2-methyl-1H-pyrrol-3-yl)(3-bromophenyl)methanone [compound No. 290]
tert-butyl(2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate [compound No. 291]
ethyl 5-(2-(2-acetoxyethyl)-2H-tetrazol-5-yl)-3-(3-bromo-4-methoxybenzoyl)-4-methyl-1H-pyrrole-2-carboxylate [compound No. 292]
ethyl 3-(3-bromo-4-methoxybenzoyl)-5-(2-(2-hydroxyethyl)-2H-tetrazol-5-yl)-4-methyl-1H-pyrrole-2-carboxylate [compound No. 293]
(5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone) [compound No. 294]
2-((5-(4-(3-bromo-4-methoxybenzoyl)-3-methyl-5-(methylcarbamoyl)-1H-pyrrol-2-yl)-2H-tetrazol-2-yl)ethyl acetate [compound No. 295] and
2-((5-(4-(3-bromobenzoyl)-3-methyl-5-(methylcarbamoyl)-1H-pyrrol-2-yl)-2H-tetrazol-2-yl)ethyl dihydrogen phosphate [compound No. 303].

[8] A pharmaceutical composition comprising a compound according to any one of [1] to [7] or a salt thereof.
[9] An anticancer agent comprising a compound according to any one of [1] to [7] or a salt thereof as an active ingredient.
[10] The anticancer agent according to [9], wherein the anticancer agent is an anticancer agent against a cancer having the potential to acquire drug resistance due to the involvement of P-glycoprotein and/or a cancer already having the resistance.
[11] The anticancer agent according to [9], wherein the anticancer agent is an anticancer agent against a cancer having the potential to acquire drug resistance due to the involvement of TUBB3 and/or a cancer already having the resistance.
[12] A tubulin polymerization inhibitor comprising a compound according to any one of [1] to [7] or a salt thereof as an active ingredient.
[13] An apoptosis inducer comprising a compound according to any one of [1] to [7] or a salt thereof as an active ingredient.
[14] The compound according to any one of [1] to [7] or a salt thereof for use in the treatment of cancer.
[15] The compound according to [14] or a salt thereof, wherein the cancer is a cancer having the potential to acquire drug resistance due to the involvement of P-glycoprotein and/or a cancer already having the resistance.
[16] The compound according to [14] or a salt thereof, wherein the cancer is a cancer having the potential to acquire drug resistance due to the involvement of TUBB3 and/or a cancer already having the resistance.
[17] The compound according to any one of [1] to [7] or a salt thereof for use in the inhibition of tubulin polymerization.
[18] The compound according to any one of [1] to [7] or a salt thereof for use in the induction of apoptosis.
[19] Use of a compound according to any one of [1] to [7] or a salt thereof for the production of an anticancer agent.
[20] The use of according to [19], wherein the anticancer agent is an anticancer agent against a cancer having the potential to acquire drug resistance due to the involvement of P-glycoprotein and/or a cancer already having the resistance.
[21] The use of according to [19], wherein the anticancer agent is an anticancer agent against a cancer having the potential to acquire drug resistance due to the involvement of TUBB3 and/or a cancer already having the resistance.
[22] Use of a compound according to any one of [1] to [7] or a salt thereof for the production of a tubulin polymerization inhibitor.
[23] Use of a compound according to any one of [1] to [7] or a salt thereof for the production of an apoptosis inducer.
[24] A method for treating cancer, comprising administering an effective amount of a compound according to any one of [1] to [7] or a salt thereof.
[25] The method according to [24], wherein the cancer is a cancer having the potential to acquire drug resistance due to the involvement of P-glycoprotein and/or a cancer already having the resistance.
[26] The method according to [24], wherein the cancer is a cancer having the potential to acquire drug resistance due to the involvement of TUBB3 and/or a cancer already having the resistance.
[27] A method for inhibiting tubulin polymerization, comprising administering an effective amount of a compound according to any one of [1] to [7] or a salt thereof.
[28] A method for inducing apoptosis, comprising administering an effective amount of a compound according to any one of [1] to [7] or a salt thereof.

[Effects of the Invention]

The pyrrole compound of the present invention or a salt thereof exhibits an excellent anticancer effect *in vitro* and *in vivo.* Moreover, the pyrrole compound (1) or a salt thereof inhibits the polymerization action of tubulin or induces apoptosis through binding to a colchicine-binding site on the tubulin molecule. The pyrrole compound (1) or a salt thereof also exhibits a potent growth inhibitory effect even on resistant lines overexpressing P-glycoprotein involved in multidrug resistance and resistant lines highly expressing Class III β-tubulin (TUBB3) involved in resistance to taxane anticancer agents (paclitaxel (PTX), docetaxel, cabazitaxel, etc.) and as such, is very useful as an anticancer agent.

[Brief Description of the Drawings]

[Figure 1] Figure 1 is a diagram showing the apoptosis-inducing effect of the compound of the present invention on HCT116 cells.
[Figure 2] Figure 2 is a diagram showing the effect of the compound of the present invention on tubulin polymerization reaction.
[Figure 3] Figure 3 is a diagram showing that the compound of the present invention binds to a colchicine-binding site on tubulin.
[Figure 4] Figure 4 is a diagram showing the antitumor effect (*in vivo*) of the compound of the present invention on the growth of human lung cancer NCI-H460 xenograft tumor.
[Figure 5] Figure 5 is a diagram showing the antitumor effect (*in vivo*) of the compound of the present invention on the growth of PV2-7 xenograft tumor.

[Modes for Carrying out the Invention]

The pyrrole compound of the present invention is represented by the general formula (1) shown above.
In the general formula (1), Ar represents an optionally substituted aryl group or an optionally substituted heteroaryl group and is preferably an optionally substituted aryl group having 6 to 14 carbon atoms or an optionally substituted 5- to 10-membered heteroaryl group, particularly preferably an optionally substituted aryl group having 6 to 14 carbon atoms.
In this context, examples of the optionally substituted aryl group having 6 to 14 carbon atoms or the optionally substituted 5- to 10-membered heteroaryl group include optionally substituted monocyclic or polycyclic aryl groups or optionally substituted monocyclic or polycyclic heteroaryl groups.
The aryl group represented by Ar is preferably an aryl group having 6 to 14 carbon atoms. Specific examples thereof include a phenyl group, a naphthyl group, an anthranyl group, and a biphenyl group. Of these groups, a phenyl group is particularly preferred.
The heteroaryl group represented by Ar is preferably 5- to 10-membered heteroaryl group, more preferably a 5-to 10-membered heteroaryl group having 1 to 4 heteroatoms selected from a nitrogen atom, an oxygen atom, and a sulfur atom. Specific examples of the heteroaryl group include a pyrrolyl group, an imidazolyl group, a triazolyl group, a tetrazolyl group, a furanyl group, a thienyl group, an oxazolyl group, a thiazolyl group, a pyridyl group, a pyrimidinyl group, an indolyl group, a benzofuranyl group, a benzothienyl group, and a benzimidazolyl group. Of these groups, a 5- or 6-membered heteroaryl group having 1 or 2 heteroatoms selected from a nitrogen atom, an oxygen atom, and a sulfur atom is preferred. Specifically, a pyrrolyl group, an imidazolyl group, a furanyl group, a thienyl group, an oxazolyl group, a thiazolyl group, a pyridyl group, a pyrimidinyl group, or the like is more preferred.
Examples of the substituent(s) by which the aryl group or the heteroaryl group may be substituted include 1 to 5 groups selected from a halogen atom, an alkyl group, an alkenyl group, a cycloalkyl group, an alkoxy group, a hydroxy group, a cyano group, a nitro group, an amino group, a formyl group, an alkylcarbonyl group, an alkylcarbonyloxy group, an alkylcarbonylamino group, an aminoalkylcarbonylamino group, an alkoxycarbonylaminoalkylcarbonylamino group, an aminoalkyloxy group, an alkylaminoalkyloxy group, a dialkylaminoalkyloxy group, an alkoxycarbonylaminoalkyloxy group, a phosphoryl group, a hydroxyalkyl group, an alkylcarbonyloxyalkyl group, an alkoxyalkyl group, a dialkoxyalkyl group, an aminoalkyl group, an alkylaminoalkyl group, a dialkylaminoalkyl group, a diaminoalkyl group, a nitro(hydroxy)alkyl group, and an alkylenedioxy group.
Of these substituents, examples of the halogen atom include a chlorine atom, a fluorine atom, a bromine atom, and an iodine atom. Examples of the alkyl group include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. Examples of the alkenyl group include linear or branched

alkenyl groups each having 2 to 8 carbon atoms. A linear or branched alkenyl group having 2 to 6 carbon atoms is preferred. Examples of the alkenyl group include a vinyl group, a 1-propenyl group, and a 2-propenyl group. Examples of the cycloalkyl group include cycloalkyl groups each having 3 to 6 carbon atoms. Examples of the cycloalkyl groups include a cyclopropyl group, a cyclobutyl group, a cyclopentyl group, and a cyclohexyl group.

Examples of the alkoxy group include linear or branched alkoxy groups each having 1 to 8 carbon atoms. A linear or branched alkoxy group having 1 to 6 carbon atoms is preferred. Examples of the alkoxy group include a methoxy group, an ethoxy group, an isopropyloxy group, a n-propyloxy group, a n-butyloxy group, and a tert-butyloxy group.

Examples of the alkylcarbonyl group include linear or branched alkylcarbonyl groups each having 1 to 8 carbon atoms. A linear or branched alkylcarbonyl group having 1 to 6 carbon atoms is preferred. Examples of the alkylcarbonyl group include an acetyl group, a propionyl group, and a butyryl group. Examples of the alkylcarbonyloxy group, the alkylcarbonylamino group, the aminoalkylcarbonylamino group, and the alkoxycarbonylaminoalkylcarbonylamino group include $C_1$-$C_6$ alkylcarbonyloxy groups, $C_1$-$C_6$ alkylcarbonylamino groups, amino-$C_1$-$C_6$ alkylcarbonylamino groups, and $C_1$-$C_6$ alkoxycarbonylamino-$C_1$-$C_6$ alkylcarbonylamino groups, respectively. Examples of the alkylcarbonyl moieties in these substituents include the same as those exemplified as the alkylcarbonyl group. Specific examples of the alkylcarbonyloxy group include an acetoxy group, a propionyloxy group, and a butyryloxy group. Specific examples of the alkylcarbonylamino group include an acetamino group, a propionylamino group, and a butyrylamino group. Specific examples of the aminoalkylcarbonylamino group include an aminoacetamino group, an aminopropionylamino group, and an aminobutyrylamino group. Specific examples of the alkoxycarbonylaminoalkylcarbonylamino group include a methoxycarbonylaminoacetamino group, an ethoxycarbonylaminoacetamino group, a tert-butoxycarbonylaminoacetamino group, a methoxycarbonylaminopropionylamino group, an ethoxycarbonylaminopropionylamino group, a tert-butoxycarbonylaminopropionylamino group, a methoxycarbonylaminobutyrylamino group, an ethoxycarbonylaminobutyrylamino group, and a tert-butoxycarbonylbutyrylamino group.

Examples of the aminoalkyloxy group, the alkylaminoalkyloxy group, the dialkylaminoalkyloxy group, and the alkoxycarbonylaminoalkyloxy group include amino-$C_1$-$C_6$ alkyloxy groups, $C_1$-$C_6$ alkylamino-$C_1$-$C_6$ alkyloxy groups, di($C_1$-$C_6$ alkyl)amino-$C_1$-$C_6$ alkyloxy groups, and $C_1$-$C_6$ alkoxycarbonylamino-$C_1$-$C_6$ alkyloxy groups, respectively. Specific examples of the aminoalkyloxy group include an aminomethyloxy group, an aminoethyloxy group, an aminopropyloxy group, an aminobutyloxy group, and an aminopentyloxy group. Specific examples of the alkylaminoalkyloxy group include a methylaminomethyloxy group, a methylaminoethyloxy group, a methylaminopropyloxy group, a methylaminobutyloxy group, an ethylaminoethyloxy group, an ethylaminopropyloxy group, and an ethylaminobutyloxy group. Specific examples of the dialkylaminoalkyloxy group include a dimethylaminomethyloxy group, a dimethylaminoethyloxy group, a dimethylaminopropyloxy group, a dimethylaminobutyloxy group, a diethylaminomethyloxy group, a diethylaminoethyloxy group, a diethylaminopropyloxy group, and a diethylaminobutyloxy group. Specific examples of the alkoxycarbonylaminoalkyloxy group include a methoxycarbonylaminomethyloxy group, a methoxycarbonylaminoethyloxy group, a methoxycarbonylaminopropyloxy group, a methoxycarbonylaminobutyloxy group, an ethoxycarbonylaminomethyloxy group, an ethoxycarbonylaminoethyloxy group, an ethoxycarbonylaminopropyloxy group, an ethoxycarbonylaminobutyloxy group, a tert-butyloxycarbonylaminomethyloxy group, a tert-butyloxycarbonylaminoethyloxy group, a tert-butyloxycarbonylaminopropyloxy group, and a tert-butyloxycarbonylaminobutyloxy group.

Examples of the hydroxyalkyl group, the alkylcarbonyloxyalkyl group, the alkoxyalkyl group, and the dialkoxyalkyl group include hydroxy-$C_1$-$C_6$ alkyl groups, $C_1$-$C_6$ alkylcarbonyloxy-$C_1$-$C_6$ alkyl groups, $C_1$-$C_6$ alkoxy-$C_1$-$C_6$ alkyl groups, and di($C_1$-$C_6$ alkoxy)$C_1$-$C_6$ alkyl groups, respectively. Specific examples of the hydroxyalkyl group include a hydroxymethyl group, a hydroxyethyl group, a hydroxypropyl group, a hydroxybutyl group, and a hydroxypentyl group. Specific examples of the alkylcarbonyloxyalkyl group include an acetyloxymethyl group, an acetyloxyethyl group, an acetyloxypropyl group, an acetyloxybutyl group, an acetyloxypentyl group, a propionyloxymethyl group, a propionyloxyethyl group, a propionyloxypropyl group, and a propionyloxybutyl group. Specific examples of the alkoxyalkyl group include a methoxymethyl group, a methoxyethyl group, a methoxypropyl group, a methoxybutyl group, an ethoxymethyl group, an ethoxyethyl group, an ethoxypropyl group, and an ethoxybutyl group. Specific examples of the dialkoxyalkyl group include a dimethoxymethyl group, a diethoxymethyl group, a dimethoxyethyl group, and a diethoxyethyl group.

Examples of the aminoalkyl group, the alkylaminoalkyl group, the dialkylaminoalkyl group, the diaminoalkyl group, and the nitro(hydroxy)alkyl group include amino-$C_1$-$C_6$ alkyl groups, $C_1$-$C_6$ alkylamino-$C_1$-$C_6$ alkyl groups, di($C_1$-$C_6$ alkyl)amino-$C_1$-$C_6$ alkyl groups, diamino-$C_1$-$C_6$ alkyl group, and nitro(hydroxy)$C_1$-$C_6$ alkyl groups, respectively. Specific examples of the aminoalkyl group include an aminomethyl group, an aminoethyl group, an aminopropyl group, an aminobutyl group, and an aminopentyl group. Specific examples of the alkylaminoalkyl group include a methylaminomethyl group, a methylaminoethyl group, a methylaminopropyl group, a methylaminobutyl group, an ethylaminomethyl group, an ethylaminoethyl group, an ethylaminopropyl group, and an ethylaminobutyl group. Specific examples of the dialkylaminoalkyl group include a dimethylaminomethyl group, a dimethylaminoethyl group, a dimethylaminopropyl group, a dimethylaminobutyl group, a diethylaminomethyl group, a diethylaminoethyl group, a diethylaminopropyl group, and a diethylaminobutyl group. Specific examples of the diaminoalkyl group include a diaminomethyl group, a diaminoethyl group, a diaminopropyl group, a diaminoisopropyl group, a diaminobutyl group, and a diaminopentyl group.

Specific examples of the nitro(hydroxy)alkyl group include a nitro(hydroxy)methyl group, a nitro(hydroxy)ethyl group, a nitro(hydroxy)propyl group, and a nitro(hydroxy)butyl group.

The alkylenedioxy group is preferably a $C_1$-$C_4$ alkylenedioxy group. Specific examples thereof include a methylenedioxy group, an ethylenedioxy group, and a propylenedioxy group.

$Z^1$, $Z^2$, $Z^3$, and $Z^4$ each independently represent C, CH, a nitrogen atom, an oxygen atom, or a sulfur atom. Preferably, at least one of $Z^1$, $Z^2$, $Z^3$, and $Z^4$ is a nitrogen atom. Examples of the ring constituted by $Z^1$, $Z^2$, $Z^3$, and $Z^4$ include a pyrrole ring, a furan ring, a thiophene ring, an oxazole ring, a thiazole ring, an imidazole ring, an oxadiazole ring, a thiadiazole ring, a triazole ring, and a tetrazole ring. Of these rings, a 5-membered heterocyclic ring such as an oxazole ring, an oxadiazole ring, a thiadiazole ring, a triazole ring, or a tetrazole ring is more preferred. A heterocyclic ring represented by any of the following formulas (2a) to (2e) is particularly preferred:

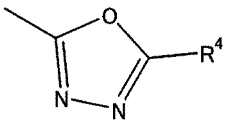
(2a)

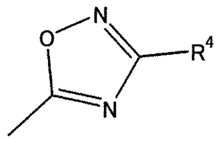
(2b)

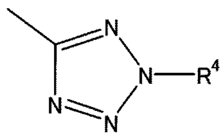
(2c)

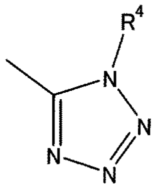
(2d)

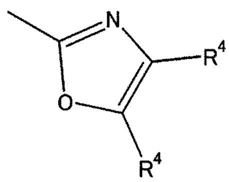
(2e)

wherein $R^4$ is as defined above.

$R^1$ represents a hydrogen atom, a halogen atom, an optionally substituted alkyl group, an optionally substituted cycloalkyl group, an optionally substituted alkylcarbonyl group, an optionally substituted alkylsulfonyl group, an optionally substituted cycloalkylsulfonyl group, an optionally substituted aryl group, an optionally substituted aralkyl group, or an optionally substituted arylcarbonyl group.

Examples of the halogen atom represented by $R^1$ include a chlorine atom, a fluorine atom, a bromine atom, and an iodine atom. Examples of the alkyl group include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. The cycloalkyl group is preferably a cycloalkyl group having 3 to 6 carbon atoms. Examples of the cycloalkyl group include a cyclopropyl group, a cyclobutyl group, a cyclopentyl group, and a cyclohexyl group.

Examples of the alkylsulfonyl group include linear or branched alkylsulfonyl groups each having 1 to 8 carbon atoms. A linear or branched alkylsulfonyl group having 1 to 6 carbon atoms is preferred. Examples of the alkylsulfonyl group include a methylsulfonyl group, an ethylsulfonyl group, a n-propylsulfonyl group, an isopropylsulfonyl group, a n-butylsulfonyl group, and an isobutylsulfonyl group. Examples of the cycloalkylsulfonyl group include cycloalkylsulfonyl groups each having 3 to 6 carbon atoms. Examples of the cycloalkylsulfonyl group include a cyclopropylsulfonyl group, a cyclobutylsulfonyl group, a cyclopentylsulfonyl group, and a cyclohexylsulfonyl group.

Examples of the alkylcarbonyl group include $C_1$-$C_6$ alkylcarbonyl groups. Examples of the alkylcarbonyl group include an acetyl group, a propionyl group, a butyryl group, a pentanoyl group, and a hexanoyl group.

The aryl group is preferably an aryl group having 6 to 14 carbon atoms. Specific examples thereof include a phenyl group, a naphthyl group, an anthranyl group, and a biphenyl group. Of these groups, a phenyl group is particularly preferred. Examples of the aralkyl group include aryl-$C_1$-$C_6$ alkyl groups each having 7 to 20 carbon atoms in total. In this context, the aryl moieties in these groups are the same as above. Specific examples of the aralkyl group include a benzyl group, a phenethyl group, and a phenylpropyl group. The arylcarbonyl group is preferably an arylcarbonyl group having 6 to 14 carbon atoms. Specific examples thereof include a benzoyl group and a naphthoyl group. Of these groups, a benzoyl group is preferred.

Examples of the substituent(s) by which the alkyl, cycloalkyl, alkylcarbonyl, alkylsulfonyl, cycloalkylsulfonyl, aryl, aralkyl, or arylcarbonyl group represented by $R^1$ may be substituted include 1 to 10 groups selected from a halogen

atom, an alkoxy group, a hydroxy group, a cyano group, a nitro group, an alkylcarbonyloxy group, an amino group, an alkoxycarbonylamino group, and an alkylcarbonylamino group. Of these substituents, examples of the halogen atom include a chlorine atom, a fluorine atom, a bromine atom, and an iodine atom. Examples of the alkoxy group include $C_1$-$C_6$ alkoxy groups, for example, a methoxy group, an ethoxy group, and a propyloxy group. Examples of the alkylcarbonyloxy group include $C_1$-$C_6$ alkylcarbonyloxy groups, for example, an acetyloxy group, a propionyloxy group, and a butyryloxy group. Examples of the alkoxycarbonylamino group include $C_1$-$C_6$ alkoxycarbonylamino groups, for example, a methoxycarbonylamino group, an ethoxycarbonylamino group, a propyloxycarbonylamino group, and a tert-butyloxycarbonylamino group. Examples of the alkylcarbonylamino group include $C_1$-$C_6$ alkylcarbonylamino groups, for example, an acetylamino group, a propionylamino group, and a butyrylamino group. These substituents are preferably added to the alkyl, cycloalkyl, alkylcarbonyl, alkylsulfonyl, and cycloalkylsulfonyl groups. The groups may be substituted by 2 to 10 identical or different groups selected from these substituents.

$R^2$ and $R^3$ are the same or different and each represent a hydrogen atom, a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, an optionally substituted arylcarbonyl group, or an optionally substituted dithianyl group. Preferably, $R^2$ is a halogen atom, a formyl group, or an optionally substituted alkyl group having 1 to 8 carbon atoms.

Examples of the halogen atom represented by $R^2$ or $R^3$ include a fluorine atom, a chlorine atom, a bromine atom, and an iodine atom. Examples of the alkoxycarbonyl group include $C_1$-$C_6$ alkoxycarbonyl groups. Specific examples thereof include a methoxycarbonyl group, an ethoxycarbonyl group, a propyloxycarbonyl group, a butyloxycarbonyl group, and a pentyloxycarbonyl group. Examples of the alkylaminocarbonyl group include $C_1$-$C_6$ alkylaminocarbonyl groups. Specific examples thereof include a methylaminocarbonyl group, an ethylaminocarbonyl group, a propylaminocarbonyl group, a butylaminocarbonyl group, and a pentylaminocarbonyl group. Examples of the cyclic aminocarbonyl group include a piperidinocarbonyl group, a piperazinocarbonyl group, $C_1$-$C_6$ alkylpiperidinocarbonyl groups, and $C_1$-$C_6$ alkylpiperazinocarbonyl groups. Examples of the imino group include an aminomethylene group.

Examples of the alkylsulfonyl group include $C_1$-$C_6$ alkylsulfonyl groups. Specific examples thereof include a methylsulfonyl group, an ethylsulfonyl group, a propylsulfonyl group, and a butylsulfonyl group. Examples of the alkyl group include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. Examples of the alkenyl group include linear or branched alkenyl groups each having 2 to 8 carbon atoms. A linear or branched alkenyl group having 2 to 6 carbon atoms is preferred. Examples of the alkenyl group include a vinyl group, a 1-propenyl group, and a 2-propenyl group. Examples of the alkylsulfonylamino group include $C_1$-$C_6$ alkylsulfonylamino groups. Specific examples thereof include a methylsulfonylamino group, an ethylsulfonylamino group, a propylsulfonylamino group, and a butylsulfonylamino group. The aryl group is preferably an aryl group having 6 to 14 carbon atoms. Specific examples thereof include a phenyl group, a naphthyl group, an anthranyl group, and a biphenyl group. Of these groups, a phenyl group is particularly preferred. The arylcarbonyl group is preferably an arylcarbonyl group having 6 to 14 carbon atoms. Specific examples thereof include a benzoyl group and a naphthoyl group. Of these groups, a benzoyl group is preferred. The dithianyl group is preferably a 1,3-dithianyl group.

Examples of the substituent(s) by which the group represented by $R^2$ or $R^3$ may be substituted include 1 to 10 groups selected from a halogen atom, a hydroxy group, a cyano group, a nitro group, an alkyl group, an alkoxy group, an amino group, a cyclic amino group, a carboxy group, an aminoalkyl group, an alkylamino group, a dialkylamino group, an alkoxycarbonyl group, an alkylcarbonyloxy group, and an aminoalkylcarbonyloxy group. Of these substituents, examples of the halogen atom include a chlorine atom, a fluorine atom, a bromine atom, and an iodine atom. Examples of the alkyl group include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. Examples of the alkoxy group include $C_1$-$C_6$ alkoxy groups. Specific examples thereof include a methoxy group, an ethoxy group, a propyloxy group, and a butyloxy group. Examples of the cyclic amino group include a piperidino group, a piperazino group, $C_1$-$C_6$ alkylpiperidino groups, and $C_1$-$C_6$ alkylpiperazino groups. Examples of the aminoalkyl group include amino-$C_1$-$C_6$ alkyl groups. Specific examples thereof include an aminomethyl group, an aminoethyl group, and an aminopropyl group. Examples of the alkylamino group include $C_1$-$C_6$ alkylamino groups. Specific examples thereof include a methylamino group, an ethylamino group, and a propylamino group. Examples of the dialkylamino group include di($C_1$-$C_6$ alkyl)amino groups. Specific examples thereof include a dimethylamino group, a diethylamino group, and a dipropylamino group. Examples of the alkoxycarbonyl group include $C_1$-$C_6$ alkoxycarbonyl groups. Specific examples thereof include a methoxycarbonyl group, an ethoxycarbonyl group, and a propyloxycarbonyl

group. Examples of the alkylcarbonyloxy group include $C_1$-$C_6$ alkylcarbonyloxy groups. Specific examples thereof include an acetyloxy group, a propionyloxy group, and a butyryloxy group. Examples of the aminoalkylcarbonyloxy group include amino-$C_1$-$C_6$ alkylcarbonyloxy groups. Specific examples thereof include an aminoacetyloxy group, an aminopropionyloxy group, and an aminobutyryloxy group. The groups may be substituted by 2 to 10 identical or different groups selected from these substituents.

n units of $R^4$ are the same or different and each represent a hydrogen atom, an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group.

Examples of the alkyl group represented by $R^4$ include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. Examples of the alkenyl group include linear or branched alkenyl groups each having 2 to 8 carbon atoms. A linear or branched alkenyl group having 2 to 6 carbon atoms is preferred. Examples of the alkenyl group include a vinyl group, a 1-propenyl group, and a 2-propenyl group. Examples of the cycloalkyl group include cycloalkyl groups each having 3 to 6 carbon atoms. Examples of the cycloalkyl groups include a cyclopropyl group, a cyclobutyl group, a cyclopentyl group, and a cyclohexyl group. The aryl group is preferably an aryl group having 6 to 14 carbon atoms. Specific examples thereof include a phenyl group, a naphthyl group, an anthranyl group, and a biphenyl group. Of these groups, a phenyl group is particularly preferred. Examples of the aralkyl group include aryl-$C_1$-$C_6$ alkyl groups each having 7 to 20 carbon atoms in total. In this context, the aryl moieties in these groups are the same as above. Specific examples of the aralkyl group include a benzyl group, a phenethyl group, and a phenylpropyl group. Examples of the alkoxycarbonyl group include $C_2$-$C_6$ alkoxycarbonyl groups. Specific examples thereof include a methoxycarbonyl group, an ethoxycarbonyl group, a propyloxycarbonyl group, and a tert-butyloxycarbonyl group.

Examples of the substituent(s) by which the group represented by $R^4$ may be substituted include 1 to 10 groups selected from a halogen atom, a cyano group, a nitro group, a hydroxy group, an amino group, an alkylcarbonyl group, an alkylcarbonyloxy group, an alkylcarbonylthio group, an alkoxycarbonyl group, a trialkylsiloxy group, an alkylamino group, a dialkylamino group, a substituted disulfide group, a substituted imide group, a cyclic amino group, an alkylsulfonylamino group, an alkylcarbonylamino group, an alkoxycarbonylamino group, an alkylureido group, an alkylthioureido group, a dialkylureido group, a dialkylthioureido group, an alkylcarbonyloxyalkylcarbonylamino group, a hydroxyalkylcarbonylamino group, an alkyl group, an aminoalkylcarbonyloxy group, and a phosphoryl group.

Examples of the halogen atom which is the substituent(s) by which the group represented by $R^4$ may be substituted include a chlorine atom, a fluorine atom, a bromine atom, and an iodine atom. Examples of the alkylcarbonyl group include $C_1$-$C_6$ alkylcarbonyl groups. Specific examples thereof include an acetyl group, a propionyl group, and a butyryl group. Examples of the alkylcarbonyloxy group include $C_1$-$C_6$ alkylcarbonyloxy groups. Specific examples thereof include an acetyloxy group, a propionyloxy group, a butyryloxy group, and a pentanoyloxy group. Examples of the alkylcarbonylthio group include $C_1$-$C_6$ alkylcarbonylthio groups. Specific examples thereof include an acetylthio group, a propionylthio group, a butyrylthio group, and a pentanoylthio group. Examples of the alkoxycarbonyl group include $C_2$-$C_6$ alkoxycarbonyl groups. Specific examples thereof include a methoxycarbonyl group, an ethoxycarbonyl group, a propyloxycarbonyl group, a butyloxycarbonyl group, and a tert-butyloxycarbonyl group. Examples of the trialkylsiloxy group include tri($C_1$-$C_6$ alkyl)silyloxy groups. Specific examples thereof include a trimethylsilyloxy group and a tert-butyldimethylsilyloxy group. Examples of the alkylamino group include $C_1$-$C_6$ alkylamino groups. Specific examples thereof include a methylamino group, an ethylamino group, and a propylamino group. Examples of the dialkylamino group include di($C_1$-$C_6$ alkyl)amino groups. Specific examples thereof include a dimethylamino group, a diethylamino group, and a dipropylamino group. Examples of the substituted disulfide group include a structure in which the group in the formula (1) forms a dimer through a disulfide bond. Examples of the substituted imide group include dicarboxylic acid imide groups such as a succinimide group and a phthalimide group. Examples of the cyclic amino group include a piperidino group, a piperazino group, $C_1$-$C_6$ alkylpiperidino groups, and $C_1$-$C_6$ alkylpiperazino groups. Examples of the alkylcarbonylamino group include $C_1$-$C_6$ alkylcarbonylamino groups. Specific examples thereof include an acetylamino group, a propionylamino group, and a butyrylamino group. Examples of the alkylsulfonylamino group include $C_1$-$C_6$ alkylsulfonylamino groups. Specific examples thereof include a methylsulfonylamino group, an ethylsulfonylamino group, and a propylsulfonylamino group. Examples of the alkoxycarbonylamino group include $C_1$-$C_6$ alkoxycarbonylamino groups. Specific examples thereof include a methoxycarbonylamino group, an ethoxycarbonylamino group, a propyloxycarbonylamino group, and a butyloxycarbonylamino group. Examples of the alkylureido group include $C_1$-$C_6$ alkylureido groups. Specific examples thereof include a methylureido group ($CH_3NHCONH$), an ethylureido group, a propylureido group, and a butylureide group. Examples of the dialkylureido group include di($C_1$-$C_6$ alkyl)ureido groups. Specific examples thereof include a dimethylureido group, a diethylureido group, a dipropylureido group, and a dibutylureido group. Examples of the alkylthioureido group include $C_1$-$C_6$ alkylthioureido groups. Specific examples thereof include a methylthioureido group ($CH_3NHCSNH$), an ethylthioureido group, and a propylthioureido group. Examples of the di-

alkylthioureido group include di($C_1$-$C_6$ alkyl)thioureido groups. Specific examples thereof include a dimethylthioureido group, a diethylthioureido group, and a dipropylthioureido group. Examples of the alkylcarbonyloxyalkylcarbonylamino group include $C_1$-$C_6$ alkylcarbonyloxy-$C_1$-$C_6$ alkylcarbonylamino groups. Specific examples thereof include an acetyloxyacetamino group, a propionyloxyacetamino group, a butyryloxyacetamino group, and an acetyloxypropionylamino group. Examples of the hydroxyalkylcarbonylamino group include hydroxy-$C_1$-$C_6$ alkylcarbonylamino groups. Specific examples thereof include a hydroxyacetamino group, a hydroxypropionylamino group, and a hydroxybutyrylamino group. Examples of the alkyl group include linear or branched alkyl groups each having 1 to 8 carbon atoms. A linear or branched alkyl group having 1 to 6 carbon atoms is preferred. Examples of the alkyl group include a methyl group, an ethyl group, a n-propyl group, an isopropyl group, a n-butyl group, an isobutyl group, a sec-butyl group, a tert-butyl group, a n-pentyl group, and a n-hexyl group. Examples of the aminoalkylcarbonyloxy group include amino-$C_1$-$C_6$ alkylcarbonyloxy groups. Specific examples thereof include an aminoacetyloxy group, an aminopropionyloxy group, and an aminobutyryloxy group. These substituents are preferably added to the alkyl group or the cycloalkyl group represented by $R^4$. The groups may be substituted by 2 to 10 identical or different groups selected from these substituents.

In the general formula (1), n represents 0 to 4 and is preferably 0 to 2, particularly preferably 0 or 1.

The compound represented by the general formula (1) is preferably a compound wherein Ar is an optionally substituted aryl group having 6 to 14 carbon atoms or an optionally substituted 5- to 10-membered heteroaryl group;
the ring constituted by $Z^1$, $Z^2$, $Z^3$, and $Z^4$ is a pyrrole ring, a furan ring, a thiophene ring, an oxazole ring, a thiazole ring, an imidazole ring, an oxadiazole ring, a thiadiazole ring, a triazole ring, or a tetrazole ring;
$R^1$ is a hydrogen atom, an optionally substituted alkyl group, an optionally substituted cycloalkyl group, an optionally substituted alkylcarbonyl group, an optionally substituted alkylsulfonyl group, an optionally substituted cycloalkylsulfonyl group, an optionally substituted aryl group, or an optionally substituted arylcarbonyl group;
$R^2$ is a halogen atom, a formyl group, or an optionally substituted alkyl group having 1 to 8 carbon atoms;
$R^3$ is a hydrogen atom, a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, or an optionally substituted arylcarbonyl group; and

$R^4$ is a hydrogen atom, an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group.

The compound represented by the general formula (1) is more preferably a compound wherein Ar is an optionally substituted aryl group having 6 to 14 carbon atoms;
the ring constituted by $Z^1$, $Z^2$, $Z^3$, and $Z^4$ is an oxazole ring, an oxadiazole ring, or a tetrazole ring;
$R^1$ is a hydrogen atom;
$R^2$ is a halogen atom, a formyl group, or an optionally substituted alkyl group having 1 to 8 carbon atoms;
$R^3$ is a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, or an optionally substituted arylcarbonyl group; and
$R^4$ is an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group.

The compound represented by the general formula (1) (also referred to as the compound (1) of the present invention) is particularly preferably any of the following compounds:

(3-bromophenyl)(2,4-dimethyl-5-(1H-tetrazol-5-yl)-1H-pyrrol-3-yl)methanone [compound No. 96]
(3-bromophenyl)(5-(2-ethyl-2H-tetrazol-5-yl)-2,4-dimethyl-1H-pyrrol-3-yl)methanone [compound No. 97]
(3-bromophenyl)(5-(1-ethyl-1H-tetrazol-5-yl)-2,4-dimethyl-1H-pyrrol-3-yl)methanone [compound No. 98]
(3-bromophenyl)(2,4-dimethyl-5-(2-(2,2,2-trifluoroethyl)-2H-tetrazol-5-yl)-1H-pyrrol-3-yl)methanone [compound No. 99]
(3-bromophenyl)(2,4-dimethyl-5-(1-(2,2,2-trifluoroethyl)-1H-tetrazol-5-yl)-1H-pyrrol-3-yl)methanone [compound No. 100]
(3-bromophenyl)(5-(2-(2-(dimethylamino)ethyl)-2H-tetrazol-5-yl)-2,4-dimethyl-1H-pyrrol-3-yl)methanone [compound No. 101]
(3-bromophenyl)(5-(2-(2-hydroxyethyl)-2H-tetrazol-5-yl)-2,4-dimethyl-1H-pyrrol-3-yl)methanone [compound No.

102]
(5-((2-(2-aminoethyl)-2H-tetrazol-5-yl)-2,4-dimethyl-1H-pyrrol-3-yl)(3-bromophenyl)methanone [compound No. 103] 2-(5-(4-(3-bromobenzoyl)-3,5-dimethyl-1H-pyrrol-2-yl)-2H-tetrazol-2-yl)ethyl acetate [compound No. 104]
(2,4-dimethyl-5-(1H-tetrazol-5-yl)-1H-pyrrol-3-yl)(3-methoxyphenyl)methanone [compound No. 105]
(3-methoxyphenyl)(5-(2-ethyl-2H-tetrazol-5-yl)-2,4-dimethyl-1H-pyrrol-3-yl)methanone [compound No. 106]
(5-((2-ethyl-2H-tetrazol-5-yl)-2,4-dimethyl-1H-pyrrol-3-yl)(3-methoxyphenyl)methanone [compound No. 107]
(3-bromo-4-methoxyphenyl)(2,4-dimethyl-5-(1H-tetrazol-5-yl)-1H-pyrrol-3-yl)methanone [compound No. 108]
(3-bromo-4-methoxyphenyl)(5-(2-ethyl-2H-tetrazol-5-yl)-2,4-dimethyl-1H-pyrrol-3-yl)methanone [compound No. 109]
(3-bromo-4-methoxyphenyl)(5-(2-(2-hydroxyethyl)-2H-tetrazol-5-yl)-2,4-dimethyl-1H-pyrrol-3-yl)methanone [compound No. 110]
2-((5-(4-(3-bromo-4-methoxybenzoyl)-3,5-dimethyl-1H-pyrrol-2-yl)-2H-tetrazol-2-yl)ethyl acetate [compound No. 111]
(3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone [compound No. 112]
(3-cyclopropylphenyl)(2,4-dimethyl-5-(1H-tetrazol-5-yl)-1H-pyrrol-3-yl)methanone [compound No. 113]
2-((5-(4-(3-cyclopropylbenzoyl)-3,5-dimethyl-1H-pyrrol-2-yl)ethyl acetate [compound No. 114]
(3-cyclopropylphenyl)(5-(2-(2-hydroxyethyl)-2H-tetrazol-5-yl)-2,4-dimethyl-1H-pyrrol-3-yl)methanone [compound No. 115]
2-((5-(4-(3-bromobenzoyl)-5-formyl-3-methyl-1H-pyrrol-2-yl)-2H-tetrazol-2-yl)ethyl acetate [compound No. 116]
3-((3-bromobenzoyl)-5-(2-(2-hydroxyethyl)-2H-tetrazol-5-yl)-4-methyl-1H-pyrrole-2-carboaldehyde [compound No. 117]
2-((5-(4-(3-bromobenzoyl)-5-(difluoromethyl)-3-methyl-1H-pyrrol-2-yl)-2H-tetrazol-2-yl)ethyl acetate [compound No. 118]
(3-bromophenyl)(2-(difluoromethyl)-5-(2-(2-hydroxyethyl)-2H-tetrazol-5-yl)-4-methyl-1H-pyrrol-3-yl)methanone [compound No. 119]
2-((5-(4-(3-bromobenzoyl)-5-(hydroxymethyl)-3-methyl-1H-pyrrol-2-yl)-2H-tetrazol-2-yl)ethyl acetate [compound No. 120]
(3-bromophenyl)(5-(2-(2-hydroxyethyl)-2H-tetrazol-5-yl)-2-(hydroxymethyl)-4-methyl-1H-pyrrol-3-yl)methanone [compound No. 121]
2-((5-(4-(3-bromobenzoyl)-5-(1-hydroxyethyl)-3-methyl-1H-pyrrol-2-yl)-2H-tetrazol-2-yl)ethyl acetate [compound No. 122]
(3-bromophenyl)(2-(1-hydroxyethyl)-5-(2-(2-hydroxyethyl)-2H-tetrazol-5-yl)-4-methyl-1H-pyrrol-3-yl)methanone [compound No. 123]
2-((5-(4-(3-bromo-4-methoxybenzoyl)-5-formyl-3-methyl-1H-pyrrol-2-yl)-2H-tetrazol-2-yl)ethyl acetate [compound No. 124]
3-((3-bromo-4-methoxybenzoyl)-5-(2-(2-hydroxyethyl)-2H-tetrazol-5-yl)-4-methyl-1H-pyrrole-2-carboaldehyde [compound No. 125]
2-((5-(4-(3-bromo-4-methoxybenzoyl)-5-(difluoromethyl)-3-methyl-1H-pyrrol-2-yl)-2H-tetrazol-2-yl)ethyl acetate [compound No. 126]
(3-bromo-4-methoxyphenyl)(2-(difluoromethyl)-5-(2-(2-hydroxyethyl)-2H-tetrazol-5-yl)-4-methyl-1H-pyrrol-3-yl)methanone [compound No. 127]
2-((5-(4-(3-bromo-4-methoxybenzoyl)-5-(hydroxymethyl)-3-methyl-1H-pyrrol-2-yl)-2H-tetrazol-2-yl)ethyl acetate [compound No. 128]
(3-bromo-4-methoxyphenyl)(5-(2-(2-hydroxyethyl)-2H-tetrazol-5-yl)-2-(hydroxymethyl)-4-methyl-1H-pyrrol-3-yl)methanone [compound No. 129]
2-((5-(4-(3-bromo-4-methoxybenzoyl)-5-(1-hydroxyethyl)-3-methyl-1H-pyrrol-2-yl)-2H-tetrazol-2-yl)ethyl acetate [compound No. 130]
(3-bromo-4-methoxyphenyl)(2-(1-hydroxyethyl)-5-(2-(2-hydroxyethyl)-2H-tetrazol-5-yl)-4-methyl-1H-pyrrol-3-yl)methanone [compound No. 131]
2-((5-(4-(3-bromobenzoyl)-5-cyano-3-methyl-1H-pyrrol-2-yl)-2H-tetrazol-2-yl)ethyl acetate [compound No. 132]
3-((3-bromobenzoyl)-5-(2-(2-hydroxyethyl)-2H-tetrazol-5-yl)-4-methyl-1H-pyrrole-2-carbonitrile [compound No. 133]
5-((2-(2-acetoxyethyl)-2H-tetrazol-5-yl)-3-(3-bromobenzoyl)-4-methyl-1H-pyrrole-2-carboxylic acid [compound No. 134]
3-((3-bromobenzoyl)-5-(2-(2-hydroxyethyl)-2H-tetrazol-5-yl)-4-methyl-1H-pyrrole-2-carboxylic acid [compound No. 135]
methyl 5-(2-(2-acetoxyethyl)-2H-tetrazol-5-yl)-3-(3-bromobenzoyl)-4-methyl-1H-pyrrole-2-carboxylate [compound

No. 136]
methyl 3-(3-bromobenzoyl)-5-(2-(2-hydroxyethyl)-2H-tetrazol-5-yl)-4-methyl-1H-pyrrole-2-carboxylate [compound No. 137]
2-((5-(4-(3-bromo-4-methoxybenzoyl)-5-cyano-3-methyl-1H-pyrrol-2-yl)-2H-tetrazol-2-yl)ethyl acetate [compound No. 138]
3-((3-bromo-4-methoxybenzoyl)-5-(2-(2-hydroxyethyl)-2H-tetrazol-5-yl)-4-methyl-1H-pyrrole-2-carbonitrile [compound No. 139]
5-((2-(2-acetoxyethyl)-2H-tetrazol-5-yl)-3-(3-bromo-4-methoxybenzoyl)-4-methyl-1H-pyrrole-2-carboxylic acid [compound No. 140]
3-((3-bromo-4-methoxybenzoyl)-5-(2-(2-hydroxyethyl)-2H-tetrazol-5-yl)-4-methyl-1H-pyrrole-2-carboxylic acid [compound No. 141]
3-((3-bromobenzoyl)-5-(2-(2-hydroxyethyl)-2H-tetrazol-5-yl)-N,4-dimethyl-1H-pyrrole-2-carboxamide [compound No. 142]
3-((3-bromobenzoyl)-5-(2-(2-hydroxyethyl)-2H-tetrazol-5-yl)-N,N,4-trimethyl-1H-pyrrole-2-carboxamide [compound No. 143]
N-(2-aminoethyl)-3-(3-bromobenzoyl)-5-(2-(2-hydroxyethyl)-2H-tetrazol-5-yl)-4-methyl-1H-pyrrole-2-carboxamide [compound No. 144]
3-((3-bromobenzoyl)-N-ethyl-5-(2-(2-hydroxyethyl)-2H-tetrazol-5-yl)-4-methyl-1H-pyrrole-2-carboxamide [compound No. 145]
3-((3-bromobenzoyl)-5-(2-(2-hydroxyethyl)-2H-tetrazol-5-yl)-N-isopropyl-4-methyl-1H-pyrrole-2-carboxamide [compound No. 146]
(3-((3-bromobenzoyl)-5-(2-(2-hydroxyethyl)-2H-tetrazol-5-yl)-4-methyl-1H-pyrrol-2-yl)(4-methylpiperazin-1-yl)methanone [compound No. 147]
(3-((3-bromobenzoyl)-5-(2-(2-hydroxyethyl)-2H-tetrazol-5-yl)-4-methyl-1H-pyrrol-2-yl)(piperazin-1-yl)methanone [compound No. 148]
3-((3-bromo-4-methoxybenzoyl)-5-(2-(2-hydroxyethyl)-2H-tetrazol-5-yl)-N,4-dimethyl-1H-pyrrole-2-carboxamide [compound No. 149]
3-((3-bromo-4-methoxybenzoyl)-5-(2-(2-hydroxyethyl)-2H-tetrazol-5-yl)-4-methyl-1H-pyrrole-2-carboxamide [compound No. 150]
2-((5-(4-(3-bromobenzoyl)-3,5-dimethyl-1H-pyrrol-2-yl)-2H-tetrazol-2-yl)ethyl 2-((tert-butoxycarbonyl)amino)acetate [compound No. 151]
2-((5-(4-(3-bromobenzoyl)-3,5-dimethyl-1H-pyrrol-2-yl)-2H-tetrazol-2-yl)ethyl 2-aminoacetate [compound No. 152]
2-((5-(4-(3-bromobenzoyl)-5-formyl-3-methyl-1H-pyrrol-2-yl)-2H-tetrazol-2-yl)ethyl 2-((tert-butoxycarbonyl)amino)acetate [compound No. 153]
2-((5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate [compound No. 154]
2-((5-(4-(3-bromobenzoyl)-5-(hydroxymethyl)-3-methyl-1H-pyrrol-2-yl)-2H-tetrazol-2-yl)ethyl 2-aminoacetate [compound No. 155]
methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate [compound No. 156]
methyl 5-(2-(2-(2-aminoacetoxy)ethyl)-2H-tetrazol-5-yl)-3-(3-bromobenzoyl)-4-methyl-1H-pyrrole-2-carboxylate [compound No. 157]
2-((5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate [compound No. 158]
2-((5-(4-(3-bromobenzoyl)-3-methyl-5-(methylcarbamoyl)-1H-pyrrol-2-yl)-2H-tetrazol-2-yl)ethyl 2-aminoacetate [compound No. 159]
2-((5-(3,5-dimethyl-4-(3-vinylbenzoyl)-1H-pyrrol-2-yl)-2H-tetrazol-2-yl)ethyl acetate [compound No. 160]
2-((5-(4-(3-formylbenzoyl)-3,5-dimethyl-1H-pyrrol-2-yl)-2H-tetrazol-2-yl)ethyl acetate [compound No. 161]
2-((5-(4-(3-(hydroxymethyl)benzoyl)-3,5-dimethyl-1H-pyrrol-2-yl)-2H-tetrazol-2-yl)ethyl acetate [compound No. 162]
(5-((2-(2-hydroxyethyl)-2H-tetrazol-5-yl)-2,4-dimethyl-1H-pyrrol-3-yl)(3-(hydroxymethyl)phenyl)methanone [compound No. 163]
(5-((2-ethyl-2H-tetrazol-5-yl)-2,4-dimethyl-1H-pyrrol-3-yl)(3-hydroxyphenyl)methanone [compound No. 164]
(3-((2-aminoethoxy)phenyl)(5-(2-ethyl-2H-tetrazol-5-yl)-2,4-dimethyl-1H-pyrrol-3-yl)methanone [compound No. 165]
(3-bromo-4-hydroxyphenyl)(5-(2-ethyl-2H-tetrazol-5-yl)-2,4-dimethyl-1H-pyrrol-3-yl)methanone [compound No. 166]
(4-((2-aminoethoxy)-3-bromophenyl)(5-(2-ethyl-2H-tetrazol-5-yl)-2,4-dimethyl-1H-pyrrol-3-yl)methanone [compound No. 167]
(4-((2-aminoethoxy)-3-bromophenyl)(5-(2-(2-hydroxyethyl)-2H-tetrazol-5-yl)-2,4-dimethyl-1H-pyrrol-3-yl)methanone [compound No. 168]
(E)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 169]
(Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 170]
2-((5-(4-(3-bromobenzoyl)-5-(chloromethyl)-3-methyl-1H-pyrrol-2-yl)-2H-tetrazol-2-yl)ethyl acetate [compound No. 171]
2-((5-(4-(3-bromo-4-methoxybenzoyl)-5-(chloromethyl)-3-methyl-1H-pyrrol-2-yl)-2H-tetrazol-2-yl)ethyl acetate [compound No. 172]
(3-bromophenyl)(5-(5-ethyl-1,3,4-oxadiazol-2-yl)-2,4-dimethyl-1H-pyrrol-3-yl)methanone [compound No. 173]
(3-bromophenyl)(5-(5-(2-chloroethyl)-1,3,4-oxadiazol-2-yl)-2,4-dimethyl-1H-pyrrol-3-yl)methanone [compound No. 174]
(3-bromophenyl)(2,4-dimethyl-5-(5-phenyl-1,3,4-oxadiazol-2-yl)-1H-pyrrol-3-yl)methanone [compound No. 175]
(5-((5-benzyl-1,3,4-oxadiazol-2-yl)-2,4-dimethyl-1H-pyrrol-3-yl)(3-bromophenyl)methanone [compound No. 176]
(3-bromophenyl)(5-(5-cyclopropyl-1,3,4-oxadiazol-2-yl)-2,4-dimethyl-1H-pyrrol-3-yl)methanone [compound No. 177]
(3-bromophenyl)(5-(5-isopropyl-1,3,4-oxadiazol-2-yl)-2,4-dimethyl-1H-pyrrol-3-yl)methanone [compound No. 178]
(3-bromophenyl)(5-(5-cyclobutyl-1,3,4-oxadiazol-2-yl)-2,4-dimethyl-1H-pyrrol-3-yl)methanone [compound No. 179]
(3-bromophenyl)(5-(5-cyclopentyl-1,3,4-oxadiazol-2-yl)-2,4-dimethyl-1H-pyrrol-3-yl)methanone [compound No. 180]
(3-bromophenyl)(5-(5-cyclohexyl-1,3,4-oxadiazol-2-yl)-2,4-dimethyl-1H-pyrrol-3-yl)methanone [compound No. 181]
methyl 3-(5-(4-(3-bromobenzoyl)-3,5-dimethyl-1H-pyrrol-2-yl)-1,3,4-oxadiazol-2-yl)propionate [compound No. 182]
ethyl 2-(5-(4-(3-bromobenzoyl)-3,5-dimethyl-1H-pyrrol-2-yl)-1,3,4-oxadiazol-2-yl)acetate [compound No. 183]
(3-bromophenyl)(5-(5-tert-butyl)-1,3,4-oxadiazol-2-yl)-2,4-dimethyl-1H-pyrrol-3-yl)methanone [compound No. 184]
(3-bromophenyl)(2,4-dimethyl-5-(5-methyl-1,3,4-oxadiazol-2-yl)-1H-pyrrol-3-yl)methanone [compound No. 185]
(3-bromophenyl)(5-(5-(difluoromethyl)-1,3,4-oxadiazol-2-yl)-2,4-dimethyl-1H-pyrrol-3-yl)methanone [compound No. 186]
(3-bromophenyl)(2,4-dimethyl-5-(5-(2,2,2-trifluoroethyl)-1,3,4-oxadiazol-2-yl)-1H-pyrrol-3-yl)methanone [compound No. 187]
(3-bromophenyl)(5-(5-(1-hydroxycyclopropyl)-1,3,4-oxadiazol-2-yl)-2,4-dimethyl-1H-pyrrol-3-yl)methanone [compound No. 188]
(3-bromophenyl)(2,4-dimethyl-5-(5-(1-methylcyclopropyl)-1,3,4-oxadiazol-2-yl)-1H-pyrrol-3-yl)methanone [compound No. 189]
(3-bromophenyl)(2,4-dimethyl-5-(5-(2-methylcyclopropyl)-1,3,4-oxadiazol-2-yl)-1H-pyrrol-3-yl)methanone [compound No. 190]
(3-bromophenyl)(5-(5-(2,2-dimethylcyclopropyl)-1,3,4-oxadiazol-2-yl)-2,4-dimethyl-1H-pyrrol-3-yl)methanone [compound No. 191]
(3-bromophenyl)(2,4-dimethyl-5-(5-(2,2,3,3-tetramethylcyclopropyl)-1,3,4-oxadiazol-2-yl)-1H-pyrrol-3-yl)methanone [compound No. 192]
(5-((5-ethyl-1,3,4-oxadiazol-2-yl)-2,4-dimethyl-1H-pyrrol-3-yl)(3-methoxyphenyl)methanone [compound No. 193]
(5-((5-cyclopropyl-1,3,4-oxadiazol-2-yl)-2,4-dimethyl-1H-pyrrol-3-yl)(3-methoxyphenyl)methanone [compound No. 194]
(5-((5-ethyl-1,3,4-oxadiazol-2-yl)-2,4-dimethyl-1H-pyrrol-3-yl)(3-nitrophenyl)methanone [compound No. 195]
(5-((5-cyclopropyl-1,3,4-oxadiazol-2-yl)-2,4-dimethyl-1H-pyrrol-3-yl)(3-nitrophenyl)methanone [compound No. 196]
(3-bromo-4-methoxyphenyl)(5-(5-ethyl-1,3,4-oxadiazol-2-yl)-2,4-dimethyl-1H-pyrrol-3-yl)methanone [compound No. 197]
(3-bromo-4-methoxyphenyl)(5-(5-cyclopropyl-1,3,4-oxadiazol-2-yl)-2,4-dimethyl-1H-pyrrol-3-yl)methanone [compound No. 198]
(5-((5-cyclopropyl-1,3,4-oxadiazol-2-yl)-2,4-dimethyl-1H-pyrrol-3-yl)(3-(hydroxymethyl)phenyl)methanone [compound No. 199]
(3-bromophenyl)(5-(5-cyclopropyl-1,3,4-oxadiazol-2-yl)-2-(1,3-dithian-2-yl)-4-methyl-1H-pyrrol-3-yl)methanone [compound No. 200]
(5-bromo-2-methoxyphenyl)(5-(5-(chloromethyl)-1,3,4-oxadiazol-2-yl)-2,4-dimethyl-1H-pyrrol-3-yl)methanone [compound No. 201]
(5-((4-(5-bromo-2-methoxybenzoyl)-3,5-dimethyl-1H-pyrrol-2-yl)-1,3,4-oxadiazol-2-yl)methyl acetate [compound

No. 202]
(5-bromo-2-hydroxyphenyl)(5-(5-(hydroxymethyl)-1,3,4-oxadiazol-2-yl)-2,4-dimethyl-1H-pyrrol-3-yl)methanone [compound No. 203]
2-((5-(4-(3-bromobenzoyl)-3,5-dimethyl-1H-pyrrol-2-yl)-1,3,4-oxadiazol-2-yl)ethyl acetate [compound No. 204]
(3-bromophenyl)(5-(5-(2-hydroxyethyl)-1,3,4-oxadiazol-2-yl)-2,4-dimethyl-1H-pyrrol-3-yl)methanone [compound No. 205]
(3-bromophenyl)(2,4-dimethyl-5-(5-vinyl-1,3,4-oxadiazol-2-yl)-1H-pyrrol-3-yl)methanone [compound No. 206]
(3-bromophenyl)(5-(5-(chloromethyl)-1,3,4-oxadiazol-2-yl)-2,4-dimethyl-1H-pyrrol-3-yl)methanone [compound No. 207]
(3-bromophenyl)(2,4-dimethyl-5-(5-((4-methylpiperazin-1-yl)methyl)-1,3,4-oxadiazol-2-yl)-1H-pyrrol-3-yl)methanone [compound No. 208]
(3-bromophenyl)(5-(5-((dimethylamino)methyl)-1,3,4-oxadiazol-2-yl)-2,4-dimethyl-1H-pyrrol-3-yl)methanone [compound No. 209]
(5-((4-(3-bromobenzoyl)-3,5-dimethyl-1H-pyrrol-2-yl)-1,3,4-oxadiazol-2-yl)methyl acetate [compound No. 210]
(3-bromophenyl)(5-(5-(hydroxymethyl)-1,3,4-oxadiazol-2-yl)-2,4-dimethyl-1H-pyrrol-3-yl)methanone [compound No. 211]
S-((5-(4-(3-bromobenzoyl)-3,5-dimethyl-1H-pyrrol-2-yl)-1,3,4-oxadiazol-2-yl)methyl)ethanethioate [compound No. 212]
2-(((5-(4-(3-bromobenzoyl)-3,5-dimethyl-1H-pyrrol-2-yl)-1,3,4-oxadiazol-2-yl)methyl)isoindole-1,3-dione [compound No. 213]
(5-((5-(aminomethyl)-1,3,4-oxadiazol-2-yl)-2,4-dimethyl-1H-pyrrol-3-yl)(3-bromophenyl)methanone [compound No. 214]
N-((5-(4-(3-bromobenzoyl)-3,5-dimethyl-1H-pyrrol-2-yl)-1,3,4-oxadiazol-2-yl)methyl)acetamide [compound No. 215]
methyl((5-(4-(3-bromobenzoyl)-3,5-dimethyl-1H-pyrrol-2-yl)-1,3,4-oxadiazol-2-yl)methyl)carbamate [compound No. 216] N-((5-(4-(3-bromobenzoyl)-3,5-dimethyl-1H-pyrrol-2-yl)-1,3,4-oxadiazol-2-yl)methyl)methanesulfonamide [compound No. 217]
1-(((5-(4-(3-bromobenzoyl)-3,5-dimethyl-1H-pyrrol-2-yl)-1,3,4-oxadiazol-2-yl)methyl)-3-ethylurea [compound No. 218]
1-(((5-(4-(3-bromobenzoyl)-3,5-dimethyl-1H-pyrrol-2-yl)-1,3,4-oxadiazol-2-yl)methyl)-3-ethylthiourea [compound No. 219]
3-(((5-(4-(3-bromobenzoyl)-3,5-dimethyl-1H-pyrrol-2-yl)-1,3,4-oxadiazol-2-yl)methyl)-1,1-dimethylthiourea [compound No. 220]
2-((((5-(4-(3-bromobenzoyl)-3,5-dimethyl-1H-pyrrol-2-yl)-1,3,4-oxadiazol-2-yl)methyl)amino)-2-oxoethyl acetate [compound No. 221]
N-((5-(4-(3-bromobenzoyl)-3,5-dimethyl-1H-pyrrol-2-yl)-1,3,4-oxadiazol-2-yl)methyl)-2-hydroxyacetamide [compound No. 222]
(3-bromophenyl)(5-(5-ethyl-1,3,4-oxadiazol-2-yl)-1,2,4-trimethyl-1H-pyrrol-3-yl)methanone [compound No. 223]
1-((3-(3-bromobenzoyl)-5-(5-ethyl-1,3,4-oxadiazol-2-yl)-2,4-dimethyl-1H-pyrrol-1-yl)ethanone [compound No. 224]
(3-bromophenyl)(5-(5-ethyl-1,3,4-oxadiazol-2-yl)-2,4-dimethyl-1-(methylsulfonyl)-1H-pyrrol-3-yl)methanone [compound No. 225]
2-((3-(3-bromobenzoyl)-5-(5-ethyl-1,3,4-oxadiazol-2-yl)-2,4-dimethyl-1H-pyrrol-1-yl)-2-oxoethyl acetate [compound No. 226]
(3-bromophenyl)(1-(cyclopropylsulfonyl)-5-(5-ethyl-1,3,4-oxadiazol-2-yl)-2,4-dimethyl-1H-pyrrol-3-yl)methanone [compound No. 227]
(3-bromophenyl)(1-(difluoromethyl)-5-(5-ethyl-1,3,4-oxadiazol-2-yl)-2,4-dimethyl-1H-pyrrol-3-yl)methanone [compound No. 228]
(3-bromophenyl)(5-(5-ethyl-1,3,4-oxadiazol-2-yl)-2,4-dimethyl-1-(2,2,2-trifluoroethyl)-1H-pyrrol-3-yl)methanone [compound No. 229]
(3-bromophenyl)(1-cyclopropyl-5-(5-ethyl-1,3,4-oxadiazol-2-yl)-2,4-dimethyl-1H-pyrrol-3-yl)methanone [compound No. 230]
tert-butyl(2-(3-(3-bromobenzoyl)-5-(5-ethyl-1,3,4-oxadiazol-2-yl)-2,4-dimethyl-1H-pyrrol-1-yl)ethyl)carbamate [compound No. 231]
(3-bromophenyl)(1-ethyl-2,4-dimethyl-5-(5-ethyl-1,3,4-oxadiazol-2yl)-1H-pyrrol-3-yl)methanone [compound No. 232]
(3-bromophenyl)(1-isopropyl-2,4-dimethyl-5-(5-ethyl-1,3,4-oxadiazol-2-yl)-1H-pyrrol-3-yl)methanone [compound No. 233]
(1-((2-aminoethyl)-5-(5-ethyl-1,3,4-oxadiazol-2-yl)-2,4-dimethyl-1H-pyrrol-3-yl)(3-bromophenyl)methanone [compound No. 234]
2-((3-(3-bromobenzoyl)-2,4-dimethyl-5-(5-ethyl-1,3,4-oxadiazol-2-yl)-1H-pyrrol-1-yl)ethyl acetate [compound No. 235]
(3-bromophenyl)(1-(2-hydroxyethyl)-2,4-dimethyl-5-(5-ethyl-1,3,4-oxadiazol-2-yl)-1H-pyrrol-3-yl)methanone [compound No. 236]
(1-benzoyl-5-(5-ethyl-1,3,4-oxadiazol-2-yl)-2,4-dimethyl-1H-pyrrol-3-yl)(3-bromophenyl)methanone [compound No. 237]
tert-butyl(2-(4-bromo-2-(5-(5-(hydroxymethyl)-1,3,4-oxadiazol-2-yl)-2,4-dimethyl-1H-pyrrole-3-carbonyl)phenoxy)ethyl)carbamate [compound No. 238]
(2-((2-aminoethoxy)-5-bromophenyl)(5-(5-(hydroxymethyl)-1,3,4-oxadiazol-2-yl)-2,4-dimethyl-1H-pyrrol-3-yl)methanone [compound No. 239]
(5-bromo-2-methoxyphenyl)(5-(5-cyclopropyl-1,3,4-oxadiazol-2-yl)-2,4-dimethyl-1H-pyrrol-3-yl)methanone [compound No. 240]
(5-bromo-2-hydroxyphenyl)(5-(5-cyclopropyl-1,3,4-oxadiazol-2-yl)-2,4-dimethyl-1H-pyrrol-3-yl)methanone [compound No. 241]
tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate [compound No. 242]
tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate [compound No. 243]
(2-((2-aminoethoxy)-5-bromophenyl)(5-(5-cyclopropyl-1,3,4-oxadiazol-2-yl)-2,4-dimethyl-1H-pyrrol-3-yl)methanone [compound No. 244]
(2-((2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone [compound No. 245]
(5-((5-ethyl-1,3,4-oxadiazol-2-yl)-2,4-dimethyl-1H-pyrrol-3-yl)(3-hydroxyphenyl)methanone [compound No. 246]
tert-butyl(2-(3-(5-(5-ethyl-1,3,4-oxadiazol-2-yl)-2,4-dimethyl-1H-pyrrole-3-carbonyl)phenoxy)ethyl)carbamate [compound No. 247]
(3-((2-aminoethoxy)phenyl)(5-(5-ethyl-1,3,4-oxadiazol-2-yl)-2,4-dimethyl-1H-pyrrol-3-yl)methanone [compound No. 248]
(3-bromo-4-hydroxyphenyl)(5-(5-ethyl-1,3,4-oxadiazol-2-yl)-2,4-dimethyl-1H-pyrrol-3-yl)methanone [compound No. 249]
tert-butyl(2-(2-bromo-4-(5-(5-ethyl-1,3,4-oxadiazol-2-yl)-2,4-dimethyl-1H-pyrrole-3-carbonyl)phenoxy)ethyl)carbamate [compound No. 250]
(4-((2-aminoethoxy)-3-bromophenyl)(5-(5-ethyl-1,3,4-oxadiazol-2-yl)-2,4-dimethyl-1H-pyrrol-3-yl)methanone [compound No. 251]
(5-((5-cyclopropyl-1,3,4-oxadiazol-2-yl)-2,4-dimethyl-1H-pyrrol-3-yl)(3-hydroxyphenyl)methanone [compound No. 252]
(3-((2-aminoethoxy)phenyl)(5-(5-cyclopropyl-1,3,4-oxadiazol-2-yl)-2,4-dimethyl-1H-pyrrol-3-yl)methanone [compound No. 253]
(3-bromo-4-hydroxyphenyl)(5-(5-cyclopropyl-1,3,4-oxadiazol-2-yl)-2,4-dimethyl-1H-pyrrol-3-yl)methanone [compound No. 254]
tert-butyl(2-(2-bromo-4-(5-(5-cyclopropyl-1,3,4-oxadiazol-2-yl)-2,4-dimethyl-1H-pyrrole-3-carbonyl)phenoxy)ethyl)carbamate [compound No. 255]
(4-((2-aminoethoxy)-3-bromophenyl)(5-(5-cyclopropyl-1,3,4-oxadiazol-2-yl)-2,4-dimethyl-1H-pyrrol-3-yl)methanone [compound No. 256]
(3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone [compound No. 257]
3-((5-bromo-2-methoxybenzoyl)-5-(5-cyclopropyl-1,3,4-oxadiazol-2-yl)-4-methyl-1H-pyrrole-2-carboaldehyde [compound No. 258]
(5-bromo-2-methoxyphenyl)(5-(5-cyclopropyl-1,3,4-oxadiazol-2-yl)-2-(hydroxymethyl)-4-methyl-1H-pyrrol-3-yl)methanone [compound No. 259]
(3-bromophenyl)(5-(5-cyclopropyl-1,3,4-oxadiazol-2-yl)-4-methyl-2-vinyl-1H-pyrrol-3-yl)methanone [compound No. 260]
3-(3-bromobenzoyl)-5-(5-cyclopropyl-1,3,4-oxadiazol-2-yl)-4-methyl-1H-pyrrole-2-carboaldehyde [compound No. 261]
(3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone [compound No. 262]
3-((3-bromo-4-methoxybenzoyl)-5-(5-cyclopropyl-1,3,4-oxadiazol-2-yl)-4-methyl-1H-pyrrole-2-carboaldehyde [compound No. 263]

(3-bromophenyl)(5-(5-cyclopropyl-1,3,4-oxadiazol-2-yl)-2-(hydroxymethyl)-4-methyl-1H-pyrrol-3-yl)methanone [compound No. 264]
(3-bromophenyl)(5-(5-cyclopropyl-1,3,4-oxadiazol-2-yl)-2-(difluoromethyl)-4-methyl-1H-pyrrol-3-yl)methanone [compound No. 265]
(3-bromo-4-methoxyphenyl)(5-(5-cyclopropyl-1,3,4-oxadiazol-2-yl)-2-(hydroxymethyl)-4-methyl-1H-pyrrol-3-yl)methanone [compound No. 266]
(3-bromo-4-methoxyphenyl)(5-(5-cyclopropyl-1,3,4-oxadiazol-2-yl)-2-(difluoromethyl)-4-methyl-1H-pyrrol-3-yl)methanone [compound No. 267]
(3-bromo-4-methoxyphenyl)(5-(5-cyclopropyl-1,3,4-oxadiazol-2-yl)-2-(1-hydroxyethyl)-4-methyl-1H-pyrrol-3-yl)methanone [compound No. 268]
(3-((3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate [compound No. 269]
(3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone [compound No. 270]
(3-bromophenyl)(2,4-dimethyl-5-(3-methyl-1,2,4-oxadiazol-5-yl)-1H-pyrrol-3-yl)methanone [compound No. 271]
(3-bromophenyl)(5-(5-ethyloxazol-2-yl)-2,4-dimethyl-1H-pyrrol-3-yl)methanone [compound No. 272]
(5-bromo-2-methoxyphenyl)(5-(5-(1-bromocyclopropyl)-1,3,4-oxadiazol-2-yl)-2,4-dimethyl-1H-pyrrol-3-yl)methanone [compound No. 273]
(5-bromo-2-methoxyphenyl)(1-bromo-5-(5-(1-bromocyclopropyl)-1,3,4-oxadiazol-2-yl)-2,4-dimethyl-1H-pyrrol-3-yl)methanone [compound No. 274]
(3-bromophenyl)(2,4-dimethyl-5-(1,3,4-oxadiazol-2-yl)-1H-pyrrol-3-yl)methanone [compound No. 275]
(5-((5-(2-aminoethyl)-1,3,4-oxadiazol-2-yl)-2,4-dimethyl-1H-pyrrol-3-yl)(3-bromophenyl)methanone [compound No. 276]
2-((2-(5-(4-(3-bromobenzoyl)-3,5-dimethyl-1H-pyrrol-2-yl)-1,3,4-oxadiazol-2-yl)ethyl)isoindoline-1,3-dione [compound No. 277]
3-((3-bromobenzoyl)-5-(2-(2-(1,3-dioxoisoindolin-2-yl)ethyl)-2H-tetrazol-5-yl)-4-methyl-1H-pyrrole-2-carboaldehyde [compound No. 278]
2-((2-(5-(4-(3-bromobenzoyl)-5-(hydroxymethyl)-3-methyl-1H-pyrrol-2-yl)-2H-tetrazol-2-yl)ethyl)isoindoline-1,3-dione [compound No. 279]
(5-((2-(2-aminoethyl)-2H-tetrazol-5-yl)-2-(hydroxymethyl)-4-methyl-1H-pyrrol-3-yl)(3-bromophenyl)methanone [compound No. 280]
3-((3-bromobenzoyl)-5-(2-(2-(1,3-dioxoisoindolin-2-yl)ethyl)-2H-tetrazol-5-yl)-N,4-dimethyl-1H-pyrrole-2-carboxamide [compound No. 281]
5-((2-(2-aminoethyl)-2H-tetrazol-5-yl)-3-(3-bromobenzoyl)-N,4-dimethyl-1H-pyrrole-2-carboxamide [compound No. 282] 3-(3-bromobenzoyl)-5-(2-(2-(1,3-dioxoisoindolin-2-yl)ethyl)-2H-tetrazol-5-yl)-4-methyl-1H-pyrrole-2-carbonitrile [compound No. 283]
5-((2-(2-aminoethyl)-2H-tetrazol-5-yl)-3-(3-bromobenzoyl)-4-methyl-1H-pyrrole-2-carbonitrile [compound No. 284]
(3-bromophenyl)(5-(2-(2-hydroxyethyl)-2H-tetrazol-5-yl)-2-(2-hydroxypropan-2-yl)-4-methyl-1H-pyrrol-3-yl)methanone [compound No. 285]
(4-bromo-2-methyl-5-(1H-tetrazol-5-yl)-1H-pyrrol-3-yl)(3-bromophenyl)methanone [compound No. 286]
2-((5-(3-bromo-4-(3-bromobenzoyl)-5-methyl-1H-pyrrol-2-yl)-2H-tetrazol-2-yl)ethyl acetate [compound No. 287]
(4-bromo-5-(2-(2-hydroxyethyl)-2H-tetrazol-5-yl)-2-methyl-1H-pyrrol-3-yl)(3-bromophenyl)methanone [compound No. 288]
tert-butyl(2-(5-(3-bromo-4-(3-bromobenzoyl)-5-methyl-1H-pyrrol-2-yl)-2H-tetrazol-2-yl)ethyl)carbamate [compound No. 289]
(5-((2-(2-aminoethyl)-2H-tetrazol-5-yl)-4-bromo-2-methyl-1H-pyrrol-3-yl)(3-bromophenyl)methanone [compound No. 290]
tert-butyl(2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate [compound No. 291]
ethyl 5-(2-(2-acetoxyethyl)-2H-tetrazol-5-yl)-3-(3-bromo-4-methoxybenzoyl)-4-methyl-1H-pyrrole-2-carboxylate [compound No. 292]
ethyl 3-(3-bromo-4-methoxybenzoyl)-5-(2-(2-hydroxyethyl)-2H-tetrazol-5-yl)-4-methyl-1H-pyrrole-2-carboxylate [compound No. 293]
(5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone) [compound No. 294]
2-((5-(4-(3-bromo-4-methoxybenzoyl)-3-methyl-5-(methylcarbamoyl)-1H-pyrrol-2-yl)-2H-tetrazol-2-yl)ethyl acetate [compound No. 295]
2-((5-(4-(3-bromobenzoyl)-3-methyl-5-(methylcarbamoyl)-1H-pyrrol-2-yl)-2H-tetrazol-2-yl)ethyl dihydrogen phosphate [compound No. 303]

Examples of the salt of the compound (1) of the present invention include: inorganic acid salts such as hydrochloride, sulfate, and nitrate; organic acid salts such as acetate, citrate, fumarate, oxalate, tartrate, methanesulfonate, and p-toluenesulfonate; metal salts such as lithium salt, sodium salt, potassium salt, magnesium salt, and calcium salt; inorganic salts such as ammonium salt; and organic amine salts such as triethylamine salt. Also, the compound (1) of the present invention or a salt thereof includes solvates such as hydrates. When the compound (1) of the present invention contains asymmetric carbon atoms, optical isomers and racemates based on these carbon atoms are also included in the scope of the present invention.

Specific examples of the salt of the compound (1) of the present invention include the following compounds:

(3-((2-aminoethoxy)phenyl)(5-(2-ethyl-2H-tetrazol-5-yl)-2,4-dimethyl-1H-pyrrol-3-yl)methanone hydrochloride [compound No. 296]

2-((5-(4-(3-bromobenzoyl)-3,5-dimethyl-1H-pyrrol-2-yl)-2H-tetrazol-2-yl)ethyl 2-aminoacetate hydrochloride [compound No. 297]

(3-bromophenyl)(2,4-dimethyl-5-(5-((4-methyl piperazin-1-yl)methyl)-1,3,4-oxadiazol-2-yl)-1H-pyrrol-3-yl)methanone hydrochloride [compound No. 298]

(3-bromophenyl)(5-(5-((dimethyl amino)methyl)-1,3,4-oxadiazol-2-yl)-2,4-dimethyl-1H-pyrrol-3-yl)methanone hydrochloride [compound No. 299]

(2-((2-aminoethoxy)-5-bromophenyl)(5-(5-cyclopropyl-1,3,4-oxadiazol-2-yl)-2,4-dimethyl-1H-pyrrol-3-yl)methanone hydrochloride [compound No. 300]

(2-((2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone hydrochloride [compound No. 301]

(4-((2-aminoethoxy)-3-bromophenyl)(5-(2-ethyl-2H-tetrazol-5-yl)-2,4-dimethyl-1H-pyrrol-3-yl)methanone hydrochloride [compound No. 302]

sodium 2-(5-(4-(3-bromobenzoyl)-3-methyl-5-(methylcarbamoyl)-1H-pyrrol-2-yl)-2H-tetrazol-2-yl)ethyl phosphate [compound No. 304]

The compound (1) of the present invention or a salt thereof can be produced, for example, according to a reaction scheme shown below. Also, compounds described later in Production Examples 1 to 95 can be converted to compound (1b), (1c), (1e), or (1f) through the same synthesis as in compound (C) in the reaction scheme.

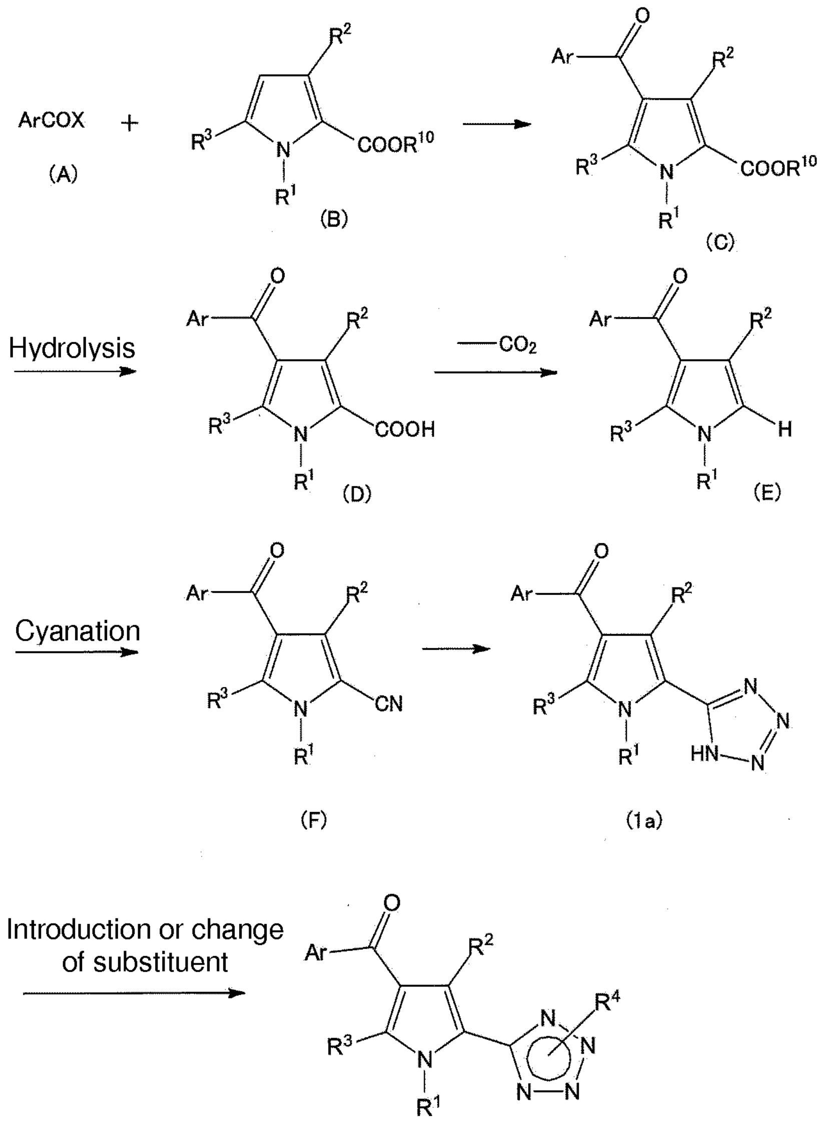


(A) (B) (C) (D) (E) (F) (1a) (1b)

wherein X represents a halogen atom; $R^{10}$ represents an alkyl group; and Ar, $R^1$, $R^2$, $R^3$, $R^4$, and n are as defined above.

Compound (A) is reacted with a pyrrole-2-carboxylic acid ester (B) in the presence of an acid catalyst to obtain compound (C), which is then hydrolyzed into compound (D). After decarboxylation of the compound (D), the resulting compound is cyanated. Subsequently, a tetrazole ring is formed through addition reaction to obtain compound (1a). The obtained compound (1a) is subjected to the introduction or change of substituents according to a routine method to obtain compound (1b).

First, the reaction of compound (A) with a pyrrole-2-carboxylic acid ester (B) is performed in the presence of an acid catalyst. The acid catalyst can be any Lewis acid usually used in Friedel-Crafts reaction. For example, $AlCl_3$, $SnCl_4$, or BF3-$OEt_2$ is used. The acid catalyst can be used at approximately 1 equivalent with respect to the compound (A). Dichloromethane or the like is used as a reaction solvent. The reaction can be performed at approximately 50°C for approximately 3 to 12 hours. This reaction can yield compound C having $R^1$, $R^2$, $R^3$, and various functional groups on the Ar ring. Specific examples of the compound C include compounds (ester forms) shown in Production Examples 1 to 95 described later. Each desired compound having the structure 2a, 2b, 2c, 2d, or 2e at position 2 of the pyrrole ring can be synthesized using this compound C as a common intermediate.

The hydrolysis reaction of compound (C) is preferably performed in the presence of an alkali. A usual alkali typified by an alkali metal hydroxide such as sodium hydroxide or potassium hydroxide is used as the alkali. Such an alkali can be used at approximately 5 equivalents with respect to the compound (C). The hydrolysis reaction can be performed at approximately 100°C for approximately 4 hours.

The decarboxylation reaction of compound (D) is performed by heating in the presence of an acid. Trifluoroacetic acid is used as the acid. An excessive amount of the acid is preferably used as a reaction solvent. The decarboxylation reaction is performed under conditions involving, for example, heating at approximately 50°C for approximately 5 minutes.

The cyanation reaction of compound (E) is performed through reaction of the compound (E) with chlorosulfonyl isocyanate or the like. An acetonitrile-dichloromethane-N,N-dimethylformamide mixed solvent or the like is preferably used as a reaction solvent. The reaction can be performed, for example, at approximately -40°C for approximately 2.5 hours and then at room temperature for approximately 12 hours.

The tetrazole ring formation reaction of compound (F) is preferably performed through reaction of the compound (F) with an azide compound such as sodium azide. For this reaction, N,N-dimethylformamide or the like is preferably used as a reaction solvent. The reaction can be performed at approximately 110°C for approximately 12 hours.

The introduction of various substituents to compound (1a) can be performed, for example, through alkylation reaction such as reaction with an alkyl halide in the presence of a base. In addition, $R^1$, $R^2$, $R^3$, $R^4$ or substituents on Ar in the obtained compound (1b) can be further subjected to functional group conversion.

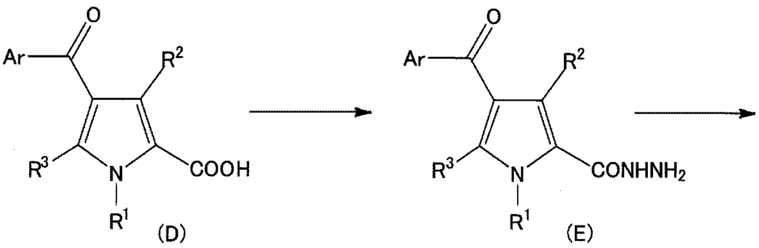

(D) (E)

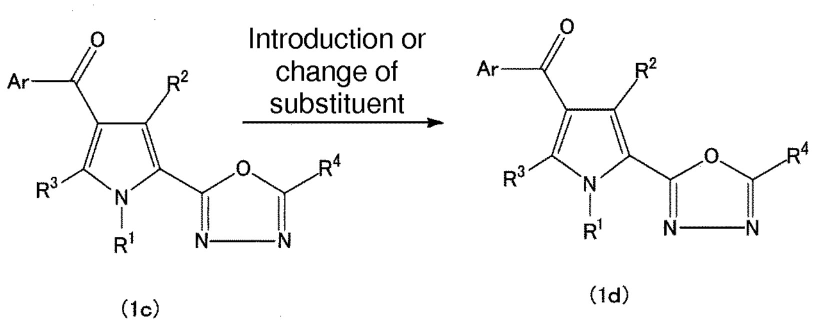


(1c) (1d)

wherein Ar, $R^1$, $R^2$, $R^3$, $R^4$, and n are as defined above.

Compound (D) is converted to a hydrazide to obtain compound (E), which is then subjected to acylation reaction with an acylating agent and intramolecular dehydration condensation reaction to obtain compound (1c).

The conversion reaction of compound (D) to a hydrazide can be performed through reaction of the compound (D) with hydrazine in the presence of a condensing agent. 1-Ethyl-3-(3-dimethylaminopropyl)carbodiimide hydrochloride or the like is used as the condensing agent. The reaction can be performed at approximately 25°C for approximately 12 hours in a solvent such as N,N-dimethylformamide.

The reaction from compound (E) to compound (1c) can be performed through reaction of the compound (E) with an acylating agent such as $R^4COOH$ or an acid halide thereof, followed by dehydration condensation reaction with p-toluenesulfonyl chloride, triethylamine, or the like. Dichloromethane or the like is preferably used as a reaction solvent. The reaction is preferably performed under conditions involving approximately 25°C for approximately 12 hours.

$R^1$, $R^2$, $R^3$, $R^4$ or substituents on Ar in the obtained compound (1c) can be further subjected to functional group conversion.

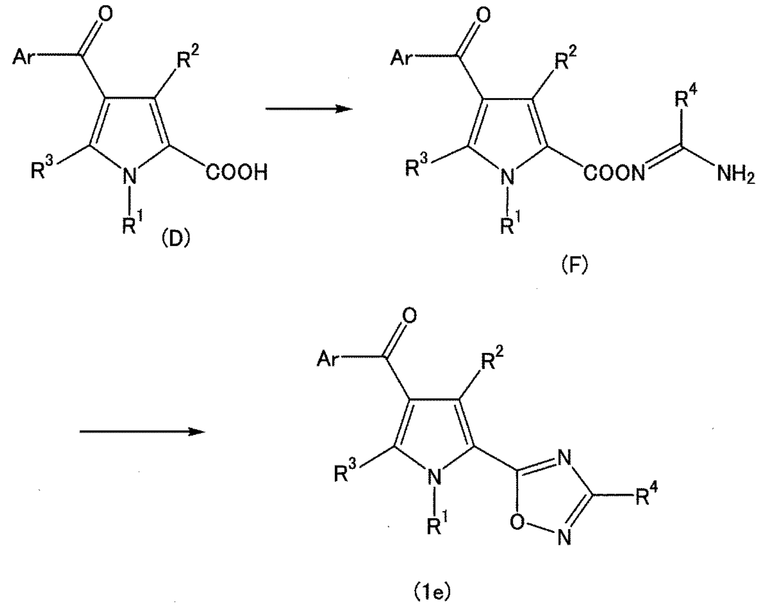

(D) (F) (1e)

wherein Ar, $R^1$, $R^2$, $R^3$, and $R^4$ are as defined above.

Compound (D) is reacted with amidoxime to obtain compound (F), which is then subjected to intramolecular dehydration condensation to obtain compound (1e).

The reaction of compound (D) with amidoxime is preferably performed in the presence of HATU. N,N-Dimethylformamide or the like is preferably used as a reaction solvent. The reaction can be performed under conditions involving approximately 25°C for approximately 12 hours.

The intramolecular dehydration condensation reaction of compound (F) can be performed, for example, by stirring at approximately 140°C in a N,N-dimethylformamide solvent.

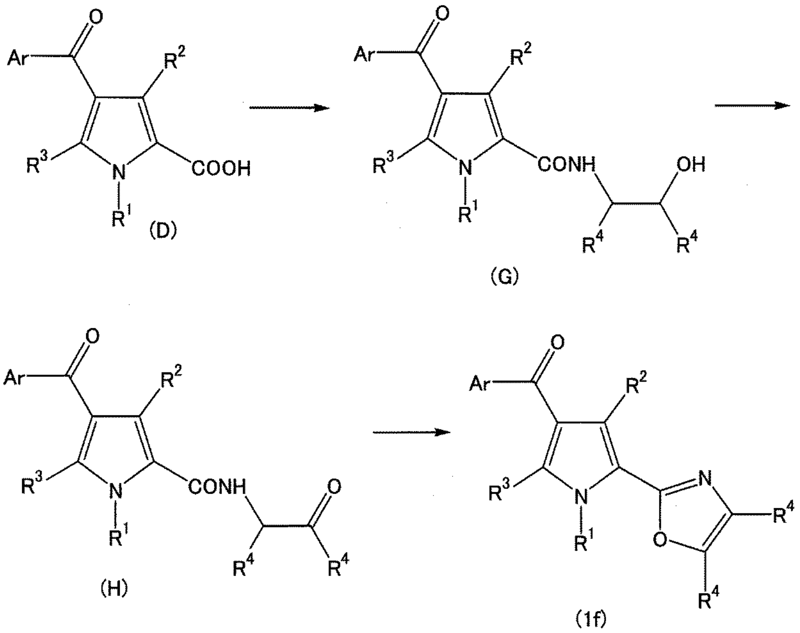

(D) (G) (H) (1f)

wherein Ar, $R^1$, $R^2$, $R^3$, and $R^4$ are as defined above.

Compound (D) is reacted with an amine compound ($H_2NCH(R^4)CH(R^4)OH$) to obtain compound (G), which is then oxidized into compound (H). The obtained compound (H) is subjected to intramolecular dehydration condensation to obtain compound (1f).

The reaction of compound (D) with an amine compound ($H_2NCH(R^4)CH(R^4)OH$) is preferably performed, for example, in the presence of 1-ethyl-3-(3-dimethylaminopropyl)carbodiimide hydrochloride or 1-hydroxybenzotriazole.

The reaction can be performed at approximately 25°C for approximately 12 hours in a solvent such as N,N-dimethylformamide.

The oxidation reaction of compound (G) is performed by use of an oxidizing agent such as Dess-Martin Periodinane.

The intramolecular dehydration condensation reaction of compound (H) can be performed with, for example, phenylphosphonic dichloride.

The obtained compound (1) of the present invention can be converted to any of the acid-addition salts described above, if desired, and can be appropriately purified by washing, recrystallization, chromatography, etc.

The compound (1) of the present invention or a salt thereof has a tubulin polymerization inhibitory effect and exhibits excellent cytotoxic activity against tumor cells and antitumor effect both *in vitro* and *in vivo,* as shown later in Examples. Thus, the compound (1) of the present invention or a salt thereof is useful as a tubulin polymerization inhibitor.

Also, the compound (1) of the present invention or a salt thereof exhibits a potent antitumor effect on drug-resistant tumor cells overexpressing P-glycoprotein, which is a factor involved in multidrug resistance. Thus, the compound (1) of the present invention or a salt thereof is useful as an anticancer agent and is particularly useful as an anticancer agent against a cancer having the potential to acquire drug resistance due to the involvement of P-glycoprotein and/or a cancer already having the resistance.

In this context, the anticancer agent is not particularly limited as long as the anticancer agent has an antitumor effect on drug-resistant tumor cells overexpressing P-glycoprotein. The anticancer agent preferably has, for example, a resistance rate of 50 or lower, more preferably 10 or lower, particularly preferably 5 or lower, as shown later in Example 5 (Table 2).

In addition, the compound (1) of the present invention or a salt thereof also exhibits a potent antitumor effect on drug-resistant tumor cells highly expressing Class III β-tubulin (TUBB3) involved in resistance to taxane anticancer agents (paclitaxel (PTX), docetaxel, cabazitaxel, etc.). Thus, the compound (1) of the present invention or a salt thereof is useful as an anticancer agent and is particularly useful as an anticancer agent against a cancer having the potential to acquire drug resistance due to the involvement of TUBB3 and/or a cancer already having the resistance.

In this context, the anticancer agent is not particularly limited as long as the anticancer agent has an antitumor effect on drug-resistant tumor cells highly expressing TUBB3. The anticancer agent preferably has, for example, a resistance rate of 2 or lower, more preferably 1 or lower, particularly preferably 0.5 or lower, as shown later in Example 7 (Table 4).

Moreover, the compound (1) of the present invention or a salt thereof exhibits an apoptosis-inducing effect, as shown later in Example 2 (Figure 1). Thus, the compound (1) of the present invention or a salt thereof is useful as an apoptosis inducer.

The compound (1) of the present invention or a salt thereof exhibits the excellent pharmacological effects and/or pharmacological activities mentioned above. Thus, a composition comprising the compound or a salt thereof is useful as a pharmaceutical composition.

Although the compound (1) of the present invention may be administered as it is, the compound (1) of the present invention can be mixed with carriers usually used in pharmaceutical formulation, such as a dispersion aid and an excipient, in the range without reducing its effects, and used in a dosage form such as an oral agent (e.g., powders, solutions, capsules, suspensions, emulsions, syrups, elixirs, granules, pills, tablets, troches, and lemonades) or an injection.

Examples of such carriers include: water-soluble monosaccharides, oligosaccharides, or polysaccharides such as mannitol, lactose, and dextran; gel-forming or water-soluble celluloses such as hydroxypropylcellulose, hydroxypropylmethylcellulose, and methylcellulose; water-absorbable and poorly water-soluble celluloses such as crystalline cellulose, α-cellulose, cross-linked sodium carboxymethylcellulose, and derivatives thereof; water-absorbable and poorly water-soluble polysaccharides such as hydroxypropyl starch, carboxymethyl starch, cross-linked starch, amylose, amylopectin, pectin, and derivatives thereof; water-absorbable and poorly water-soluble gums such as gam arabic, gum tragacanth, glucomannan, and derivatives thereof; cross-linked vinyl polymers such as polyvinylpyrrolidone, cross-linked polyacrylic acid and a salt thereof, cross-linked polyvinyl alcohol, polyhydroxyethyl methacrylate, and derivatives thereof; and molecular assembly (e.g., liposomes)-forming lipids such as phospholipids and cholesterols.

The compound (1) of the present invention having low solubility can be subjected to solubilization treatment. Examples of the solubilization treatment include usual pharmaceutically applicable methods, for example, a method involving adding a surfactant such as polyoxyethylene alcohol ethers, polyoxyethylene acyl esters, sorbitan acyl esters, or polyoxyethylene sorbitan acyl esters, and a method using a water-soluble polymer such as polyethylene glycol. Alternatively, for example, a method of forming a soluble salt or a method of forming an inclusion compound using cyclodextrin or the like may be used, if necessary.

The dose of the pharmaceutical drug of the present invention can be appropriately adjusted according to an administration method, symptoms of a patient, etc. and is preferably a daily dose of 1 mg to 10 g, more preferably 100 mg to 10 g, particularly preferably 500 mg to 10 g, for an adult in terms of the amount of the compound (1) of the present

invention or a salt thereof.

[Examples]

Next, the present invention will be described in detail with reference to Examples.

Example 1 Inhibitory effect on growth of human cancer cells *(in vitro)*

Each pyrrole compound of the present invention was studied for its effects on the growth of human non-small cell lung cancer A549 cells and human colorectal cancer HT-29 cells and HCT116 cells. The cells of each line were suspended in an RPMI1640 medium supplemented with 10% fetal bovine serum and inoculated at a cell density of 1000 cells/50 μL/well to a 96-well plate. After overnight culture, each compound diluted with a medium was added thereto at a concentration of 50 μL/well, and the cells were further cultured for 96 hours. After the completion of culture, WST-8 (Seikagaku Corp.) was added thereto at a concentration of 10 μL/well, and the cells were cultured at 37°C for approximately 1 hour under 5% $CO_2$ conditions, followed by measurement of absorbance at 450 nm. The concentration at which the compound inhibited cell growth by 50% with respect to a control ($IC_{50}$ value) was calculated from the number of live cells at each concentration. As a result, the compound of the present invention exhibited a cell growth inhibitory effect on all of the cell lines, as shown in Tables 1-1 to 1-3.

[Table 1-1]

| | IC50 (nM) | | |
|---|---|---|---|
| Compound No. | A549 | HT29 | HCT116 |
| 97 | 2.4 | 3.5 | 4.1 |
| 102 | 19.3 | 8.7 | 9.5 |
| 103 | 87.8 | 43.3 | 181.6 |
| 104 | 11.0 | 8.5 | 8.5 |
| 107 | 54.9 | 16.1 | 27.4 |
| 109 | 9.2 | 1.0 | 2.0 |
| 110 | 39.3 | 8.2 | 14.2 |
| 115 | 49.2 | 42.5 | 41.0 |
| 117 | 83.0 | 42.1 | 118.0 |
| 119 | 50.1 | 42.6 | 145.0 |
| 121 | 122.0 | 43.8 | 43.5 |
| 123 | 325.7 | 39.5 | 46.1 |
| 124 | 38.6 | 30.4 | 37.8 |
| 125 | 45.8 | 35.2 | 43.2 |
| 126 | 38.2 | 33.1 | 38.2 |
| 127 | 44.7 | 36.9 | 40.4 |
| 128 | 27.8 | 27.4 | 22.4 |
| 129 | 46.9 | 34.2 | 41.2 |
| 130 | 35.5 | 30.7 | 36.9 |
| 131 | 45.1 | 34.8 | 40.4 |
| 132 | 248.5 | 179.4 | 166.6 |
| 133 | 238.5 | 191.5 | 158.0 |
| 137 | 277.5 | 155.6 | 185.5 |
| 138 | 239.3 | 169.7 | 166.6 |

(continued)

| | IC50 (nM) | | |
|---|---|---|---|
| Compound No. | A549 | HT29 | HCT116 |
| 139 | 245.4 | 173.5 | 170.9 |
| 142 | 256.9 | 175.5 | 194.1 |
| 149 | 306.0 | 232.1 | 218.6 |
| 150 | 191.9 | 145.2 | 180.6 |
| 152 | 10.8 | 8.3 | 7.8 |
| 163 | 305.3 | 73.8 | 67.5 |
| 165 | 359.0 | 126.5 | 40.2 |
| 167 | 138.6 | 31.5 | 39.5 |
| 168 | 885.8 | 670.5 | 506.5 |
| 169 | 224.9 | 167.4 | 161.6 |
| 170 | 196.6 | 142.9 | 132.6 |
| 171 | 47.4 | 35.7 | 37.7 |
| 172 | 47.0 | 34.5 | 36.9 |
| 173 | 5.1 | 1.0 | 1.1 |
| 174 | 77.1 | 38.6 | 44.6 |
| 177 | 1.5 | 0.4 | 0.4 |
| 178 | 71.8 | 36.9 | 45.1 |
| 179 | 800.1 | 335.8 | 328.6 |

[Table 1-2]

| | | | |
|---|---|---|---|
| 185 | 9.1 | 2.8 | 2.9 |
| 186 | 288.9 | 41.7 | 64.1 |
| 188 | 37.8 | 35.1 | 40.4 |
| 189 | 163.5 | 135.2 | 199.4 |
| 190 | 266.5 | 191.5 | 218.7 |
| 193 | 4.3 | 2.1 | 2.5 |
| 195 | 12.2 | 8.5 | 9.9 |
| 196 | 9.9 | 6.2 | 5.5 |
| 197 | 3.9 | 0.9 | 1.4 |
| 198 | 1.8 | 0.3 | 0.9 |
| 199 | 14.5 | 9.0 | 10.6 |
| 200 | 389.1 | 209.2 | 646.7 |
| 202 | 127.3 | 50.5 | 62.4 |
| 203 | 209.9 | 170.7 | 151.7 |
| 204 | 31.1 | 5.9 | 8.9 |
| 205 | 64.9 | 20.3 | 37.0 |

(continued)

| | | | |
|---|---|---|---|
| 206 | 31.5 | 8.8 | 9.5 |
| 210 | 204.7 | 39.3 | 42.9 |
| 211 | 183.2 | 44.1 | 49.1 |
| 214 | 998.8 | 229.8 | 285.8 |
| 223 | 230.8 | 28.1 | 46.9 |
| 224 | 1.8 | 1.3 | 1.7 |
| 225 | 9.7 | 6.6 | 9.2 |
| 226 | 0.7 | 0.3 | 0.4 |
| 227 | 554.8 | 290.1 | 447.2 |
| 235 | 738.3 | 172.5 | 238.7 |
| 236 | 468.7 | 167.5 | 230.7 |
| 237 | 1.9 | 0.3 | 0.6 |
| 240 | 2.1 | 1.1 | 1.9 |
| 241 | 2.1 | 2.6 | 1.4 |
| 251 | 191.4 | 102.1 | 55.3 |
| 253 | 351.4 | 145.9 | 45.2 |
| 254 | 55.1 | 33.2 | 44.5 |
| 256 | 95.9 | 36.2 | 43.2 |
| 257 | 865.3 | 383.7 | 864.6 |
| 258 | 50.5 | 30.9 | 45.4 |
| 259 | 69.5 | 37.2 | 39.2 |
| 260 | 0.4 | 0.3 | 0.3 |
| 261 | 12.9 | 9.4 | 88.9 |
| 262 | 905.4 | 845.4 | 902.5 |
| 263 | 36.5 | 32.5 | 53.8 |
| 264 | 8.2 | 1.8 | 1.8 |
| 265 | 46.0 | 37.1 | 157.9 |
| 266 | 4.7 | 2.7 | 1.6 |
| 267 | 13.0 | 10.7 | 39.0 |

[Table 1-3]

| | | | |
|---|---|---|---|
| 268 | 4.1 | 5.2 | 2.5 |
| 270 | 858.9 | 724.7 | 818.6 |
| 271 | 92.9 | 44.1 | 50.4 |
| 272 | 66.6 | 42.0 | 48.4 |
| 280 | 250.2 | 194.4 | 250.2 |
| 282 | 524.6 | 314.6 | 754.3 |
| 285 | 594.9 | 597.5 | 246.4 |

(continued)

| | | | |
|---|---|---|---|
| 288 | 8.2 | 6.7 | 7.1 |
| 290 | 166.9 | 123.4 | 183.9 |
| 295 | 509.3 | 459.9 | 456.6 |
| 296 | 637.3 | 197.6 | 207.9 |
| 297 | 12.2 | 8.2 | 7.3 |
| 302 | 47.4 | 33.0 | 35.0 |

Example 2 Apoptosis-inducing effect

Each compound of the present invention shown in Figure 1 was studied for its apoptosis-inducing effect on HCT116 cells. In this experiment, a topoisomerase inhibitor SN-38 known to exhibit an apoptosis-inducing effect was used as a positive control drug. The HCT116 cells were suspended in a medium and inoculated at a cell density of 3000 cells/50 μL/well to a 96-well plate. After overnight culture, each test sample diluted with a medium was added thereto at a concentration of 50 μL/well (final concentration: 0.1 mM), and the cells were further cultured for 48 hours. After the completion of culture, the amount of intracellular fragmented DNAs was measured using Cell Death Detection ELISA PLUS kit (Roche Diagnostics K.K.) according to the attached protocol.
The amount of fragmented DNAs detected in each sample was compared with that in an untreated control to calculate a relative amount (fold). As a result, the studied compounds induced DNA fragmentation at the concentration at which each compound exhibited cell growth inhibitory effect, as shown in Figure 1. This suggested that the compound of the present invention has an apoptosis-inducing effect.

Example 3 Effect on polymerization of purified tubulin

A compound of the present invention was studied for its effect on tubulin polymerization reaction using HTS-Tubulin Polymerization Assay kit (Cytoskeleton, Inc.). Each test sample diluted with a G-PEM buffer (80 mM PIPES pH 6.9, 2 mM $MgCl_2$, 0.5 mM EGTA, 10% glycerol, and 1 mM GTP) was added at a concentration of 4 μL/well to a 96-well half-area plate and incubated at 37°C for 5 minutes. A 3 mg/mL purified tubulin solution prepared using a G-PEM buffer was further added thereto at a concentration of 36 μL/well. Polymerization reaction was started in SPECTRA Max PLUS384 kept at 37°C. After the start of polymerization reaction, absorbance at a wavelength of 340 nm was measured over time. As a result, the compound of the present invention exhibited a polymerization inhibitory effect on purified tubulin, as shown in Figure 2.

Example 4 Study of binding site on tubulin (*in vitro)*

Subsequently, a pyrrole compound of the present invention was studied for its binding site on tubulin. In this experiment, vincristine or colchicine known to bind to a vinca alkaloid-binding site or a colchicine-binding site on tubulin was used as a positive control drug. First, a purified tubulin solution (0.5 mg/mL) was prepared using a G-PEM buffer (80 mM PIPES pH 6.9, 2 mM $MgCl_2$, 0.5 mM EGTA, 10% glycerol, and 1 mM GTP). Subsequently, 3 μM $^3$H-vincristine or $^3$H-colchicine containing 1.8 x $10^4$ dpm/nmol isotopes was added to the tubulin solution. The test compound was further added thereto at a final concentration of 0.1, 1, 10, or 100 μM. Polymerization reaction was performed under conditions involving 37°C for 1 hour. After the polymerization reaction, tubulin-unbound labeled compounds were removed using a Sephadex G-25 column. A liquid scintillator (PerkinElmer, Inc.) was added to the purified reaction solution. The amount of radiation was measured using LS6500 (Beckman Coulter Inc.). As a result, the compound of the present invention exhibited a competitive binding effect only against labeled colchicine, as shown in Figure 3, suggesting that the compound of the present invention binds to a colchicine-binding site on tubulin.

Example 5 Inhibitory effect on growth of multidrug-resistant cell line *(in vitro)*

Each compound of the present invention was studied for its effect on the growth of a multidrug-resistant cell line. A drug efflux pump called P-glycoprotein is well known to be involved in the drug resistance mechanism of the multidrug-resistant line MES-SA/Dx5 used in this experiment. Accordingly, in this experiment, paclitaxel and vincristine, which are proven substrates of P-glycoprotein, were used as control drugs. Human uterus cancer MES-SA cells and their multidrug-resistant line MES-SA/Dx5 cells were suspended in a medium and inoculated at a cell density of 2000

cells/50 μL/well to a 96-well plate. After overnight culture, each test sample diluted with a medium was added thereto at a concentration of 50 μL/well, and the cells were further cultured for 48 hours. After the completion of culture, WST-8 was added thereto at a concentration of 10 μL/well, and the cells were cultured at 37°C for approximately 1 hour under 5% $CO_2$ conditions, followed by measurement of absorbance at a wavelength of 450 nm. The concentration at which the compound inhibited the growth of the cells of each line by 50% with respect to the control ($IC_{50}$ value) was calculated from the number of live cells at each concentration. The resistance rate was calculated according to the following expression:

$$\text{Resistance rate} = IC_{50}\ \text{value (MES-SA/Dx5)} / IC_{50}\ \text{value (MES-SA)}$$

As a result, the compound of the present invention exhibited a cell growth inhibitory effect even on the multidrug-resistant line at a level equivalent to that on the parent line, as shown in Table 2. This suggested that the compound of the present invention is also effective for tumor resistant to anticancer agents serving as substrates of P-glycoprotein.

[Table 2]

| | $IC_{50}$ (nM) | | |
|---|---|---|---|
| | MES-SA | MES-SA/Dx5 | Resistance rate |
| Paclitaxel | 15.2 | 3697.8 | 243.3 |
| Vincristine | 36.2 | 2443.0 | 67.5 |
| Compound 102 | 3.8 | 4.3 | 1.1 |
| Compound 110 | 5.5 | 22.7 | 4.2 |
| Compound 121 | 49.8 | 88.4 | 1.8 |
| Compound 123 | 63.3 | 135.0 | 2.1 |
| Compound 142 | 237.7 | 656.2 | 2.8 |
| Compound 198 | 0.21 | 0.28 | 1.3 |

Example 6 Antitumor effect on growth of human lung cancer NCI-H460 xenograft tumor (*in vivo*)

Each compound of the present invention was studied for its *in vivo* antitumor effect using human lung cancer NCI-H460 cell-transplanted mice. An NCI-H460 cell suspension containing $2 \times 10^6$ cells was subcutaneously transplanted in an amount of 0.1 mL/mouse to the left groin areas of 6-week-old male BALB/c nude mice. The mice were further raised for approximately 1 week to 10 days and divided into groups (4 mice per group) having equal estimated tumor volumes at the point in time when the tumor size reached approximately 100 to 200 $mm^3$ (Day 1). Each administration sample was administered at a dose of 10 mL/kg into the tail vein of the mice. The dose and administration schedule of each compound were as described in Table 3. At Day 22, tumor was excised from each mouse. The tumor growth inhibition rate (IR: Inhibition Rate) was calculated from the weight of the excised tumor according to the expression shown below. The Dunnett's test was used in the significant difference test.

$$\text{Tumor growth inhibition rate (Inhibition Rate, \%)} = (1 - (\text{Excised tumor weight of the administration group}) / (\text{Excised tumor weight of the control group})) \times 100$$

As a result, the compound of the present invention was shown to exert an antitumor effect on the human lung cancer NCI-H460 cell-transplanted mice, as shown in Table 3 and Figure 4. The administration of the compound of the present invention was confirmed to be free from toxicity such as the weight loss of recipient mice.

[Table 3]

| Compound No. | Dose (mg/kg) | Administration (Days) schedule | Inhibition Rate (%) | Significance |
|---|---|---|---|---|
| 97 | 30 | 1. 3, 5, 8, 10, 12, 15, 17, 19 | 31.2 | p<0.05 |
| 102 | 12.5 | 1, 3, 5, 8, 10, 12, 15, 17, 19 | 43.4 | p<0.001 |
| 102 | 25 | 1, 3, 5, 8, 10, 12, 15, 17, 19 | 61.1 | p<0.001 |
| 102 | 37.5 | 1, 3, 5, 8, 10, 12, 15, 17, 19 | 80.5 | p<0.001 |
| 102 | 40 | 1, 5, 9 | 46.5 | p<0.001 |
| 107 | 25 | 1, 3, 5, 8, 10, 12, 15, 17, 19 | 32.6 | p<0.001 |
| 109 | 25 | 1, 3, 8, 10, 12, 15, 17, 19 | 29.0 | p<0.05 |
| 110 | 12.5 | 1, 3, 5, 8, 10, 12, 15, 17, 19 | 55.8 | p<0.001 |
| 110 | 18.75 | 1, 3, 5, 8, 10, 12, 15, 17, 19 | 77.4 | p<0.001 |
| 121 | 60 | 1, 3, 5, 8, 10, 12, 15, 17, 19 | 60.6 | p<0.001 |
| 121 | 120 | 1, 8, 15 | 75.5 | p<0.001 |
| 123 | 50 | 1, 3, 5, 8, 10, 12, 15, 17, 19 | 58.2 | p<0.001 |
| 123 | 100 | 1, 5, 9 | 56.5 | p<0.001 |
| 142 | 140 | 1, 8, 15 | 73.8 | p<0.001 |
| 173 | 10 | 1, 3, 5, 8, 10, 12, 15, 17, 19 | 40.2 | p<0.01 |
| 193 | 10 | 1, 3, 8, 10, 12, 15, 17, 19 | 33.9 | p<0.01 |
| 198 | 10 | 1, 3, 8, 10, 12, 15, 17, 19 | 51.6 | P<0.001 |
| 240 | 12.5 | 1, 3, 5, 8, 10, 12, 15, 17, 19 | 33.2 | p<0.001 |
| 263 | 50 | 1, 3, 5, 8, 10, 12, 15, 17, 19 | 59.6 | p<0.001 |
| 266 | 20 | 1, 3, 5, 8, 10, 12, 15, 17, 19 | 39.4 | p<0.01 |
| 267 | 20 | 1, 3, 5, 8, 10, 12, 15, 17, 19 | 35.6 | p<0.01 |
| 268 | 20 | 1, 3, 5, 8, 10, 12, 15, 17, 19 | 41.2 | p<0.01 |

Example 7 Inhibitory effect on growth of PTX-resistant tumor cell line highly expressing TUBB3 (*in vitro*)

Each compound of the present invention was studied for its effect on the growth of a tumor cell line having a PTX resistance mechanism different from that attributed to the high expression of P-glycoprotein. Human non-small cell lung cancer NCI-H460 cells were exposed to 1 nM paclitaxel (PTX) and 10 μM verapamil at the same time. Only the PTX concentration was increased to 2, 3, 4, 5, and 7 nM in order over approximately 6 months to establish PTX-resistant H460/PV2-7 cells (PV2-7). An evaluation test on sensitivity to PTX revealed that the PV2-7 cells had approximately 4.6 times the resistance of the parent line. Also, the PV2-7 cells did not highly express the P-glycoprotein gene in expression analysis, and the PTX sensitivity of the PV2-7 cells did not vary upon exposure to verapamil inhibiting the drug efflux function of P-glycoprotein, demonstrating that the high expression of P-glycoprotein is not involved in the PTX resistance mechanism of the PV2-7 cells. As for the PTX resistance mechanism different from that attributed to the high expression of P-glycoprotein, an isotype called Class III β-tubulin (TUBB3), which is one isotype of β-tubulin, has been reported to be highly expressed in PTX-resistant established lines *in vitro* or in clinical samples from patients having resistance to PTX therapy. As a result of expression analysis, the PV2-7 cells were shown to highly express the TUBB3 gene and protein, suggesting that the high expression of TUBB3 is involved in the PTX resistance mechanism of the PV2-7 cells.

The PV2-7 cells and the parent line NCI-H460 cells were suspended in a medium and inoculated at a cell density of 1000 cells/50 μL/well to a 96-well plate. After overnight culture, each compound diluted 2-fold (final concentration) was added thereto at a concentration of 50 μL/well, and the cells were cultured for 96 hours. After the completion of culture, WST-8 (Seikagaku Corp.) was added thereto at a concentration of 10 μL/well, and the cells were cultured at 37°C for approximately 1 hour under 5% $CO_2$ conditions, followed by measurement of absorbance at 450 nm. The concentration at which the compound inhibited cell growth by 50% with respect to a control ($IC_{50}$ value) was calculated from the number of live cells at each concentration. As a result, the compound of the present invention exhibited a potent

cell growth inhibitory effect on both the cell lines, as shown in Table 4.

[Table 4]

| | $IC_{50}$ (nM) | | |
|---|---|---|---|
| | H460 | H460/PV2-7 | Resistance rate |
| Paclitaxel | 3.5 | 16.5 | 4.6 |
| Docetaxel | 1.5 | 4.0 | 2.6 |
| Cabazitaxel | 1.3 | 4.0 | 3.2 |
| Compound 121 | 76.2 | 20.6 | 0.27 |
| Compound 123 | 68.8 | 21.0 | 0.31 |
| Compound 142 | 374.33 | 107.77 | 0.29 |

Example 8 Antitumor effect on growth of PV2-7 xenograft tumor (*in vivo*)

A compound of the present invention was studied for its *in vivo* antitumor effect using PTX-resistant PV2-7 cell-transplanted mice. A PV2-7 cell suspension containing $2 \times 10^6$ cells was subcutaneously transplanted in an amount of 0.1 mL/mouse to the left groin areas of 6-week-old male BALB/c nude mice. The mice were further raised for approximately 1 week to 10 days and divided into groups (4 or 5 mice per group) having equal estimated tumor volumes at the point in time when the tumor size reached approximately 100 to 200 $mm^3$ (Day 1). Each administration sample was administered at a dose of 10 mL/kg into the tail vein of the mice. In an administration schedule, each sample was administered once a week, three times in total (Days 1, 8, and 15). As a result, PTX was hardly effective for the PV2-7 cell-transplanted mice, whereas the compound of the present invention exhibited a dose-dependent antitumor effect, as shown in Figure 5. The administration of the compound of the present invention was confirmed to be free from toxicity such as the weight loss of recipient mice. This suggested that the compound of the present invention exerts an antitumor effect on tumor highly expressing TUBB3.

Production Example 1: Synthesis of ethyl 4-benzoyl-3,5-dimethyl-1H-pyrrole-2-carboxylate [compound No. 1] Production Example 1-1: Synthesis of ethyl 4-benzoyl-3,5-dimethyl-1H-pyrrole-2-carboxylate [compound No. 1]

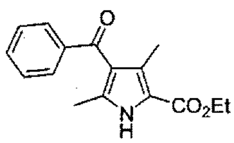

To a solution of benzoyl chloride (59 μl, 0.5 mmol) in dichloromethane (2 ml), tin tetrachloride (1 M solution in dichloromethane, 500 μl, 0.5 mmol) and ethyl 3,5-dimethyl-1H-pyrrole-2-carboxylate (84 mg, 0.5 mmol) were added in the argon atmosphere, and the reaction solution was heated to reflux. After 12 hours, the reaction solution was allowed to cool and quenched by the addition of water. The aqueous layer was extracted with chloroform. The obtained organic layer was dried over anhydrous magnesium sulfate. Magnesium sulfate was filtered off, and the solvent was distilled off under reduced pressure. The residue was purified by column chromatography to obtain the compound of interest (116 mg, yield: 85.5%, white solid).
$^1$H-NMR (400 MHz, $CDCl_3$) δ: 1.38 (3H, t, J = 7.1 Hz), 2.24 (3H, s), 2.25 (3H, s), 4.35 (2H, q, J = 7.1 Hz), 7.41-7.49 (2H, m), 7.51-7.57 (1H, m), 7.69-7.76 (2H, m), 9.59 (1H, s).

Production Example 2: Synthesis of ethyl 3,5-dimethyl-4-(2-methylbenzoyl)-1H-pyrrole-2-carboxylate [compound No. 2]

Production Example 2-1: Synthesis of ethyl 3,5-dimethyl-4-(2-methylbenzoyl)-1H-pyrrole-2-carboxylate [compound No. 2]

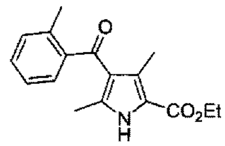

The compound of interest was obtained by the same procedures as in Production Example 1-1 using 2-methylbenzoyl chloride. Yield: quant., white solid.
$^1$H-NMR (400 MHz, $CDCl_3$) δ: 1.36 (3H, t, J = 7.1 Hz), 2.15 (3H, s), 2.22 (3H, s), 2.35 (3H, s), 4.33 (2H, q, J = 7.1 Hz), 7.19-7.28 (3H, m), 7.31-7.36 (1H, m), 8.92 (1H, s).

Production Example 3: Synthesis of ethyl 4-(2-methoxybenzoyl)-3,5-dimethyl-1H-pyrrole-2-carboxylate [compound No. 3]

Production Example 3-1: Synthesis of ethyl 4-(2-methoxybenzoyl)-3,5-dimethyl-1H-pyrrole-2-carboxylate [compound No. 3]

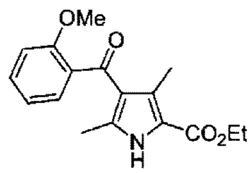

The compound of interest was obtained by the same procedures as in Production Example 1-1 using 2-methoxybenzoyl chloride. Yield: quant., white solid.
$^1$H-NMR (400 MHz, $CDCl_3$) δ: 1.36 (3H, t, J = 7.1 Hz), 2.21 (3H, s), 2.25 (3H, s), 3.78 (3H, s), 4.32 (2H, q, J = 7.1 Hz), 6.93-7.03 (2H, m), 7.25-7.29 (1H, m), 7.38-7.44 (1H, m), 8.89 (1H, s).

Production Example 4: Synthesis of ethyl 4-(2-fluorobenzoyl)-3,5-dimethyl-1H-pyrrole-2-carboxylate [compound No. 4]

Production Example 4-1: Synthesis of ethyl 4-(2-fluorobenzoyl)-3,5-dimethyl-1H-pyrrole-2-carboxylate [compound No. 4]

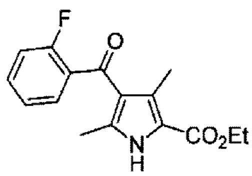

The compound of interest was obtained by the same procedures as in Production Example 1-1 using 2-fluorobenzoyl chloride. Yield: 44.2 %, white solid. $^1$H-NMR (400 MHz, $CDCl_3$) δ: 1.37 (3H, t, J = 7.2 Hz), 2.26 (3H, s), 2.27 (3H, s), 4.33 (2H, q, J = 7.2 Hz), 7.08-7.15 (1H, m), 7.20-7.25 (1H, m), 7.44-7.52 (2H,m), 8.90 (1H, s).

Production Example 5: Synthesis of ethyl 4-(2-chlorobenzoyl)-3,5-dimethyl-1H-pyrrole-2-carboxylate [compound No. 5]

Production Example 5-1: Synthesis of ethyl 4-(2-chlorobenzoyl)-3,5-dimethyl-1H-pyrrole-2-carboxylate [compound No. 5]

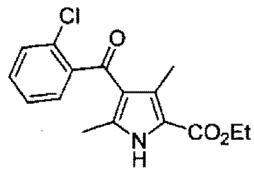

The compound of interest was obtained by the same procedures as in Production Example 1-1 using 2-chlorobenzoyl chloride. Yield: 54.9 %, white solid. $^1$H-NMR (400 MHz, $CDCl_3$) δ: 1.36 (3H, t, J = 7.1 Hz), 2.20 (3H, s), 2.26 (3H, s), 4.33 (2H, q, J = 7.2 Hz), 7.29-7.45 (4H, m), 8.96 (1H, s).

Production Example 6: Synthesis of ethyl 4-(2-bromobenzoyl)-3,5-dimethyl-1H-pyrrole-2-carboxylate [compound No. 6]

Production Example 6-1: Synthesis of ethyl 4-(2-bromobenzoyl)-3,5-dimethyl-1H-pyrrole-2-carboxylate [compound No. 6]

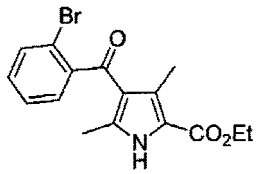

The compound of interest was obtained by the same procedures as in Production Example 1-1 using 2-bromobenzoyl chloride. Yield: 61.7 %, white solid.
$^1$H-NMR (400 MHz, $CDCl_3$) δ: 1.36 (3H, t, J = 7.2 Hz), 2.19 (3H, s), 2.26 (3H, s), 4.33 (2H, q, J = 7.2 Hz), 7.27-7.33 (2H, m), 7.36-7.42 (1H, m), 7.59-7.63 (1H, m), 8.93 (1H, s).

Production Example 7: Synthesis of ethyl 3,5-dimethyl-4-(3-methylbenzoyl)-1H-pyrrole-2-carboxylate [compound No. 7]

Production Example 7-1: Synthesis of ethyl 3,5-dimethyl-4-(3-methylbenzoyl)-1H-pyrrole-2-carboxylate [compound No. 7]

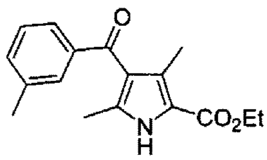

The compound of interest was obtained by the same procedures as in Production Example 1-1 using 3-methylbenzoyl chloride. Yield: quant., white solid.
$^1$H-NMR (400 MHz, $CDCl_3$) δ: 1.38 (3H, t, J = 7.2 Hz), 2.23 (3H, s), 2.25 (3H, s), 2.40 (3H, s), 4.34 (2H, q, J = 7.2 Hz), 7.30-7.37 (2H, m), 7.48-7.56 (2H, m), 8.94 (1H, s).

Production Example 8: Synthesis of ethyl 4-(3-methoxybenzoyl)-3,5-dimethyl-1H-pyrrole-2-carboxylate [compound No. 8]

Production Example 8-1: Synthesis of ethyl 4-(3-methoxybenzoyl)-3,5-dimethyl-1H-pyrrole-2-carboxylate [compound No. 8]

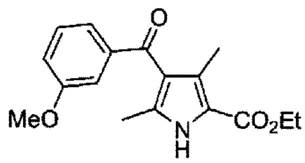

The compound of interest was obtained by the same procedures as in Production Example 1-1 using 3-methoxybenzoyl chloride. Yield: 87.6 %, white solid.
$^1$H-NMR (400 MHz, $CDCl_3$) δ: 1.37 (3H, t, J = 7.1 Hz), 2.24 (3H, s), 2.25 (3H, s), 3.85 (3H, s), 4.34 (2H, q, J = 7.1 Hz), 7.06-7.11 (1H, m), 7.26-7.30 (2H, m), 7.31-7.37 (1H, m), 8.91 (1H, s).

Production Example 9: Synthesis of ethyl 4-(3-fluorobenzoyl)-3,5-dimethyl-1H-pyrrole-2-carboxylate [compound No. 9]

Production Example 9-1: Synthesis of ethyl 4-(3-fluorobenzoyl)-3,5-dimethyl-1H-pyrrole-2-carboxylate [compound No. 9]

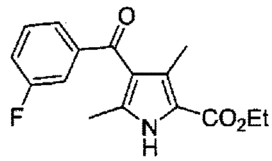

The compound of interest was obtained by the same procedures as in Production Example 1-1 using 3-fluorobenzoyl chloride. Yield: 77.4 %, white solid. $^1$H-NMR (400 MHz, $CDCl_3$) δ: 1.38 (3H, t, J = 7.2 Hz), 2.23 (3H, s), 2.27 (3H, s), 4.34 (2H, q, J = 7.2 Hz), 7.21-7.26 (1H, m), 7.39-7.45 (2H, m), 7.48-7.52 (1H,m), 8.95 (1H, s).

Production Example 10: Synthesis of ethyl 4-(3-chlorobenzoyl)-3,5-dimethyl-1H-pyrrole-2-carboxylate [compound No. 10]

Production Example 10-1: Synthesis of ethyl 4-(3-chlorobenzoyl)-3,5-dimethyl-1H-pyrrole-2-carboxylate [compound No. 10]

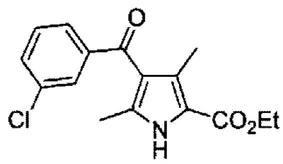

The compound of interest was obtained by the same procedures as in Production Example 1-1 using 3-chlorobenzoyl chloride. Yield: 20.9 %, white solid. $^1$H-NMR (400 MHz, $CDCl_3$) δ: 1.38 (3H, t, J = 7.2 Hz), 2.22 (3H, s), 2.26 (3H, s), 4.34 (2H, q, J = 7.2 Hz), 7.37-7.42 (1H, m), 7.49-7.53 (1H, m), 7.57-7.61 (1H,m), 7.68-7.70 (1H, m), 8.91 (1H, s).

Production Example 11: Synthesis of ethyl 4-(3-bromobenzoyl)-3,5-dimethyl-1H-pyrrole-2-carboxylate [compound No. 11]

Production Example 11-1: Synthesis of ethyl 4-(3-bromobenzoyl)-3,5-dimethyl-1H-pyrrole-2-carboxylate [compound No. 11]

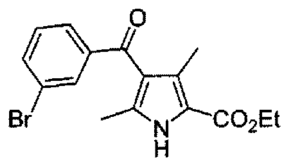

The compound of interest was obtained by the same procedures as in Production Example 1-1 using 3-bromobenzoyl chloride. Yield: 76.5 %, pale yellow solid. $^1$H-NMR (400 MHz, $CDCl_3$) δ: 1.38 (3H, t, J = 7.1 Hz), 2.22 (3H, s), 2.27 (3H, s), 4.35 (2H, q, J = 7.2 Hz), 7.31-7.36 (1H, m), 7.62-7.69 (2H, m), 7.83-7.85 (1H,m), 8.99 (1H, s).

Production Example 12: Synthesis of ethyl 4-(3-cyanobenzoyl)-3,5-dimethyl-1H-pyrrole-2-carboxylate [compound No. 12]

Production Example 12-1: Synthesis of ethyl 4-(3-cyanobenzoyl)-3,5-dimethyl-1H-pyrrole-2-carboxylate [compound No. 12]

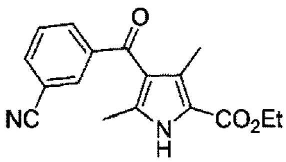

The compound of interest was obtained by the same procedures as in Production Example 1-1 using 3-cyanobenzoyl chloride. Yield: 21.6 %, white solid. $^1$H-NMR (400 MHz, $CDCl_3$) δ: 1.38 (3H, t, J = 7.1 Hz), 2.18 (3H, s), 2.29 (3H, s), 4.35 (2H, q, J = 7.1 Hz), 7.57-7.63 (1H, m), 7.80-7.85 (1H, m), 7.93-8.00 (2H,m), 8.96 (1H, s).

Production Example 13: Synthesis of ethyl 3,5-dimethyl-4-(4-methylbenzoyl)-1H-pyrrole-2-carboxylate [compound No. 13]

Production Example 13-1: Synthesis of ethyl 3,5-dimethyl-4-(4-methylbenzoyl)-1H-pyrrole-2-carboxylate [compound No. 13]

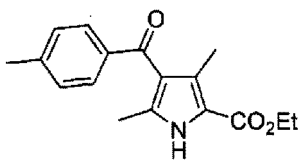

The compound of interest was obtained by the same procedures as in Production Example 1-1 using 4-methylbenzoyl chloride. Yield: quant., yellow solid.
$^1$H-NMR (400 MHz, $CDCl_3$) δ: 1.37 (3H, t, J = 7.1 Hz), 2.23 (3H, s), 2.25 (3H, s), 2.42 (3H, s), 4.34 (2H, q, J = 7.1 Hz), 7.22-7.26 (2H, m), 7.61-7.66 (2H, m), 8.95 (1H, s).

Production Example 14: Synthesis of ethyl 4-(4-methoxybenzoyl)-3,5-dimethyl-1H-pyrrole-2-carboxylate [compound No. 14]

Production Example 14-1: Synthesis of ethyl 4-(4-methoxybenzoyl)-3,5-dimethyl-1H-pyrrole-2-carboxylate [compound No. 14]

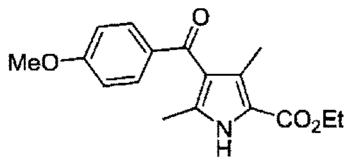

The compound of interest was obtained by the same procedures as in Production Example 1-1 using 4-methoxybenzoyl chloride. Yield: 92.9 %, white solid.
$^1$H-NMR (400 MHz, $CDCl_3$) δ: 1.37 (3H, t, J = 7.2 Hz), 2.24 (3H, s), 2.26 (3H, s), 3.88 (3H, s), 4.34 (2H, q, J = 7.2 Hz), 6.91-6.95 (2H, m), 7.72-7.76 (2H, m), 8.87 (1H, s).

Production Example 15: Synthesis of ethyl 4-(4-fluorobenzoyl)-3,5-dimethyl-1H-pyrrole-2-carboxylate [compound No. 15]

Production Example 15-1: Synthesis of ethyl 4-(4-fluorobenzoyl)-3,5-dimethyl-1H-pyrrole-2-carboxylate [compound No. 15]

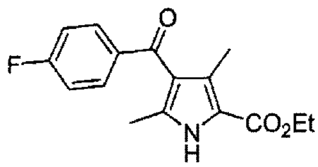

The compound of interest was obtained by the same procedures as in Production Example 1-1 using 4-fluorobenzoyl chloride. Yield: quant., yellow solid.
$^1$H-NMR (400 MHz, $CDCl_3$) δ: 1.38 (3H, t, J = 7.2 Hz), 2.22 (3H, s), 2.27 (3H, s), 4.34 (2H, q, J = 7.2 Hz), 7.10-7.16 (2H, m), 7.74-7.79 (2H, m), 8.96 (1H, s).

Production Example 16: Synthesis of ethyl 4-(4-chlorobenzoyl)-3,5-dimethyl-1H-pyrrole-2-carboxylate [compound No. 16]

Production Example 16-1: Synthesis of ethyl 4-(4-chlorobenzoyl)-3,5-dimethyl-1H-pyrrole-2-carboxylate [compound No. 16]

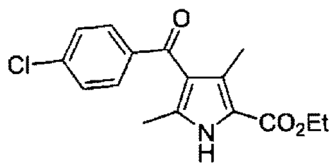

The compound of interest was obtained by the same procedures as in Production Example 1-1 using 4-chlorobenzoyl chloride. Yield: 34.0 %, white solid. $^1$H-NMR (400 MHz, $CDCl_3$) δ: 1.38 (3H, t, J = 7.1 Hz), 2.22 (3H, s), 2.26 (3H, s), 4.34 (2H, q, J = 7.1 Hz), 7.40-7.45 (2H, m), 7.65-7.70 (2H, m), 8.89 (1H, s).

Production Example 17: Synthesis of ethyl 4-(4-bromobenzoyl)-3,5-dimethyl-1H-pyrrole-2-carboxylate [compound No. 17]

Production Example 17-1: Synthesis of ethyl 4-(4-bromobenzoyl)-3,5-dimethyl-1H-pyrrole-2-carboxylate [compound No. 17]

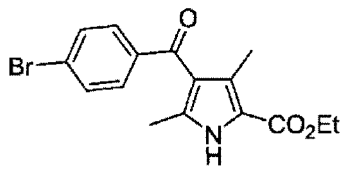

The compound of interest was obtained by the same procedures as in Production Example 1-1 using 4-bromobenzoyl chloride. Yield: 43.4 %, white solid. $^1$H-NMR (400 MHz, $CDCl_3$) δ: 1.38 (3H, t, J = 7.1 Hz), 2.22 (3H, s), 2.26 (3H, s), 4.34 (2H, q, J = 7.1 Hz), 7.59 (4H, s), 8.97 (1H, s).

Production Example 18: Synthesis of ethyl 4-(4-cyanobenzoyl)-3,5-dimethyl-1H-pyrrole-2-carboxylate [compound No. 18]

Production Example 18-1: Synthesis of ethyl 4-(4-cyanobenzoyl)-3,5-dimethyl-1H-pyrrole-2-carboxylate [compound No. 18]

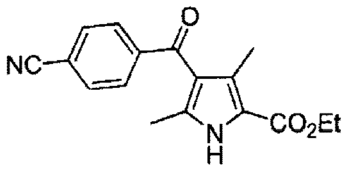

The compound of interest was obtained by the same procedures as in Production Example 1-1 using 4-cyanobenzoyl chloride. Yield: 16.2 %, white solid. $^1$H-NMR (400 MHz, $CDCl_3$) δ: 1.38 (3H, t, J = 7.1 Hz), 2.18 (3H, s), 2.27 (3H, s), 4.35 (2H, q, J = 7.1 Hz), 7.73-7.81 (4H, m), 8.97 (1H, s).

Production Example 19: Synthesis of ethyl 3,5-dimethyl-4-(3-nitrobenzoyl)-1H-pyrrole-2-carboxylate [compound No. 19]

Production Example 19-1: Synthesis of ethyl 3,5-dimethyl-4-(3-nitrobenzoyl)-1H-pyrrole-2-carboxylate [compound No. 19]

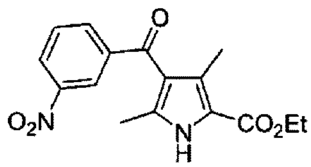

The compound of interest was obtained by the same procedures as in Production Example 1-1 using 3-nitrobenzoyl chloride. Yield: 35.6 %, yellow solid. $^1$H-NMR (400 MHz, $CDCl_3$) δ: 1.38 (3H, t, J = 7.1 Hz), 2.20 (3H, s), 2.30 (3H, s), 4.36 (2H, q, J = 7.1 Hz), 7.65-7.70 (1H, m), 8.05-8.09 (1H, m), 8.38-8.42 (1H, m), 8.51-8.53 (1H, m), 9.07 (1H, s).

Production Example 20: Synthesis of ethyl 4-([1,1'-biphenyl]-3-carbonyl)-3,5-dimethyl-1H-pyrrole-2-carboxylate [compound No. 20]

Production Example 20-1: Synthesis of ethyl 4-([1,1'-biphenyl]-3-carbonyl)-3,5-dimethyl-1H-pyrrole-2-carboxylate [compound No. 20]

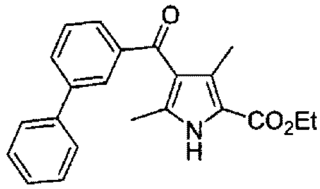

The compound of interest was obtained by the same procedures as in Production Example 1-1 using 3-phenylbenzoyl chloride. Yield: 53.8 %, white solid. $^1$H-NMR (400 MHz, DMSO-$d_6$) δ: 1.30 (3H, t, J = 7.2 Hz), 2.11 (3H, s), 2.19 (3H, s), 4.26 (2H, q, J = 7.2 Hz), 7.40 (1H, t, J = 7.3 Hz), 7.49 (2H, t, J = 7.6 Hz), 7.61-7.59 (2H, m), 7.70-7.68 (2H, m), 7.82 (1H, s), 7.91-7.89 (1H, m), 11.95 (1H, br s).

Production Example 21: Synthesis of ethyl 4-(3,5-dibromobenzoyl)-3,5-dimethyl-1H-pyrrole-2-carboxylate [compound No. 21]

Production Example 21-1: Synthesis of ethyl 4-(3,5-dibromobenzoyl)-3,5-dimethyl-1H-pyrrole-2-carboxylate [compound No. 21]

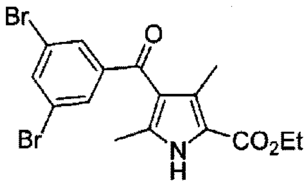

The compound of interest was obtained by the same procedures as in Production Example 1-1 using 3,5-dibromobenzoyl chloride. Yield: 85.4 %, white solid. $^1$H-NMR (400 MHz, $CDCl_3$) δ: 1.37 (3H, t, J = 7.1 Hz), 2.24 (3H, s), 2.26 (3H, s), 4.33 (2H, q, J = 7.1 Hz), 7.39-7.53 (3H, m), 8.94 (1H, s).

Production Example 22: Synthesis of ethyl 4-(3-bromo-4-methoxybenzoyl)-3,5-dimethyl-1H-pyrrole-2-carboxylate [compound No. 22]

Production Example 22-1: Synthesis of ethyl 4-(3-bromo-4-methoxybenzoyl)-3,5-dimethyl-1H-pyrrole-2-carboxylate [compound No. 22]

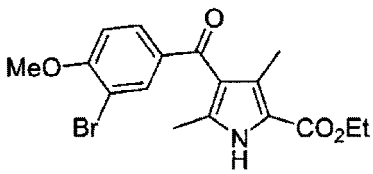

The compound of interest was obtained by the same procedures as in Production Example 1-1 using 3-bromo-4-methoxybenzoyl chloride. Yield: 71.0 %, white solid.
$^1$H-NMR (400 MHz, $CDCl_3$) δ: 1.38 (3H, t, J = 7.2 Hz), 2.24 (3H, s), 2.27 (3H, s), 3.97 (3H, s), 4.34 (2H, q, J = 7.2 Hz), 6.92-6.95 (1H, m), 7.70-7.74 (1H, m), 7.96-7.97 (1H, m), 8.88 (1H, s).

Production Example 23: Synthesis of ethyl 4-(3-bromo-4-methylbenzoyl)-3,5-dimethyl-1H-pyrrole-2-carboxylate [compound No. 23]

Production Example 23-1: Synthesis of ethyl 4-(3-bromo-4-methylbenzoyl)-3,5-dimethyl-1H-pyrrole-2-carboxylate [compound No. 23]

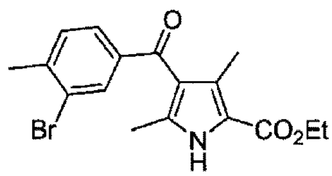

The compound of interest was obtained by the same procedures as in Production Example 1-1 using 3-bromo-4-methylbenzoyl chloride. Yield: 67.7 %, white solid. $^1$H-NMR (400 MHz, $CDCl_3$) δ: 1.38 (3H, t, J = 7.1 Hz), 2.23 (7H, s), 2.26 (7H, s), 2.46 (3H, s), 4.34 (2H, q, J = 7.2 Hz), 7.29-7.33 (1H, m), 7.55-7.59 (1H, m), 7.88-7.89 (1H, m), 8.99 (1H, s).

Production Example 24: Synthesis of ethyl 4-(benzo[d][1,3]dioxole-5-carbonyl)-3,5-dimethyl-1H-pyrrole-2-carboxylate [compound No. 24]

Production Example 24-1: Synthesis of ethyl 4-(benzo[d][1,3]dioxole-5-carbonyl)-3,5-dimethyl-1H-pyrrole-2-carboxylate [compound No. 24]

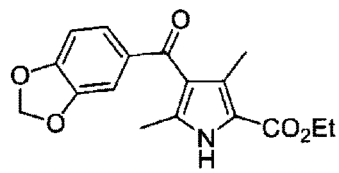

The compound of interest was obtained by the same procedures as in Production Example 1-1 using 3,4-methylenedioxybenzoyl chloride. Yield: 79.1 %, white solid.
$^1$H-NMR (400 MHz, DMSO-$d_6$) δ: 1.30 (3H, t, J = 7.2 Hz), 2.10 (3H, s), 2.16 (3H,s), 4.25 (2H, q, J = 7.2 Hz), 6.14 (2H, s), 6.99 (1H, d, J = 7.8 Hz), 7.18 (2H, td, J = 8.4, 1.7 Hz), 11.85 (1H, br s).

Production Example 25: Synthesis of ethyl 4-(2-naphthoyl)-3,5-dimethyl-1H-pyrrole-2-carboxylate [compound No. 25]

Production Example 25-1: Synthesis of ethyl 4-(2-naphthoyl)-3,5-dimethyl-1H-pyrrole-2-carboxylate [compound No. 25]

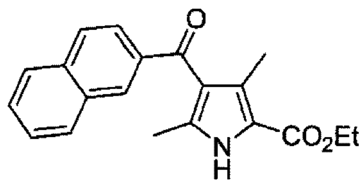

The compound of interest was obtained by the same procedures as in Production Example 1-1 using 2-naphthalenecarbonyl chloride. Yield: 30.5 %, white solid. $^1$H-NMR (400 MHz, DMSO-$d_6$) δ: 1.31 (3H, t, J = 7.1 Hz), 2.10 (3H, s), 2.19 (3H,s), 4.27 (2H, q, J = 7.1 Hz), 7.62 (2H, dtd, J = 23.1, 7.5, 1.3 Hz), 7.75 (1H, dd, J = 8.5, 1.7 Hz), 8.02 (2H, t, J = 8.5 Hz), 8.10 (1H, d, J = 8.1 Hz), 8.21 (1H, s), 11.95 (1H, s).

Production Example 26: Synthesis of ethyl 4-(furan-2-carbonyl)-3,5-dimethyl-1H-pyrrole-2-carboxylate [compound No. 26]

Production Example 26-1: Synthesis of ethyl 4-(furan-2-carbonyl)-3,5-dimethyl-1H-pyrrole-2-carboxylate [compound No. 26]

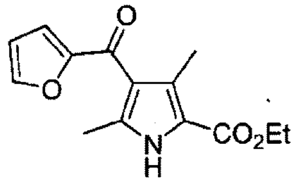

The compound of interest was obtained by the same procedures as in Production Example 1-1 using furan-2-carbonyl chloride. Yield: 15.3 %, white solid.
$^1$H-NMR (400 MHz, $CDCl_3$) $\delta$: 1.38 (3H, t, J = 7.2 Hz), 2.33 (6H, s), 4.34 (2H, q, J = 7.2 Hz), 6.55-6.57 (1H, m), 7.10-7.12 (1H, m), 7.60-7.62 (1H, m), 8.96 (1H,s).

Production Example 27: Synthesis of ethyl 4-(furan-3-carbonyl)-3,5-dimethyl-1H-pyrrole-2-carboxylate [compound No. 27]

Production Example 27-1: Synthesis of ethyl 4-(furan-3-carbonyl)-3,5-dimethyl-1H-pyrrole-2-carboxylate [compound No. 27]

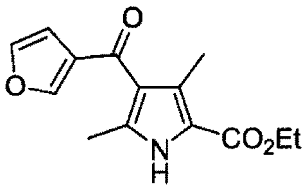

The compound of interest was obtained by the same procedures as in Production Example 1-1 using furan-3-carbonyl chloride. Yield: 33.7 %, white solid.
$^1$H-NMR (400 MHz, $CDCl_3$) $\delta$: 1.38 (3H, t, J = 7.1 Hz), 2.34 (6H, s), 4.34 (2H, q, J = 7.1 Hz), 6.76-6.78 (1H, m), 7.45-7.47 (1H, m), 7.77-7.79 (1H, m), 8.97 (1H,s).

Production Example 28: Synthesis of ethyl 3,5-dimethyl-4-(thiophene-2-carbonyl)-1H-pyrrole-2-carboxylate [compound No. 28]

Production Example 28-1: Synthesis of ethyl 3,5-dimethyl-4-(thiophene-2-carbonyl)-1H-pyrrole-2-carboxylate [compound No. 28]

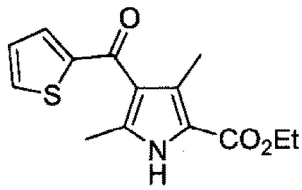

The compound of interest was obtained by the same procedures as in Production Example 1-1 using thiophene-2-carbonyl chloride. Yield: 69.2 %, white solid.
$^1$H-NMR (400 MHz, $CDCl_3$) δ: 1.38 (3H, t, J = 7.2 Hz), 2.33 (6H, s), 4.34 (2H, q, J = 7.2 Hz), 7.12 (1H, dd, J = 4.9, 3.7 Hz), 7.49 (1H, dd, J = 3.7, 1.1 Hz), 7.67 (1H, dd, J = 4.9, 1.1 Hz), 8.90 (1H, s).

Production Example 29: Synthesis of ethyl 3,5-dimethyl-4-(thiophene-3-carbonyl)-1H-pyrrole-2-carboxylate [compound No. 29]

Production Example 29-1: Synthesis of ethyl 3,5-dimethyl-4-(thiophene-3-carbonyl)-1H-pyrrole-2-carboxylate [compound No. 29]

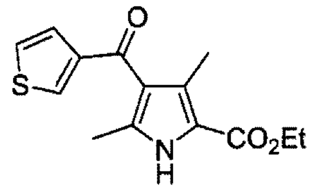

The compound of interest was obtained by the same procedures as in Production Example 1-1 using thiophene-3-carbonyl chloride. Yield: 77.9 %, white solid.
$^1$H-NMR (400 MHz, $CDCl_3$) δ: 1.38 (3H, t, J = 7.2 Hz), 2.29 (6H, s), 4.34 (2H, q, J = 7.2 Hz), 7.32-7.35 (1H, m), 7.45-7.47 (1H, m), 7.81-7.82 (1H, m), 8.85 (1H,s).

Production Example 30: Synthesis of ethyl 4-(4-bromopicolinoyl)-3,5-dimethyl-1H-pyrrole-2-carboxylate [compound No. 30]

Production Example 30-1: Synthesis of ethyl 4-(4-bromopicolinoyl)-3,5-dimethyl-1H-pyrrole-2-carboxylate [compound No. 30]

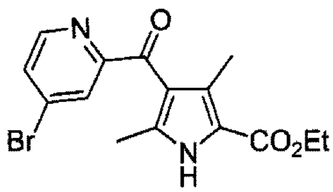

The compound of interest was obtained by the same procedures as in Production Example 1-1 using 4-bromopyridine-2-carbonyl chloride. Yield: 11.4 %, yellow solid.
$^1$H-NMR (400 MHz, DMSO-$d_6$) δ: 1.29 (3H, t, J = 7.2 Hz), 2.04 (3H, s), 2.17 (3H,s), 4.25 (2H, q, J = 7.2 Hz), 7.88 (1H, dd, J = 5.4, 2.0 Hz), 7.93 (1H, d, J = 2.0 Hz), 8.51 (1H, d, J = 5.4 Hz), 11.98 (1H, br s).

Production Example 31: Synthesis of ethyl 4-(5-bromonicotinoyl)-3,5-dimethyl-1H-pyrrole-2-carboxylate [compound No. 31]

Production Example 31-1: Synthesis of ethyl 4-(5-bromonicotinoyl)-3,5-dimethyl-1H-pyrrole-2-carboxylate [compound No. 31]

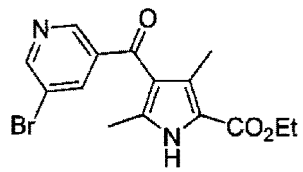

The compound of interest was obtained by the same procedures as in Production Example 1-1 using 5-bromopyridine-3-carbonyl chloride. Yield: 4.6 %, yellow solid.
$^1$H-NMR (400 MHz, DMSO-$d_6$) δ: 1.30 (3H, t, J = 7.1 Hz), 2.10 (3H, s), 2.19 (3H,s), 4.26 (2H, q, J = 7.1 Hz), 8.17 (1H, t, J = 2.2 Hz), 8.69 (1H, d, J = 1.7 Hz), 8.91 (1H, d, J = 2.2 Hz), 12.07 (1H, s).

Production Example 32: Synthesis of ethyl 4-(2-bromoisonicotinoyl)-3,5-dimethyl-1H-pyrrole-2-carboxylate [compound No. 32]

Production Example 32-1: Synthesis of ethyl 4-(2-bromoisonicotinoyl)-3,5-dimethyl-1H-pyrrole-2-carboxylate [compound No. 32]

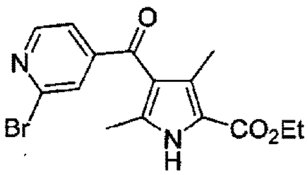

The compound of interest was obtained by the same procedures as in Production Example 1-1 using 2-bromopyridine-4-carbonyl chloride. Yield: 9.2 %, yellow solid.
$^1$H-NMR (400 MHz, DMSO-$d_6$) δ: 1.30 (3H, t, J = 7.1 Hz), 2.10 (3H, s), 2.19 (3H,s), 4.26 (2H, q, J = 7.1 Hz), 7.50-7.54 (1H, m), 7.59-7.71 (1H, m), 8.53-8.57 (1H, m), 12.12 (1H, s).

Production Example 33: Synthesis of ethyl 4-(6-bromopicolinoyl)-3,5-dimethyl-1H-pyrrole-2-carboxylate [compound No. 33]

Production Example 33-1: Synthesis of ethyl 4-(6-bromopicolinoyl)-3,5-dimethyl-1H-pyrrole-2-carboxylate [compound No. 33]

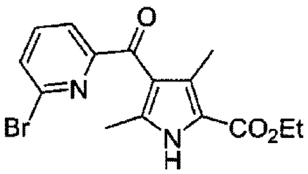

The compound of interest was obtained by the same procedures as in Production Example 1-1 using 6-bromopyridine-2-carbonyl chloride. Yield: 16.2 %, yellow solid.
$^1$H-NMR (400 MHz, DMSO-$d_6$) δ: 1.30 (3H, t, J = 7.1 Hz), 2.08 (3H, s), 2.18 (3H,s), 4.26 (2H, q, J = 7.1 Hz), 7.78 (1H, dd, J = 7.4, 1.0 Hz), 7.86 (1H, dd, J= 8.1, 1.0 Hz), 7.94-7.98 (1H, m), 12.01 (1H, s).

Production Example 34: Synthesis of ethyl 4-(3-bromo-2-methoxybenzoyl)-3,5-dimethyl-1H-pyrrole-2-carboxylate [compound No. 34]

Production Example 34-1: Synthesis of ethyl 4-(3-bromo-2-methoxybenzoyl)-3,5-dimethyl-1H-pyrrole-2-carboxylate [compound No. 34]

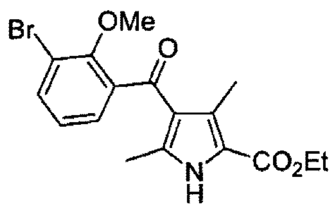

The compound of interest was obtained by the same procedures as in Production Example 1-1 using 3-bromo-2-methoxybenzoyl chloride. Yield: quant, white solid. $^1$H-NMR (400 MHz, $CDCl_3$) $\delta$: 1.36 (3H, t, J = 7.2 Hz), 2.25 (6H, s), 3.77 (3H, s), 4.33 (2H, q, J = 7.1 Hz), 6.84 (1H, d, J = 8.8 Hz), 7.36 (1H, d, J = 2.4 Hz), 7.50 (1H, dd, J = 8.8, 2.7 Hz), 8.93 (1H, s).

Production Example 35: Synthesis of ethyl 4-(5-bromo-2-methoxybenzoyl)-3,5-dimethyl-1H-pyrrole-2-carboxylate [compound No. 35]

Production Example 35-1: Synthesis of ethyl 4-(5-bromo-2-methoxybenzoyl)-3,5-dimethyl-1H-pyrrole-2-carboxylate [compound No. 35]

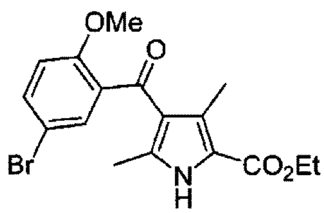

The compound of interest was obtained by the same procedures as in Production Example 1-1 using 5-bromo-2-methoxybenzoyl chloride. Yield: quant, yellow solid. $^1$H-NMR (400 MHz, $CDCl_3$) $\delta$: 1.36 (3H, t, J = 7.2 Hz), 2.18 (3H, s), 2.28 (3H, s), 3.79 (3H, s), 4.32 (2H, q, J = 7.2 Hz), 7.03-7.08 (1H, m), 7.23-7.26 (1H, m), 7.63-7.67 (1H, m), 8.91 (1H, s).

Production Example 36: Synthesis of ethyl 4-(3-cyclopropylbenzoyl)-3,5-dimethyl-1H-pyrrole-2-carboxylate [compound No. 36]

Production Example 36-1: Synthesis of ethyl 4-(3-cyclopropylbenzoyl)-3,5-dimethyl-1H-pyrrole-2-carboxylate [compound No. 36]

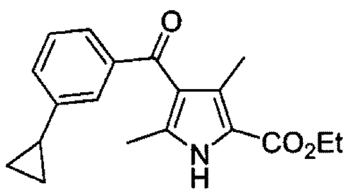

The compound of interest was obtained by the same procedures as in Production Example 1-1 using 3-cyclopropylbenzoyl chloride. Yield: 53.0 %, white solid. $^1$H-NMR (400 MHz, $CDCl_3$) δ: 0.70-0.75 (2H, m), 0.96-1.03 (2H, m), 1.38 (3H, t, J= 7.2 Hz), 1.90-1.99 (1H, m), 2.23 (3H, s), 2.24 (3H, s), 4.34 (2H, q, J = 7.1 Hz), 7.23-7.28 (1H, m), 7.29-7.34 (1H, m), 7.41-7.51 (2H, m), 9.01 (1H, s).

Production Example 37: Synthesis of ethyl 4-(4-acetoxy-3-bromobenzoyl)-3,5-dimethyl-1H-pyrrole-2-carboxylate [compound No. 37]

Production Example 37-1: Synthesis of ethyl 4-(4-acetoxy-3-bromobenzoyl)-3,5-dimethyl-1H-pyrrole-2-carboxylate [compound No. 37]

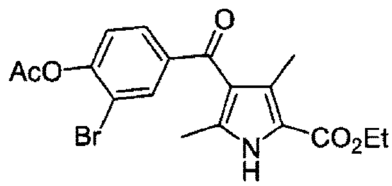

The compound of interest was obtained by the same procedures as in Production Example 1-1 using 4-acetoxy-3-bromobenzoyl chloride. 44.9 %, pale yellow solid. $^1$H-NMR (400 MHz, DMSO-$d_6$) δ: 1.30 (3H, t, J = 7.1 Hz), 2.11 (3H, s), 2.17 (3H, s), 2.36 (3H, s), 4.26 (2H, q, J = 6.9 Hz), 7.42 (1H, d, J = 8.1 Hz), 7.65 (1H, dd, J = 8.2, 2.1 Hz), 7.88 (1H, d, J = 2.0 Hz), 11.98 (1H, s).

Production Example 38: Synthesis of ethyl 4-(4-acetoxy-3-chlorobenzoyl)-3,5-dimethyl-1H-pyrrole-2-carboxylate [compound No. 38]

Production Example 38-1: Synthesis of ethyl 4-(4-acetoxy-3-chlorobenzoyl)-3,5-dimethyl-1H-pyrrole-2-carboxylate [compound No. 38]

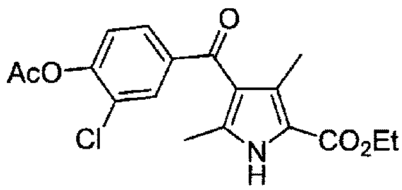

The compound of interest was obtained by the same procedures as in Production Example 1-1 using 4-acetoxy-3-chlorobenzoyl chloride. 32.1 %, pale yellow solid. $^1$H-NMR (400 MHz, DMSO-$d_6$) δ: 1.30 (3H, t, J = 7.1 Hz), 2.11 (3H, s), 2.17 (3H, s), 2.37 (3H, s), 4.26 (2H, q, J = 7.1 Hz), 7.44 (1H, d, J = 8.3 Hz), 7.61 (1H, dd, J = 8.3, 2.0 Hz), 7.75 (1H, d, J = 2.0 Hz), 11.98 (1H, s).

Production Example 39: Synthesis of ethyl 4-(3-bromobenzoyl)-3-methyl-1H-pyrrole-2-carboxylate [compound No. 39]

Production Example 39-1: Synthesis of ethyl 4-(3-bromobenzoyl)-3-methyl-1H-pyrrole-2-carboxylate [compound No. 39]

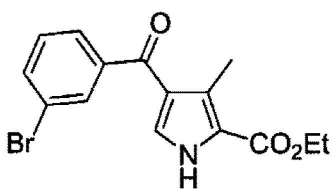

The compound of interest was obtained by the same procedures as in Production Example 1-1 using ethyl 3-methyl-1H-pyrrole-2-carboxylate and 3-bromobenzoyl chloride. Yield: 13.7 %, orange solid.
$^1$H-NMR (400 MHz, $CDCl_3$) δ: 1.40 (3H, t, J = 7.1 Hz), 2.63 (3H, s), 4.38 (2H, q, J = 7.2 Hz), 7.19-7.21 (1H, m), 7.32-7.37 (1H, m), 7.65-7.70 (2H, m), 7.87-7.90(1H, m), 9.18 (1H, s).

Production Example 40: Synthesis of methyl 4-(3-bromobenzoyl)-3-ethyl-5-methyl-1H-pyrrole-2-carboxylate [compound No. 40]

Production Example 40-1: Synthesis of methyl 4-(3-bromobenzoyl)-3-ethyl-5-methyl-1H-pyrrole-2-carboxylate [compound No. 40]

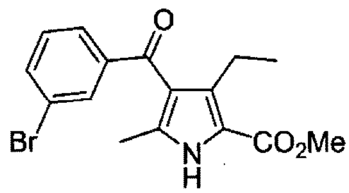

The compound of interest was obtained by the same procedures as in Production Example 1-1 using methyl 3-ethyl-5-methyl-1H-pyrrole-2-carboxylate and 3-bromobenzoyl chloride. Yield: 46.2 %, brown solid. $^1$H-NMR (400 MHz, $CDCl_3$) δ: 1.06 (3H, t, J = 7.5 Hz), 2.17 (3H, s), 2.78 (2H, q, J = 7.5 Hz), 3.89 (3H, s), 7.31-7.36 (1H, m), 7.64-7.69 (2H, m), 7.84-7.86 (1H,m), 8.91 (1H, s).

Production Example 41: Synthesis of ethyl 4-(3-bromobenzoyl)-5-methyl-1H-pyrrole-2-carboxylate [compound No. 41]

Production Example 41-1: Synthesis of ethyl 4-(3-bromobenzoyl)-5-methyl-1H-pyrrole-2-carboxylate [compound No. 41]

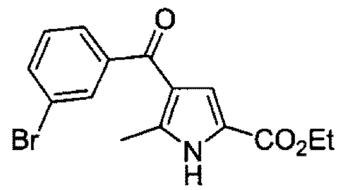

The compound of interest was obtained by the same procedures as in Production Example 1-1 using ethyl 5-methyl-1H-pyrrole-2-carboxylate and 3-bromobenzoyl chloride. Yield: 58.9 %, white solid.
$^1$H-NMR (400 MHz, $CDCl_3$) δ: 1.36 (3H, t, J = 7.1 Hz), 2.63 (3H, s), 4.33 (2H, q, J = 7.2 Hz), 7.02-7.06 (1H, m), 7.33-7.38 (1H, m), 7.65-7.73 (2H, m), 7.89-7.93(1H, m), 9.11-9.22 (1H, m).

Production Example 42: Synthesis of ethyl 4-benzoyl-5-methyl-1H-pyrrole-2-carboxylate [compound No. 42]

Production Example 42-1: Synthesis of ethyl 4-benzoyl-5-methyl-1H-pyrrole-2-carboxylate [compound No. 42]

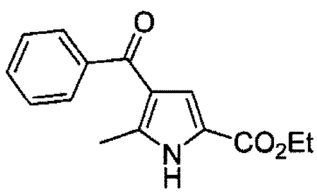

The compound of interest was obtained by the same procedures as in Production Example 1-1 using ethyl 5-methyl-1H-pyrrole-2-carboxylate. Yield: quant., white solid.
$^1$H-NMR (400 MHz, $CDCl_3$) δ: 1.35 (3H, t, J = 7.2 Hz), 2.64 (3H, s), 4.33 (2H, q, J = 7.2 Hz), 7.07-7.10 (1H, m), 7.44-7.51 (2H, m), 7.52-7.58 (1H, m), 7.77-7.82(2H, m), 9.34 (1H, s).

Production Example 43: Synthesis of methyl 4-(3-bromobenzoyl)-3-(2-methoxyethyl)-5-methyl-1H-pyrrole-2-carboxylate [compound No. 43]

Production Example 43-1: Synthesis of methyl 4-(3-bromobenzoyl)-3-(2-methoxyethyl)-5-methyl-1H-pyrrole-2-carboxylate [compound No. 43]

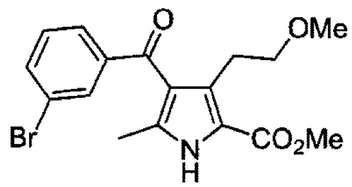

The compound of interest was obtained by the same procedures as in Production Example 1-1 using methyl 3-(2-methoxyethyl)-5-methyl-1H-pyrrole-2-carboxylate and 3-bromobenzoyl chloride. Yield: 22.2 %, yellow solid. $^1$H-NMR (400 MHz, $CDCl_3$) δ: 2.14 (3H, s), 3.12 (2H, t, J = 7.1 Hz), 3.22 (3H, s), 3.45 (2H, t, J = 7.1 Hz), 3.89 (3H, s), 7.31-7.36 (1H, m), 7.64-7.69 (2H, m), 7.84-7.86 (1H, m), 8.98 (1H, s).

Production Example 44: Synthesis of ethyl 4-(3-(hydroxymethyl)benzoyl)-3,5-dimethyl-1H-pyrrole-2-carboxylate [compound No. 44]

Production Example 44-1: Synthesis of ethyl 4-(3-(hydroxymethyl)benzoyl)-3,5-dimethyl-1H-pyrrole-2-carboxylate [compound No. 44]

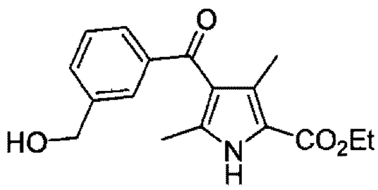

The compound of interest was obtained by the same procedures as in Production Example 1-1 using 3-hydroxymethylbenzoyl chloride. Yield: 76.2 %, white solid.
$^1$H-NMR (400 MHz, DMSO-$d_6$) δ: 1.30 (3H, t, J = 7.1 Hz), 2.07 (3H, s), 2.16 (3H, s), 4.26 (2H, q, J = 7.1 Hz), 4.55 (2H, d, J = 5.6 Hz), 5.31 (1H, t, J = 5.6 Hz), 7.41-7.54 (3H, m), 7.56-7.59 (1H, m), 11.91 (1H, s).

Production Example 45: Synthesis of methyl 4-benzoyl-3-(methoxymethyl)-5-methyl-1H-pyrrole-2-carboxylate [compound No. 45]

Production Example 45-1: Synthesis of methyl 4-benzoyl-3-(methoxymethyl)-5-methyl-1H-pyrrole-2-carboxylate [compound No. 45]

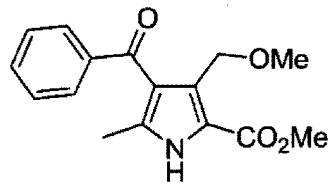

To a solution of methyl 4-methoxyacetoacetate (725 mg, 5 mmol) in acetic acid (4 ml), an aqueous solution (4 ml) of sodium nitrite (345 mg, 5 mmol) was added at 5°C. After 12 hours, a solution of benzoylacetone (811 mg, 5 mmol) in acetic acid (2 ml) and subsequently zinc powder (649 mg, 9.9 mmol) and sodium acetate (562 mg, 6.9 mmol) were added to the mixture. Then, the reaction mixture was heated to reflux. After 1 hour, the reaction solution was quenched by the addition of ice water. The reaction solution was rendered weakly basic by the addition of a saturated aqueous solution of sodium bicarbonate. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (295 mg, yield: 20.5%, yellow oil).

$^1$H-NMR (400 MHz, $CDCl_3$) δ: 2.28 (3H, s), 3.06 (3H, s), 3.89 (3H, s), 4.49 (2H, s), 7.44-7.47 (2H, m), 7.52-7.56 (1H, m), 7.74-7.79 (2H, m), 9.10 (1H, s).

Production Example 46: Synthesis of ethyl 4-(3-aminobenzoyl)-3,5-dimethyl-1H-pyrrole-2-carboxylate [compound No. 46]

Production Example 46-1: Synthesis of ethyl 4-(3-aminobenzoyl)-3,5-dimethyl-1H-pyrrole-2-carboxylate [compound No. 46]

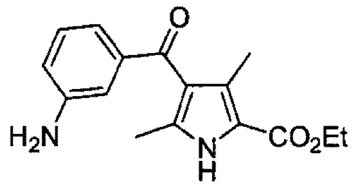

To a suspension of compound 19 (330 mg, 1.04 mmol) in methanol (5 ml), 5% palladium carbon (40 mg) was added, and the mixture was stirred in the hydrogen atmosphere. After 1 hour, palladium carbon was filtered off through celite. The filtrate was purified by column chromatography to obtain the compound of interest (279 mg, yield: 93.7%, pale yellow solid).

$^1$H-NMR (400 MHz, DMSO-$d_6$) δ: 1.29 (3H, t, J = 7.1 Hz), 2.09 (3H, s), 2.16 (3H, s), 4.25 (2H, q, J = 7.1 Hz), 5.28 (2H, s), 6.71-6.76 (2H, m), 6.81-6.83 (1H, m), 7.08-7.13 (1H, m), 11.81 (1H, s).

Production Example 47: Synthesis of ethyl 4-(3-(2-((tert-butoxycarbonyl)amino)acetamide)benzoyl)-3,5-dimethyl-1H-pyrrole-2-carboxylate [compound No. 47]

Production Example 47-1: Synthesis of ethyl 4-(3-(2-((tert-butoxycarbonyl)amino)acetamide)benzoyl)-3,5-dimethyl-1H-pyrrole-2-carboxylate [compound No. 47]

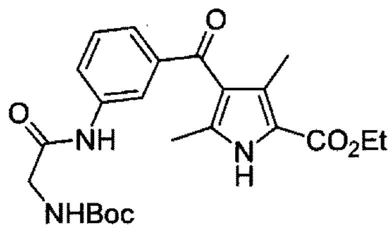

To a solution of Boc-glycine (31 mg, 0.18 mmol) in N,N-dimethylformamide (3 ml), 1-ethyl-3-(3-dimethylaminopropyl)carbodiimide hydrochloride (41 mg, 0.21 mmol) and 1-hydroxybenzotriazole (29 mg, 0.21 mmol) were added in the argon atmosphere. After 1 hour, compound 46 (50 mg, 0.18 mmol) was added to the mixture. After 12 hours, the reaction solution was quenched by the addition of water. The aqueous layer was extracted with chloroform. The obtained organic layer was dried over anhydrous magnesium sulfate, and magnesium sulfate was filtered off. The solvent was distilled off under reduced pressure. The residue was purified by column chromatography to obtain the compound of interest (88 mg, yield: quant., white solid).
$^1$H-NMR (400 MHz, $CDCl_3$) δ: 1.37 (3H, t, J = 7.2 Hz), 1.48 (9H, s), 2.25 (6H, s), 3.93 (2H, d, J = 5.9 Hz), 4.33 (2H, q, J = 7.2 Hz), 5.21 (1H, s), 7.37-7.50 (2H, m), 7.72-7.77 (1H, m), 7.79-7.84 (1H, m), 8.25 (1H, s), 8.93 (1H, s).

Production Example 48: Synthesis of ethyl 4-(3-(2-aminoacetamide)benzoyl)-3,5-dimethyl-1H-pyrrole-2-carboxylate [compound No. 48]

Production Example 48-1: Synthesis of ethyl 4-(3-(2-aminoacetamide)benzoyl)-3,5-dimethyl-1H-pyrrole-2-carboxylate [compound No. 48]

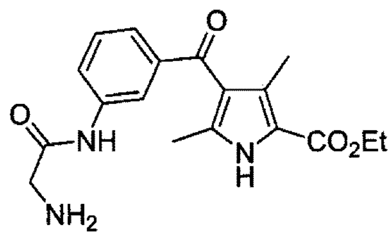

Trifluoroacetic acid (1 ml) was added to a solution of compound 47 (80 mg, 0.18 mmol) in dichloromethane (1 ml). After 2 hours, saturated saline was added to the reaction solution, and the aqueous layer was washed with chloroform. The obtained organic layer was extracted with 1 M hydrochloric acid. The obtained aqueous layer was combined with the preceding washed aqueous layer. After addition of chloroform, the reaction solution was rendered weakly basic by the addition of a saturated aqueous solution of sodium bicarbonate under ice cooling. This aqueous layer was extracted with chloroform. The obtained organic layer was dried over anhydrous magnesium sulfate. Magnesium sulfate was filtered off, and the solvent was distilled off under reduced pressure to obtain the compound of interest (60 mg, yield: quant., white solid).
$^1$H-NMR (400 MHz, DMSO-$d_6$) δ: 1.30 (3H, t, J = 7.2 Hz), 2.10 (3H, s), 2.17 (3H, s), 3.27 (2H, s), 4.26 (2H, q, J = 7.2 Hz), 7.26-7.32 (1H, m), 7.38-7.45 (1H, m), 7.78-7.85 (1H, m), 7.90-7.95 (1H, m), 11.91 (1H, s).

Production Example 49: Synthesis of ethyl 4-(3-bromo-4-hydroxybenzoyl)-3,5-dimethyl-1H-pyrrole-2-carboxylate [compound No. 49]

Production Example 49-1: Synthesis of ethyl 4-(3-bromo-4-hydroxybenzoyl)-3,5-dimethyl-1H-pyrrole-2-carboxylate [compound No. 49]

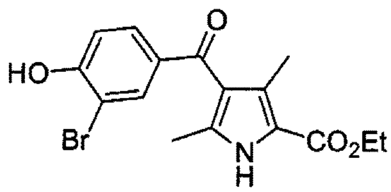

To a solution of compound 37 (550 mg, 1.5 mmol) in tetrahydrofuran (5 ml), a 1 M aqueous sodium hydroxide solution (1.5 ml, 1.5 mmol) was added at 0°C. After 1 hour, water was added to the reaction solution, and the aqueous layer was extracted with chloroform. The obtained organic layer was dried over anhydrous magnesium sulfate, and magnesium sulfate was filtered off. The solvent was distilled off under reduced pressure. The residue was purified by

column chromatography to obtain the compound of interest (478 mg, yield: 97.0%, orange solid).
$^1$H-NMR (400 MHz, DMSO-$d_6$) δ: 1.30 (3H, t, J = 7.2 Hz), 2.10 (3H, s), 2.15 (3H, s), 4.25 (2H, q, J = 7.0 Hz), 7.03 (1H, d, J = 8.5 Hz), 7.51 (1H, dd, J = 8.4, 2.1 Hz), 7.74 (1H, d, J = 2.0 Hz), 11.16 (1H, s), 11.87 (1H, s).

Production Example 50: Synthesis of ethyl 4-(3-bromo-4-(2-((tert-butoxycarbonyl)amino)ethoxy)benzoyl)-3,5-dimethyl-1H-pyrrole-2-carboxylate [compound No. 50]

Production Example 50-1: Synthesis of ethyl 4-(3-bromo-4-(2-((tert-butoxycarbonyl)amino)ethoxy)benzoyl)-3,5-dimethyl-1H-pyrrole-2-carboxylate [compound No. 50]

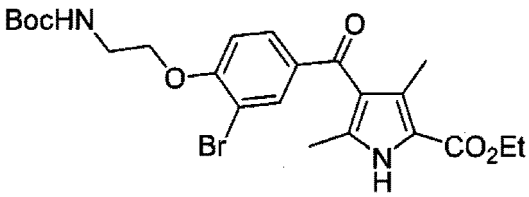

To a suspension of compound 49 (100 mg, 0.27 mmol) in acetone (10 ml), potassium carbonate (193 mg, 1.4 mmol) and 2-Boc-aminoethyl bromide (314 mg, 1.4 mmol) were added in the argon atmosphere, and the mixture was heated to reflux. After 12 hours, the reaction solution was cooled in ice and quenched by the addition of water. The aqueous layer was extracted with chloroform. The obtained organic layer was dried over anhydrous magnesium sulfate. Magnesium sulfate was filtered off, and the solvent was distilled off under reduced pressure. The residue was purified by column chromatography to obtain the compound of interest (89 mg, yield: 64.9%, white solid).
$^1$H-NMR (400 MHz, $CDCl_3$) δ: 1.38 (3H, t, J = 7.1 Hz), 1.46 (9H, s), 2.23 (3H, s), 2.27 (3H, s), 3.59-3.66 (2H, m), 4.12-4.18 (2H, m), 4.34 (2H, q, J = 7.1 Hz), 5.05 (1H, s), 6.91 (1H, d, J = 8.8 Hz), 7.69 (1H, dd, J = 8.8, 2.2 Hz), 7.97 (1H, d, J = 2.2 Hz), 8.88 (1H, s).

Production Example 51: Synthesis of ethyl 4-(4-(2-aminoethoxy)-3-bromobenzoyl)-3,5-dimethyl-1H-pyrrole-2-carboxylate [compound No. 51]

Production Example 51-1: Synthesis of ethyl 4-(4-(2-aminoethoxy)-3-bromobenzoyl)-3,5-dimethyl-1H-pyrrole-2-carboxylate [compound No. 51]

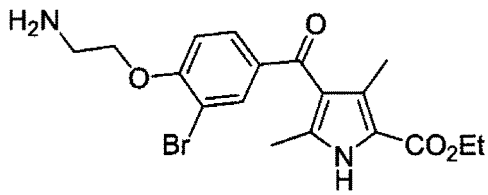

The compound of interest was obtained by the same procedures as in Production Example 48-1 using compound 50 as a starting material. Yield: 51.8 %, white solid.
$^1$H-NMR (400 MHz, DMSO-$d_6$) δ: 1.30 (3H, t, J = 7.2 Hz), 2.11 (3H, s), 2.16 (3H, s), 2.96 (2H, t, J = 5.6 Hz), 4.11 (2H, t, J = 5.6 Hz), 4.26 (2H, q, J = 7.1 Hz), 7.21 (1H, d, J = 8.5 Hz), 7.62 (1H, dd, J = 8.5, 2.2 Hz), 7.80 (1H, d, J = 2.2Hz), 11. 91 (1H, s).

Production Example 52: Synthesis of ethyl 4-(3-chloro-4-hydroxybenzoyl)-3,5-dimethyl-1H-pyrrole-2-carboxylate [compound No. 52]

Production Example 52-1: Synthesis of ethyl 4-(3-chloro-4-hydroxybenzoyl)-3,5-dimethyl-1H-pyrrole-2-carboxylate [compound No. 52]

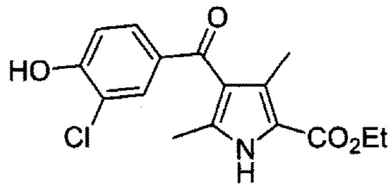

The compound of interest was obtained by the same procedures as in Production Example 49-1 using compound 38 as a starting material. Yield: 91.6 %, orange solid. $^1$H-NMR (400 MHz, DMSO-$d_6$) $\delta$: 1.30 (3H, t, J = 7.1 Hz), 2.10 (3H, s), 2.15 (3H, s), 4.25 (2H, q, J = 7.1 Hz), 7.04 (1H, d, J = 8.3 Hz), 7.46 (1H, dd, J = 8.4, 2.1 Hz), 7.59 (1H, d, J = 2.0 Hz), 11.09 (1H, s), 11.87 (1H, s).

Production Example 53: Synthesis of ethyl 4-(4-(2-((tert-butoxycarbonyl)amino)ethoxy)-3-chlorobenzoyl)-3,5-dimethyl-1H-pyrrole-2-carboxylate [compound No. 53]

Production Example 53-1: Synthesis of ethyl 4-(4-(2-((tert-butoxycarbonyl)amino)ethoxy)-3-chlorobenzoyl)-3,5-dimethyl-1H-pyrrole-2-carboxylate [compound No. 53]

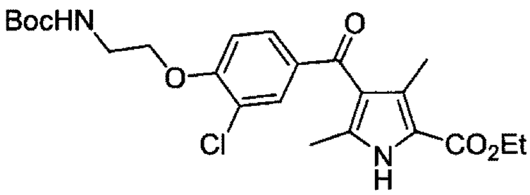

The compound of interest was obtained by the same procedures as in Production Example 50-1 using compound 52 as a starting material. Yield: 88.1 %, white solid. $^1$H-NMR (400 MHz, $CDCl_3$) $\delta$: 1.38 (3H, t, J = 7.1 Hz), 1.46 (9H, s), 2.23 (3H, s), 2.27 (3H, s), 3.58-3.66 (2H, m), 4.13-4.19 (2H, m), 4.34 (3H, q, J = 7.2 Hz), 5.04 (1H, s), 6.95 (1H, d, J = 8.5 Hz), 7.64 (1H, dd, J = 8.4, 2.1 Hz), 7.80 (1H, d, J = 2.0 Hz), 8.85 (1H, s).

Production Example 54: Synthesis of ethyl 4-(4-(2-aminoethoxy)-3-chlorobenzoyl)-3,5-dimethyl-1H-pyrrole-2-carboxylate [compound No. 54]

Production Example 54-1: Synthesis of ethyl 4-(4-(2-aminoethoxy)-3-chlorobenzoyl)-3,5-dimethyl-1H-pyrrole-2-carboxylate [compound No. 54]

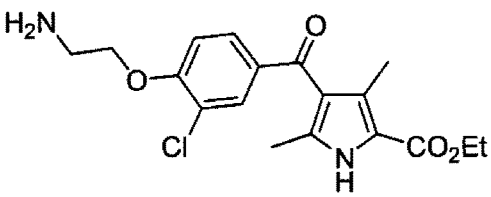

The compound of interest was obtained by the same procedures as in Production Example 48-1 using compound 53 as a starting material. Yield: 70.6 %, white solid. $^1$H-NMR (400 MHz, DMSO-$d_6$) δ: 1.30 (3H, t, J = 7.1 Hz), 2.11 (3H, s), 2.16 (3H, s), 2.95 (2H, t, J = 5.7 Hz), 4.11 (2H, t, J = 5.6 Hz), 4.26 (2H, q, J = 7.2 Hz), 7.24 (1H, d, J = 8.5 Hz), 7.58 (1H, dd, J = 8.5, 2.2 Hz), 7.65 (1H, d, J = 2.0Hz), 11.92 (1H, s).

Production Example 55: Synthesis of ethyl 4-(3-bromobenzoyl)-5-formyl-3-methyl-1H-pyrrole-2-carboxylate [compound No. 55]

Production Example 55-1: Synthesis of ethyl 4-(3-bromobenzoyl)-5-formyl-3-methyl-1H-pyrrole-2-carboxylate [compound No. 55]

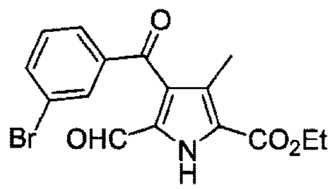

A mixture of compound 11 (500 mg, 1.43 mmol), cerium ammonium nitrate (3133 mg, 5.72 mmol), tetrahydrofuran (3 ml), acetic acid (3 ml), and water (3 ml) was heated to reflux. After 12 hours, the reaction solution was allowed to cool, and water was added thereto. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (393 mg, yield: 75.5%, yellow solid).
$^1$H-NMR (400 MHz, $CDCl_3$) δ: 1.42 (3H, t, J = 7.2 Hz), 2.12 (3H, s), 2.26 (3H, s), 4.42 (2H, q, J = 7.2 Hz), 7.36-7.42 (1H, m), 7.71-7.80 (2H, m), 7.96-7.99 (1H,m), 9.55 (1H, s), 10.13 (1H, s).

Production Example 56: Synthesis of ethyl 4-(3-bromo-4-methoxybenzoyl)-5-formyl-3-methyl-1H-pyrrole-2-carboxylate [compound No. 56]

Production Example 56-1: Synthesis of ethyl 4-(3-bromo-4-methoxybenzoyl)-5-formyl-3-methyl-1H-pyrrole-2-carboxylate [compound No. 56]

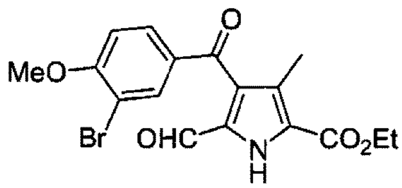

The compound of interest was obtained by the same procedures as in Production Example 55-1 using compound 22 as a starting material. Yield: 44.2%, yellow solid. $^1$H-NMR (400 MHz, DMSO-$d_6$) δ: 1.34 (3H, t, J = 7.1 Hz), 2.15 (3H, s), 3.94 (3H, s), 4.33 (2H, q, J = 7.1 Hz), 7.20 (1H, d, J = 8.8 Hz), 7.73 (1H, dd, J = 8.8, 2.2 Hz), 7.92 (1H, d, J = 2.2 Hz), 9.59 (1H, s), 13.25 (1H, br s).

Production Example 57: Synthesis of ethyl 4-(3-bromobenzoyl)-5-(difluoromethyl)-3-methyl-1H-pyrrole-2-carboxylate [compound No. 57]

Production Example 57-1: Synthesis of ethyl 4-(3-bromobenzoyl)-5-(difluoromethyl)-3-methyl-1H-pyrrole-2-carboxylate [compound No. 57]

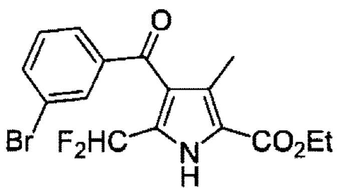

To a solution of compound 55 (100 mg, 0.27 mmol) in dichloromethane (3 ml), bis(2-methoxyethyl)aminosulfur trifluoride (142 μl, 0.81 mmol) was added in the argon atmosphere. After 8 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (71 mg, yield: 68.1%, white solid).
$^1$H-NMR (400 MHz, $CDCl_3$) δ: 1.41 (3H, t, J = 7.1 Hz), 2.18 (3H, s), 4.40 (2H, q, J = 7.2 Hz), 6.79 (1H, t, J = 54.2 Hz), 7.34-7.39 (1H, m), 7.65-7.75 (2H, m), 7.87-7.90 (1H, m), 9.61 (1H, s).

Production Example 58: Synthesis of ethyl 4-(3-bromobenzoyl)-5-(hydroxymethyl)-3-methyl-1H-pyrrole-2-carboxylate [compound No. 58]

Production Example 58-1: Synthesis of ethyl 4-(3-bromobenzoyl)-5-(hydroxymethyl)-3-methyl-1H-pyrrole-2-carboxylate [compound No. 58]

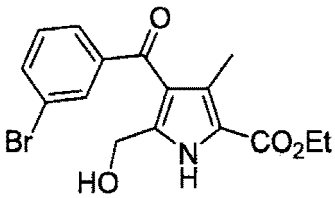

To a solution of compound 55 (50 mg, 0.14 mmol) in methanol (5 ml) and acetic acid (50 μl), sodium cyanoborohydride (9 mg, 0.14 mmol) was added at 0°C in the argon atmosphere. Then, the reaction temperature was increased to room temperature. After 12 hours, the reaction solution was quenched by the addition of water. The aqueous layer was extracted with chloroform. The obtained organic layer was dried over anhydrous magnesium sulfate, and magnesium sulfate was filtered off. The solvent was distilled off under reduced pressure. The residue was purified by column chromatography to obtain the compound of interest (27 mg, yield: 52.7%, white solid).
$^1$H-NMR (400 MHz, $CDCl_3$) δ: 1.38 (4H, t, J = 7.2 Hz), 2.12 (3H, s), 3.19 (1H, t, J = 6.2 Hz), 4.36 (2H, q, J = 7.2 Hz), 4.68 (2H, d, J = 6.2 Hz), 7.32-7.38 (1H,m), 7.61-7.65 (1H, m), 7.67-7.72 (1H, m), 7.81-7.84 (1H, m), 9.44 (1H, s).

Production Example 59: Synthesis of ethyl 4-(3-bromo-4-methoxybenzoyl)-5-(hydroxymethyl)-3-methyl-1H-pyrrole-2-carboxylate [compound No. 59]

Production Example 59-1: Synthesis of ethyl 4-(3-bromo-4-methoxybenzoyl)-5-(hydroxymethyl)-3-methyl-1H-pyrrole-2-carboxylate [compound No. 59]

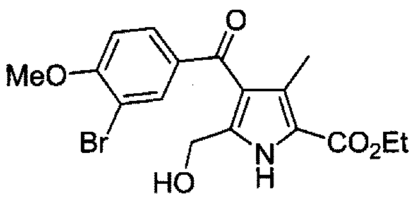

The compound of interest was obtained by the same procedures as in Production Example 58-1 using compound 56 as a starting material. Yield: 65.9 %, white solid. $^1$H-NMR (400 MHz, DMSO-$d_6$) δ: 1.31 (3H, t, J = 7.1 Hz), 2.16 (3H, s), 3.94 (3H, s), 4.27 (2H, q, J = 7.1 Hz), 4.31 (2H, d, J = 5.6 Hz), 4.95 (1H, t, J = 5.6 Hz), 7.21 (1H, d, J = 8.5 Hz), 7.68 (1H, dd, J = 8.5, 2.0 Hz), 7.84 (1H, d, J = 2.0Hz), 11.86 (1H, s).

Production Example 60: Synthesis of ethyl 4-(3-bromobenzoyl)-5-(fluoromethyl)-3-methyl-1H-pyrrole-2-carboxylate [compound No. 60]

Production Example 60-1: Synthesis of ethyl 4-(3-bromobenzoyl)-5-(fluoromethyl)-3-methyl-1H-pyrrole-2-carboxylate [compound No. 60]

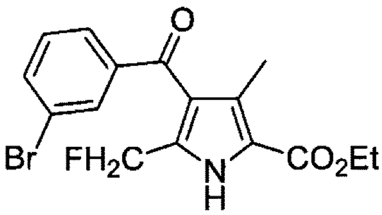

To a suspension of compound 58 (83 mg, 0.23 mmol) in dichloromethane (3 ml), diethylaminosulfur trifluoride (30 μl, 0.23 mmol) was added at 0°C in the argon atmosphere. After 3 minutes, the reaction solution was purified by column chromatography to obtain the compound of interest (15 mg, yield: 17.7%, pale yellow solid).
$^1$H-NMR (400 MHz, $CDCl_3$) δ: 1.39 (3H, t, J = 7.2 Hz), 2.17 (3H, s), 4.37 (2H, q, J = 7.2 Hz), 5.50 (2H, d, J = 47.3 Hz), 7.32-7.37 (1H, m), 7.61-7.65 (1H, m), 7.67-7.72 (1H, m), 7.82-7.85 (1H, m), 9.42 (1H, s).

Production Example 61: Synthesis of ethyl 4-(3-bromobenzoyl)-5-(1-hydroxyethyl)-3-methyl-1H-pyrrole-2-carboxylate [compound No. 61]

Production Example 61-1: Synthesis of ethyl 4-(3-bromobenzoyl)-5-(1-hydroxyethyl)-3-methyl-1H-pyrrole-2-carboxylate [compound No. 61]

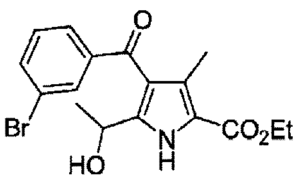

To a solution of compound 55 (200 mg, 0.55 mmol) in tetrahydrofuran (5 ml), methyl magnesium bromide (1.4 M solution in toluene and tetrahydrofuran, 862 μl, 1.2 mmol) was added at 0°C in the argon atmosphere. After 2 hours, the reaction solution was quenched by the addition of 1 M hydrochloric acid. The aqueous layer was extracted with chloroform. The obtained organic layer was dried over anhydrous magnesium sulfate, and magnesium sulfate was filtered off. The solvent was distilled off under reduced pressure. The residue was purified by column chromatography to obtain the compound of interest (155 mg, yield: 74.0%, yellow solid).
$^1$H-NMR (400 MHz, DMSO-$d_6$) δ: 1.28-1.37 (6H, m), 2.06 (3H, s), 4.21-4.31 (2H, m), 4.64-4.72 (1H, m), 5.20 (1H, d, J = 5.4 Hz), 7.45-7.52 (1H, m), 7.61-7.66 (1H,m), 7.74-7.77 (1H, m), 7.80-7.85 (1H, m), 11.64 (1H, s).

Production Example 62: Synthesis of ethyl 4-(3-bromobenzoyl)-5-ethyl-3-methyl-1H-pyrrole-2-carboxylate [compound No. 62]

Production Example 62-1: Synthesis of ethyl 4-(3-bromobenzoyl)-5-ethyl-3-methyl-1H-pyrrole-2-carboxylate [compound No. 62]

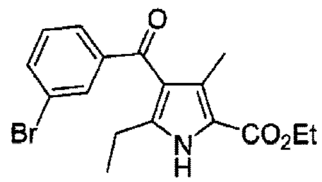

To a solution of compound 61 (30 mg, 0.08 mmol) in dichloromethane (3 ml), a boron trifluoride-diethyl ether complex (15 μl, 0.12 mmol) and triethylsilane (52 μl, 0.32 mmol) were added in the argon atmosphere. After 4 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (19 mg, yield: 66.1%, colorless oil).
$^1$H-NMR (400 MHz, $CDCl_3$) δ: 1.21 (3H, t, J = 7.7 Hz), 1.38 (3H, t, J = 7.2 Hz), 2.19 (3H, s), 2.66 (2H, q, J = 7.7 Hz), 4.35 (2H, q, J = 7.2 Hz), 7.31-7.36 (1H,m), 7.64-7.69 (2H, m), 7.84-7.86 (1H, m), 9.03 (1H, s).

Production Example 63: Synthesis of ethyl 4-(3-bromobenzoyl)-5-(bromomethyl)-3-methyl-1H-pyrrole-2-carboxylate [compound No. 63]

Production Example 63-1: Synthesis of ethyl 4-(3-bromobenzoyl)-5-(bromomethyl)-3-methyl-1H-pyrrole-2-carboxylate [compound No. 63]

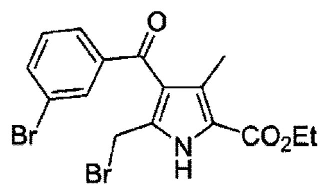

To a suspension of compound 58 (70 mg, 0.19 mmol) in chloroform (3 ml), triethylamine (27 μl, 0.19 mmol) and phosphorus tribromide (18 μl, 0.19 mmol) were added at 0°C in the argon atmosphere. After 3 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (44 mg, yield: 53.4%, white solid).
$^1$H-NMR (400 MHz, $CDCl_3$) δ: 1.39 (3H, t, J = 7.2 Hz), 2.16 (3H, s), 4.37 (2H, q, J = 7.2 Hz), 4.51 (2H, s), 7.33-7.39 (1H, m), 7.66-7.74 (2H, m), 7.87-7.90 (1H,m), 9.23-9.30 (1H, m).

Production Example 64: Synthesis of ethyl 5-(aminomethyl)-4-(3-bromobenzoyl)-3-methyl-1H-pyrrole-2-carboxylate [compound No. 64]

Production Example 64-1: Synthesis of ethyl 5-(aminomethyl)-4-(3-bromobenzoyl)-3-methyl-1H-pyrrole-2-carboxylate [compound No. 64]

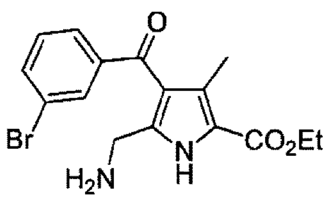

To a solution of compound 63 (100 mg, 0.23 mmol) in N,N-dimethylformamide (3 ml), phthalimide potassium salt (52 mg, 0.28 mmol) was added in the argon atmosphere. After 12 hours, the reaction solution was quenched by the

addition of water. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain a phthalimide intermediate (73 mg, yellow solid). Methanol (3 ml) and hydrazine monohydrate (111 ml, 2.30 mmol) were added thereto. After 24 hours, a saturated aqueous solution of sodium bicarbonate was added to the reaction solution. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (32 mg, yield: 37.6%, pale yellow solid).
$^1$H-NMR (400 MHz, DMSO-$d_6$) δ: 1.30 (3H, t, J = 7.1 Hz), 2.11 (3H, s), 3.48-3.52 (2H, m), 4.27 (2H, q, J = 7.1 Hz), 7.44-7.49 (1H, m), 7.59-7.63 (1H, m), 7.74-7.76 (1H, m), 7.79-7.83 (1H, m).

Production Example 65: Synthesis of ethyl 4-(3-bromobenzoyl)-5-((dimethylamino)methyl)-3-methyl-1H-pyrrole-2-carboxylate [compound No. 65]

Production Example 65-1: Synthesis of ethyl 4-(3-bromobenzoyl)-5-((dimethylamino)methyl)-3-methyl-1H-pyrrole-2-carboxylate [compound No. 65]

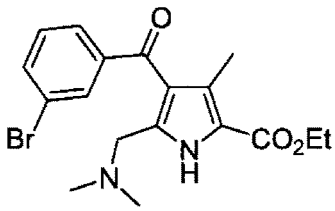

To a solution of compound 63 (94 mg, 0.22 mmol) in N,N-dimethylformamide (3 ml), potassium carbonate (36 mg, 0.26 mmol), dimethylamine hydrochloride (22 mg, 0.26 mmol), and triethylamine (36 μl, 0.26 mmol) were added in the argon atmosphere, and the mixture was heated with stirring at 80°C. After 4 hours, the reaction solution was allowed to cool and quenched by the addition of water. The aqueous layer was extracted with chloroform. The obtained organic layer was dried over anhydrous magnesium sulfate. After filtering off of magnesium sulfate, the solvent was distilled off under reduced pressure to obtain the compound of interest (71 mg, yield: 82.5%, brown solid).
$^1$H-NMR (400 MHz, DMSO-$d_6$) δ: 1.31 (3H, t, J = 7.1 Hz), 2.11 (3H, s), 4.28 (2H, q, J = 7.1 Hz), 7.46-7.52 (1H, m), 7.61-7.66 (1H, m), 7.75-7.78 (1H, m), 7.81-7.86 (1H, m).

Production Example 66: Synthesis of ethyl 4-(3-bromobenzoyl)-5-(1,3-dithian-2-yl)-3-methyl-1H-pyrrole-2-carboxylate [compound No. 66]

Production Example 66-1: Synthesis of ethyl 4-(3-bromobenzoyl)-5-(1,3-dithian-2-yl)-3-methyl-1H-pyrrole-2-carboxylate [compound No. 66]

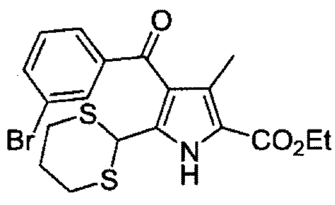

To a solution of compound 55 (1649 mg, 4.53 mmol) in acetic acid (10 ml), 1,3-propanedithiol (683 μl, 6.80 mmol) and a boron trifluoride-diethyl ether complex (174 μl, 1.37 mmol) were added at 0°C in the argon atmosphere. Then, the reaction temperature was increased to room temperature. After 2 hours, the precipitated yellow solid was collected by filtration and washed with water and hexane. After replacement of the receiver, the solid thus collected by filtration was dissolved in chloroform. Saturated saline was added to the obtained chloroform solution to separate an organic layer. The aqueous layer was extracted with chloroform. Combined organic layers were dried over anhydrous

magnesium sulfate. After filtering off of magnesium sulfate, the solvent was distilled off under reduced pressure to obtain the compound of interest (1532 mg, yield: 74.4%, pale yellow solid).
$^1$H-NMR (400 MHz, $CDCl_3$) δ: 1.39 (3H, t, J = 7.2 Hz), 1.84-1.96 (1H, m), 2.07-2.16 (1H, m), 2.19 (3H, s), 2.83-2.95 (4H, m), 4.36 (2H, q, J = 7.2 Hz), 5.32 (1H,s), 7.33-7.38 (1H, m), 7.68-7.73 (2H, m), 7.90-7.93 (1H, m), 9.42 (1H, s).

Production Example 67: Synthesis of ethyl 4-(3-bromo-4-methoxybenzoyl)-5-(1,3-dithian-2-yl)-3-methyl-1H-pyrrole-2-carboxylate [compound No. 67]

Production Example 67-1: Synthesis of ethyl 4-(3-bromo-4-methoxybenzoyl)-5-(1,3-dithian-2-yl)-3-methyl-1H-pyrrole-2-carboxylate [compound No. 67]

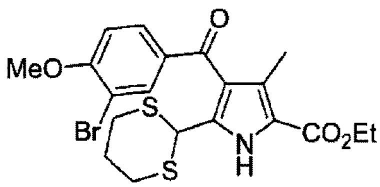

The compound of interest was obtained by the same procedures as in Production Example 66-1 using compound 56 as a starting material. Yield: 81.5 %, white solid. $^1$H-NMR (400 MHz, DMSO-$d_6$) δ: 1.31 (3H, t, J = 7.1 Hz), 1.60-1.70 (1H, m), 1.98-2.02 (1H, m), 2.05 (3H, s), 2.83 (2H, d, J = 3.2 Hz), 2.85 (2H, d, J = 3.2 Hz), 3.95 (3H, s), 4.25 (2H, q, J = 7.1 Hz), 5.36 (1H, s), 7.24 (1H, d, J = 8.4 Hz), 7.71 (1H, dd, J = 8.4, 2.0 Hz), 7.87 (1H, dd, J = 2.0, 0.7 Hz), 12.04 (1H, br s).

Production Example 68: Synthesis of (E)-ethyl 4-(3-bromobenzoyl)-5-(3-methoxy-3-oxoprop-1-en-1-yl)-3-methyl-1H-pyrrole-2-carboxylate [compound No. 68]

Production Example 68-1: Synthesis of (E)-ethyl 4-(3-bromobenzoyl)-5-(3-methoxy-3-oxoprop-1-en-1-yl)-3-methyl-1H-pyrrole-2-carboxylate [compound No. 68]

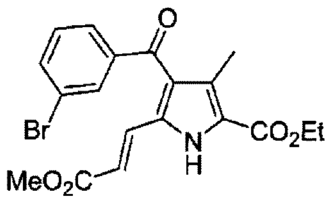

To a solution of methyl diethylphosphonoacetate (26 μl, 0.14 mmol) in tetrahydrofuran (3 ml), normal butyllithium (1.6 M solution in hexane, 88 μl, 0.14 mmol) was added at 0°C in the argon atmosphere. After 10 minutes, a solution of compound 55 (50 mg, 0.14 mmol) in tetrahydrofuran (2 ml) was added to the mixture. After 12 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (45 mg, yield: 78.2%, light brown solid).
$^1$H-NMR (400 MHz, $CDCl_3$) δ: 1.41 (3H, t, J = 7.2 Hz), 2.20 (3H, s), 3.75 (3H, s), 4.41 (2H, q, J = 7.2 Hz), 6.23-6.29 (1H, m), 7.32-7.38 (1H, m), 7.43-7.48 (1H,m), 7.67-7.74 (2H, m), 7.90-7.92 (1H, m), 9.51 (1H, s).

Production Example 69: Synthesis of ethyl 4-(3-bromobenzoyl)-3-methyl-5-vinyl-1H-pyrrole-2-carboxylate [compound No. 69]

Production Example 69-1: Synthesis of ethyl 4-(3-bromobenzoyl)-3-methyl-5-vinyl-1H-pyrrole-2-carboxylate [compound No. 69]

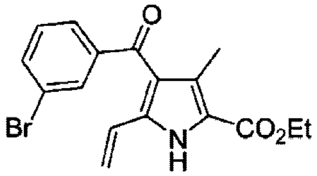

The compound of interest was obtained by the same procedures as in Production Example 68-1 using methyltriphenylphosphonium bromide. Yield: 82.8%, yellow solid.
$^1$H-NMR (400 MHz, DMSO-$d_6$) δ: 1.32 (3H, t, J = 7.1 Hz), 2.12 (3H, s), 4.30 (2H, q, J = 7.1 Hz), 5.25 (1H, d, J = 12.0 Hz), 6.00 (1H, d, J = 18.1 Hz), 6.37 (1H, dd, J = 17.8, 11.5 Hz), 7.47 (1H, t, J = 7.8 Hz), 7.62-7.64 (1H, m), 7.77 (1H, t, J = 1.8 Hz), 7.82-7.85 (1H, m), 12.18 (1H, br s).

Production Example 70: Synthesis of ethyl 4-(3-bromo-4-methoxybenzoyl)-3-methyl-5-vinyl-1H-pyrrole-2-carboxylate [compound No. 70]

Production Example 70-1: Synthesis of ethyl 4-(3-bromo-4-methoxybenzoyl)-3-methyl-5-vinyl-1H-pyrrole-2-carboxylate [compound No. 70]

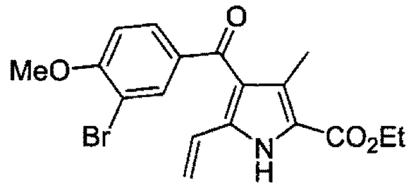

The compound of interest was obtained by the same procedures as in Production Example 68-1 using methyltriphenylphosphonium bromide and compound 56. Yield: 55.6%, white solid.
$^1$H-NMR (400 MHz, DMSO-$d_6$) δ: 1.32 (3H, t, J = 7.1 Hz), 2.13 (3H, s), 3.94 (3H, s), 4.30 (2H, q, J = 7.1 Hz), 5.23 (1H, d, J = 11.6 Hz), 5.96 (1H, dd, J = 17.8,0.7 Hz), 6.37 (1H, dd, J = 17.8, 11.6 Hz), 7.22 (1H, d, J = 8.8 Hz), 7.68 (1H, dd, J = 8.8, 2.2 Hz), 7.85 (1H, d, J = 2.2 Hz), 12.10 (1H, br s).

Production Example 71: Synthesis of ethyl 4-(3-bromobenzoyl)-5-(3-methoxy-3-oxopropyl)-3-methyl-1H-pyrrole-2-carboxylate [compound No. 71]

Production Example 71-1: Synthesis of ethyl 4-(3-bromobenzoyl)-5-(3-methoxy-3-oxopropyl)-3-methyl-1H-pyrrole-2-carboxylate [compound No. 71]

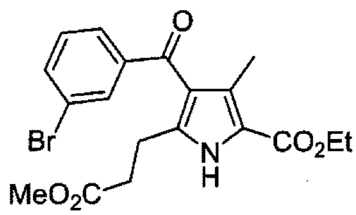

To a mixture of compound 68 (45 mg, 0.11 mmol), cobalt chloride hexahydrate (9 mg, 0.04 mmol), methanol (2 ml), and tetrahydrofuran (1 ml), sodium borohydride (9 mg, 0.21 mmol) was added in the argon atmosphere. After 3 days, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (30 mg, yield: 66.4%, colorless oil).
$^1$H-NMR (400 MHz, $CDCl_3$) δ: 1.38 (3H, t, J = 7.1 Hz), 2.12 (3H, s), 2.69 (2H, t, J = 6.3 Hz), 2.96 (2H, t, J = 6.3 Hz), 3.71

(3H, s), 4.34 (3H, q, J = 7.1 Hz), 7.31-7.36 (1H, m), 7.63-7.69 (2H, m), 7.83-7.85 (1H, m), 9.71 (1H, s).

Production Example 72: Synthesis of ethyl 4-(3-bromobenzoyl)-5-(1-hydroxy-3-oxopropyl)-3-methyl-1H-pyrrole-2-carboxylate [compound No. 72]

Production Example 72-1: Synthesis of ethyl 4-(3-bromobenzoyl)-5-(1-hydroxy-3-oxopropyl)-3-methyl-1H-pyrrole-2-carboxylate [compound No. 72]

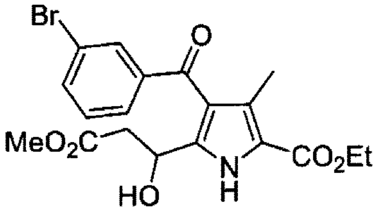

To a mixture of zinc powder (12 mg, 0.18 mmol) and tetrahydrofuran (3 ml), chlorotrimethylsilane (3 ml) was added in the argon atmosphere, and the mixture was stirred at 55°C for 15 minutes. Subsequently, methyl bromoacetate (25 mg, 0.16 mmol) was added thereto, and the mixture was stirred at 45°C for 15 minutes. Then, the reaction temperature was decreased to room temperature, and compound 55 (25 mg, 0.07 mmol) was added thereto. After 24 hours, the reaction solution was quenched by the addition of water. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (19 mg, yield: 62.9%, yellow solid).
$^1$H-NMR (400 MHz, $CDCl_3$) δ: 1.41 (3H, t, J = 7.2 Hz), 2.20 (3H, s), 3.75 (3H, s), 4.40 (2H, q, J = 7.2 Hz), 6.23 (1H, d, J = 16.3 Hz), 7.33-7.37 (1H, m), 7.45 (1H, d, J = 16.3 Hz), 7.67-7.74 (2H, m), 7.90-7.92 (1H, m), 9.36 (1H, s).

Production Example 73: Synthesis of ethyl 4-(3-bromobenzoyl)-5-(1,3-dinitropropan-2-yl)-3-methyl-1H-pyrrole-2-carboxylate [compound No. 73]

Production Example 73-1: Synthesis of ethyl 4-(3-bromobenzoyl)-5-(1,3-dinitropropan-2-yl)-3-methyl-1H-pyrrole-2-carboxylate [compound No. 73]

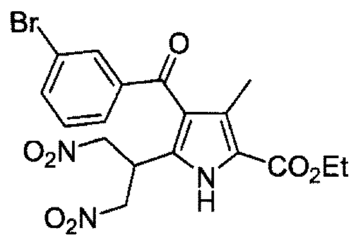

To a solution of compound 55 (100 mg, 0.28 mmol) in tetrahydrofuran (5 ml), nitromethane (778 μl, 14.5 mmol) and tetrabutylammonium fluoride (1 M solution in tetrahydrofuran, 2.8 ml, 2.8 mmol) were added at 0°C in the argon atmosphere. After 12 hours, the reaction solution was quenched by the addition of water. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (100 mg, yield: 76.3%, yellow solid).
$^1$H-NMR (400 MHz, $CDCl_3$) δ: 1.40 (3H, t, J = 7.2 Hz), 2.01 (3H, s), 4.40 (2H, q, J = 7.2 Hz), 4.70 (1H, heptet, J = 7.1 Hz), 4.92-5.05 (4H, m), 7.34-7.39 (1H, m), 7.58-7.62 (1H, m), 7.71-7.75 (1H, m), 7.82-7.85 (1H, m), 10.26 (1H, s).

Production Example 74: Synthesis of ethyl 4-(3-bromobenzoyl)-5-(1,3-diaminopropan-2-yl)-3-methyl-1H-pyrrole-2-carboxylate [compound No. 74]

Production Example 74-1: Synthesis of ethyl 4-(3-bromobenzoyl)-5-(1,3-diaminopropan-2-yl)-3-methyl-1H-pyrrole-2-carboxylate [compound No. 74]

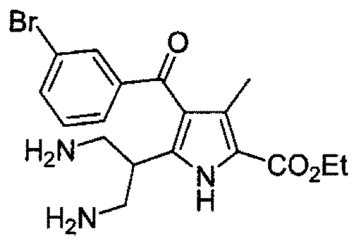

Iron powder (158 mg, 2.8 mmol), ammonium chloride (42 mg, 0.76 mmol), and water (5 ml) were added to a solution of compound 73 (100 mg, 0.22 mmol) in ethanol (5 ml), and the mixture was heated to reflux. After 3 hours, the reaction solution was allowed to cool, and 1 M hydrochloric acid and saturated saline were added thereto. The aqueous layer was washed with chloroform. The obtained organic layer was extracted with 1 M hydrochloric acid. The obtained aqueous layer was combined with the preceding washed aqueous layer. After addition of chloroform, the mixture was rendered weakly basic by the addition of a saturated aqueous solution of sodium bicarbonate under ice cooling. This aqueous layer was extracted with chloroform. The obtained organic layer was dried over anhydrous magnesium sulfate. Magnesium sulfate was filtered off, and the solvent was distilled off under reduced pressure to obtain the compound of interest (34 mg, yield: 37.9%, pale yellow solid).
$^1$H-NMR (400 MHz, DMSO-$d_6$) $\delta$: 1.28 (3H, t, J = 7.0 Hz), 1.89 (3H, s), 4.24 (2H, q, J = 7.0 Hz), 7.38-7.42 (1H, m), 7.43-7.47 (1H, m), 7.58-7.60 (1H, m), 7.62-7.66 (1H, m).

Production Example 75: Synthesis of ethyl 4-(3-bromobenzoyl)-5-(1-hydroxy-2-nitroethyl)-3-methyl-1H-pyrrole-2-carboxylate [compound No. 75]

Production Example 75-1: Synthesis of ethyl 4-(3-bromobenzoyl)-5-(1-hydroxy-2-nitroethyl)-3-methyl-1H-pyrrole-2-carboxylate [compound No. 75]

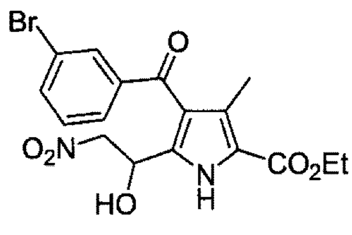

To a solution of compound 55 (70 mg, 0.19 mmol) in tetrahydrofuran (2 ml), nitromethane (11 $\mu$l, 0.19 mmol) and tetrabutylammonium fluoride (1 M solution in tetrahydrofuran, 193 $\mu$l, 0.19 mmol) were added at 0°C in the argon atmosphere. After 15 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (40 mg, yield: 48.9%, pale yellow solid).
$^1$H-NMR (400 MHz, $CDCl_3$) $\delta$: 1.39 (3H, t, J = 6.9 Hz), 2.06 (3H, s), 3.86-3.91 (1H, m), 4.37 (2H, q, J = 6.9 Hz), 4.54-4.62 (1H, m), 4.98-5.05 (1H, m), 5.65-5.73(1H, m), 7.33-7.39 (1H, m), 7.59-7.63 (1H, m), 7.69-7.73 (1H, m), 7.80-7.82 (1H, m), 9.68 (1H, s).

Production Example 76: Synthesis of ethyl 4-(3-bromobenzoyl)-3-methyl-5-(2-nitroethyl)-1H-pyrrole-2-carboxylate [compound No. 76]

Production Example 76-1: Synthesis of ethyl 4-(3-bromobenzoyl)-3-methyl-5-(2-nitroethyl)-1H-pyrrole-2-carboxylate [compound No. 76]

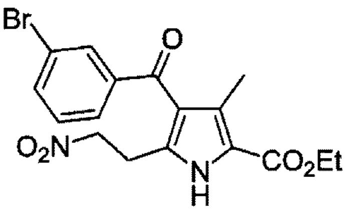

To a solution of compound 75 (40 mg, 0.1 mmol) in dichloromethane (5 ml), a boron trifluoride-diethyl ether complex (59 μl, 0.48 mmol) and triethylsilane (202 μl, 1.27 mmol) were added at 0°C in the argon atmosphere. After 5 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (24 mg, yield: 61.6%, orange solid).
$^1$H-NMR (400 MHz, $CDCl_3$) δ: 1.38 (3H, t, J = 7.2 Hz), 2.06 (3H, s), 3.41 (2H, t, J = 6.2 Hz), 4.35 (2H, q, J = 7.2 Hz), 4.79 (2H, t, J = 6.2 Hz), 7.32-7.37 (1H,m), 7.60-7.64 (1H, m), 7.68-7.71 (1H, m), 7.82-7.83 (1H, m), 9.36 (1H, s).

Production Example 77: Synthesis of ethyl 5-(2-aminoethyl)-4-(3-bromobenzoyl)-3-methyl-1H-pyrrole-2-carboxylate [compound No. 77]

Production Example 77-1: Synthesis of ethyl 5-(2-aminoethyl)-4-(3-bromobenzoyl)-3-methyl-1H-pyrrole-2-carboxylate [compound No. 77]

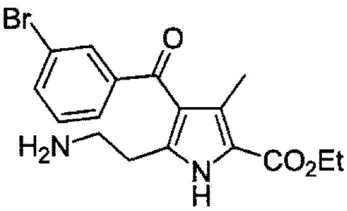

The compound of interest was obtained by the same procedures as in Production Example 74-1 using compound 76 as a starting material. Yield: 80.5 %, pale yellow solid.
$^1$H-NMR (400 MHz, DMSO-$d_6$) δ: 1.10-1.39 (3H, m), 1.78-2.02 (3H, m), 2.58-2.74 (2H, m), 3.62-3.79 (2H, m), 4.09-4.34 (2H, m), 7.29-7.50 (2H, m), 7.52-7.76 (2H, m), 12.01 (1H, s).

Production Example 78: Synthesis of ethyl 4-(3-((dimethylamino)methyl)benzoyl)-3,5-dimethyl-1H-pyrrole-2-carboxylate [compound No. 78]

Production Example 78-1: Synthesis of ethyl 4-(3-((dimethylamino)methyl)benzoyl)-3,5-dimethyl-1H-pyrrole-2-carboxylate [compound No. 78]

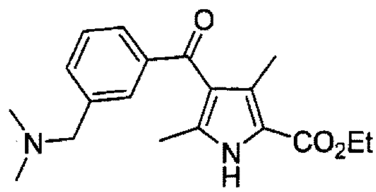

To a suspension of compound 44 (50 mg, 0.17 mmol) in dichloromethane (4 ml), carbon tetrabromide (64 mg, 0.19 mmol) and triphenylphosphine (50 mg, 0.19 mmol) were added at 0°C in the argon atmosphere. Then, the reaction temperature was increased to room temperature. After 24 hours, the reaction solution was quenched by the addition of water. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain a bromo intermediate (27 mg).
To a solution of the bromo intermediate in N,N-dimethylformamide (4 ml), triethylamine (13 μl, 0.09 mmol), dimethylamine hydrochloride (8 mg, 0.09 mmol), and potassium carbonate (13 mg, 0.09 mmol) were added in the argon atmosphere, and the mixture was heated with stirring at 80°C. After 2 hours, the reaction solution was allowed to cool and quenched by the addition of water. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (15 mg, yield: 27.5%, light brown solid).
$^1$H-NMR (400 MHz, DMSO-$d_6$) δ: 1.30 (3H, t, J = 7.1 Hz), 2.07 (3H, s), 2.16 (3H, s), 2.21 (6H, s), 4.26 (2H, q, J = 7.1 Hz), 7.42-7.60 (4H, m), 11.92 (1H, s).

Production Example 79: Synthesis of ethyl 4-(3-formylbenzoyl)-3,5-dimethyl-1H-pyrrole-2-carboxylate [compound No. 79]

Production Example 79-1: Synthesis of ethyl 4-(3-formylbenzoyl)-3,5-dimethyl-1H-pyrrole-2-carboxylate [compound No. 79]

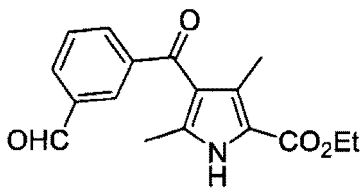

To a solution of compound 44 (892 mg, 2.96 mmol) in dichloromethane (10 ml), Dess-Martin periodinane (1381 mg, 3.26 mmol) was added in the argon atmosphere. After confirmation that the starting material was consumed, the reaction solution was quenched by the addition of a saturated aqueous solution of sodium bicarbonate. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the compound of interest (785 mg, yield: 88.7%, pale yellow solid). $^1$H-NMR (400 MHz, $CDCl_3$) δ: 1.38 (3H, t, J = 7.2 Hz), 2.20 (3H, s), 2.28 (3H, s), 4.35 (2H, q, J = 7.2 Hz), 7.62-7.68 (1H, m), 7.98-8.03 (1H, m), 8.05-8.10 (1H,m), 8.18-8.21 (1H, m), 9.01 (1H, s), 10.08 (1H, s).

Production Example 80: Synthesis of ethyl 4-(3-(1-hydroxy-2-nitroethyl)benzoyl)-3,5-dimethyl-1H-pyrrole-2-carboxylate [compound No. 80]

Production Example 80-1: Synthesis of ethyl 4-(3-(1-hydroxy-2-nitroethyl)benzoyl)-3,5-dimethyl-1H-pyrrole-2-carboxylate [compound No. 80]

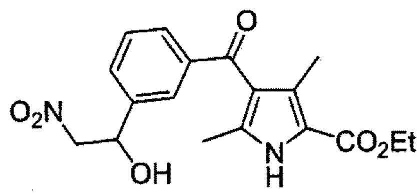

The compound of interest was obtained by the same procedures as in Production Example 75-1 using compound 79 as a starting material. Yield: quant, orange solid. $^1$H-NMR (400 MHz, $CDCl_3$) δ: 1.38 (3H, t, J = 7.2 Hz), 2.20 (3H, s), 2.26 (3H, s), 4.35 (3H, q, J = 7.2 Hz), 4.52-4.66 (2H, m), 5.50-5.57 (1H, m), 7.48-7.53 (1H,m), 7.58-7.63 (1H, m), 7.69-7.73 (1H, m), 7.76-7.78 (1H, m), 9.05 (1H, s).

Production Example 81: Synthesis of ethyl 4-(3-(1,3-dinitropropan-2-yl)benzoyl)-3,5-dimethyl-1H-pyrrole-2-carboxylate [compound No. 81]

Production Example 81-1: Synthesis of ethyl 4-(3-(1,3-dinitropropan-2-yl)benzoyl)-3,5-dimethyl-1H-pyrrole-2-carboxylate [compound No. 81]

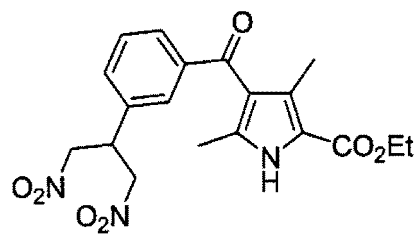

The compound of interest was obtained by the same procedures as in Production Example 73-1 using compound 79 as a starting material. Yield: 15.1 %, white solid. $^1$H-NMR (400 MHz, $CDCl_3$) δ: 1.38 (3H, t, J = 7.2 Hz), 2.21 (3H, s), 2.21 (3H, s), 4.35 (2H, q, J = 7.2 Hz), 4.37 (1H, t, J = 7.1 Hz), 4.80 (2H, d, J = 7.1 Hz), 4.81 (2H, d, J = 7.1 Hz), 7.40-7.45 (1H, m), 7.46-7.52 (1H, m), 7.57-7.61 (1H, m), 7.68-7.73 (1H, m), 8.99 (1H, s).

Production Example 82: Synthesis of ethyl 4-(3-(1,3-diaminopropan-2-yl)benzoyl)-3,5-dimethyl-1H-pyrrole-2-carboxylate [compound No. 82]

Production Example 82-1: Synthesis of ethyl 4-(3-(1,3-diaminopropan-2-yl)benzoyl)-3,5-dimethyl-1H-pyrrole-2-carboxylate [compound No. 82]

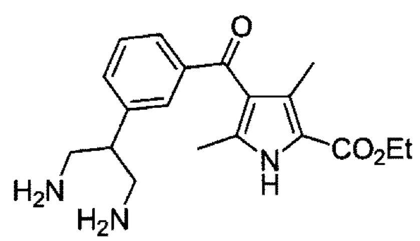

The compound of interest was obtained by the same procedures as in Production Example 74-1 using compound 81 as a starting material. Yield: quant, white solid. $^1$H-NMR (400 MHz, DMSO-$d_6$) δ: 1.30 (3H, t, J = 7.2 Hz), 2.07 (3H, s), 2.17 (3H, s), 4.25 (2H, q, J = 7.2 Hz), 7.37-7.59 (4H, m).

Production Example 83: Synthesis of ethyl 3,5-dimethyl-4-(3-vinylbenzoyl)-1H-pyrrole-2-carboxylate [compound No. 83]

Production Example 83-1: Synthesis of ethyl 3,5-dimethyl-4-(3-vinylbenzoyl)-1H-pyrrole-2-carboxylate [compound No. 83]

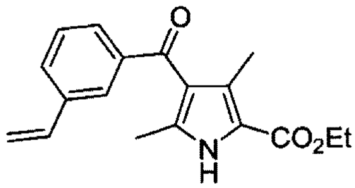

A mixture of compound 79 (300 mg, 0.86 mmol), toluene (10 ml), tetrakis(triphenylphosphine)palladium (20 mg, 0.02 mmol), and vinyltributyltin (277 μl, 0.95 mmol) was heated to reflux in the argon atmosphere.
After 12 hours, the reaction solution was allowed to cool and quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (207 mg, yield: 80.9%, yellow solid). $^1$H-NMR (400 MHz, $CDCl_3$) δ: 1.38 (3H, t, J = 7.2 Hz), 2.24 (3H, s), 2.26 (3H, s), 4.34 (2H, q, J = 7.2 Hz), 5.32 (1H, d, J = 10.7 Hz), 5.80 (1H, d, J = 17.6 Hz), 6.75 (1H, dd, J = 17.6, 11.0 Hz), 7.38-7.44 (1H, m), 7.56-7.63 (2H, m), 7.74-7.77 (1H, m), 9.08 (1H, s).

Production Example 84: Synthesis of ethyl 4-(3-ethylbenzoyl)-3,5-dimethyl-1H-pyrrole-2-carboxylate [compound No. 84]

Production Example 84-1: Synthesis of ethyl 4-(3-ethylbenzoyl)-3,5-dimethyl-1H-pyrrole-2-carboxylate [compound No. 84]

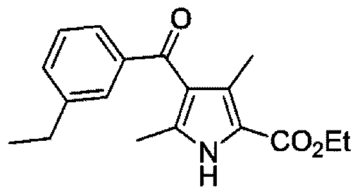

To a solution of compound 83 (40 mg, 0.13 mmol) in methanol (2 ml), 10% palladium carbon (5 mg) was added, and the mixture was stirred in the hydrogen atmosphere. After 6 hours, palladium carbon was filtered off through celite, and the celite was thoroughly washed with chloroform. The filtrate was concentrated, and the residue was purified by column chromatography to obtain the compound of interest (17 mg, yield: 42.1%, colorless oil).
$^1$H-NMR (400 MHz, $CDCl_3$) δ: 1.26 (3H, t, J = 7.6 Hz), 1.38 (3H, t, J = 7.2 Hz), 2.23 (3H, s), 2.25 (3H, s), 2.70 (2H, q, J = 7.6 Hz), 4.34 (2H, q, J = 7.0 Hz), 7.33-7.40 (2H, m), 7.51-7.58 (2H, m), 8.93 (1H, s).

Production Example 85: Synthesis of ethyl 4-benzoyl-3-bromo-5-methyl-1H-pyrrole-2-carboxylate [compound No. 85]

Production Example 85-1: Synthesis of ethyl 4-benzoyl-3-bromo-5-methyl-1H-pyrrole-2-carboxylate [compound No. 85]

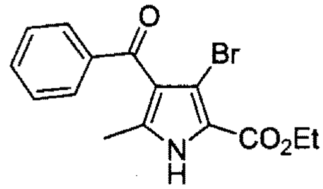

To a solution of compound 42 (289 mg, 1.12 mmol) and pyridine (191 μl, 2.35 mmol) in dichloromethane (10 ml), a solution of bromine (64 μl, 1.24 mmol) in dichloromethane (5 ml) was added at 0°C in the argon atmosphere. After 15 minutes, the reaction solution was quenched by the addition of a saturated aqueous solution of sodium thiosulfate. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the compound of interest (223 mg, yield: 59.2%, white solid).
$^1$H-NMR (400 MHz, $CDCl_3$) δ: 1.40 (3H, t, J = 7.1 Hz), 2.36 (3H, s), 4.38 (2H, q, J = 7.1 Hz), 7.43-7.49 (2H, m), 7.55-7.60 (1H, m), 7.77-7.81 (2H, m), 9.25 (1H,s).

Production Example 86: Synthesis of ethyl 4-benzoyl-5-methyl-3-vinyl-1H-pyrrole-2-carboxylate [compound No. 86]

Production Example 86-1: Synthesis of ethyl 4-benzoyl-5-methyl-3-vinyl-1H-pyrrole-2-carboxylate [compound No. 86]

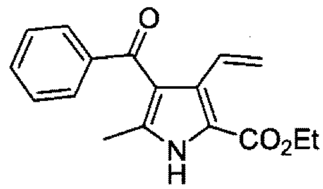

The compound of interest was obtained by the same procedures as in Production Example 83-1 using compound 85 as a starting material. Yield: 52.9 %, pale yellow solid.
$^1$H-NMR (400 MHz, $CDCl_3$) δ: 1.39 (3H, t, J = 7.2 Hz), 2.30 (3H, s), 4.36 (2H, q, J = 7.2 Hz), 5.06 (1H, d, J = 18.1 Hz), 5.07 (1H, d, J = 11.2 Hz), 7.01 (1H, dd, J = 18.1, 11.2 Hz), 7.38-7.44 (2H, m), 7.50-7.55 (1H, m), 7.77-7.81 (2H, m), 8.97 (1H, s).

Production Example 87: Synthesis of ethyl 4-benzoyl-3-formyl-5-methyl-1H-pyrrole-2-carboxylate [compound No. 87]

Production Example 87-1: Synthesis of ethyl 4-benzoyl-3-formyl-5-methyl-1H-pyrrole-2-carboxylate [compound No. 87]

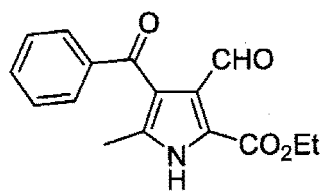

To a solution of compound 86 (100 mg, 0.36 mmol) in tetrahydrofuran (15 ml), osmium tetraoxide (64 mg, 0.25 mmol), carbon tetrachloride (5 ml), sodium periodate (1302 mg, 6.09 mmol), and water (10 ml) were added in the argon atmosphere. After 12 hours, water was added to the reaction solution. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (22 mg, yield: 21.4%, light brown solid).
$^1$H-NMR (400 MHz, $CDCl_3$) δ: 1.42 (3H, t, J = 7.2 Hz), 2.32 (3H, s), 4.45 (2H, q, J = 7.2 Hz), 7.39-7.44 (2H, m), 7.51-7.56

(1H, m), 7.77-7.81 (2H, m), 9.26 (1H,s), 10.35 (1H, s).

Production Example 88: Synthesis of ethyl 4-benzoyl-3-(hydroxymethyl)-5-methyl-1H-pyrrole-2-carboxylate [compound No. 88]

Production Example 88-1: Synthesis of ethyl 4-benzoyl-3-(hydroxymethyl)-5-methyl-1H-pyrrole-2-carboxylate [compound No. 88]

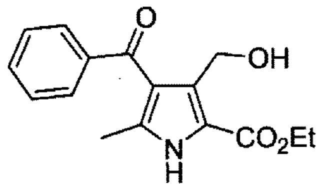

The compound of interest was obtained by the same procedures as in Production Example 58-1 using compound 87 as a starting material. Yield: 24.9 %, white solid. $^1$H-NMR (400 MHz, $CDCl_3$) δ: 1.41 (3H, t, J = 7.2 Hz), 2.08 (3H, s), 4.39 (2H, q, J = 7.2 Hz), 4.81 (2H, s), 7.44-7.50 (2H, m), 7.55-7.60 (1H, m), 7.68-7.73 (2H,m), 9.02 (1H, s).

Production Example 89: Synthesis of ethyl 4-benzoyl-3-(difluoromethyl)-5-methyl-1H-pyrrole-2-carboxylate [compound No. 89]

Production Example 89-1: Synthesis of ethyl 4-benzoyl-3-(difluoromethyl)-5-methyl-1H-pyrrole-2-carboxylate [compound No. 89]

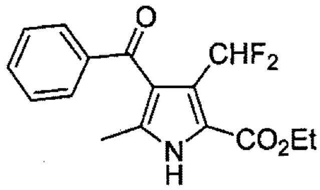

The compound of interest was obtained by the same procedures as in Production Example 57-1 using compound 87 as a starting material. Yield: 5.4 %, white solid.
$^1$H-NMR (400 MHz, $CDCl_3$) δ: 1.40 (3H, t, J = 7.2 Hz), 2.24 (3H, s), 4.40 (2H, q, J = 7.2 Hz), 7.10 (1H, t, J = 55.1 Hz), 7.43-7.48 (2H, m), 7.55-7.61 (1H, m), 7.81-7.85 (2H, m), 9.11 (1H, s).

Production Example 90: Synthesis of ethyl 4-(3-bromobenzoyl)-5-chloro-3-methyl-1H-pyrrole-2-carboxylate [compound No. 90]

Production Example 90-1: Synthesis of ethyl 4-(3-bromobenzoyl)-5-chloro-3-methyl-1H-pyrrole-2-carboxylate [compound No. 90]

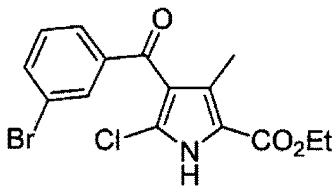

To a solution of N-chlorosuccinimide (6 mg, 0.05 mmol) in dichloromethane (2 ml), zirconium chloride (0.5 mg, 0.003 mmol) was added at 0°C in the argon atmosphere, and subsequently compound 39 (15 mg, 0.05 mmol) was added thereto. Then, the reaction temperature was increased to room temperature. After 6 hours, the reaction solution was purified by column chromatography to obtain the compound of interest (3 mg, yield: 18.0%, white solid). $^1$H-NMR (400 MHz, $CDCl_3$) δ: 1.39 (3H, t, J = 7.2 Hz), 2.37 (3H, s), 4.37 (2H, q, J = 7.2 Hz), 7.32-7.37 (1H, m), 7.67-7.72 (2H, m), 7.88-7.91 (1H, m).

Production Example 91: Synthesis of ethyl 4-(2-hydroxybenzoyl)-3,5-dimethyl-1H-pyrrole-2-carboxylate [compound No. 91]

Production Example 91-1: Synthesis of ethyl 4-(2-hydroxybenzoyl)-3,5-dimethyl-1H-pyrrole-2-carboxylate [compound No. 91]

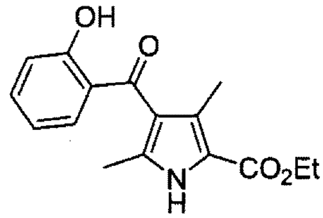

To a solution of compound 3 (70 mg, 0.24 mmol) in dichloromethane (3 ml), boron tribromide (1 M solution in dichloromethane, 520 μl, 0.52 mmol) was added in the argon atmosphere. After 12 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the compound of interest (25 mg, yield: 36.3%, white solid).
$^1$H-NMR (400 MHz, DMSO-$d_6$) δ: 1.29 (3H, t, J = 7.2 Hz), 2.10 (3H, s), 2.21 (3H, s), 4.25 (2H, q, J = 7.1 Hz), 6.87-6.96 (2H, m), 7.28-7.32 (1H, m), 7.38-7.44 (1H, m), 10.80 (1H, s), 11.91 (1H, s).

Production Example 92: Synthesis of ethyl 4-(3-hydroxybenzoyl)-3,5-dimethyl-1H-pyrrole-2-carboxylate [compound No. 92]

Production Example 92-1: Synthesis of ethyl 4-(3-hydroxybenzoyl)-3,5-dimethyl-1H-pyrrole-2-carboxylate [compound No. 92]

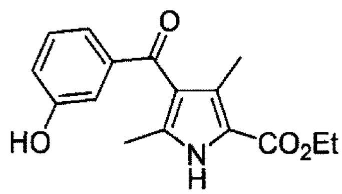

The compound of interest was obtained by the same procedures as in Production Example 91-1 using compound 8 as a starting material. Yield: 39.2 %, white solid. $^1$H-NMR (400 MHz, DMSO-$d_6$) δ: 1.30 (3H, t, J = 7.1 Hz), 2.09 (3H, s), 2.16 (3H, s), 4.25 (2H, q, J = 7.1 Hz), 6.93-7.05 (3H, m), 7.25-7.32 (1H, m), 9.70 (1H, s), 11.89 (1H, s).

Production Example 93: Synthesis of ethyl 4-(3-(2-aminoethoxy)benzoyl)-3,5-dimethyl-1H-pyrrole-2-carboxylate [compound No. 93]

Production Example 93-1: Synthesis of ethyl 4-(3-(2-aminoethoxy)benzoyl)-3,5-dimethyl-1H-pyrrole-2-carboxylate [compound No. 93]

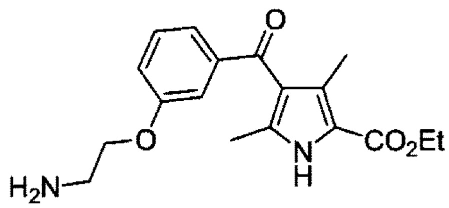

The compound of interest was obtained by the same procedures as in Production Examples 50-1 and 48-1 using compound 92 as a starting material. Yield: 70.0 %, white solid.
$^1$H-NMR (400 MHz, DMSO-$d_6$) δ: 1.30 (3H, t, J = 7.2 Hz), 2.08 (3H, s), 2.16 (3H, s), 2.88 (2H, t, J = 5.7 Hz), 3.95 (2H, t, J = 5.7 Hz), 4.25 (2H, q, J = 7.1 Hz), 7.09-7.12 (1H, m), 7.13-7.19 (2H, m), 7.37-7.43 (1H, m).

Production Example 94: Synthesis of ethyl 4-(3-(2-(dimethylamino)ethoxy)benzoyl)-3,5-dimethyl-1H-pyrrole-2-carboxylate [compound No. 94]

Production Example 94-1: Synthesis of ethyl 4-(3-(2-(dimethylamino)ethoxy)benzoyl)-3,5-dimethyl-1H-pyrrole-2-carboxylate [compound No. 94]

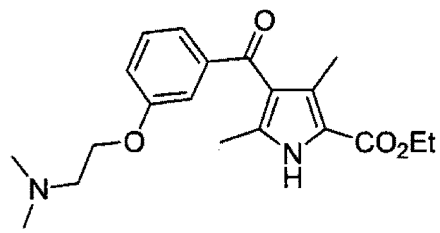

To compound 93 (60 mg, 0.18 mmol), acetic acid (1 ml), and 37% formalin (33 μl, 0.4 mmol), sodium cyanoborohydride (32 mg, 0.5 mmol) was added at 0°C. Then, the reaction temperature was increased to room temperature. After 2 hours, a saturated aqueous solution of sodium bicarbonate was added to the reaction solution. The aqueous layer was extracted with chloroform. The obtained organic layer was dried over anhydrous magnesium sulfate. After filtering off of magnesium sulfate, the solvent was distilled off under reduced pressure to obtain the compound of interest (63 mg, yield: 97.6%, colorless oil).
$^1$H-NMR (400 MHz, DMSO-$d_6$) δ: 1.29 (3H, t, J = 7.0 Hz), 2.07-2.10 (6H, m), 2.16 (3H, s), 2.20 (3H, s), 2.62 (2H, t, J = 5.6 Hz), 4.08 (2H, t, J = 5.7 Hz), 4.25 (2H, q, J = 7.1 Hz), 7.09-7.20 (3H, m), 7.36-7.44 (1H, m).

Production Example 95: Synthesis of ethyl 4-(4-hydroxybenzoyl)-3,5-dimethyl-1H-pyrrole-2-carboxylate [compound No. 95]

Production Example 95-1: Synthesis of ethyl 4-(4-hydroxybenzoyl)-3,5-dimethyl-1H-pyrrole-2-carboxylate [compound No. 95]

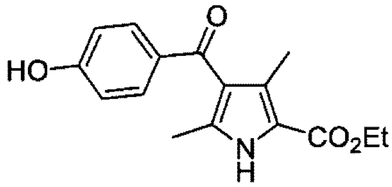

The compound of interest was obtained by the same procedures as in Production Example 91-1 using compound 14 as a starting material. Yield: 37.7 %, white solid. $^1$H-NMR (400 MHz, DMSO-$d_6$) δ: 1.30 (3H, t, J = 7.1 Hz), 2.09 (3H, s), 2.15 (3H, s), 4.25 (2H, q, J = 7.2 Hz), 6.81-6.85 (2H, m), 7.51-7.56 (2H, m), 10.24 (1H, s), 11.79 (1H, s).

Production Example 96: Synthesis of (3-bromophenyl)(2,4-dimethyl-5-(1H-tetrazol-5-yl)-1H-pyrrol-3-yl)methanone [compound No. 96]

Production Example 96-1: Synthesis of 4-(3-bromobenzoyl)-3,5-dimethyl-1H-pyrrole-2-carboxylic acid

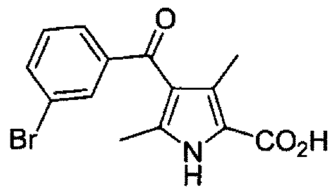

A 1 M aqueous sodium hydroxide solution (4 ml) was added to a suspension of compound 11 (100 mg, 0.29 mmol) in methanol (4 ml), and the mixture was heated to reflux. After 2 hours, the consumption of the starting material was confirmed, and the solvent was distilled off. The residue was cooled in ice and rendered acidic by the addition of 1 M hydrochloric acid. The precipitated solid was collected by filtration, washed with hexane, and then dried to obtain the compound of interest (86 mg, yield: 93.7%, white solid).
$^1$H-NMR (400 MHz, DMSO-$d_6$) δ: 2.08 (3H, s), 2.15 (3H, s), 7.43-7.49 (1H, m), 7.56-7.60 (1H, m), 7.69-7.72 (1H, m), 7.77-7.81 (1H, m), 11.91 (1H, s), 12.51 (1H, s).

Production Example 96-2: Synthesis of (3-bromophenyl)(2,4-dimethyl-1H-pyrrol-3-yl)methanone

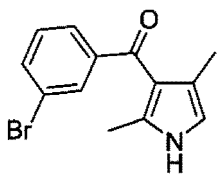

A solution of the compound (500 mg, 1.55 mmol) obtained in Production Example 96-1 in trifluoroacetic acid (5 ml) was heated with stirring at 40°C. After 5 minutes, the reaction solution was cooled in ice and rendered basic by the addition of a 5 M aqueous sodium hydroxide solution. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the compound of interest (364 mg, yield: 84.5%, yellow solid).
$^1$H-NMR (400 MHz, $CDCl_3$) δ: 1.99 (3H, s), 2.21 (3H, s), 6.42-6.44 (1H, m), 7.28-7.35 (1H, m), 7.60-7.65 (2H, m), 7.81-7.84 (1H, m), 7.96 (1H, s).

Production Example 96-3: Synthesis of 4-(3-bromobenzoyl)-3,5-dimethyl-1H-pyrrole-2-carbonitrile

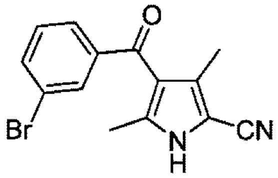

To a solution of the compound (200 mg, 0.72 mmol) obtained in Production Example 96-2 in acetonitrile (4 ml) and dichloromethane (1 ml), chlorosulfonyl isocyanate (67 μl, 0.76 mmol) was added at -42°C in the argon atmosphere. After 2.5 hours, N,N-dimethylformamide (1 ml) was added to the mixture. The reaction temperature was increased to room temperature. After 12 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the compound of interest (200 mg, yield: 92.0%, pink solid).
$^1$H-NMR (400 MHz, $CDCl_3$) δ: 2.12 (3H, s), 2.28 (3H, s), 7.33-7.39 (1H, m), 7.59-7.63 (1H, m), 7.68-7.72 (1H, m), 7.77-7.83 (1H, m), 8.72 (1H, s).

Production Example 96-4: Synthesis of (3-bromophenyl)(2,4-dimethyl-5-(1H-tetrazol-5-yl)-1H-pyrrol-3-yl)methanone [compound No. 96]

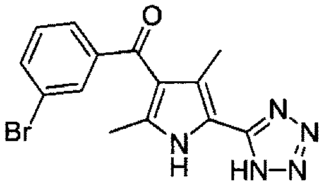

To a solution of the compound (100 mg, 0.33 mmol) obtained in Production Example 96-3 in N,N-dimethylformamide (20 ml), sodium azide (1073 mg, 16.5 mmol) and ammonium chloride (883 mg, 16.5 mmol) were added in the argon atmosphere, and the mixture was heated with stirring at 110°C. After 12 hours, the precipitated solid was filtered off and thoroughly washed with N,N-dimethylformamide. After filtering off of the solvent in the filtrate, ethyl acetate was added to the residue. The reaction solution was rendered basic by the addition of a 1 M aqueous sodium hydroxide solution. The organic layer was washed. The obtained aqueous layer was rendered acidic by the addition of 1 M hydrochloric acid, followed by extraction with ethyl acetate. The obtained organic layer was dried over anhydrous magnesium sulfate. Magnesium sulfate was filtered off, and the solvent was distilled off under reduced pressure to obtain the compound of interest (65 mg, yield: 57.1%, pale yellow solid).
$^1$H-NMR (400 MHz, DMSO-$d_6$) δ: 2.14 (3H, s), 2.18 (3H, s), 7.44-7.51 (1H, m), 7.60-7.64 (1H, m), 7.73-7.76 (1H, m), 7.78-7.82 (1H, m), 12.09 (1H, s).

Production Example 97: Synthesis of (3-bromophenyl)(5-(2-ethyl-2H-tetrazol-5-yl)-2,4-dimethyl-1H-pyrrol-3-yl)methanone [compound No. 97]

Production Example 97-1: Synthesis of (3-bromophenyl)(5-(2-ethyl-2H-tetrazol-5-yl)-2,4-dimethyl-1H-pyrrol-3-yl)methanone [compound No. 97]

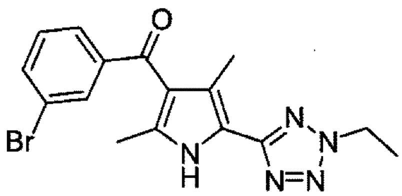

To a solution of compound 96 (53 mg, 0.154 mg) in acetone (4 ml), triethylamine (24 μl, 0.169 mmol) and ethyl

iodide (14 μl, 0.169 mmol) were added in the argon atmosphere. After 4 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the compound of interest (45 mg, yield: 78.3%, yellow solid).
$^1$H-NMR (400 MHz, $CDCl_3$) δ: 1.68 (3H, t, J = 7.3 Hz), 2.30 (3H, s), 2.32 (3H, s), 4.68 (2H, q, J = 7.3 Hz), 7.31-7.36 (1H, m), 7.64-7.70 (2H, m), 7.87-7.89 (1H,m), 9.05 (1H, s).

Production Example 98: Synthesis of (3-bromophenyl)(5-(1-ethyl-1H-tetrazol-5-yl)-2,4-dimethyl-1H-pyrrol-3-yl)methanone [compound No. 98]

Production Example 98-1: Synthesis of (3-bromophenyl)(5-(1-ethyl-1H-tetrazol-5-yl)-2,4-dimethyl-1H-pyrrol-3-yl)methanone [compound No. 98]

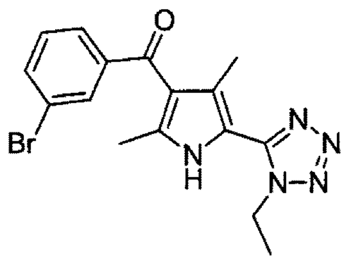

The compound of interest was obtained as a positional isomer by the procedures of Production Example 97-1. Yield: 7.0%, brown solid.
$^1$H-NMR (400 MHz, $CDCl_3$) δ: 1.61 (3H, t, J = 7.3 Hz), 2.11 (3H, s), 2.23 (3H, s), 4.41 (2H, q, J = 7.3 Hz), 7.33-7.39 (1H, m), 7.65-7.72 (2H, m), 7.86-7.89 (1H,m), 9.37 (1H, s).

Production Example 99: Synthesis of (3-bromophenyl)(2,4-dimethyl-5-(2-(2,2,2-trifluoroethyl)-2H-tetrazol-5-yl)-1H-pyrrol-3-yl)methanone [compound No. 99]

Production Example 99-1: Synthesis of (3-bromophenyl)(2,4-dimethyl-5-(2-(2,2,2-trifluoroethyl)-2H-tetrazol-5-yl)-1H-pyrrol-3-yl)methanone [compound No. 99]

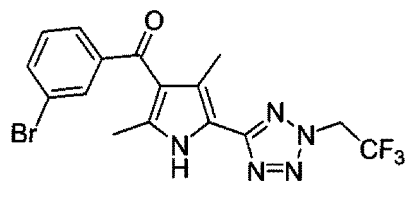

The compound of interest was obtained by the same procedures as in Production Example 97-1 using trifluoromethanesulfonic acid 2,2,2-trifluoroethyl ester. Yield: 58.0%, pale yellow solid.
$^1$H-NMR (400 MHz, $CDCl_3$) δ: 2.32 (3H, s), 2.33 (3H, s), 5.24 (2H, q, J = 7.7 Hz), 7.32-7.37 (1H, m), 7.66-7.71 (2H, m), 7.87-7.90 (1H, m), 9.03 (1H, s).

Production Example 100: Synthesis of (3-bromophenyl)(2,4-dimethyl-5-(1-(2,2,2-trifluoroethyl)-1H-tetrazol-5-yl)-1H-pyrrol-3-yl)methanone [compound No. 100]

Production Example 100-1: Synthesis of (3-bromophenyl)(2,4-dimethyl-5-(1-(2,2,2-trifluoroethyl)-1H-tetrazol-5-yl)-1H-pyrrol-3-yl)methanone [compound No. 100]

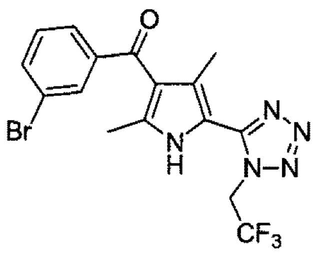

The compound of interest was obtained as a positional isomer by the procedures of Production Example 99-1. Yield: 6.4%, yellow solid.
$^1$H-NMR (400 MHz, $CDCl_3$) δ: 2.22 (3H, s), 2.23 (3H, s), 5.24 (2H, q, J = 7.8 Hz), 7.27-7.33 (1H, m), 7.58-7.67 (2H, m), 7.85-7.88 (1H, m), 8.94 (1H, s).

Production Example 101: Synthesis of (3-bromophenyl)(5-(2-(2-(dimethylamino)ethyl)-2H-tetrazol-5-yl)-2,4-dimethyl-1H-pyrrol-3-yl)methanone [compound No. 101]

Production Example 101-1: Synthesis of (3-bromophenyl)(5-(2-(2-(dimethylamino)ethyl)-2H-tetrazol-5-yl)-2,4-dimethyl-1H-pyrrol-3-yl)methanone [compound No. 101]

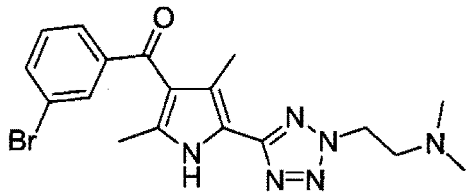

The compound of interest was obtained by the same procedures as in Production Example 97-1 using 2-dimethylaminoethyl chloride hydrochloride. Yield: 23.1%, yellow solid.
$^1$H-NMR (400 MHz, $CDCl_3$) δ: 2.30 (3H, s), 2.33 (3H, s), 2.49 (6H, s), 3.07-3.25 (2H, m), 4.80-4.94 (2H, m), 7.31-7.37 (1H, m), 7.64-7.71 (2H, m), 7.86-7.89 (1H,m), 9.34 (1H, s).

Production Example 102: Synthesis of (3-bromophenyl)(5-(2-(2-hydroxyethyl)-2H-tetrazol-5-yl)-2,4-dimethyl-1H-pyrrol-3-yl)methanone [compound No. 102]

Production Example 102-1: Synthesis of (3-bromophenyl)(5-(2-(2-hydroxyethyl)-2H-tetrazol-5-yl)-2,4-dimethyl-1H-pyrrol-3-yl)methanone [compound No. 102]

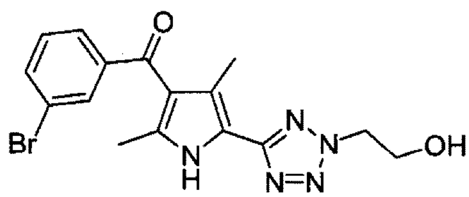

The compound of interest was obtained by the same procedures as in Production Example 97-1 using 2-iodoethanol. Yield: 74.2 %, pale yellow solid.
$^1$H-NMR (400 MHz, $CDCl_3$) δ: 2.31 (3H, s), 2.32 (3H, s), 4.20-4.27 (2H, m), 4.77-4.82 (2H, m), 7.32-7.37 (1H, m), 7.65-7.70 (2H, m), 7.86-7.89 (1H, m), 9.04 (1H,s).

Production Example 103: Synthesis of (5-(2-(2-aminoethyl)-2H-tetrazol-5-yl)-2,4-dimethyl-1H-pyrrol-3-yl)(3-bromophenyl)methanone [compound No. 103]

Production Example 103-1: Synthesis of (5-(2-(2-aminoethyl)-2H-tetrazol-5-yl)-2,4-dimethyl-1H-pyrrol-3-yl)(3-bromophenyl)methanone [compound No. 103]

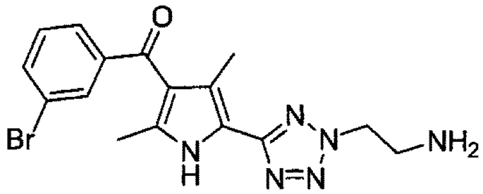

The compound of interest was obtained by the same procedures as in Production Example 97-1 using 2-bromoethylamine. Yield: 21.3 %, pale yellow solid. $^1$H-NMR (400 MHz, DMSO-$d_6$) δ: 2.13 (3H, s), 2.25 (3H, s), 3.09-3.18 (2H, m), 4.64-4.70 (2H, m), 7.45-7.50 (1H, m), 7.59-7.63 (1H, m), 7.72-7.74 (1H, m), 7.78-7.82 (1H, m), 12.12 (1H, s).

Production Example 104: Synthesis of 2-(5-(4-(3-bromobenzoyl)-3,5-dimethyl-1H-pyrrol-2-yl)-2H-tetrazol-2-yl)ethyl acetate [compound No. 104]

Production Example 104-1: Synthesis of 2-(5-(4-(3-bromobenzoyl)-3,5-dimethyl-1H-pyrrol-2-yl)-2H-tetrazol-2-yl)ethyl acetate [compound No. 104]

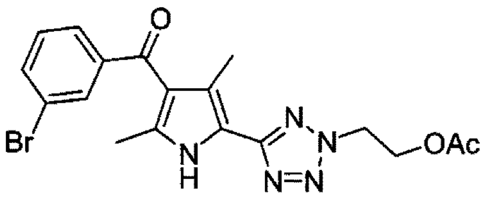

The compound of interest was obtained by the same procedures as in Production Example 97-1 using 2-bromoethyl acetate. Yield: 81.9 %, brown solid.
$^1$H-NMR (400 MHz, $CDCl_3$) δ: 2.05 (3H, s), 2.30 (3H, s), 2.32 (3H, s), 4.64 (2H, t, J = 5.2 Hz), 4.89 (2H, t, J = 5.2 Hz), 7.31-7.37 (1H, m), 7.65-7.70 (2H, m), 7.86-7.89 (1H, m), 9.06 (1H, s).

Production Example 105: Synthesis of (2,4-dimethyl-5-(1H-tetrazol-5-yl)-1H-pyrrol-3-yl)(3-methoxyphenyl)methanone [compound No. 105]

Production Example 105-1: Synthesis of 4-(3-methoxybenzoyl)-3,5-dimethyl-1H-pyrrole-2-carboxylic acid

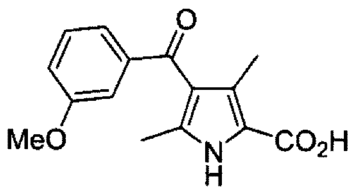

The compound of interest was obtained by the same procedures as in Production Example 96-1 using compound 8 as a starting material. Yield: 95.5 %, light red solid.

$^1$H-NMR (400 MHz, DMSO-$d_6$) δ: 2.08 (3H, s), 2.16 (3H, s), 3.78 (3H, s), 6.94-7.04 (3H, m), 7.25-7.31 (1H, m), 9.70 (1H, s), 11.95 (1H, s).

Production Example 105-2: Synthesis of (2,4-dimethyl-1H-pyrrol-3-yl)(3-methoxyphenyl)methanone

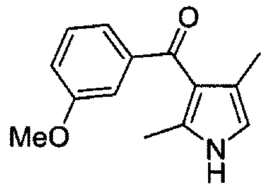

The compound of interest was obtained by the same procedures as in Production Example 96-2 using the compound obtained in Production Example 105-1. Yield: 86.3%, pale yellow solid.
$^1$H-NMR (400 MHz, $CDCl_3$) δ: 2.01 (3H, s), 2.19 (3H, s), 3.84 (3H, s), 6.39-6.44 (1H, m), 7.01-7.07 (1H, m), 7.18-7.35 (2H, m), 7.98 (1H, s).

Production Example 105-3: Synthesis of 4-(3-methoxybenzoyl)-3,5-dimethyl-1H-pyrrole-2-carbonitrile

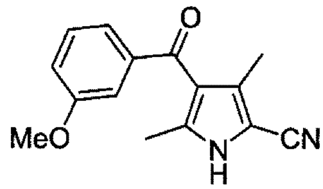

The compound of interest was obtained by the same procedures as in Production Example 96-3 using the compound obtained in Production Example 105-2. Yield: 92.9 %, orange solid.
$^1$H-NMR (400 MHz, $CDCl_3$) δ: 2.14 (3H, s), 2.27 (3H, s), 3.86 (3H, s), 7.08-7.13 (1H, m), 7.22-7.26 (2H, m), 7.33-7.38 (1H, m), 8.61 (1H, s).

Production Example 105-4: Synthesis of (2,4-dimethyl-5-(1H-tetrazol-5-yl)-1H-pyrrol-3-yl)(3-methoxyphenyl)methanone [compound No. 105]

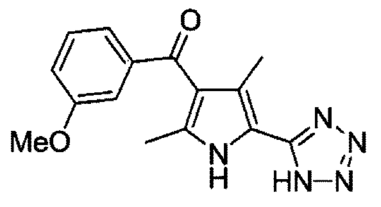

The compound of interest was obtained by the same procedures as in Production Example 96-4 using the compound obtained in Production Example 105-3. Yield: 81.5 %, yellow solid.
$^1$H-NMR (400 MHz, DMSO-$d_6$) δ: 2.13 (3H, s), 2.19 (3H, s), 3.81 (3H, s), 7.13-7.21 (3H, m), 7.39-7.45 (1H, m), 12.02 (1H, s).

Production Example 106: Synthesis of (3-methoxyphenyl)(5-(2-ethyl-2H-tetrazol-5-yl)-2,4-dimethyl-1H-pyrrol-3-yl)methanone [compound No. 106]

Production Example 106-1: Synthesis of (3-methoxyphenyl)(5-(2-ethyl-2H-tetrazol-5-yl)-2,4-dimethyl-1H-pyrrol-3-yl)methanone [compound No. 106]

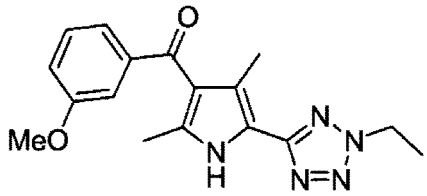

The compound of interest was obtained by the same procedures as in Production Example 97-1 using compound 105 as a starting material. Yield: 56.4 %, yellow oil. $^1$H-NMR (400 MHz, $CDCl_3$) δ: 1.68 (3H, t, J = 7.3 Hz), 2.14 (3H, s), 2.27 (3H, s), 3.86 (3H, s), 4.68 (2H, q, J = 7.3 Hz), 7.08-7.13 (1H, m), 7.22-7.26 (2H, m), 7.33-7.38 (1H, m), 8.61 (1H, s).

Production Example 107: Synthesis of (5-(2-ethyl-2H-tetrazol-5-yl)-2,4-dimethyl-1H-pyrrol-3-yl)(3-methoxyphenyl)methanone [compound No. 107]

Production Example 107-1: Synthesis of (5-(2-ethyl-2H-tetrazol-5-yl)-2,4-dimethyl-1H-pyrrol-3-yl)(3-methoxyphenyl)methanone [compound No. 107]

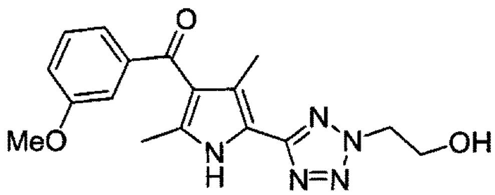

The compound of interest was obtained by the same procedures as in Production Example 97-1 using compound 105 and 2-iodoethanol. Yield: 17.8 %, brown solid. $^1$H-NMR (400 MHz, DMSO-$d_6$) δ: 2.12 (3H, s), 2.26 (3H, s), 3.80 (3H, s), 3.92-3.98 (2H, m), 4.70-4.77 (2H, m), 5.08 (1H, s), 7.11-7.21 (3H, m), 7.38-7.44 (1H, m), 12.03 (1H, s).

Production Example 108: Synthesis of (3-bromo-4-methoxyphenyl)(2,4-dimethyl-5-(1H-tetrazol-5-yl)-1H-pyrrol-3-yl)methanone [compound No. 108]

Production Example 108-1: Synthesis of 4-(3-bromo-4-methoxybenzoyl)-3,5-dimethyl-1H-pyrrole-2-carboxylic acid

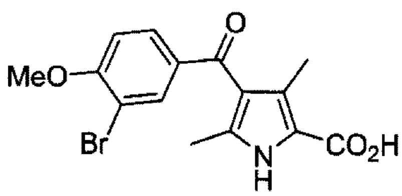

The compound of interest was obtained by the same procedures as in Production Example 96-1 using compound 22 as a starting material. Yield: quant, flesh-colored solid.
$^1$H-NMR (400 MHz, DMSO-$d_6$) δ: 2.09 (3H, s), 2.15 (3H, s), 3.94 (3H, s), 7.21 (1H, d, J = 8.8 Hz), 7.64 (1H, dd, J = 8.7,

2.1 Hz), 7.79 (1H, d, J = 2.2 Hz), 11.84 (1H, s), 12.46 (1H, s).

Production Example 108-2: Synthesis of (3-bromo-4-methoxyphenyl)(2,4-dimethyl-1H-pyrrol-3-yl)methanone

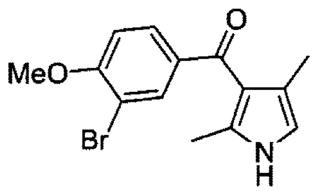

The compound of interest was obtained by the same procedures as in Production Example 96-2 using the compound obtained in Production Example 108-1. Yield: 99.9 %, yellow solid.
$^1$H-NMR (400 MHz, $CDCl_3$) δ: 2.01 (3H, s), 2.22 (3H, s), 3.97 (3H, s), 6.41-6.46 (1H, m), 6.93 (1H, d, J = 8.5 Hz), 7.72 (1H, dd, J = 8.4, 2.1 Hz), 7.91-8.01 (1H, m), 7.96 (1H, d, J = 2.0 Hz).

Production Example 108-3: Synthesis of 4-(3-bromo-4-methoxybenzoyl)-3,5-dimethyl-1H-pyrrole-2-carbonitrile

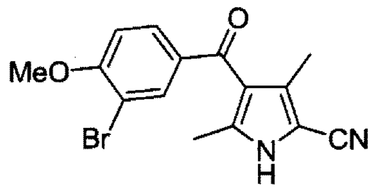

The compound of interest was obtained by the same procedures as in Production Example 96-3 using the compound obtained in Production Example 108-2. Yield: 92.9 %, green solid.
$^1$H-NMR (400 MHz, $CDCl_3$) δ: 2.14 (3H, s), 2.29 (3H, s), 3.98 (3H, s), 6.95 (1H, d, J = 8.5 Hz), 7.70 (1H, dd, J = 8.5, 2.1 Hz), 7.94 (1H, d, J = 2.1 Hz), 8.58 (1H, s).

Production Example 108-4: Synthesis of (3-bromo-4-methoxyphenyl)(2,4-dimethyl-5-(1H-tetrazol-5-yl)-1H-pyrrol-3-yl)methanone [compound No. 108]

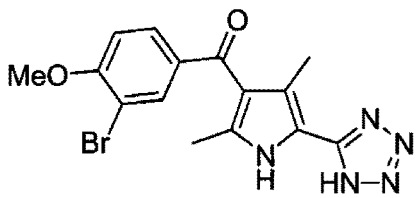

The compound of interest was obtained by the same procedures as in Production Example 96-4 using the compound obtained in Production Example 108-3. Yield: 91.4 %, yellow solid.
$^1$H-NMR (400 MHz, DMSO-$d_6$) δ: 2.16 (3H, s), 2.18 (3H, s), 3.95 (3H, s), 7.23 (1H, d, J = 8.8 Hz), 7.69 (1H, dd, J = 8.5, 2.0 Hz), 7.83 (1H, d, J = 2.0 Hz), 12.01 (1H, s).

Production Example 109: Synthesis of (3-bromo-4-methoxyphenyl)(5-(2-ethyl-2H-tetrazol-5-yl)-2,4-dimethyl-1H-pyrrol-3-yl)methanone [compound No. 109]

Production Example 109-1: Synthesis of (3-bromo-4-methoxyphenyl)(5-(2-ethyl-2H-tetrazol-5-yl)-2,4-dimethyl-1H-pyrrol-3-yl)methanone [compound No. 109]

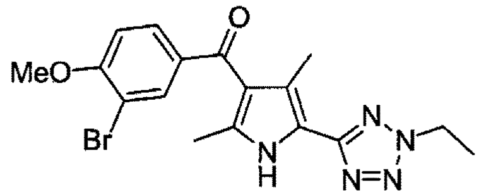

The compound of interest was obtained by the same procedures as in Production Example 97-1 using compound 108 as a starting material. Yield: 53.2 %, white solid. $^1$H-NMR (400 MHz, $CDCl_3$) δ: 1.43 (3H, t, J = 7.6 Hz), 2.29 (6H, s), 2.94 (2H, q, J = 7.6 Hz), 3.98 (3H, s), 6.93-6.98 (1H, m), 7.73-7.78 (1H, m), 7.97-8.01 (1H,m), 9.49 (1H, s).

Production Example 110: Synthesis of (3-bromo-4-methoxyphenyl)(5-(2-(2-hydroxyethyl)-2H-tetrazol-5-yl)-2,4-dimethyl-1H-pyrrol-3-yl)methanone [compound No. 110]

Production Example 110-1: Synthesis of (3-bromo-4-methoxyphenyl)(5-(2-(2-hydroxyethyl)-2H-tetrazol-5-yl)-2,4-dimethyl-1H-pyrrol-3-yl)methanone [compound No. 110]

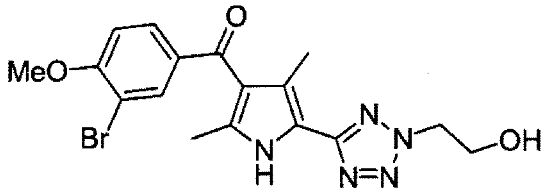

The compound of interest was obtained by the same procedures as in Production Example 97-1 using compound 108 and 2-iodoethanol. Yield: 27.4 %, pale yellow oil. $^1$H-NMR (400 MHz, DMSO-$d_6$) δ: 2.15 (3H, s), 2.25 (3H, s), 3.92-3.98 (2H, m), 3.94 (3H, s), 4.71-4.76 (2H, m), 7.22 (1H, d, J = 8.8 Hz), 7.68 (1H, dd, J = 8.5, 2.0 Hz), 7.82 (1H, d, J = 2.2 Hz), 12.03 (1H, s).

Production Example 111: Synthesis of 2-(5-(4-(3-bromo-4-methoxybenzoyl)-3,5-dimethyl-1H-pyrrol-2-yl)-2H-tetrazol-2-yl)ethyl acetate [compound No. 111]

Production Example 111-1: Synthesis of 2-(5-(4-(3-bromo-4-methoxybenzoyl)-3,5-dimethyl-1H-pyrrol-2-yl)-2H-tetrazol-2-yl)ethyl acetate [compound No. 111]

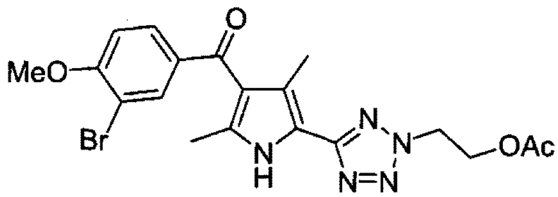

The compound of interest was obtained by the same procedures as in Production Example 97-1 using compound

108 and 2-bromoethyl acetate. Yield: 60.3 %, white solid. $^1$H-NMR (400 MHz, DMSO-$d_6$) δ: 2.15 (3H, s), 2.25 (3H, s), 3.94 (3H, s), 4.56 (2H, t, J = 5.1 Hz), 4.99 (2H, t, J = 5.1 Hz), 7.22 (1H, d, J = 8.5 Hz), 7.68 (1H, dd, J = 8.5, 2.2 Hz), 7.82 (1H, d, J = 2.2 Hz), 12.04 (1H, br s).

Production Example 112: Synthesis of (3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone [compound No. 112]

Production Example 112-1: Synthesis of (3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone [compound No. 112]

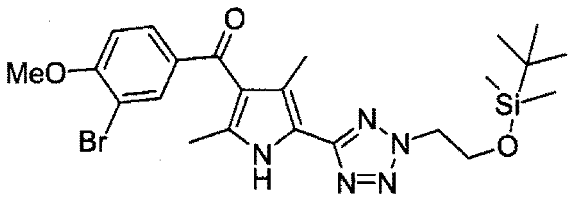

The compound of interest was obtained by the same procedures as in Production Example 97-1 using compound 108 and (2-bromoethoxy)-tert-butyldimethylsilane. Yield: 56.1 %, white solid.
$^1$H-NMR (400 MHz, DMSO-$d_6$) δ: -0.10 (6H, s), 0.73 (9H, s), 2.15 (3H, s), 2.24 (3H, s), 3.94 (3H, s), 4.13 (2H, t, J = 4.9 Hz), 4.80 (2H, t, J = 4.9 Hz), 7.22 (1H, d, J = 8.5 Hz), 7.68 (1H, dd, J = 8.5, 2.2 Hz), 7.81 (1H, d, J = 2.2 Hz), 12.05 (1H, br s).
ESI-MS m/z: 534[M+H]$^+$, 536[M+2+H]$^+$.

Production Example 113: Synthesis of (3-cyclopropylphenyl)(2,4-dimethyl-5-(1H-tetrazol-5-yl)-1H-pyrrol-3-yl)methanone [compound No. 113]

Production Example 113-1: Synthesis of 4-(3-cyclopropylbenzoyl)-3,5-dimethyl-1H-pyrrole-2-carboxylic acid

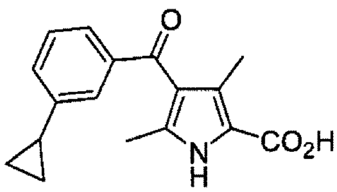

The compound of interest was obtained by the same procedures as in Production Example 96-1 using compound 36 as a starting material. Yield: quant, orange solid.
$^1$H-NMR (400 MHz, DMSO-$d_6$) δ: 0.65-0.73 (2H, m), 0.94-1.01 (2H, m), 1.96-2.03 (1H, m), 2.05 (3H, s), 2.14 (3H, s), 7.25-7.31 (2H, m), 7.32-7.38 (2H, m), 11.82 (1H, s), 12.42 (1H, s).

Production Example 113-2: Synthesis of (3-cyclopropylphenyl)(2,4-dimethyl-1H-pyrrol-3-yl)methanone

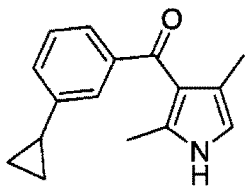

The compound of interest was obtained by the same procedures as in Production Example 96-2 using the compound obtained in Production Example 113-1. Yield: quant, brown solid.
$^1$H-NMR (400 MHz, DMSO-$d_6$) δ: 0.64-0.70 (2H, m), 0.94-1.00 (2H, m), 1.89 (3H, s), 1.95-2.03 (1H, m), 2.00 (3H, s), 6.45-6.48 (1H, m), 7.19-7.36 (4H, m), 10.96 (1H, s).

Production Example 113-3: Synthesis of 4-(3-cyclopropylbenzoyl)-3,5-dimethyl-1H-pyrrole-2-carbonitrile

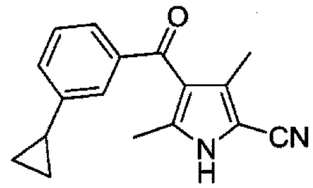

The compound of interest was obtained by the same procedures as in Production Example 96-3 using the compound obtained in Production Example 113-2. Yield: 98.3 %, pale yellow solid.
$^1$H-NMR (400 MHz, $CDCl_3$) δ: 0.70-0.76 (2H, m), 0.98-1.04 (2H, m), 1.90-1.99 (1H, m), 2.12 (3H, s), 2.26 (3H, s), 7.26-7.29 (1H, m), 7.31-7.36 (1H, m), 7.39-7.41 (1H, m), 7.42-7.46 (1H, m), 8.54 (1H, s).

Production Example 113-4: Synthesis of (3-cyclopropylphenyl)(2,4-dimethyl-5-(1H-tetrazol-5-yl)-1H-pyrrol-3-yl)methanone [compound No. 113]

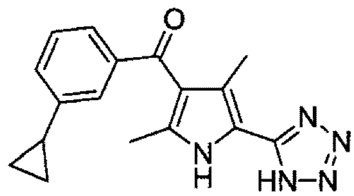

The compound of interest was obtained by the same procedures as in Production Example 96-4 using the compound obtained in Production Example 113-3. Yield: 74.3 %, pale yellow solid.
$^1$H-NMR (400 MHz, DMSO-$d_6$) δ: 0.67-0.73 (2H, m), 0.95-1.01 (2H, m), 1.97-2.06 (1H, m), 2.11 (3H, s), 2.17 (3H, s), 7.28-7.39 (4H, m), 12.01 (1H, s).

Production Example 114: Synthesis of 2-(5-(4-(3-cyclopropylbenzoyl)-3,5-dimethyl-1H-pyrrol-2-yl)ethyl acetate [compound No. 114]

Production Example 114-1: Synthesis of 2-(5-(4-(3-cyclopropylbenzoyl)-3,5-dimethyl-1H-pyrrol-2-yl)ethyl acetate [compound No. 114]

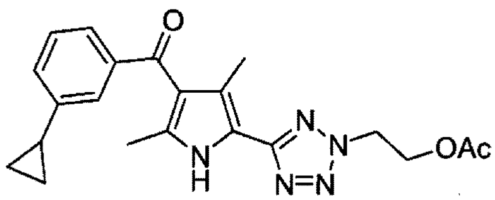

The compound of interest was obtained by the same procedures as in Production Example 97-1 using compound 113 and 2-bromoethyl acetate. Yield: 50.8 %, pale yellow solid.

$^1$H-NMR (400 MHz, $CDCl_3$) δ: 0.70-0.77 (2H, m), 0.96-1.03 (2H, m), 1.91-2.00 (1H,m), 2.05 (3H, s), 2.28 (3H, s), 2.33 (3H, s), 4.64 (2H, t, J = 5.4 Hz), 4.89 (2H, t, J = 5.4 Hz), 7.24-7.28 (1H, m), 7.30-7.35 (1H, m), 7.45-7.48 (1H, m), 7.49-7.55 (1H, m), 9.02 (1H, s).

Production Example 115: Synthesis of (3-cyclopropylphenyl)(5-(2-(2-hydroxyethyl)-2H-tetrazol-5-yl)-2,4-dimethyl-1H-pyrrol-3-yl)methanone [compound No. 115]

Production Example 115-1: Synthesis of (3-cyclopropylphenyl)(5-(2-(2-hydroxyethyl)-2H-tetrazol-5-yl)-2,4-dimethyl-1H-pyrrol-3-yl)methanone [compound No. 115]

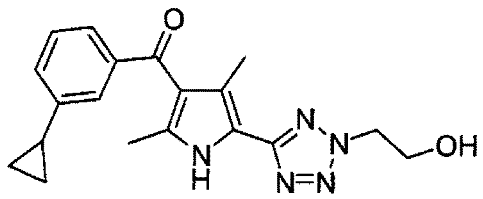

The compound of interest was obtained by the same procedures as in Production Example 97-1 using compound 113 and 2-iodoethanol. Yield: 90.6 %, pale yellow solid. $^1$H-NMR (400 MHz, $CDCl_3$) δ: 0.70-0.76 (2H, m), 0.96-1.03 (2H, m), 1.91-2.00 (1H,m), 2.28 (3H, s), 2.33 (3H, s), 4.21-4.25 (2H, m), 4.77-4.82 (2H, m), 7.22-7.27 (1H, m), 7.30-7.35 (1H, m), 7.45-7.48 (1H, m), 7.49-7.54 (1H, m), 8.99 (1H, s).

Production Example 116: Synthesis of 2-(5-(4-(3-bromobenzoyl)-5-formyl-3-methyl-1H-pyrrol-2-yl)-2H-tetrazol-2-yl)ethyl acetate [compound No. 116]

Production Example 116-1: Synthesis of 2-(5-(4-(3-bromobenzoyl)-5-formyl-3-methyl-1H-pyrrol-2-yl)-2H-tetrazol-2-yl)ethyl acetate [compound No. 116]

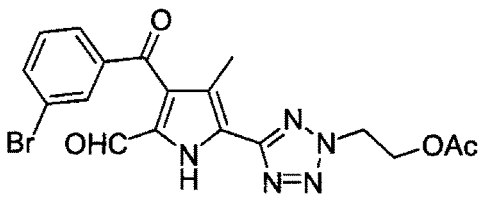

To a solution of compound 104 (300 mg, 0.69 mmol) in acetic acid (4 ml), sulfuryl chloride (112 μl, 1.38 mmol) was added in the argon atmosphere, and the mixture was heated with stirring at 50°C. After 2 hours, the reaction solution was allowed to cool. A solid was precipitated by the addition of water. The solid was collected by filtration through celite. After replacement of the receiver, the solid remaining on the celite was dissolved in chloroform. The obtained organic layer was washed with saturated saline and dried over anhydrous magnesium sulfate. Magnesium sulfate was filtered off, and the solvent was distilled off under reduced pressure to obtain a crude product. This crude product was purified by column chromatography to obtain the compound of interest (225 mg, yield: 72.6%, yellow oil).
$^1$H-NMR (400 MHz, $CDCl_3$) δ: 2.05 (3H, s), 2.38 (3H, s), 4.66 (2H, t, J = 5.2 Hz), 4.95 (2H, t, J = 5.2 Hz), 7.37-7.42 (1H, m), 7.74-7.82 (2H, m), 8.01-8.04 (1H,m), 9.52 (1H, s), 10.19 (1H, s).

Production Example 117: Synthesis of 3-(3-bromobenzoyl)-5-(2-(2-hydroxyethyl)-2H-tetrazol-5-yl)-4-methyl-1H-pyrrole-2-carboaldehyde [compound No. 117]

Production Example 117-1: Synthesis of 3-(3-bromobenzoyl)-5-(2-(2-hydroxyethyl)-2H-tetrazol-5-yl)-4-methyl-1H-pyrrole-2-carboaldehyde [compound No. 117]

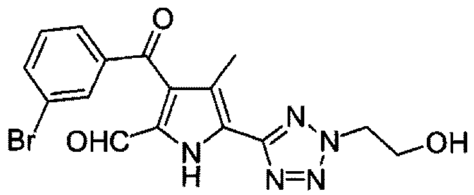

To a solution of compound 116 (280 mg, 0.63 mmol) in methanol (5 ml), a 1 M aqueous sodium hydroxide solution (1.9 ml, 1.89 mmol) was added under ice cooling. After 30 minutes, water was added to the reaction solution. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (158 mg, yield: 62.2%, pale yellow solid). $^1$H-NMR (400 MHz, $CDCl_3$) δ: 2.37 (3H, s), 4.28 (2H, t, J = 5.0 Hz), 4.86 (2H, t, J = 5.0 Hz), 7.37-7.42 (1H, m), 7.74-7.81 (2H, m), 8.01-8.04 (1H, m), 9.52 (1H,s), 10.29 (1H, s).

Production Example 118: Synthesis of 2-(5-(4-(3-bromobenzoyl)-5-(difluoromethyl)-3-methyl-1H-pyrrol-2-yl)-2H-tetrazol-2-yl)ethyl acetate [compound No. 118]

Production Example 118-1: Synthesis of 2-(5-(4-(3-bromobenzoyl)-5-(difluoromethyl)-3-methyl-1H-pyrrol-2-yl)-2H-tetrazol-2-yl)ethyl acetate [compound No. 118]

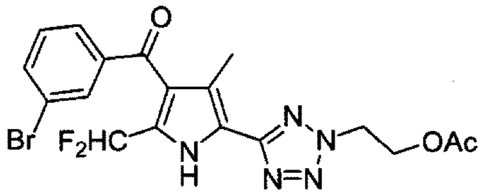

The compound of interest was obtained by the same procedures as in Production Example 57-1 using compound 116 as a starting material. Yield: 40.6 %, yellow solid. $^1$H-NMR (400 MHz, $CDCl_3$) δ: 2.05 (3H, s), 2.28 (3H, s), 4.64-4.68 (2H, m), 4.91-4.95 (2H, m), 6.81 (1H, t, J = 54.0 Hz), 7.35-7.41 (1H, m), 7.70-7.77 (2H, m), 7.91-7.95 (1H, m), 9.79 (1H, s).

Production Example 119: Synthesis of (3-bromophenyl)(2-(difluoromethyl)-5-(2-(2-hydroxyethyl)-2H-tetrazol-5-yl)-4-methyl-1H-pyrrol-3-yl)methanone [compound No. 119]

Production Example 119-1: Synthesis of (3-bromophenyl)(2-(difluoromethyl)-5-(2-(2-hydroxyethyl)-2H-tetrazol-5-yl)-4-methyl-1H-pyrrol-3-yl)methanone [compound No. 119]

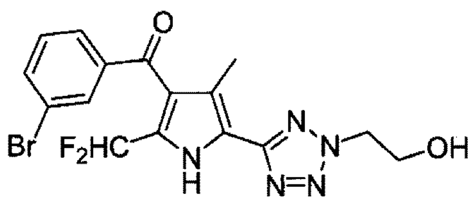

The compound of interest was obtained by the same procedures as in Production Example 117-1 using compound 118 as a starting material. Yield: 91.0 %, white solid. $^1$H-NMR (400 MHz, $CDCl_3$) δ: 2.27 (3H, s), 4.23-4.29 (2H, m), 4.81-4.85 (2H, m), 6.81 (1H, t, J = 54.0 Hz), 7.34-7.41 (1H, m), 7.70-7.76 (2H, m), 7.91-7.95 (1H, m), 9.81 (1H, s).

Production Example 120: Synthesis of 2-(5-(4-(3-bromobenzoyl)-5-(hydroxymethyl)-3-methyl-1H-pyrrol-2-yl)-2H-tetrazol-2-yl)ethyl acetate [compound No. 120]

Production Example 120-1: Synthesis of 2-(5-(4-(3-bromobenzoyl)-5-(hydroxymethyl)-3-methyl-1H-pyrrol-2-yl)-2H-tetrazol-2-yl)ethyl acetate [compound No. 120]

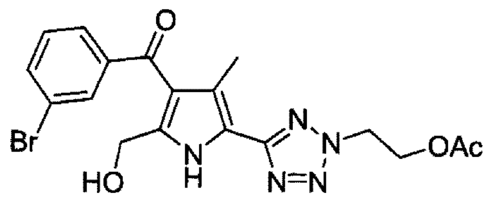

The compound of interest was obtained by the same procedures as in Production Example 58-1 using compound 116 as a starting material. Yield: 57.3 %, pale yellow solid.
$^1$H-NMR (400 MHz, $CDCl_3$) δ: 2.05 (3H, s), 2.19 (3H, s), 4.62-4.66 (2H, m), 4.72 (2H, s), 4.88-4.92 (2H, m), 7.33-7.39 (1H, m), 7.65-7.72 (2H, m), 7.86-7.89 (1H,m), 9.67 (1H, s).

Production Example 121: Synthesis of (3-bromophenyl)(5-(2-(2-hydroxyethyl)-2H-tetrazol-5-yl)-2-(hydroxymethyl)-4-methyl-1H-pyrrol-3-yl)methanone [compound No. 121]

Production Example 121-1: Synthesis of (3-bromophenyl)(5-(2-(2-hydroxyethyl)-2H-tetrazol-5-yl)-2-(hydroxymethyl)-4-methyl-1H-pyrrol-3-yl)methanone [compound No. 121]

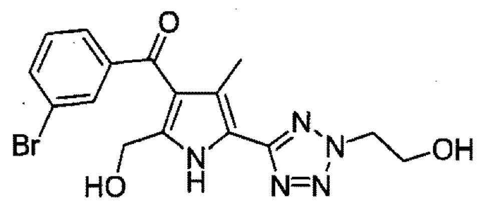

The compound of interest was obtained by the same procedures as in Production Example 117-1 using compound 120 as a starting material. Yield: 82.5 %, yellow solid. $^1$H-NMR (400 MHz, DMSO-$d_6$) δ: 2.24 (3H, s), 3.95 (2H, q, J = 5.3 Hz), 4.33 (2H, d, J = 5.4 Hz), 4.75 (2H, t, J = 5.4 Hz), 4.95 (1H, t, J = 5.6 Hz), 5.10 (1H, t, J = 5.4 Hz), 7.44-7.51 (1H, m), 7.63-7.69 (1H, m), 7.76-7.84 (2H, m), 12.06 (1H, s).

Production Example 122: Synthesis of 2-(5-(4-(3-bromobenzoyl)-5-(1-hydroxyethyl)-3-methyl-1H-pyrrol-2-yl)-2H-tetrazol-2-yl)ethyl acetate [compound No. 122]

Production Example 122-1: Synthesis of 2-(5-(4-(3-bromobenzoyl)-5-(1-hydroxyethyl)-3-methyl-1H-pyrrol-2-yl)-2H-tetrazol-2-yl)ethyl acetate [compound No. 122]

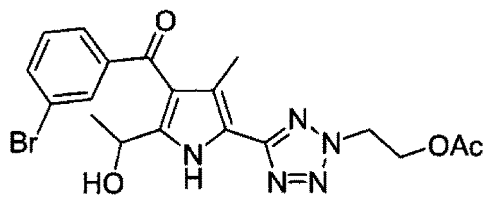

The compound of interest was obtained by the same procedures as in Production Example 61-1 using compound 116 as a starting material. Yield: 60.1 %, yellow solid. $^1$H-NMR (400 MHz, $CDCl_3$) δ: 1.61 (3H, d, J = 6.6 Hz), 2.04 (3H, s), 2.15 (3H, s), 4.62-4.67 (2H, m), 4.87-4.93 (2H, m), 5.08 (1H, q, J = 6.6 Hz), 7.34-7.39 (1H,m) 7.67-7.73 (2H, m), 7.87-7.91 (1H, m), 9.54 (1H, s).

Production Example 123: Synthesis of (3-bromophenyl)(2-(1-hydroxyethyl)-5-(2-(2-hydroxyethyl)-2H-tetrazol-5-yl)-4-methyl-1H-pyrrol-3-yl)methanone [compound No. 123]

Production Example 123-1: Synthesis of (3-bromophenyl)(2-(1-hydroxyethyl)-5-(2-(2-hydroxyethyl)-2H-tetrazol-5-yl)-4-methyl-1H-pyrrol-3-yl)methanone [compound No. 123]

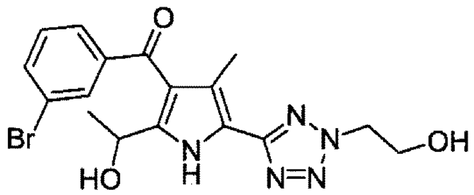

The compound of interest was obtained by the same procedures as in Production Example 117-1 using compound 122 as a starting material. Yield: quant, pale yellow solid.
$^1$H-NMR (400 MHz, DMSO-$d_6$) δ: 1.38 (3H, d, J = 6.6 Hz), 2.13 (3H, s), 3.90-3.99 (2H, m), 4.65-4.78 (3H, m), 5.01-5.22 (1H, m), 7.46-7.52 (1H, m), 7.64-7.68 (1H,m) 7.76-7.79 (1H, m), 7.80-7.84 (1H, m), 11.80 (1H, s).

Production Example 124: Synthesis of 2-(5-(4-(3-bromo-4-methoxybenzoyl)-5-formyl-3-methyl-1H-pyrrol-2-yl)-2H-tetrazol-2-yl)ethyl acetate [compound No. 124]

Production Example 124-1: Synthesis of 2-(5-(4-(3-bromo-4-methoxybenzoyl)-5-formyl-3-methyl-1H-pyrrol-2-yl)-2H-tetrazol-2-yl)ethyl acetate [compound No. 124]

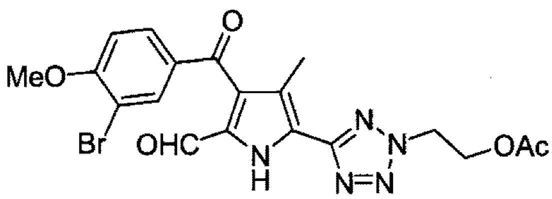

The compound of interest was obtained by the same procedures as in Production Example 116-1 using compound 111 as a starting material. Yield: 45.1 %, yellow solid. $^1$H-NMR (400 MHz, DMSO-$d_6$) δ: 1.97 (3H, s), 2.22 (3H, s), 3.95 (3H, s), 4.59 (2H, t, J = 5.1 Hz), 5.07 (2H, t, J = 5.1 Hz), 7.22 (1H, d, J = 8.8 Hz), 7.79 (1H, dd, J = 8.8, 2.1 Hz), 7.97 (1H, d, J = 2.1 Hz), 9.56 (1H, s), 13.42 (1H, br s).

Production Example 125: Synthesis of 3-(3-bromo-4-methoxybenzoyl)-5-(2-(2-hydroxyethyl)-2H-tetrazol-5-yl)-4-methyl-1H-pyrrole-2-carboaldehyde [compound No. 125]

Production Example 125-1: Synthesis of 3-(3-bromo-4-methoxybenzoyl)-5-(2-(2-hydroxyethyl)-2H-tetrazol-5-yl)-4-methyl-1H-pyrrole-2-carboaldehyde [compound No. 125]

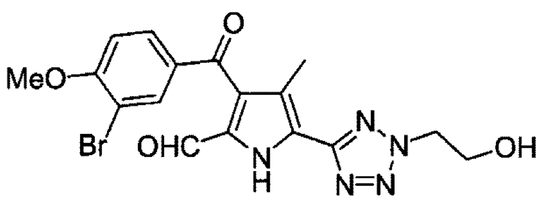

The compound of interest was obtained by the same procedures as in Production Example 117-1 using compound 124 as a starting material. Yield: 79.6 %, purple solid. $^1$H-NMR (400 MHz, DMSO-$d_6$) δ: 2.23 (3H, s), 3.95 (3H, s), 3.98 (2H, t, J = 5.1 Hz), 4.81 (2H, t, J = 5.1 Hz), 5.11 (1H, br s), 7.22 (1H, d, J = 8.5 Hz), 7.78 (1H, dd, J = 8.5, 2.2 Hz), 7.97 (1H, d, J = 2.2 Hz), 9.56 (1H, s), 13.40 (1H, br s).

Production Example 126: Synthesis of 2-(5-(4-(3-bromo-4-methoxybenzoyl)-5-(difluoromethyl)-3-methyl-1H-pyrrol-2-yl)-2H-tetrazol-2-yl)ethyl acetate [compound No. 126]

Production Example 126-1: Synthesis of 2-(5-(4-(3-bromo-4-methoxybenzoyl)-5-(difluoromethyl)-3-methyl-1H-pyrrol-2-yl)-2H-tetrazol-2-yl)ethyl acetate [compound No. 126]

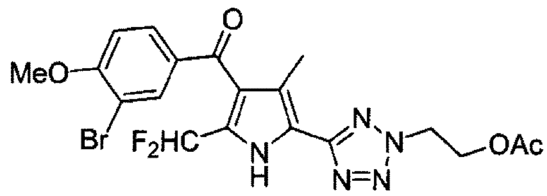

The compound of interest was obtained by the same procedures as in Production Example 57-1 using compound 124 as a starting material. Yield: 84.5 %, white solid. $^1$H-NMR (400 MHz, DMSO-$d_6$) δ: 1.96 (3H, s), 2.17 (3H, s), 3.96 (3H, s), 4.58 (2H, t, J = 5.1 Hz), 5.04 (2H, t, J = 5.1 Hz), 6.86 (1H, t, J = 53.4 Hz), 7.26 (1H,d, J = 8.5 Hz), 7.77 (1H, dd, J = 8.5, 2.2 Hz), 7.92 (1H, d, J = 2.2 Hz), 13.11 (1H, br s).

Production Example 127: Synthesis of (3-bromo-4-methoxyphenyl)(2-(difluoromethyl)-5-(2-(2-hydroxyethyl)-2H-tetrazol-5-yl)-4-methyl-1H-pyrrol-3-yl)methanone [compound No. 127]

Production Example 127-1: Synthesis of (3-bromo-4-methoxyphenyl)(2-(difluoromethyl)-5-(2-(2-hydroxyethyl)-2H-tetrazol-5-yl)-4-methyl-1H-pyrrol-3-yl)methanone [compound No. 127]

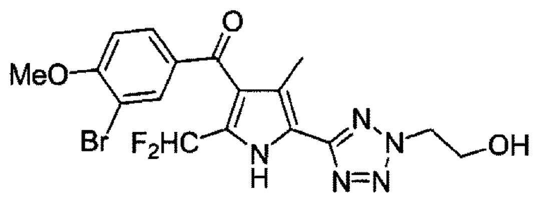

The compound of interest was obtained by the same procedures as in Production Example 117-1 using compound 126 as a starting material. Yield: 15.5 %, white solid.
$^1$H-NMR (400 MHz, DMSO-$d_6$) δ: 2.16 (3H, s), 3.92-3.94 (1H, m), 3.95 (3H, s), 3.96-3.97 (1H, m), 4.76 (2H, t, J = 5.2 Hz), 5.08 (1H, t, J = 5.6 Hz), 6.85 (1H, t, J = 53.4 Hz), 7.24 (1H, d, J = 8.8 Hz), 7.75 (1H, dd, J = 8.8, 2.2 Hz), 7.90 (1H, d, J = 2.2 Hz), 13.08 (1H, br s).

Production Example 128: Synthesis of 2-(5-(4-(3-bromo-4-methoxybenzoyl)-5-(hydroxymethyl)-3-methyl-1H-pyrrol-2-yl)-2H-tetrazol-2-yl)ethyl acetate [compound No. 128]

Production Example 128-1: Synthesis of 2-(5-(4-(3-bromo-4-methoxybenzoyl)-5-(hydroxymethyl)-3-methyl-1H-pyrrol-2-yl)-2H-tetrazol-2-yl)ethyl acetate [compound No. 128]

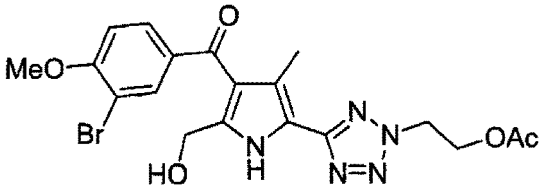

The compound of interest was obtained by the same procedures as in Production Example 58-1 using compound 124 as a starting material. Yield: 86.0 %, white solid. $^1$H-NMR (400 MHz, DMSO-$d_6$) δ: 1.96 (3H, s), 2.24 (3H, s), 3.95 (3H, s), 4.35 (2H, s), 4.57 (2H, t, J = 5.1 Hz), 4.92 (1H, br s), 5.01 (2H, t, J = 5.1 Hz), 7.22 (1H, d, J = 8.5 Hz), 7.73 (1H, dd, J = 8.5, 2.2 Hz), 7.87 (1H, d, J = 2.2 Hz), 12.02 (1H, br s).

Production Example 129: Synthesis of (3-bromo-4-methoxyphenyl)(5-(2-(2-hydroxyethyl)-2H-tetrazol-5-yl)-2-(hydroxymethyl)-4-methyl-1H-pyrrol-3-yl)methanone [compound No. 129]

Production Example 129-1: Synthesis of (3-bromo-4-methoxyphenyl)(5-(2-(2-hydroxyethyl)-2H-tetrazol-5-yl)-2-(hydroxymethyl)-4-methyl-1H-pyrrol-3-yl)methanone [compound No. 129]

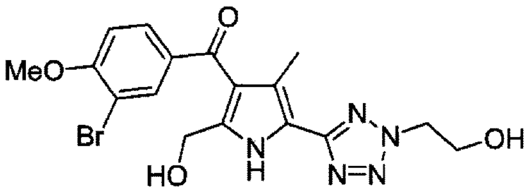

The compound of interest was obtained by the same procedures as in Production Example 117-1 using compound 128 as a starting material. Yield: 65.7 %, purple solid. $^1$H-NMR (400 MHz, DMSO-$d_6$) δ: 2.24 (3H, s), 3.95 (3H, s), 3.96 (2H, t, J = 5.6 Hz), 4.35 (2H, d, J = 5.6 Hz), 4.75 (2H, t, J = 5.2 Hz), 4.92 (1H, t, J = 5.6 Hz), 5.09 (1H, t, J = 5.6 Hz), 7.22 (1H, d, J = 8.5 Hz), 7.72 (1H, dd, J = 8.5, 2.2Hz), 7.87 (1H, d, J = 2.2 Hz), 11.99 (1H, br s).

Production Example 130: Synthesis of 2-(5-(4-(3-bromo-4-methoxybenzoyl)-5-(1-hydroxyethyl)-3-methyl-1H-pyrrol-2-yl)-2H-tetrazol-2-yl)ethyl acetate [compound No. 130]

Production Example 130-1: Synthesis of 2-(5-(4-(3-bromo-4-methoxybenzoyl)-5-(1-hydroxyethyl)-3-methyl-1H-pyrrol-2-yl)-2H-tetrazol-2-yl)ethyl acetate [compound No. 130]

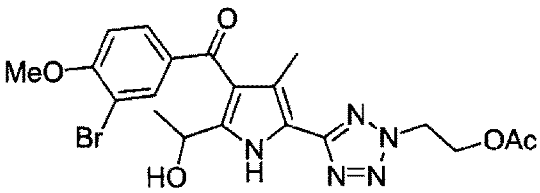

The compound of interest was obtained by the same procedures as in Production Example 61-1 using compound 124 as a starting material. Yield: 56.2 %, white solid. $^1$H-NMR (400 MHz, DMSO-$d_6$) δ: 1.38 (3H, d, J = 6.6 Hz), 1.96 (3H, s), 2.15 (3H, s), 3.95 (3H, s), 4.57 (2H, t, J = 5.1 Hz), 4.71 (1H, q, J = 6.3 Hz), 5.00 (2H, t, J = 5.1 Hz), 5.07 (1H, t, J = 5.1 Hz), 7.24 (1H, d, J = 8.5 Hz), 7.74 (1H, dd, J = 8.5, 2.2 Hz), 7.86 (1H, d, J = 2.2 Hz), 11.78 (1H, br s).

Production Example 131: Synthesis of (3-bromo-4-methoxyphenyl)(2-(1-hydroxyethyl)-5-(2-(2-hydroxyethyl)-2H-tetrazol-5-yl)-4-methyl-1H-pyrrol-3-yl)methanone [compound No. 131]

Production Example 131-1: Synthesis of (3-bromo-4-methoxyphenyl)(2-(1-hydroxyethyl)-5-(2-(2-hydroxyethyl)-2H-tetrazol-5-yl)-4-methyl-1H-pyrrol-3-yl)methanone [compound No. 131]

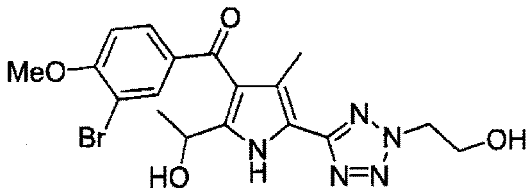

The compound of interest was obtained by the same procedures as in Production Example 117-1 using compound 130 as a starting material. Yield: 65.7 %, purple solid. $^1$H-NMR (400 MHz, DMSO-$d_6$) δ: 1.38 (3H, d, J = 6.6 Hz), 2.15 (3H, s), 3.93-3.94 (1H, m), 3.95 (3H, s), 3.96-3.97 (2H, m), 4.74 (2H, t, J = 5.2 Hz), 5.09 (2H, brs), 7.24 (1H, d, J = 8.5 Hz), 7.73 (1H, dd, J = 8.5, 2.2 Hz), 7.86 (1H, d, J = 2.2 Hz), 11.74 (1H, br s).

Production Example 132: Synthesis of 2-(5-(4-(3-bromobenzoyl)-5-cyano-3-methyl-1H-pyrrol-2-yl)-2H-tetrazol-2-yl)ethyl acetate [compound No. 132]

Production Example 132-1: Synthesis of 2-(5-(4-(3-bromobenzoyl)-5-cyano-3-methyl-1H-pyrrol-2-yl)-2H-tetrazol-2-yl)ethyl acetate [compound No. 132]

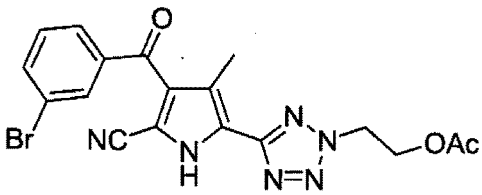

Compound 116 (164 mg, 0.37 mmol) was dissolved in pyridine (2.6 ml). To the solution, hydroxylamine hydrochloride (28 mg, 0.407 mmol) was added, and the mixture was stirred at room temperature for 7 hours. Acetic anhydride (0.077 ml, 0.814 mmol) was added thereto, and the mixture was stirred overnight at 100°C. After reaction, the solvent was distilled off. Water was added to the residue, followed by extraction with ethyl acetate. The organic layer was dried over magnesium sulfate, and the solvent was distilled off. The residue was purified by silica gel column chromatography to obtain the compound of interest (105 mg, yield: 64.3%, yellow solid). $^1$H-NMR (400 MHz, DMSO-$d_6$) δ: 1.96 (3H, s), 2.37 (3H, s), 4.58 (2H, t, J = 5.1 Hz), 5.07 (2H, t, J = 5.1 Hz), 7.53 (1H, t, J = 7.7 Hz), 7.78 (1H, d, J = 7.7 Hz), 7.88-7.91 (1H, m), 7.91-7.92 (1H, m), 14.01 (1H, br s).

Production Example 133: Synthesis of 3-(3-bromobenzoyl)-5-(2-(2-hydroxyethyl)-2H-tetrazol-5-yl)-4-methyl-1H-pyrrole-2-carbonitrile [compound No. 133]

Production Example 133-1: Synthesis of 3-(3-bromobenzoyl)-5-(2-(2-hydroxyethyl)-2H-tetrazol-5-yl)-4-methyl-1H-pyrrole-2-carbonitrile [compound No. 133]

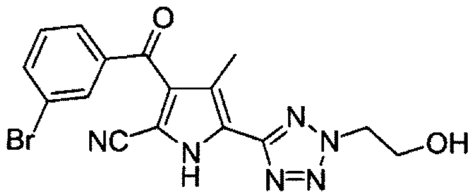

The compound of interest was obtained by the same procedures as in Production Example 117-1 using compound 132 as a starting material. Yield: 83.4 %, yellow solid. $^1$H-NMR (400 MHz, DMSO-$d_6$) δ: 2.37 (3H, s), 3.97 (2H, t, J = 5.1 Hz), 4.80 (2H, t, J = 5.1 Hz), 5.10 (1H, br s), 7.53 (1H, t, J = 7.7 Hz), 7.77 (1H, dt, J = 7.7, 1.3 Hz), 7.88-7.92 (2H, m), 14.00 (1H, br s).

Production Example 134: Synthesis of 5-(2-(2-acetoxyethyl)-2H-tetrazol-5-yl)-3-(3-bromobenzoyl)-4-methyl-1H-pyrrole-2-carboxylic acid [compound No. 134]

Production Example 134-1: Synthesis of 5-(2-(2-acetoxyethyl)-2H-tetrazol-5-yl)-3-(3-bromobenzoyl)-4-methyl-1H-pyrrole-2-carboxylic acid [compound No. 134]

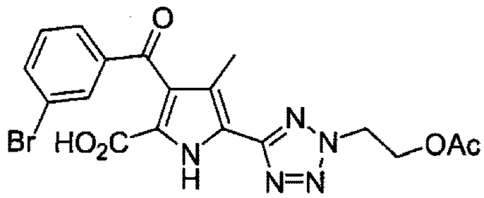

An aqueous solution (3 ml) of potassium dihydrogen phosphate (275 mg, 2.02 mmol) and subsequently an aqueous solution (3 ml) of potassium permanganate (427 mg, 2.70 mmol) were added to a solution of compound 116 (600 mg, 1.35 mmol) in tert-butyl alcohol (6 ml). After 1 hour, the reaction solution was quenched by the addition of 1 M hydrochloric acid and an aqueous sodium sulfite solution. The aqueous layer was extracted with chloroform. The obtained organic layer was dried over anhydrous magnesium sulfate. Magnesium sulfate was filtered off, and the solvent was distilled off under reduced pressure to obtain the compound of interest (611 mg, yield: 98.0%, yellow oil).
$^1$H-NMR (400 MHz, DMSO-$d_6$) δ: 1.97 (3H, s), 2.18 (3H, s), 4.58 (2H, t, J = 5.1 Hz), 5.04 (2H, t, J = 5.0 Hz), 7.45-7.51 (1H, m), 7.70-7.76 (1H, m), 7.81-7.88 (2H, m), 12.77 (1H, s), 12.88 (1H, s).

Production Example 135: Synthesis of 3-(3-bromobenzoyl)-5-(2-(2-hydroxyethyl)-2H-tetrazol-5-yl)-4-methyl-1H-pyrrole-2-carboxylic acid [compound No. 135]

Production Example 135-1: Synthesis of 3-(3-bromobenzoyl)-5-(2-(2-hydroxyethyl)-2H-tetrazol-5-yl)-4-methyl-1H-pyrrole-2-carboxylic acid [compound No. 135]

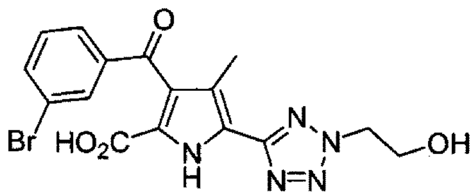

The compound of interest was obtained by the same procedures as in Production Example 117-1 using compound 134 as a starting material. Yield: 86.2 %, white solid.
$^1$H-NMR (400 MHz, DMSO-$d_6$) δ: 2.19 (3H, s), 3.97 (2H, q, J = 5.4 Hz), 4.78 (2H, t, J = 5.1 Hz), 5.09 (1H, t, J = 5.6 Hz), 7.44-7.51 (1H, m), 7.70-7.74 (1H, m), 7.81-7.88 (2H, m), 12.73 (1H, s), 12.85 (1H, s).

Production Example 136: Synthesis of methyl 5-(2-(2-acetoxyethyl)-2H-tetrazol-5-yl)-3-(3-bromobenzoyl)-4-methyl-1H-pyrrole-2-carboxylate [compound No. 136]

Production Example 136-1: Synthesis of methyl 5-(2-(2-acetoxyethyl)-2H-tetrazol-5-yl)-3-(3-bromobenzoyl)-4-methyl-1H-pyrrole-2-carboxylate compound No. 136]

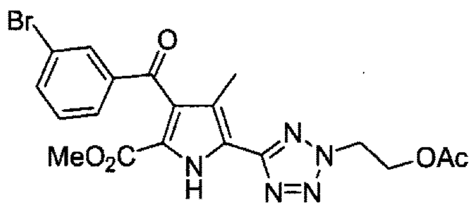

To a solution of compound 134 (200 mg, 0.44 mmol) in dichloromethane (5 ml), oxalyl chloride (38 μl, 0.44 mmol) and N,N-dimethylformamide (one drop) were added in the argon atmosphere. After 1 hour, methanol (1 ml) was added to the reaction solution. After 1 hour, the reaction solution was purified by column chromatography to obtain the compound of interest (71 mg, yield: 35.2%, yellow oil).
$^1$H-NMR (400 MHz, $CDCl_3$) δ: 2.06 (3H, s), 2.36 (3H, s), 3.62 (3H, s), 4.64-4.68 (2H, m), 4.92-4.96 (2H, m), 7.30-7.38 (1H, m), 7.68-7.72 (1H, m), 7.76-7.80 (1H,m) 7.99-8.03 (1H, m), 9.96 (1H, s).

Production Example 137: Synthesis of methyl 3-(3-bromobenzoyl)-5-(2-(2-hydroxyethyl)-2H-tetrazol-5-yl)-4-methyl-1H-pyrrole-2-carboxylate [compound No. 137]

Production Example 137-1: Synthesis of methyl 3-(3-bromobenzoyl)-5-(2-(2-hydroxyethyl)-2H-tetrazol-5-yl)-4-methyl-1H-pyrrole-2-carboxylate [compound No.137]

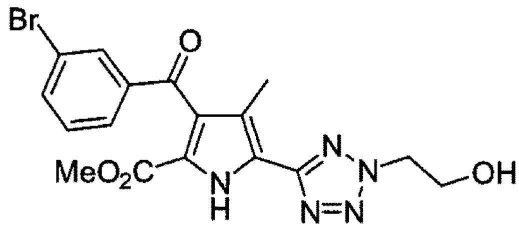

The compound of interest was obtained by the same procedures as in Production Example 117-1 using compound 136 as a starting material. Yield: 75.4 %, white solid. $^1$H-NMR (400 MHz, $CDCl_3$) δ: 2.36 (3H, s), 3.62 (3H, s), 4.27 (2H, t, J = 5.1 Hz), 4.85 (2H, t, J = 5.1 Hz), 7.31-7.36 (1H, m), 7.68-7.72 (1H, m), 7.76-7.80 (1H,m) 7.99-8.03 (1H, m), 9.96 (1H, s).

Production Example 138: Synthesis of 2-(5-(4-(3-bromo-4-methoxybenzoyl)-5-cyano-3-methyl-1H-pyrrol-2-yl)-2H-tetrazol-2-yl)ethyl acetate [compound No. 138]

Production Example 138-1: Synthesis of 2-(5-(4-(3-bromo-4-methoxybenzoyl)-5-cyano-3-methyl-1H-pyrrol-2-yl)-2H-tetrazol-2-yl)ethyl acetate [compound No. 138]

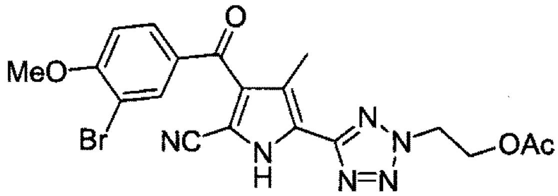

The compound of interest was obtained by the same procedures as in Production Example 132-1 using compound 124 as a starting material. Yield: 21.2 %, white solid. $^1$H-NMR (400 MHz, DMSO-$d_6$) δ: 1.96 (3H, s), 2.34 (3H, s), 3.97 (3H, s), 4.58 (2H, t, J = 5.1 Hz), 5.06 (2H, t, J = 5.1 Hz), 7.27 (1H, d, J = 8.8 Hz), 7.84 (1H, dd, J = 8.8, 2.2 Hz), 7.99 (1H, d, J = 2.2 Hz), 13.91 (1H, br s).

Production Example 139: Synthesis of 3-(3-bromo-4-methoxybenzoyl)-5-(2-(2-hydroxyethyl)-2H-tetrazol-5-yl)-4-methyl-1H-pyrrole-2-carbonitrile [compound No. 139]

Production Example 139-1: Synthesis of 3-(3-bromo-4-methoxybenzoyl)-5-(2-(2-hydroxyethyl)-2H-tetrazol-5-yl)-4-methyl-1H-pyrrole-2-carbonitrile [compound No. 139]

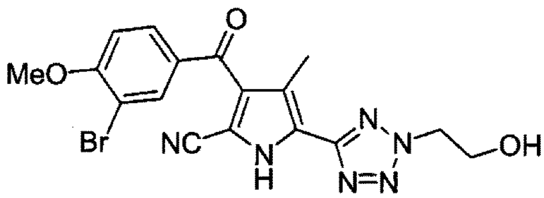

The compound of interest was obtained by the same procedures as in Production Example 117-1 using compound 138 as a starting material. Yield: 77.3 %, yellow solid. $^1$H-NMR (400 MHz, DMSO-$d_6$) δ: 2.35 (3H, s), 3.94-3.96 (2H, m), 3.97 (3H, s), 4.80 (2H, t, J = 5.2 Hz), 5.10 (1H, br s), 7.27 (1H, d, J = 8.8 Hz), 7.83 (1H, dd, J = 8.8, 2.2 Hz), 7.98 (1H, d, J = 2.2 Hz), 13.90 (1H, br s).

Production Example 140: Synthesis of 5-(2-(2-acetoxyethyl)-2H-tetrazol-5-yl)-3-(3-bromo-4-methoxybenzoyl)-4-methyl-1H-pyrrole-2-carboxylic acid [compound No. 140]

Production Example 140-1: Synthesis of 5-(2-(2-acetoxyethyl)-2H-tetrazol-5-yl)-3-(3-bromo-4-methoxybenzoyl)-4-methyl-1H-pyrrole-2-carboxylic acid [compound No. 140]

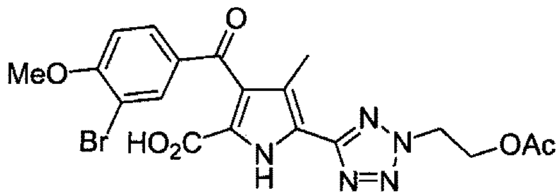

The compound of interest was obtained by the same procedures as in Production Example 134-1 using compound 124 as a starting material. Yield: 60.4 %, yellow solid. $^1$H-NMR (400 MHz, DMSO-$d_6$) δ: 1.96 (3H, s), 2.16 (3H, s), 3.92 (3H, s), 4.57 (2H, t, J = 5.1 Hz), 5.01 (2H, t, J = 5.1 Hz), 7.19 (1H, d, J = 8.8 Hz), 7.74 (1H, dd, J = 8.8, 2.2 Hz), 7.91 (1H, d, J = 2.2 Hz).

Production Example 141: Synthesis of 3-(3-bromo-4-methoxybenzoyl)-5-(2-(2-hydroxyethyl)-2H-tetrazol-5-yl)-4-methyl-1H-pyrrole-2-carboxylic acid [compound No. 141]

Production Example 141-1: Synthesis of 3-(3-bromo-4-methoxybenzoyl)-5-(2-(2-hydroxyethyl)-2H-tetrazol-5-yl)-4-methyl-1H-pyrrole-2-carboxylic acid [compound No. 141]

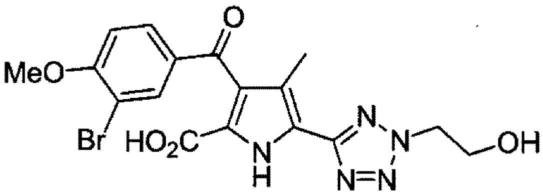

The compound of interest was obtained by the same procedures as in Production Example 117-1 using compound 140 as a starting material. Yield: quant., yellow solid. $^1$H-NMR (400 MHz, DMSO-$d_6$) δ: 2.16 (3H, s), 3.94 (3H, s), 3.95-3.99(2H, m), 4.78(2H, t, J = 5.1 Hz), 5.10 (1H, t, J = 5.7 Hz), 7.22 (1H, d, J = 8.8 Hz), 7.73 (1H, dd, J = 8.8, 2.2 Hz), 7.92 (1H, d, J = 2.2 Hz), 12.65 (1H, br s), 12.75 (1H,br s).

Production Example 142: Synthesis of 3-(3-bromobenzoyl)-5-(2-(2-hydroxyethyl)-2H-tetrazol-5-yl)-N,4-dimethyl-1H-pyrrole-2-carboxamide [compound No. 142]

Production Example 142-1: Synthesis of 3-(3-bromobenzoyl)-5-(2-(2-hydroxyethyl)-2H-tetrazol-5-yl)-N,4-dimethyl-1H-pyrrole-2-carboxamide [compound No. 142]

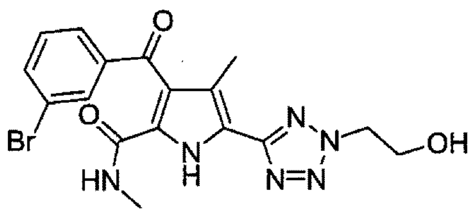

To a solution of compound 134 (50 mg, 0.11 mmol) in N,N-dimethylformamide (3 ml), 1-ethyl-3-(3-dimethylaminopropyl)carbodiimide hydrochloride (25 mg, 0.13 mmol) and 1-hydroxybenzotriazole (18 mg, 0.13 mmol) were added in the argon atmosphere. After 1 hour, methylamine hydrochloride (9 mg, 0.13 mmol) and triethylamine (18 μl, 0.13 mmol) were added to the mixture. After 12 hours, the reaction solution was quenched by the addition of water. The aqueous layer was extracted with chloroform. The obtained organic layer was dried over anhydrous magnesium sulfate, and magnesium sulfate was filtered off. The solvent was distilled off under reduced pressure to obtain a crude product. This crude product was dissolved in methanol (3 ml). To the solution, a 1 M aqueous sodium hydroxide solution (390 μl, 0.39 mmol) was added under ice cooling. After 30 minutes, water was added to the reaction solution, followed by extraction with chloroform. The obtained organic layer was dried over anhydrous magnesium sulfate, and magnesium sulfate was filtered off. The solvent was distilled off under reduced pressure. The residue was purified by column chromatography to obtain the compound of interest (34 mg, yield: 72.7%, white solid).
$^1$H-NMR (400 MHz, DMSO-$d_6$) δ: 2.25 (3H, s), 2.56 (3H, d, J = 4.6 Hz), 3.97 (2H, q, J = 5.3 Hz), 4.78 (2H, t, J = 5.4 Hz), 5.10 (1H, t, J = 5.6 Hz), 7.40-7.46 (1H, m), 7.62-7.66 (1H, m), 7.75-7.82 (2H, m), 8.26-8.34 (1H, m), 12.41 (1H, s).

Production Example 143: Synthesis of 3-(3-bromobenzoyl)-5-(2-(2-hydroxyethyl)-2H-tetrazol-5-yl)-N,N,4-trimethyl-1H-pyrrole-2-carboxamide [compound No. 143]

Production Example 143-1: Synthesis of 3-(3-bromobenzoyl)-5-(2-(2-hydroxyethyl)-2H-tetrazol-5-yl)-N,N,4-trimethyl-1H-pyrrole-2-carboxamide [compound No. 143]

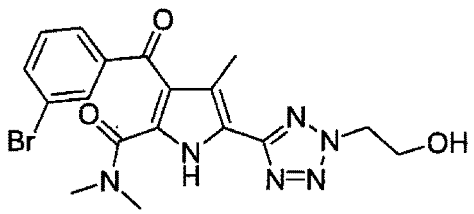

The compound of interest was obtained by the same procedures as in Production Example 142-1 using dimethylamine hydrochloride. Yield: 58.0%, white solid. $^1$H-NMR (400 MHz, DMSO-$d_6$) δ: 2.45 (3H, s), 2.56 (3H, s), 2.91 (3H, s), 3.96 (2H, q, J = 5.2 Hz), 4.77 (2H, t, J = 5.2 Hz), 5.10 (1H, t, J = 5.6 Hz), 7.41-7.46 (1H, m), 7.58-7.63 (1H, m), 7.71-7.74 (1H, m), 7.75-7.80 (1H, m), 12.70 (1H, s).

Production Example 144: Synthesis of N-(2-aminoethyl)-3-(3-bromobenzoyl)-5-(2-(2-hydroxyethyl)-2H-tetrazol-5-yl)-4-methyl-1H-pyrrole-2-carboxamide [compound No. 144]

Production Example 144-1: Synthesis of N-(2-aminoethyl)-3-(3-bromobenzoyl)-5-(2-(2-hydroxyethyl)-2H-tetrazol-5-yl)-4-methyl-1H-pyrrole-2-carboxamide [compound No. 144]

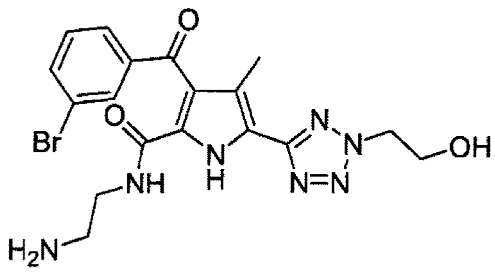

The compound of interest was obtained by the same procedures as in Production Example 142-1 and Production Example 48-1 using N-(2-aminoethyl)carbamic acid tert-butyl ester. Yield: 72.1%, yellow solid.
$^1$H-NMR (400 MHz, DMSO-$d_6$) δ: 2.26 (3H, s), 2.60-2.66 (2H, m), 3.09-3.16 (2H, m), 3.97 (2H, t, J = 5.1 Hz), 4.78 (2H, t, J = 5.1 Hz), 7.40-7.46 (1H, m), 7.64-7.69 (1H, m), 7.76-7.83 (2H, m), 8.39-8.46 (1H, m).

Production Example 145: Synthesis of 3-(3-bromobenzoyl)-N-ethyl-5-(2-(2-hydroxyethyl)-2H-tetrazol-5-yl)-4-methyl-1H-pyrrole-2-carboxamide [compound No. 145]

Production Example 145-1: Synthesis of 3-(3-bromobenzoyl)-N-ethyl-5-(2-(2-hydroxyethyl)-2H-tetrazol-5-yl)-4-methyl-1H-pyrrole-2-carboxamide [compound No. 145]

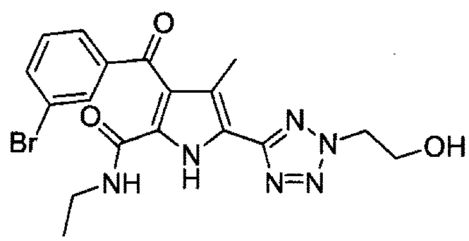

The compound of interest was obtained by the same procedures as in Production Example 142-1 using ethylamine hydrochloride. Yield: 38.6 %, white solid.
$^1$H-NMR (400 MHz, DMSO-$d_6$) δ: 0.96 (3H, t, J = 7.2 Hz), 2.26 (3H, s), 3.04 (2H, dq, J = 7.2, 5.2 Hz), 3.97 (2H, q, J = 5.4 Hz), 4.78 (2H, t, J = 5.1 Hz), 5.10 (1H, t, J = 5.7 Hz), 7.39-7.45 (1H, m), 7.63-7.67 (1H, m), 7.75-7.81 (2H, m), 8.30 (1H, t, J = 5.2 Hz), 12.44 (1H, s).

Production Example 146: Synthesis of 3-(3-bromobenzoyl)-5-(2-(2-hydroxyethyl)-2H-tetrazol-5-yl)-N-isopropyl-4-methyl-1H-pyrrole-2-carboxamide [compound No. 146]

Production Example 146-1: Synthesis of 3-(3-bromobenzoyl)-5-(2-(2-hydroxyethyl)-2H-tetrazol-5-yl)-N-isopropyl-4-methyl-1H-pyrrole-2-carboxamide [compound No. 146]

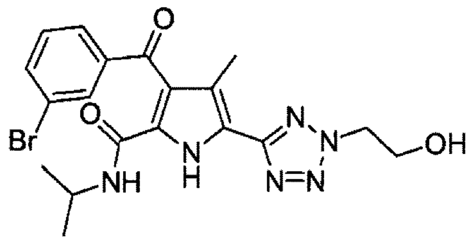

The compound of interest was obtained by the same procedures as in Production Example 142-1 using isopropyl amine hydrochloride. Yield: 41.4 %, white solid.
$^1$H-NMR (400 MHz, DMSO-$d_6$) δ: 1.00 (3H, s), 1.02 (3H, s), 2.26 (3H, s), 3.69-3.79 (1H, m), 3.97 (2H, q, J = 5.4 Hz), 4.78 (2H, t, J = 5.2 Hz), 5.10 (1H, t, J = 5.6 Hz), 7.39-7.45 (1H, m), 7.63-7.68 (1H, m), 7.75-7.81 (2H, m), 8.17 (1H, d, J= 7.3 Hz), 12.46 (1H, s).

Production Example 147: Synthesis of (3-(3-bromobenzoyl)-5-(2-(2-hydroxyethyl)-2H-tetrazol-5-yl)-4-methyl-1H-pyrrol-2-yl)(4-methylpiperazin-1-yl)methanone [compound No. 147]

Production Example 147-1: Synthesis of (3-(3-bromobenzoyl)-5-(2-(2-hydroxyethyl)-2H-tetrazol-5-yl)-4-methyl-1H-pyrrol-2-yl)(4-methylpiperazin-1-yl)methanone [compound No. 147]

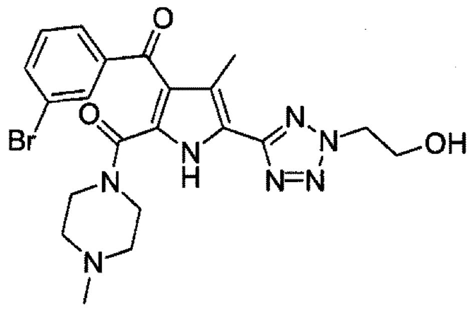

The compound of interest was obtained by the same procedures as in Production Example 142-1 using 1-methylpiperazine. Yield: 56.1 %, pale yellow solid.
$^1$H-NMR (400 MHz, DMSO-$d_6$) δ: 2.08-2.21 (8H, m), 2.43 (3H, s), 3.20 (3H, s), 3.96 (2H, q, J = 5.3 Hz), 4.77 (2H, t, J = 5.2 Hz), 5.09 (1H, t, J = 5.5 Hz), 7.41-7.46 (1H, m), 7.60-7.64 (1H, m), 7.72-7.75 (1H, m), 7.76-7.80 (1H, m), 12.71 (1H, s).

Production Example 148: Synthesis of (3-(3-bromobenzoyl)-5-(2-(2-hydroxyethyl)-2H-tetrazol-5-yl)-4-methyl-1H-pyrrol-2-yl)(piperazin-1-yl)methanone [compound No. 148]

Production Example 148-1: Synthesis of (3-(3-bromobenzoyl)-5-(2-(2-hydroxyethyl)-2H-tetrazol-5-yl)-4-methyl-1H-pyrrol-2-yl)(piperazin-1-yl)methanone [compound No. 148]

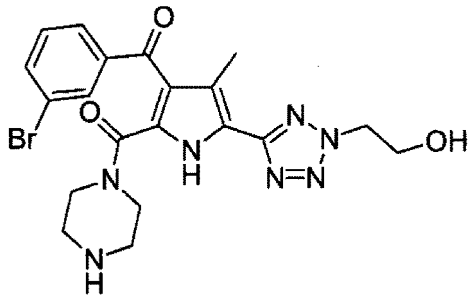

The compound of interest was obtained by the same procedures as in Production Example 142-1 and Production Example 48-1 using 1-(tert-butoxycarbonyl)piperazine. Yield: 67.0 %, yellow solid.
$^1$H-NMR (400 MHz, DMSO-$d_6$) δ: 2.41 (3H, s), 2.72-2.87 (2H, m), 3.96 (2H, q, J = 5.4 Hz), 4.77 (2H, t, J = 5.2 Hz), 5.09 (1H, t, J = 5.6 Hz), 7.41-7.47 (1H, m), 7.61-7.66 (1H, m), 7.74-7.82 (2H, m).

Production Example 149: Synthesis of 3-(3-bromo-4-methoxybenzoyl)-5-(2-(2-hydroxyethyl)-2H-tetrazol-5-yl)-N,4-dimethyl-1H-pyrrole-2-carboxamide [compound No. 149]

Production Example 149-1: Synthesis of 3-(3-bromo-4-methoxybenzoyl)-5-(2-(2-hydroxyethyl)-2H-tetrazol-5-yl)-N,4-dimethyl-1H-pyrrole-2-carboxamide [compound No. 149]

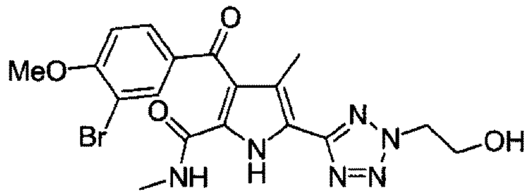

The compound of interest was obtained by the same procedures as in Production Example 142-1 using compound 140 as a starting material. Yield: 59.8 %, white solid. $^1$H-NMR (400 MHz, DMSO-$d_6$) δ: 2.20 (3H, s), 2.61 (3H, d, J = 4.4 Hz), 3.92 (3H, s), 3.95-3.99 (2H, m), 4.78 (2H, t, J = 5.1 Hz), 5.11 (1H, t, J = 5.6 Hz), 7.17 (1H, d, J = 8.8 Hz), 7.67 (1H, dd, J = 8.8, 2.1 Hz), 7.87 (1H, d, J = 2.1 Hz), 8.28 (1H, q, J = 4.4 Hz), 12.33 (1H, br s).

Production Example 150: Synthesis of 3-(3-bromo-4-methoxybenzoyl)-5-(2-(2-hydroxyethyl)-2H-tetrazol-5-yl)-4-methyl-1H-pyrrole-2-carboxamide [compound No. 150]

Production Example 150-1: Synthesis of 3-(3-bromo-4-methoxybenzoyl)-5-(2-(2-hydroxyethyl)-2H-tetrazol-5-yl)-4-methyl-1H-pyrrole-2-carboxamide [compound No. 150]

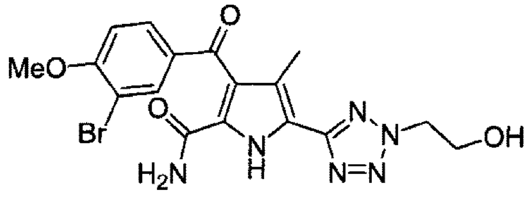

The compound of interest was obtained by the same procedures as in Production Example 142-1 using compound 140 and ammonia water. Yield: 6.1 %, yellow solid.
$^1$H-NMR (400 MHz, DMSO-$d_6$) δ: 2.18 (3H, s), 3.92 (3H, s), 3.95-3.99 (2H, m), 4.78 (2H, t, J = 5.0 Hz), 5.10 (1H, t, J = 5.6 Hz), 7.17 (1H, d, J = 8.7 Hz), 7.31 (1H, br s), 7.68 (1H, dd, J = 8.7, 2.1 Hz), 7.76 (1H, br s), 7.87 (1H, d, J = 2.1 Hz), 12.34 (1H, br s).

Production Example 151: Synthesis of 2-(5-(4-(3-bromobenzoyl)-3,5-dimethyl-1H-pyrrol-2-yl)-2H-tetrazol-2-yl)ethyl 2-((tert-butoxycarbonyl)amino)acetate [compound No. 151]

Production Example 151-1: Synthesis of 2-(5-(4-(3-bromobenzoyl)-3,5-dimethyl-1H-pyrrol-2-yl)-2H-tetrazol-2-yl)ethyl 2-((tert-butoxycarbonyl)amino)acetate [compound No. 151]

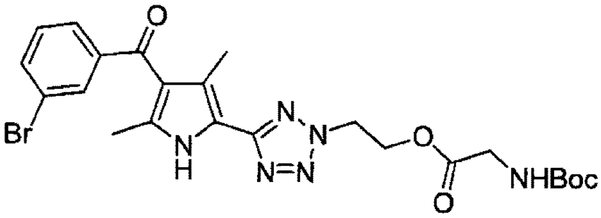

To a solution of compound 102 (42 mg, 0.11 mmol) in dichloromethane (3 ml), 1-ethyl-3-(3-dimethylaminopropyl)carbodiimide hydrochloride (23 mg, 0.12 mmol), 4-dimethylaminopyridine (14 mg, 0.11 mmol), and Boc-glycine (20 mg, 0.11 mmol) were added at 0°C in the argon atmosphere. After 1 hour, the reaction temperature was increased to room temperature. After 17 hours, the reaction solution was directly purified by column chromatography to obtain the compound of interest (50 mg, yield: 83.0%, yellow oil).
$^1$H-NMR (400 MHz, $CDCl_3$) δ: 1.45 (9H, s), 2.32 (3H, s), 2.34 (3H, s), 3.88-3.95 (2H, m), 4.69 (2H, t, J = 5.1 Hz), 4.93 (2H, t, J = 5.1 Hz), 4.94-5.02 (1H, m), 7.31-7.37 (1H, m), 7.64-7.70 (2H, m), 7.87-7.90 (1H, m), 9.47 (1H, s).

Production Example 152: Synthesis of 2-(5-(4-(3-bromobenzoyl)-3,5-dimethyl-1H-pyrrol-2-yl)-2H-tetrazol-2-yl)ethyl 2-aminoacetate [compound No. 152]

Production Example 152-1: Synthesis of 2-(5-(4-(3-bromobenzoyl)-3,5-dimethyl-1H-pyrrol-2-yl)-2H-tetrazol-2-yl)ethyl 2-aminoacetate [compound No. 152]

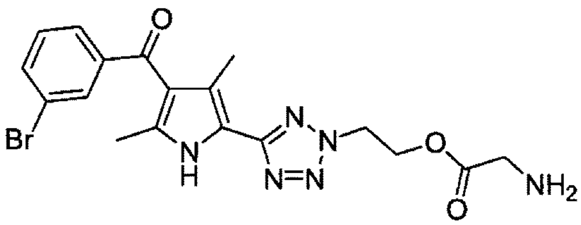

Trifluoroacetic acid (1 ml) was added to a solution of compound 151 (50 mg, 0.09 mmol) in dichloromethane (3 ml). After 30 minutes, saturated saline was added to the reaction solution. The aqueous layer was washed with chloroform. The obtained organic layer was extracted with 1 M hydrochloric acid. The obtained aqueous layer was combined with the preceding washed aqueous layer. After addition of chloroform, the reaction solution was rendered weakly basic by the addition of a saturated aqueous solution of sodium bicarbonate under ice cooling. This aqueous layer was extracted with chloroform. The obtained organic layer was dried over anhydrous magnesium sulfate. Magnesium sulfate was filtered off, and the organic layer was distilled under reduced pressure to obtain the compound of interest (31 mg, yield: 75.9%, yellow solid).
$^1$H-NMR (400 MHz, DMSO-$d_6$) δ: 2.13 (3H, s), 2.25 (3H, s), 3.21 (2H, s), 4.60 (2H, t, J = 5.0 Hz), 5.00 (2H, t, J = 5.1 Hz), 7.45-7.50 (1H, m), 7.59-7.63 (1H, m), 7.72-7.75 (1H, m), 7.78-7.82 (1H, m), 12.16 (1H, s).

Production Example 153: Synthesis of 2-(5-(4-(3-bromobenzoyl)-5-formyl-3-methyl-1H-pyrrol-2-yl)-2H-tetrazol-2-yl)ethyl 2-((tert-butoxycarbonyl)amino)acetate [compound No. 153]

Production Example 153-1: Synthesis of 2-(5-(4-(3-bromobenzoyl)-5-formyl-3-methyl-1H-pyrrol-2-yl)-2H-tetrazol-2-yl)ethyl 2-((tert-butoxycarbonyl)amino)acetate [compound No. 153]

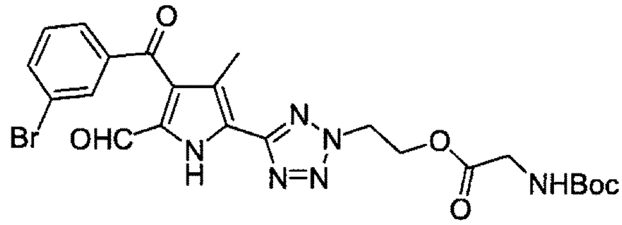

The compound of interest was obtained by the same procedures as in Production Example 151-1 using compound 125 as a starting material. Yield: 93.7 %, light red solid.
$^1$H-NMR (400 MHz, $CDCl_3$) δ: 1.44 (9H, s), 2.38 (3H, s), 3.86-3.95 (2H, m), 4.74 (2H, t, J = 5.1 Hz), 4.89-5.01 (1H, m), 4.97 (2H, t, J = 5.2 Hz), 7.35-7.43 (1H,m) 7.73-7.83 (2H, m), 8.00-8.05 (1H, m), 9.53 (1H, s), 10.31 (1H, s).

Production Example 154: Synthesis of 2-(5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate [compound No. 154]

Production Example 154-1: Synthesis of 2-(5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate [compound No. 154]

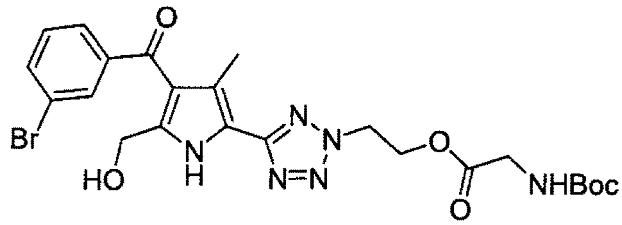

The compound of interest was obtained by the same procedures as in Production Example 58-1 using compound 153 as a starting material. Yield: 75.4 %, white oil. $^1$H-NMR (400 MHz, $CDCl_3$) δ: 1.44 (9H, s), 2.38 (3H, s), 3.86-3.95 (2H, m), 4.74 (2H, t, J = 5.1 Hz), 4.89-5.01 (1H, m), 4.97 (2H, t, J = 5.2 Hz), 7.35-7.43 (1H,m) 7.73-7.83 (2H, m), 8.00-8.05 (1H, m), 9.53 (1H, s), 10.31 (1H, s).

Production Example 155: Synthesis of 2-(5-(4-(3-bromobenzoyl)-5-(hydroxymethyl)-3-methyl-1H-pyrrol-2-yl)-2H-tetrazol-2-yl)ethyl 2-aminoacetate [compound No. 155]

Production Example 155-1: Synthesis of 2-(5-(4-(3-bromobenzoyl)-5-(hydroxymethyl)-3-methyl-1H-pyrrol-2-yl)-2H-tetrazol-2-yl)ethyl 2-aminoacetate [compound No. 155]

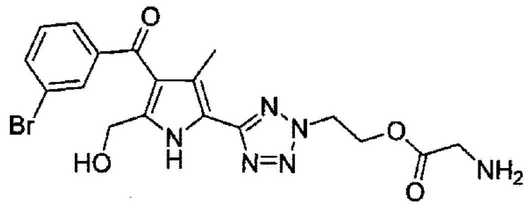

The compound of interest was obtained by the same procedures as in Production Example 152-1 using compound 154 as a starting material. Yield: trace, yellow solid. $^1$H-NMR (400 MHz, DMSO-$d_6$) δ: 2.22 (3H, s), 3.21 (2H, s), 4.28-4.35 (2H, m), 4.60 (2H, t, J = 5.0 Hz), 4.92-4.98 (1H, m), 5.01 (2H, t, J = 5.1 Hz), 7.43-7.49 (1H, m), 7.62-7.67 (1H, m), 7.76-7.83 (2H, m).

Production Example 156: Synthesis of methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate [compound No. 156]

Production Example 156-1: Synthesis of methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate [compound No. 156]

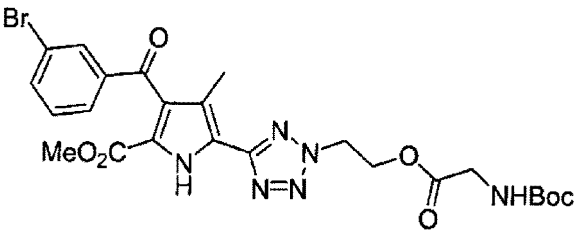

The compound of interest was obtained by the same procedures as in Production Example 151-1 using compound 137 as a starting material. Yield: 96.2 %, colorless oil. $^1$H-NMR (400 MHz, $CDCl_3$) δ: 1.44 (9H, s), 2.36 (3H, s), 3.62 (3H, s), 3.88-3.94 (2H, m), 4.74 (2H, t, J = 5.2 Hz), 4.93-4.98 (1H, m), 4.96 (2H, t, J = 5.1 Hz), 7.31-7.36 (1H, m), 7.67-7.72 (1H, m), 7.76-7.80 (1H, m), 8.00-8.04 (1H, m), 10.01 (1H, s).

Production Example 157: Synthesis of methyl 5-(2-(2-(2-aminoacetoxy)ethyl)-2H-tetrazol-5-yl)-3-(3-bromobenzoyl)-4-methyl-1H-pyrrole-2-carboxylate [compound No. 157]

Production Example 157-1: Synthesis of methyl 5-(2-(2-(2-aminoacetoxy)ethyl)-2H-tetrazol-5-yl)-3-(3-bromobenzoyl)-4-methyl-1H-pyrrole-2-carboxylate [compound No. 157]

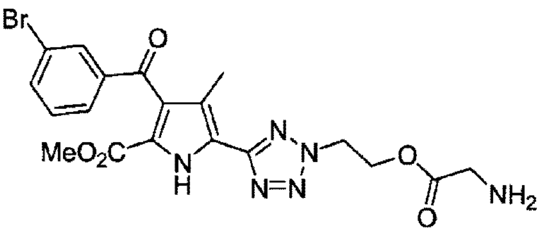

The compound of interest was obtained by the same procedures as in Production Example 152-1 using compound 156 as a starting material. Yield: 69.6 %, white solid. $^1$H-NMR (400 MHz, DMSO-$d_6$) δ: 2.21 (3H, s), 3.27 (2H, s), 3.48 (3H, s), 4.63 (2H, t, J = 5.1 Hz), 5.06 (2H, t, J = 5.0 Hz), 7.47-7.52 (1H, m), 7.67-7.72 (1H, m), 7.83-7.88 (2H, m).

Production Example 158: Synthesis of 2-(5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate [compound No. 158]

Production Example 158-1: Synthesis of 2-(5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate [compound No. 158]

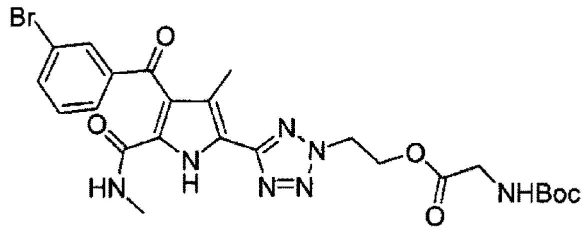

The compound of interest was obtained by the same procedures as in Production Example 151-1 using compound 142 as a starting material. Yield: 76.0 %, white solid.
$^1$H-NMR(400 MHz, $CDCl_3$) δ: 1.43 (9H, s), 2.09 (3H, s), 2.99 (3H, d, J = 4.9 Hz), 3.86-3.93 (2H, m), 4.73 (2H, t, J = 5.2 Hz), 4.91-4.96 (1H, m), 4.94 (2H, t, J= 5.2 Hz), 7.34-7.42 (1H, m), 7.65-7.72 (1H, m), 7.72-7.78 (1H, m), 7.89-7.95 (1H, m), 8.92 (1H, s), 10.51 (1H, s).

Production Example 159: Synthesis of 2-(5-(4-(3-bromobenzoyl)-3-methyl-5-(methylcarbamoyl)-1H-pyrrol-2-yl)-2H-tetrazol-2-yl)ethyl 2-aminoacetate [compound No. 159]

Production Example 159-1: Synthesis of 2-(5-(4-(3-bromobenzoyl)-3-methyl-5-(methylcarbamoyl)-1H-pyrrol-2-yl)-2H-tetrazol-2-yl)ethyl 2-aminoacetate [compound No. 159]

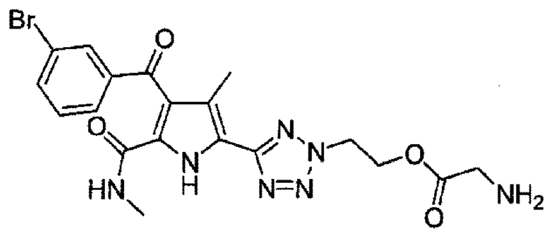

The compound of interest was obtained by the same procedures as in Production Example 152-1 using compound 158 as a starting material. Yield: 66.4 %, white solid. $^1$H-NMR (400 MHz, DMSO-$d_6$) δ: 2.25 (3H, s), 2.55 (3H, d, J = 4.6 Hz), 4.65 (2H, t, J = 4.9 Hz), 5.06 (2H, t, J = 4.9 Hz), 7.39-7.47 (1H, m), 7.62-7.68 (1H, m), 7.75-7.82 (2H, m), 8.27-8.34 (1H, m).

Production Example 160: Synthesis of 2-(5-(3,5-dimethyl-4-(3-vinylbenzoyl)-1H-pyrrol-2-yl)-2H-tetrazol-2-yl)ethyl acetate [compound No. 160]

Production Example 160-1: Synthesis of 2-(5-(3,5-dimethyl-4-(3-vinylbenzoyl)-1H-pyrrol-2-yl)-2H-tetrazol-2-yl)ethyl acetate [compound No. 160]

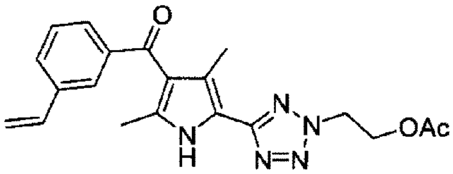

The compound of interest was obtained by the same procedures as in Production Example 83-1 using compound 104 as a starting material. Yield: 61.1 %, pale yellow solid.
$^1$H-NMR (400 MHz, $CDCl_3$) δ: 2.05 (3H, s), 2.29 (3H, s), 2.34 (3H, s), 4.64 (2H, t, J = 5.2 Hz), 4.89 (2H, t, J = 5.4 Hz), 5.31 (1H, d, J = 11.0 Hz), 5.81 (1H, d, J = 17.6 Hz), 6.76 (1H, dd, J = 17.6, 11.0 Hz), 7.39-7.44 (1H, m), 7.57-7.61 (1H, m), 7.62-7.66 (1H, m), 7.78-7.81 (1H, m), 9.11 (1H, s).

Production Example 161: Synthesis of 2-(5-(4-(3-formylbenzoyl)-3,5-dimethyl-1H-pyrrol-2-yl)-2H-tetrazol-2-yl)ethyl acetate [compound No. 161]

Production Example 161-1: Synthesis of 2-(5-(4-(3-formylbenzoyl)-3,5-dimethyl-1H-pyrrol-2-yl)-2H-tetrazol-2-yl)ethyl acetate [compound No. 161]

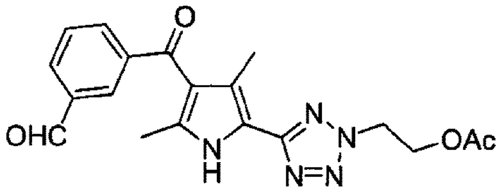

The compound of interest was obtained by the same procedures as in Production Example 87-1 using compound 160 as a starting material. Yield: 41.1 %, pale yellow solid.
$^1$H-NMR (400 MHz, $CDCl_3$) δ: 2.05 (3H, s), 2.31 (3H, s), 2.32 (3H, s), 4.64 (2H, t, J = 5.1 Hz), 4.89 (2H, t, J = 5.2 Hz), 7.63-7.69 (1H, m), 8.02-8.10 (2H, m), 8.22-8.26 (1H, m), 9.09 (1H, s), 10.09 (1H, s).

Production Example 162: Synthesis of 2-(5-(4-(3-(hydroxymethyl)benzoyl)-3,5-dimethyl-1H-pyrrol-2-yl)-2H-tetrazol-2-yl)ethyl acetate [compound No. 162]

Production Example 162-1: Synthesis of 2-(5-(4-(3-(hydroxymethyl)benzoyl)-3,5-dimethyl-1H-pyrrol-2-yl)-2H-tetrazol-2-yl)ethyl acetate [compound No. 162]

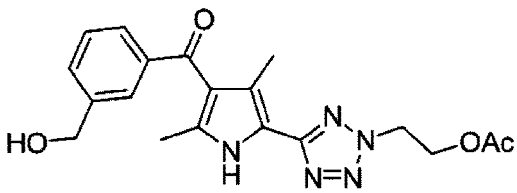

The compound of interest was obtained by the same procedures as in Production Example 58-1 using compound 161 as a starting material. Yield: 67.4 %, pale yellow solid.
$^1$H-NMR (400 MHz, $CDCl_3$) δ: 2.05 (3H, s), 2.29 (3H, s), 2.32 (3H, s), 4.61-4.66 (2H, m), 4.77 (2H, s), 4.86-4.91 (2H, m), 7.42-7.48 (1H, m), 7.54-7.58 (1H, m), 7.66-7.70 (1H, m), 7.75-7.78 (1H, m), 9.04 (1H, s).

Production Example 163: Synthesis of (5-(2-(2-hydroxyethyl)-2H-tetrazol-5-yl)-2,4-dimethyl-1H-pyrrol-3-yl)(3-(hydroxymethyl)phenyl)methanone [compound No. 163]

Production Example 163-1: Synthesis of (5-(2-(2-hydroxyethyl)-2H-tetrazol-5-yl)-2,4-dimethyl-1H-pyrrol-3-yl)(3-(hydroxymethyl)phenyl)methanone [compound No. 163]

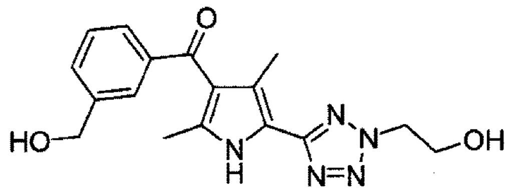

The compound of interest was obtained by the same procedures as in Example 117-1 using compound 162 as a starting material. Yield: 96.1 %, pale yellow solid. $^1$H-NMR (400 MHz, DMSO-$d_6$) δ: 2.12 (3H, s), 2.25 (3H, s), 3.89-3.99 (2H, m), 4.56 (2H, s), 4.69-4.75 (2H, m), 5.01-5.14 (1H, m), 5.21-5.38 (1H, m), 7.41-7.47 (1H, m), 7.48-7.53 (2H, m), 7.58-7.61 (1H, m), 12.01 (1H, s).

Production Example 164: Synthesis of (5-(2-ethyl-2H-tetrazol-5-yl)-2,4-dimethyl-1H-pyrrol-3-yl)(3-hydroxyphenyl)methanone [compound No. 164]

Production Example 164-1: Synthesis of (5-(2-ethyl-2H-tetrazol-5-yl)-2,4-dimethyl-1H-pyrrol-3-yl)(3-hydroxyphenyl)methanone [compound No. 164]

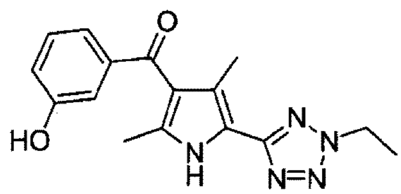

The compound of interest was obtained by the same procedures as in Production Example 91-1 using compound 106 as a starting material. Yield: 84.2 %, yellow solid. $^1$H-NMR (400 MHz, DMSO-$d_6$) δ: 1.56 (3H, t, J = 7.3 Hz), 2.12 (3H, s), 2.25 (3H, s), 4.73 (2H, q, J = 7.3 Hz), 6.93-6.99 (1H, m), 7.00-7.08 (2H, m), 7.26-7.32 (1H, m), 9.68 (1H, s), 12.01 (1H, s).

Production Example 165: Synthesis of (3-(2-aminoethoxy)phenyl)(5-(2-ethyl-2H-tetrazol-5-yl)-2,4-dimethyl-1H-pyrrol-3-yl)methanone [compound No. 165]

Production Example 165-1: Synthesis of (3-(2-aminoethoxy)phenyl)(5-(2-ethyl-2H-tetrazol-5-yl)-2,4-dimethyl-1H-pyrrol-3-yl)methanone [compound No. 165]

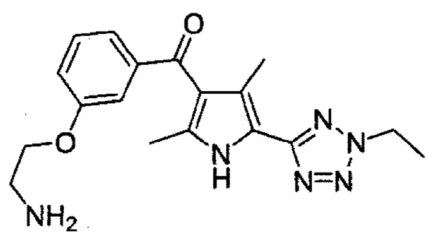

The compound of interest was obtained by the same procedures as in Production Examples 50-1 and 48-1 using compound 164 as a starting material. Yield: 81.3 %, yellow solid.
$^1$H-NMR (400 MHz, DMSO-$d_6$) δ: 1.56 (3H, t, J = 7.3 Hz), 2.12 (3H, s), 2.26 (3H, s), 2.93-2.98 (2H, m), 3.98-4.04 (2H, m), 4.73 (2H, q, J = 7.3 Hz), 7.13-7.21 (3H, m), 7.39-7.44 (1H, m), 12.04 (1H, s).

Production Example 166: Synthesis of (3-bromo-4-hydroxyphenyl)(5-(2-ethyl-2H-tetrazol-5-yl)-2,4-dimethyl-1H-pyrrol-3-yl)methanone [compound No. 166]

Production Example 166-1: Synthesis of (3-bromo-4-hydroxyphenyl)(5-(2-ethyl-2H-tetrazol-5-yl)-2,4-dimethyl-1H-pyrrol-3-yl)methanone [compound No. 166]

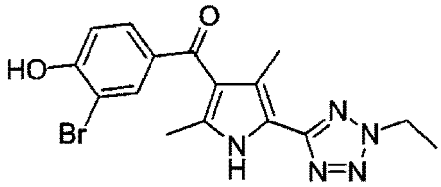

The compound of interest was obtained by the same procedures as in Production Example 91-1 using compound 109 as a starting material. Yield: quant, yellow solid. $^1$H-NMR (400 MHz, $CDCl_3$) δ: 1.68 (3H, t, J = 7.3 Hz), 2.32 (3H, s), 2.33 (3H, s), 4.69 (2H, q, J = 7.4 Hz), 5.92 (1H, s), 7.08 (1H, d, J = 8.3 Hz), 7.70 (1H, dd, J = 8.4, 2.1 Hz), 7.97 (1H, d, J = 2.0 Hz), 9.02 (1H, s).

Production Example 167: Synthesis of (4-(2-aminoethoxy)-3-bromophenyl)(5-(2-ethyl-2H-tetrazol-5-yl)-2,4-dimethyl-1H-pyrrol-3-yl)methanone [compound No. 167]

Production Example 167-1: Synthesis of (4-(2-aminoethoxy)-3-bromophenyl)(5-(2-ethyl-2H-tetrazol-5-yl)-2,4-dimethyl-1H-pyrrol-3-yl)methanone [compound No. 167]

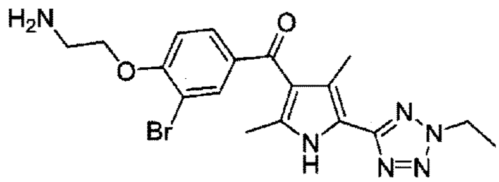

The compound of interest was obtained by the same procedures as in Production Examples 50-1 and 48-1 using compound 166 as a starting material. Yield: 65.0 %, pale yellow solid.
$^1$H-NMR (400 MHz, DMSO-$d_6$) δ: 1.57 (3H, t, J = 7.3 Hz), 2.15 (3H, s), 2.25 (3H, s), 2.95 (2H, t, J = 5.5 Hz), 4.10 (2H, t, J = 5.7 Hz), 4.73 (2H, q, J = 7.3 Hz), 7.21 (1H, d, J = 8.8 Hz), 7.66 (1H, dd, J = 8.5, 2.0 Hz), 7.82 (1H, d, J = 2.0Hz), 12.03 (1H, s).

Production Example 168: Synthesis of (4-(2-aminoethoxy)-3-bromophenyl)(5-(2-(2-hydroxyethyl)-2H-tetrazol-5-yl)-2,4-dimethyl-1H-pyrrol-3-yl)methanone [compound No. 168]

Production Example 168-1: Synthesis of (4-(2-aminoethoxy)-3-bromophenyl)(5-(2-(2-hydroxyethyl)-2H-tetrazol-5-yl)-2,4-dimethyl-1H-pyrrol-3-yl)methanone [compound No. 168]

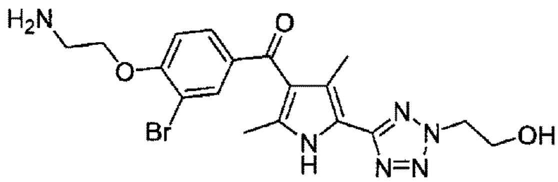

The compound of interest was obtained by the same procedures as in Production Examples 91-1, 50-1, 48-1, and 117-1 using compound 111 as a starting material. Yield: 28.1 %, white solid.
$^1$H-NMR (400 MHz, DMSO-$d_6$) δ: 2.16 (3H, s), 2.25 (3H, s), 3.22-3.28 (2H, m), 3.90-3.99 (2H, m), 4.30-4.37 (2H, m), 4.70-4.77 (2H, m), 5.06-5.12 (1H, m), 7.23-7.29 (1H, m), 7.66-7.71 (1H, m), 7.83-7.86 (1H, m), 12.06 (1H, s).

Production Example 169: Synthesis of (E)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 169]

Production Example 169-1: Synthesis of (E)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 169]

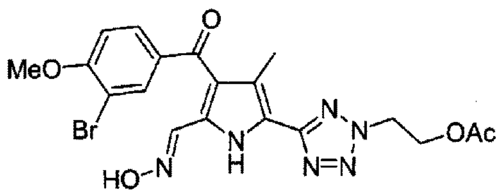

Compound 124 (111 mg, 0.23 mmol) was dissolved in pyridine (1.8 ml). To the solution, hydroxylamine hydrochloride (18 mg, 0.25 mmol) was added, and the mixture was stirred overnight at room temperature.
Acetic anhydride (0.048 ml, 0.51 mmol) was added thereto, and the mixture was stirred at 80°C for 1 day. After reaction, the solvent was distilled off. Water was added to the residue, followed by extraction with ethyl acetate. The organic layer was dried over magnesium sulfate, and the solvent was distilled off. The residue was purified using a preparative LC system with MS triggers manufactured by Waters Corp. to obtain the title compound (24 mg, yield: 21.2%) as a yellow solid.
$^1$H-NMR (400 MHz, DMSO-$d_6$) δ: 1.96 (3H, s), 2.23 (3H, s), 3.95 (3H, s), 4.56 (2H, t, J = 5.1 Hz), 5.04 (2H, t, J = 5.1 Hz), 7.24 (1H, d, J = 8.5 Hz), 7.31 (1H, s), 7.74 (1H, dd, J = 8.5, 2.2 Hz), 7.92 (1H, d, J = 2.2 Hz), 11.64 (1H, br s), 12.16 (1H, br s).
ESI-MS m/z: 491[M+H]$^+$, 493[M+2+H]$^+$.

Production Example 170: Synthesis of (Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 170]

Production Example 170-1: Synthesis of (Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate [compound No. 170]

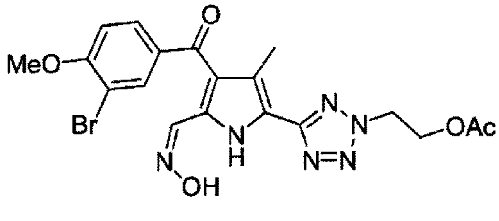

The compound of interest was obtained as an isomer by the procedures of Production Example 169-1. Yield: 29.2%, yellow solid.
$^1$H-NMR (400 MHz, DMSO-$d_6$) δ: 1.97 (3H, s), 2.20 (3H, s), 3.94 (3H, s), 4.57 (2H, t, J = 5.1 Hz), 5.02 (2H, t, J = 5.1

Hz), 7.21 (1H, d, J = 8.8 Hz), 7.73 (1H, dd, J = 8.8, 2.0 Hz), 7.90 (1H, s), 7.91 (1H, d, J = 2.0 Hz), 11.20 (1H, br s), 12.37 (1H, br s).
ESI-MS m/z: 491[M+H]$^+$, 493[M+2+H]$^+$.

Production Example 171: Synthesis of 2-(5-(4-(3-bromobenzoyl)-5-(chloromethyl)-3-methyl-1H-pyrrol-2-yl)-2H-tetrazol-2-yl)ethyl acetate [compound No. 171]

Production Example 171-1: Synthesis of 2-(5-(4-(3-bromobenzoyl)-5-(chloromethyl)-3-methyl-1H-pyrrol-2-yl)-2H-tetrazol-2-yl)ethyl acetate [compound No. 171]

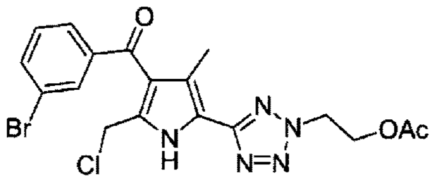

Compound 104 (516 mg, 1.19 mmol) was dissolved in acetic acid (15 ml). To the solution, sulfuryl chloride (0.193 ml, 2.38 mmol) was added, and the mixture was stirred at 50°C for 2 hours. After reaction, the reaction solution was poured into ice water. After extraction with chloroform, the organic layer was washed with a 1 M aqueous sodium hydroxide solution. The organic layer was dried over magnesium sulfate, and the solvent was distilled off. The residue was purified by silica gel column chromatography to obtain the title compound (97 mg, yield: 17.5%) as a brown solid.
$^1$H-NMR (400 MHz, DMSO-$d_6$) $\delta$: 1.96 (3H, s), 2.23 (3H, s), 4.33 (2H, s), 4.57 (2H, t, J = 5.0 Hz), 5.01 (2H, t, J = 5.0 Hz), 7.48 (1H, t, J = 7.8 Hz), 7.66 (1H, dt, J = 7.8, 1.2 Hz), 7.78-7.83 (2H, m), 12.07 (1H, br s).

Production Example 172: Synthesis of 2-(5-(4-(3-bromo-4-methoxybenzoyl)-5-(chloromethyl)-3-methyl-1H-pyrrol-2-yl)-2H-tetrazol-2-yl)ethyl acetate [compound No. 172]

Production Example 172-1: Synthesis of 2-(5-(4-(3-bromo-4-methoxybenzoyl)-5-(chloromethyl)-3-methyl-1H-pyrrol-2-yl)-2H-tetrazol-2-yl)ethyl acetate [compound No. 172]

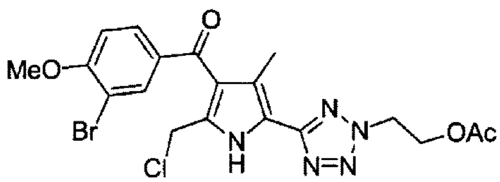

The compound of interest was obtained by the same procedures as in Production Example 171-1 using compound 111 as a starting material. Yield: 15.3 %, brown solid. $^1$H-NMR (400 MHz, DMSO-$d_6$) $\delta$: 1.96 (3H, s), 2.24 (3H, s), 3.95 (3H, s), 4.35 (2H, s), 4.57 (2H, t, J = 5.1 Hz), 5.01 (2H, t, J = 5.1 Hz), 7.22 (1H, d, J = 8.5 Hz), 7.73 (1H, dd, J = 8.5, 2.2 Hz), 7.87 (1H, d, J = 2.2 Hz), 12.01 (1H, br s).

Production Example 173: Synthesis of (3-bromophenyl)(5-(5-ethyl-1,3,4-oxadiazol-2-yl)-2,4-dimethyl-1H-pyrrol-3-yl)methanone [compound No. 173]

Production Example 173-1: Synthesis of 4-(3-bromobenzoyl)-3,5-dimethyl-1H-pyrrole-2-carbohydrazide

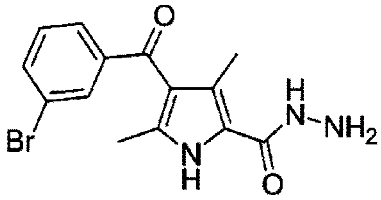

To a solution of the compound (322 mg, 1 mmol) obtained in Production Example 96-1 in N,N-dimethylformamide (5 ml), 1-ethyl-3-(3-dimethylaminopropyl)carbodiimide hydrochloride (231 mg, 1.2 mmol) and 1-hydroxybenzotriazole (163 mg, 1.2 mmol) were added in the argon atmosphere. After 1 hour, hydrazine monohydrate (59 μl, 1.2 mmol) was added to the mixture. After overnight stirring, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure to obtain the title compound (397 mg, yield: quant., pale yellow solid). $^1$H-NMR (400 MHz, DMSO-$d_6$) δ: 2.05 (3H, s), 2.12 (3H, s), 4.38 (2H, s), 7.43-7.48 (1H, m), 7.55-7.59 (1H, m), 7.68-7.70 (1H, m), 7.77-7.81 (1H, m), 8.77 (1H, s), 11.57 (1H, s).

Production Example 173-2: Synthesis of (3-bromophenyl)(5-(5-ethyl-1,3,4-oxadiazol-2-yl)-2,4-dimethyl-1H-pyrrol-3-yl)methanone [compound No. 173]

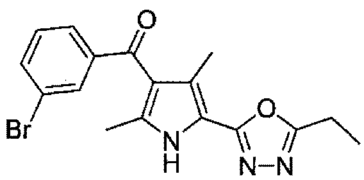

To a suspension of the compound (120 mg, 0.33 mmol) obtained in Production Example 173-1 in acetonitrile (4 ml), triethylamine (46 μl, 0.33 mmol) and propionyl chloride (29 μl, 0.33 mmol) were added at 0°C in the argon atmosphere. After 3 hours, phenylphosphonic dichloride (47 μl, 0.33 mmol) was added to the mixture, and the mixture was heated with stirring at 80°C for 12 hours. Then, the reaction solution was allowed to cool and quenched by the addition of water. The aqueous layer was extracted with chloroform. The obtained organic layer was dried over anhydrous magnesium sulfate. Magnesium sulfate was filtered off, and the solvent was distilled off under reduced pressure to obtain a crude product. This crude product was purified by column chromatography to obtain the title compound (75 mg, yield: 60.9%, light brown solid).
$^1$H-NMR (400 MHz, $CDCl_3$) δ: 1.43 (3H, t, J = 7.6 Hz), 2.28 (6H, s), 2.94 (2H, q, J = 7.6 Hz), 7.32-7.37 (1H, m), 7.64-7.70 (2H, m), 7.85-7.88 (1H, m), 9.31 (1H,s).

Production Example 174: Synthesis of (3-bromophenyl)(5-(5-(2-chloroethyl)-1,3,4-oxadiazol-2-yl)-2,4-dimethyl-1H-pyrrol-3-yl)methanone [compound No. 174]

Production Example 1-1: Synthesis of (3-bromophenyl)(5-(5-(2-chloroethyl)-1,3,4-oxadiazol-2-yl)-2,4-dimethyl-1H-pyrrol-3-yl)methanone [compound No. 174]

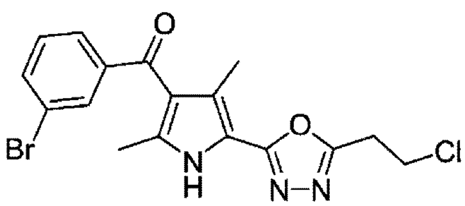

The title compound was obtained by the same procedures as in Production Example 173-2 using 3-chloropropionyl chloride.

Yield: 11.0 %, pale yellow solid.
$^1$H-NMR (400 MHz, $CDCl_3$) δ: 2.29 (6H, s), 3.41 (2H, t, J = 6.8 Hz), 3.94 (2H, t, J = 7.0 Hz), 7.32-7.38 (1H, m), 7.65-7.71 (2H, m), 7.86-7.88 (1H, m), 9.31 (1H,s).

Production Example 175: Synthesis of (3-bromophenyl)(2,4-dimethyl-5-(5-phenyl-1,3,4-oxadiazol-2-yl)-1H-pyrrol-3-yl)methanone [compound No. 175]

Production Example 175-1: Synthesis of (3-bromophenyl)(2,4-dimethyl-5-(5-phenyl-1,3,4-oxadiazol-2-yl)-1H-pyrrol-3-yl)methanone [compound No. 175]

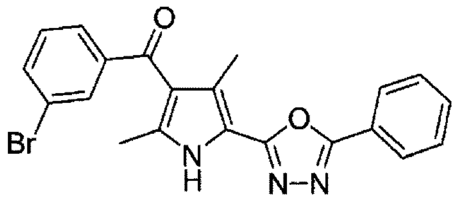

The title compound was obtained by the same procedures as in Production Example 173-2 using benzoyl chloride.
Yield: 11.1 %, white solid.
$^1$H-NMR (400 MHz, $CDCl_3$) δ: 2.32 (3H, s), 2.40 (3H, s), 7.34-7.39 (1H, m), 7.52-7.57 (3H, m), 7.68-7.72 (2H, m), 7.88-7.90 (1H, m), 8.07-8.11 (2H, m), 9.56 (1H,s).

Production Example 176: Synthesis of (5-(5-benzyl-1,3,4-oxadiazol-2-yl)-2,4-dimethyl-1H-pyrrol-3-yl)(3-bromophenyl)methanone [compound No. 176]

Production Example 176-1: Synthesis of (5-(5-benzyl-1,3,4-oxadiazol-2-yl)-2,4-dimethyl-1H-pyrrol-3-yl)(3-bromophenyl)methanone [compound No. 176]

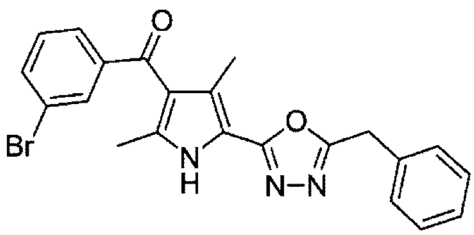

The title compound was obtained by the same procedures as in Production Example 173-2 using 2-phenylacetyl chloride.
Yield: 13.8 %, light brown solid.
$^1$H-NMR (400 MHz, $CDCl_3$) δ: 2.22 (3H, s), 2.26 (3H, s), 4.25 (2H, s), 7.30-7.37 (6H, m), 7.62-7.69 (2H, m), 7.83-7.86 (1H, m), 9.13 (1H, s).

Production Example 177: Synthesis of (3-bromophenyl)(5-(5-cyclopropyl-1,3,4-oxadiazol-2-yl)-2,4-dimethyl-1H-pyrrol-3-yl)methanone [compound No. 177]

Production Example 177-1: Synthesis of (3-bromophenyl)(5-(5-cyclopropyl-1,3,4-oxadiazol-2-yl)-2,4-dimethyl-1H-pyrrol-3-yl)methanone [compound No. 177]

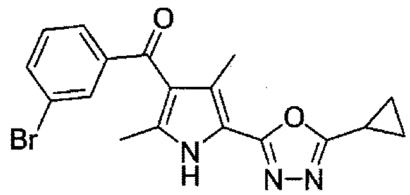

The title compound was obtained by the same procedures as in Production Example 173-2 using cyclopropanecarbonyl chloride.
Yield: 42.6 %, white solid.
$^1$H-NMR (400 MHz, $CDCl_3$) δ: 1.19 (4H, d, J = 6.6 Hz), 2.20 (1H, quin, J = 6.6 Hz), 2.25 (3H, s), 2.27 (3H, s), 7.32-7.37 (1H, m), 7.64-7.70 (2H, m), 7.85-7.87 (1H, m), 9.35 (1H, s).

Production Example 178: Synthesis of (3-bromophenyl)(5-(5-isopropyl-1,3,4-oxadiazol-2-yl)-2,4-dimethyl-1H-pyrrol-3-yl)methanone [compound No. 178]

Production Example 178-1: Synthesis of (3-bromophenyl)(5-(5-isopropyl-1,3,4-oxadiazol-2-yl)-2,4-dimethyl-1H-pyrrol-3-yl)methanone [compound No178]

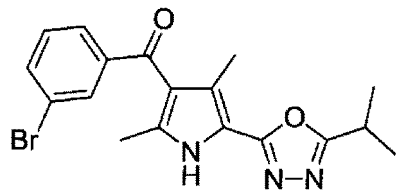

The title compound was obtained by the same procedures as in Production Example 173-2 using isobutyryl chloride.
Yield: 53.6 %, light brown solid.
$^1$H-NMR (400 MHz, $CDCl_3$) δ: 1.45 (6H, d, J = 7.1 Hz), 2.28 (3H, s), 2.29 (3H, s), 3.25 (1H, heptet, J = 7.0 Hz), 7.32-7.37 (1H, m), 7.65-7.70 (2H, m), 7.86-7.87(1H, m), 9.61 (1H, s).

Production Example 179: Synthesis of (3-bromophenyl)(5-(5-cyclobutyl-1,3,4-oxadiazol-2-yl)-2,4-dimethyl-1H-pyrrol-3-yl)methanone [compound No. 179]

Production Example 179-1: Synthesis of (3-bromophenyl)(5-(5-cyclobutyl-1,3,4-oxadiazol-2-yl)-2,4-dimethyl-1H-pyrrol-3-yl)methanone [compound No. 179]

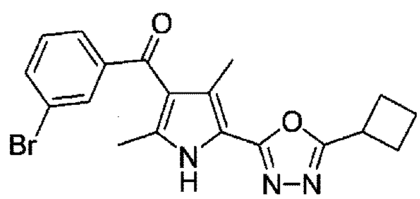

The title compound was obtained by the same procedures as in Production Example 173-2 using cyclobutanecarbonyl chloride.
Yield: 46.9 %, pale yellow solid.
$^1$H-NMR (400 MHz, $CDCl_3$) δ: 2.02-2.20 (2H, m), 2.29 (3H, s), 2.29 (3H, s), 2.43-2.53 (4H, m), 3.78 (1H, quin, J = 8.1 Hz), 7.32-7.37 (1H, m), 7.65-7.70 (2H, m), 7.85-7.88 (1H, m), 9.47 (1H, s).

Production Example 180: Synthesis of (3-bromophenyl)(5-(5-cyclopentyl-1,3,4-oxadiazol-2-yl)-2,4-dimethyl-1H-pyrrol-3-yl)methanone [compound No. 180]

Production Example 180-1: Synthesis of (3-bromophenyl)(5-(5-cyclopentyl-1,3,4-oxadiazol-2-yl)-2,4-dimethyl-1H-pyrrol-3-yl)methanone [compound No. 180]

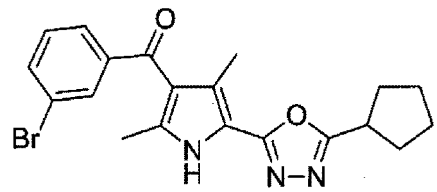

The title compound was obtained by the same procedures as in Production Example 173-2 using cyclopentanecarbonyl chloride.
Yield: 68.1 %, pale yellow solid.
$^1$H-NMR (400 MHz, $CDCl_3$) δ: 1.69-1.79 (2H, m), 1.80-1.89 (2H, m), 1.92-2.02 (2H,m), 2.10-2.20 (2H, m), 2.27 (3H, s), 2.28 (3H, s), 3.37 (1H, quin, J = 8.1 Hz), 7.32-7.37 (1H, m), 7.65-7.70 (2H, m), 7.85-7.87 (1H, m), 9.37 (1H, s) .

Production Example 181: Synthesis of (3-bromophenyl)(5-(5-cyclohexyl-1,3,4-oxadiazol-2-yl)-2,4-dimethyl-1H-pyrrol-3-yl)methanone [compound No. 181]

Production Example 181-1: Synthesis of (3-bromophenyl)(5-(5-cyclohexyl-1,3,4-oxadiazol-2-yl)-2,4-dimethyl-1H-pyrrol-3-yl)methanone [compound No. 181]

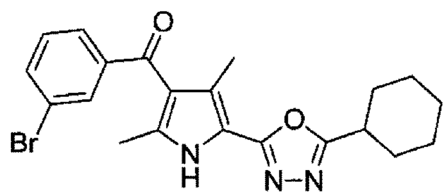

The title compound was obtained by the same procedures as in Production Example 173-2 using cyclohexanecarbonyl chloride.
Yield: 69.0 %, pale yellow solid.
$^1$H-NMR (400 MHz, $CDCl_3$) δ: 1.24-1.48 (3H, m), 1.61-1.78 (3H, m), 1.81-1.91 (2H,m), 2.09-2.19 (2H, m), 2.28 (6H, s), 2.91-3.01 (1H, m), 7.32-7.37 (1H, m), 7.64-7.70 (2H, m), 7.85-7.87 (1H, m), 9.29 (1H, s).

Production Example 182: Synthesis of methyl 3-(5-(4-(3-bromobenzoyl)-3,5-dimethyl-1H-pyrrol-2-yl)-1,3,4-oxadiazol-2-yl)propionate [compound No. 182]

Production Example 182-1: Synthesis of methyl 3-(5-(4-(3-bromobenzoyl)-3,5-dimethyl-1H-pyrrol-2-yl)-1,3,4-oxadiazol-2-yl)propionate [compound No. 182]

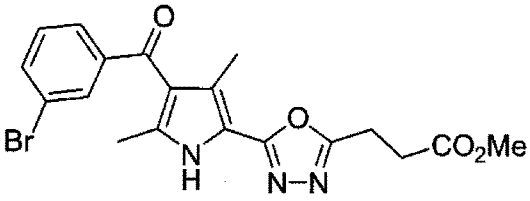

The title compound was obtained by the same procedures as in Production Example 173-2 using methyl 4-chloro-4-oxobutanoate.
Yield: 23.1 %, pale yellow solid.
$^1$H-NMR (400 MHz, DMSO-$d_6$) δ: 2.13 (3H, s), 2.20 (3H, s), 2.88 (2H, t, J = 7.0 Hz), 3.16 (2H, t, J = 7.1 Hz), 3.63 (3H, s), 7.45-7.50 (1H, m), 7.59-7.63 (1H, m), 7.73-7.75 (1H, m), 7.79-7.83 (1H, m), 12.31 (1H, s).

Production Example 183: Synthesis of ethyl 2-(5-(4-(3-bromobenzoyl)-3,5-dimethyl-1H-pyrrol-2-yl)-1,3,4-oxadiazol-2-yl)acetate [compound No. 183]

Production Example 183-1: Synthesis of ethyl 2-(5-(4-(3-bromobenzoyl)-3,5-dimethyl-1H-pyrrol-2-yl)-1,3,4-oxadiazol-2-yl)acetate [compound No. 183]

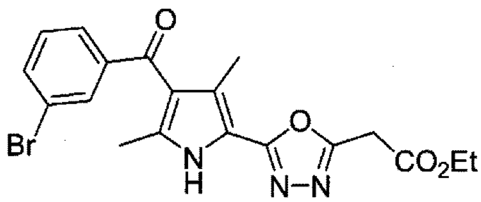

The title compound was obtained by the same procedures as in Production Example 173-2 using ethyl 3-chloro-3-oxopropanoate.
Yield: 43.5 %, pale yellow solid.
$^1$H-NMR (400 MHz, $CDCl_3$) δ: 1.30 (3H, t, J = 7.2 Hz), 2.28 (3H, s), 2.29 (3H, s), 4.01 (2H, s), 4.25 (2H, q, J = 7.1 Hz), 7.32-7.39 (1H, m), 7.64-7.71 (2H, m), 7.85-7.89 (1H, m), 9.46 (1H, s).

Production Example 184: Synthesis of (3-bromophenyl)(5-(5-tert-butyl)-1,3,4-oxadiazol-2-yl)-2,4-dimethyl-1H-pyrrol-3-yl)methanone [compound No. 184]

Production Example 184-1: Synthesis of (3-bromophenyl)(5-(5-tert-butyl)-1,3,4-oxadiazol-2-yl)-2,4-dimethyl-1H-pyrrol-3-yl)methanone [compound No. 184]

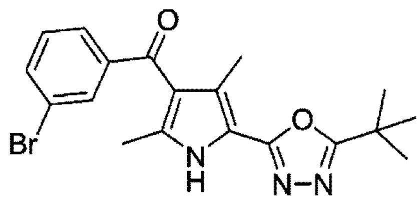

The title compound was obtained by the same procedures as in Production Example 173-2 using pivaloyl chloride.
Yield: 26.7 %, yellow solid.
$^1$H-NMR (400 MHz, $CDCl_3$) δ: 1.47 (9H, s), 2.28 (3H, s), 2.29 (3H, s), 3.49 (1H, s), 7.32-7.37 (1H, m), 7.65-7.70 (2H, m), 7.86-7.88 (1H, m), 9.68 (1H, s).

Production Example 185: Synthesis of (3-bromophenyl)(2,4-dimethyl-5-(5-methyl-1,3,4-oxadiazol-2-yl)-1H-pyrrol-3-yl)methanone [compound No. 185]

Production Example 185-1: Synthesis of (3-bromophenyl)(2,4-dimethyl-5-(5-methyl-1,3,4-oxadiazol-2-yl)-1H-pyrrol-3-yl)methanone [compound No. 185]

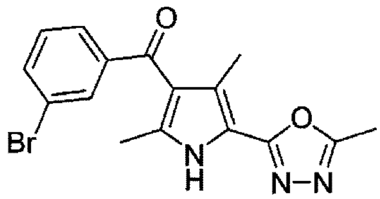

The title compound was obtained by the same procedures as in Production Example 173-2 using acetyl chloride.
Yield: 35.4 %, yellow solid.
$^1$H-NMR (400 MHz, $CDCl_3$) δ: 2.28 (3H, s), 2.28 (3H, s), 2.60 (3H, s), 7.32-7.37 (1H, m), 7.65-7.70 (2H, m), 7.85-7.87 (1H, m), 9.23 (1H, s).

Production Example 186: Synthesis of (3-bromophenyl)(5-(5-(difluoromethyl)-1,3,4-oxadiazol-2-yl)-2,4-dimethyl-1H-pyrrol-3-yl)methanone [compound No. 186]

Production Example 186-1: Synthesis of (3-bromophenyl)(5-(5-(difluoromethyl)-1,3,4-oxadiazol-2-yl)-2,4-dimethyl-1H-pyrrol-3-yl)methanone [compound No. 186]

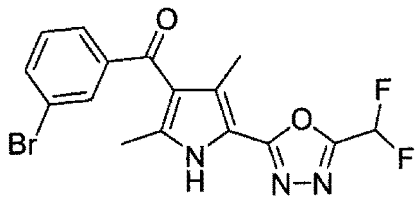

The title compound was obtained by the same procedures as in Production Example 173-2 using difluoroacetyl chloride.
Yield: 5.5 %, white solid.
$^1$H-NMR (400 MHz, DMSO-$d_6$) δ: 2.16 (3H, s), 2.25 (3H, s), 7.46-7.51 (1H, m), 7.53 (1H, t, J = 51.4 Hz), 7.61-7.65 (1H, m), 7.74-7.78 (1H, m), 7.80-7.85 (1H, m), 12.55 (1H, s).

Production Example 187: Synthesis of (3-bromophenyl)(2,4-dimethyl-5-(5-(2,2,2-trifluoroethyl)-1,3,4-oxadiazol-2-yl)-1H-pyrrol-3-yl)methanone [compound No. 187]

Production Example 187-1: Synthesis of (3-bromophenyl)(2,4-dimethyl-5-(5-(2,2,2-trifluoroethyl)-1,3,4-oxadiazol-2-yl)-1H-pyrrol-3-yl)methanone [compound No. 187]

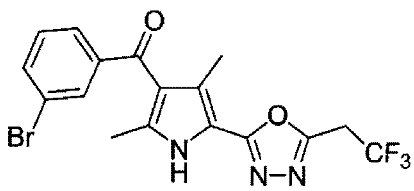

The title compound was obtained by the same procedures as in Production Example 173-2 using 3,3,3-trifluoropropionyl chloride.
Yield: 7.1 %, yellow solid.
$^1$H-NMR (400 MHz, DMSO-$d_6$) δ: 2.14 (3H, s), 2.22 (3H, s), 4.37 (2H, q, J = 10.6 Hz), 7.45-7.50 (1H, m), 7.61-7.64 (1H, m), 7.74-7.76 (1H, m), 7.80-7.83 (1H, m), 12.43 (1H, s).

Production Example 188: Synthesis of (3-bromophenyl)(5-(5-(1-hydroxycyclopropyl)-1,3,4-oxadiazol-2-yl)-2,4-dimethyl-1H-pyrrol-3-yl)methanone [compound No. 188]

Production Example 188-1: Synthesis of (3-bromophenyl)(5-(5-(1-hydroxycyclopropyl)-1,3,4-oxadiazol-2-yl)-2,4-dimethyl-1H-pyrrol-3-yl)methanone [compound No. 188]

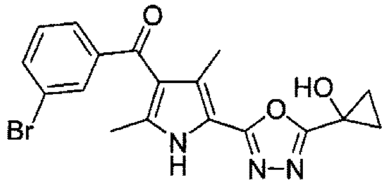

The title compound was obtained by the same procedures as in Production Example 173-2 using 1-hydroxycyclopropane carbonyl chloride.
Yield: 13.3 %, light brown solid.
$^1$H-NMR (400 MHz, $CDCl_3$) δ: 1.40-1.49 (4H, m), 2.24 (3H, s), 2.29 (3H, s), 7.32-7.37 (1H, m), 7.64-7.70 (2H, m), 7.84-7.87 (1H, m), 9.47 (1H, s).

Production Example 189: Synthesis of (3-bromophenyl)(2,4-dimethyl-5-(5-(1-methylcyclopropyl)-1,3,4-oxadiazol-2-yl)-1H-pyrrol-3-yl)methanone [compound No. 189]

Production Example 189-1: Synthesis of (3-bromophenyl)(2,4-dimethyl-5-(5-(1-methylcyclopropyl)-1,3,4-oxadiazol-2-yl)-1H-pyrrol-3-yl)methanone [compound No. 189]

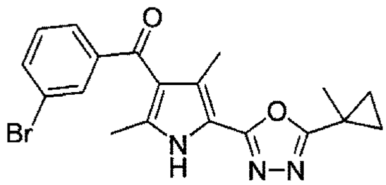

The title compound was obtained by the same procedures as in Production Example 173-2 using 1-methylcyclopropane carbonyl chloride.
Yield: 51.1 %, light brown solid.
$^1$H-NMR (400 MHz, $CDCl_3$) δ: 0.96-1.01 (2H, m), 1.31-1.37 (2H, m), 1.60 (3H, s), 2.23 (3H, s), 2.26-2.29 (3H, m), 7.31-7.37 (1H, m), 7.63-7.70 (2H, m), 7.84-7.87(1H, m).

Production Example 190: Synthesis of (3-bromophenyl)(2,4-dimethyl-5-(5-(2-methylcyclopropyl)-1,3,4-oxadiazol-2-yl)-1H-pyrrol-3-yl)methanone [compound No. 190]

Production Example 190-1: Synthesis of (3-bromophenyl)(2,4-dimethyl-5-(5-(2-methylcyclopropyl)-1,3,4-oxadiazol-2-yl)-1H-pyrrol-3-yl)methanone [compound No. 190]

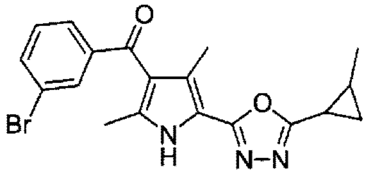

The title compound was obtained by the same procedures as in Production Example 173-2 using 2-methylcyclopropane carbonyl chloride.
Yield: 60.8 %, white solid.
$^1$H-NMR (400 MHz, $CDCl_3$) δ: 0.94-1.01 (1H, m), 1.12-1.18 (1H, m), 1.24 (3H, d, J= 6.1 Hz), 1.32-1.38 (1H, m), 1.86-1.93 (1H, m), 2.24 (3H, s), 2.27 (3H, s), 7.32-7.37 (1H, m), 7.64-7.70 (2H, m), 7.84-7.88 (1H, m).

Production Example 191: Synthesis of (3-bromophenyl)(5-(5-(2,2-dimethylcyclopropyl)-1,3,4-oxadiazol-2-yl)-2,4-dimethyl-1H-pyrrol-3-yl)methanone [compound No. 191]

Production Example 191-1: Synthesis of (3-bromophenyl)(5-(5-(2,2-dimethylcyclopropyl)-1,3,4-oxadiazol-2-yl)-2,4-dimethyl-1H-pyrrol-3-yl)methanone [compound No. 191]

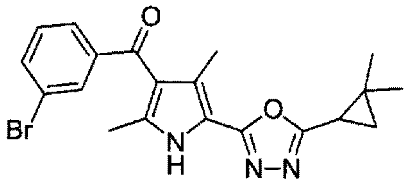

The title compound was obtained by the same procedures as in Production Example 173-2 using 2,2-dimethylcyclopropane carbonyl chloride.
Yield: 40.9 %, light brown solid.
$^1$H-NMR (400 MHz, $CDCl_3$) δ: 1.16 (3H, s), 1.28 (3H, s), 1.29-1.32 (2H, m), 1.96-2.01 (1H, m), 2.27 (3H, s), 2.28 (3H, s), 7.32-7.37 (1H, m), 7.64-7.70 (2H, m), 7.84-7.88 (1H, m), 9.27 (1H, s).

Production Example 192: Synthesis of (3-bromophenyl)(2,4-dimethyl-5-(5-(2,2,3,3-tetramethylcyclopropyl)-1,3,4-oxadiazol-2-yl)-1H-pyrrol-3-yl)methanone [compound No. 192]

Production Example 192-1: Synthesis of (3-bromophenyl)(2,4-dimethyl-5-(5-(2,2,3,3-tetramethylcyclopropyl)-1,3,4-oxadiazol-2-yl)-1H-pyrrol-3-yl)methanone [compound No. 192]

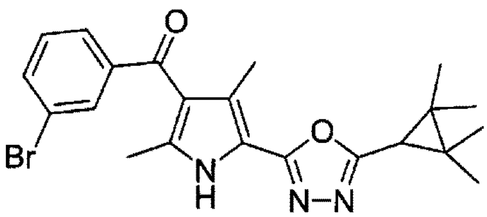

The title compound was obtained by the same procedures as in Production Example 173-2 using 2,2,3,3-tetramethylcyclopropane carbonyl chloride.
Yield: 41.3 %, white solid.
$^1$H-NMR (400 MHz, $CDCl_3$) δ: 1.17 (6H, s), 1.68 (6H, s), 2.28 (3H, s), 2.29 (3H, s), 3.17 (1H, s), 7.32-7.38 (1H, m), 7.65-7.70 (2H, m), 7.85-7.88 (1H, m).

Production Example 193: Synthesis of (5-(5-ethyl-1,3,4-oxadiazol-2-yl)-2,4-dimethyl-1H-pyrrol-3-yl)(3-methoxyphenyl)methanone [compound No. 193]

Production Example 193-1: Synthesis of 4-(3-methoxybenzoyl)-3,5-dimethyl-1H-pyrrole-2-carbohydrazide

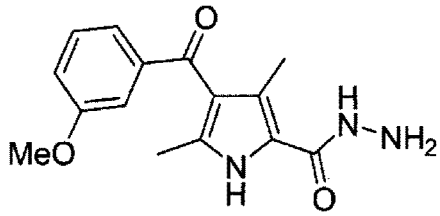

The title compound was obtained in the same way as in Production Example 173-1 using the compound obtained in Production Example 105-1 as a starting material. Yield: quant, orange solid.
$^1$H-NMR (400 MHz, DMSO-$d_6$) δ: 2.05 (3H, s), 2.13 (3H, s), 3.79 (3H, s), 7.08-7.11 (1H, m), 7.12-7.17 (2H, m), 7.37-7.42 (1H, m), 8.73 (1H, s), 11.49 (1H, s).

Production Example 193-2: Synthesis of (5-(5-ethyl-1,3,4-oxadiazol-2-yl)-2,4-dimethyl-1H-pyrrol-3-yl)(3-methoxyphenyl)methanone [compound No. 193]

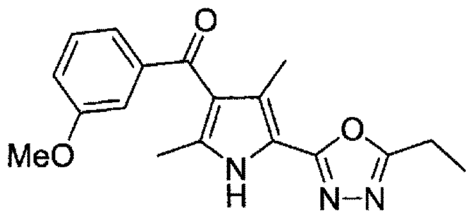

The title compound was obtained by the same procedures as in Production Example 173-2 using the compound obtained in Production Example 193-1. Yield: 43.3 %, yellow solid.
$^1$H-NMR (400 MHz, DMSO-$d_6$) δ: 1.68 (3H, t, J = 7.3 Hz), 2.29 (3H, s), 2.35 (3H, s), 3.86 (3H, s), 4.68 (2H, q, J = 7.3 Hz), 7.06-7.11 (1H, m), 7.30-7.38 (3H, m), 9.06 (1H, s) .

Production Example 194: Synthesis of (5-(5-cyclopropyl-1,3,4-oxadiazol-2-yl)-2,4-dimethyl-1H-pyrrol-3-yl)(3-methoxyphenyl)methanone [compound No. 194]

Production Example 194-1: Synthesis of (5-(5-cyclopropyl-1,3,4-oxadiazol-2-yl)-2,4-dimethyl-1H-pyrrol-3-yl)(3-methoxyphenyl)methanone [compound No. 194]

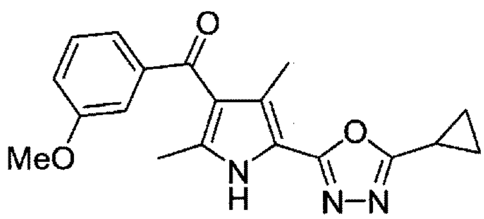

The title compound was obtained by the same procedures as in Production Example 173-2 using the compound obtained in Production Example 193-1 and cyclopropanecarbonyl chloride.
Yield: 61.7 %, yellow solid.
$^1$H-NMR (400 MHz, $CDCl_3$) δ: 1.14-1.21 (4H, m), 2.15-2.22 (1H, m), 2.26 (3H, s), 2.27 (3H, s), 3.86 (3H, s), 7.07-7.11

(1H, m), 7.27-7.31 (2H, m), 7.32-7.38 (1H,m), 10.29 (1H, s).

Production Example 195: Synthesis of (5-(5-ethyl-1,3,4-oxadiazol-2-yl)-2,4-dimethyl-1H-pyrrol-3-yl)(3-nitrophenyl)methanone [compound No. 195]

Production Example 195-1: Synthesis of 3,5-dimethyl-4-(3-nitrobenzoyl)-1H-pyrrole-2-carboxylic acid

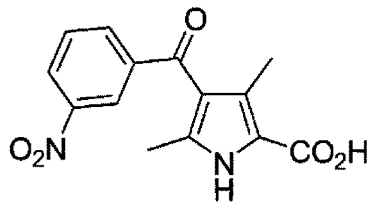

The title compound was obtained by the same procedures as in Production Example 96-1 using compound 19 as a starting material.
Yield: quant, orange solid.
$^1$H-NMR (400 MHz, DMSO-$d_6$) δ: 2.09 (3H, s), 2.16 (3H, s), 7.77-7.83 (1H, m), 8.02-8.06 (1H, m), 8.28-8.31 (1H, m), 8.41-8.46 (1H, m), 12.00 (1H, s).

Production Example 195-2: Synthesis of 3,5-dimethyl-4-(3-nitrobenzoyl)-1H-pyrrole-2-carbohydrazide

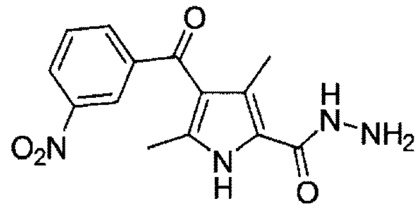

The title compound was obtained by the same procedures as in Production Example 173-1 using the compound obtained in Production Example 195-1.
Yield: 67.8 %, yellow solid.
$^1$H-NMR (400 MHz, DMSO-$d_6$) δ: 2.07 (3H, s), 2.13 (3H, s), 7.76-7.83 (1H, m), 8.00-8.04 (1H, m), 8.27-8.30 (1H, m), 8.40-8.45 (1H, m), 8.80 (1H, s), 11.64 (1H, s).

Production Example 195-3: Synthesis of (5-(5-ethyl-1,3,4-oxadiazol-2-yl)-2,4-dimethyl-1H-pyrrol-3-yl)(3-nitrophenyl)methanone [compound No. 195]

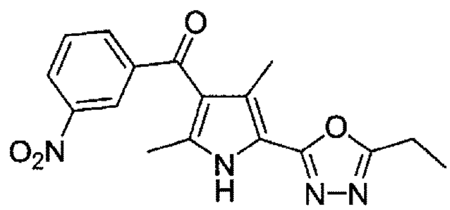

The title compound was obtained by the same procedures as in Production Example 173-2 using the compound obtained in Production Example 195-2 as a starting material.
Yield: 28.9%, yellow solid.
$^1$H-NMR (400 MHz, DMSO-$d_6$) δ: 1.32 (3H, t, J = 7.6 Hz), 2.15 (3H, s), 2.23 (3H, s), 2.92 (2H, q, J = 7.6 Hz), 7.79-7.84

(1H, m), 8.05-8.09 (1H, m), 8.32-8.34 (1H, m), 8.43-8.47 (1H, m), 12.40 (1H, s).

Production Example 196: Synthesis of (5-(5-cyclopropyl-1,3,4-oxadiazol-2-yl)-2,4-dimethyl-1H-pyrrol-3-yl)(3-nitrophenyl)methanone [compound No. 196]

Production Example 196-1: Synthesis of (5-(5-cyclopropyl-1,3,4-oxadiazol-2-yl)-2,4-dimethyl-1H-pyrrol-3-yl)(3-nitrophenyl)methanone [compound No. 196]

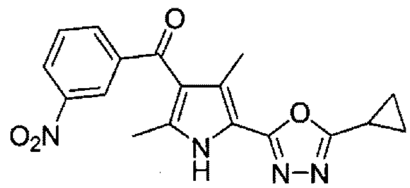

The title compound was obtained by the same procedures as in Production Example 173-2 using the compound obtained in Production Example 195-1 and cyclopropanecarbonyl chloride.
Yield: 44.8%, yellow solid.
$^1$H-NMR (400 MHz, DMSO-$d_6$) δ: 1.04-1.30 (4H, m), 2.14 (3H, s), 2.20 (3H, s), 2.21-2.36 (1H, m), 7.79-7.84 (1H, m), 8.04-8.09 (1H, m), 8.31-8.34 (1H, m), 8.43-8.47 (1H, m), 12.32 (1H, s).

Production Example 197: Synthesis of (3-bromo-4-methoxyphenyl)(5-(5-ethyl-1,3,4-oxadiazol-2-yl)-2,4-dimethyl-1H-pyrrol-3-yl)methanone [compound No. 197]

Production Example 197-1: Synthesis of 4-(3-bromo-4-methoxybenzoyl)-3,5-dimethyl-1H-pyrrole-2-carbohydrazide

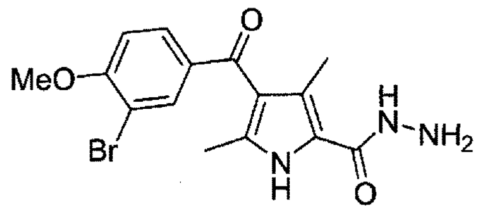

The title compound was obtained by the same procedures as in Production Example 173-1 using the compound obtained in Production Example 108-1 as a starting material.
Yield: 48.9 %, white solid.
$^1$H-NMR (400 MHz, DMSO-$d_6$) δ: 2.08 (3H, s), 2.12 (3H, s), 3.94 (3H, s), 4.27-4.58 (2H, m), 7.20 (1H, d, J = 8.5 Hz), 7.64 (1H, dd, J = 8.5, 2.0 Hz), 7.78 (1H, d, J = 2.2 Hz), 8.74 (1H, s), 11.48 (1H, s).

Production Example 197-2: Synthesis of (3-bromo-4-methoxyphenyl)(5-(5-ethyl-1,3,4-oxadiazol-2-yl)-2,4-dimethyl-1H-pyrrol-3-yl)methanone [compound No. 197]

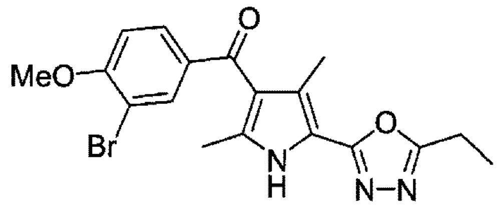

The title compound was obtained by the same procedures as in Production Example 173-2 using the compound

obtained in Production Example 197-1 as a starting material.
Yield: 60.8 %, light brown solid.
$^1$H-NMR (400 MHz, $CDCl_3$) δ: 1.43 (3H, t, J = 7.6 Hz), 2.29 (6H, s), 2.94 (2H, q, J = 7.6 Hz), 3.98 (3H, s), 6.94-6.98 (1H, m), 7.73-7.78 (1H, m), 7.98-8.00 (1H,m), 9.49 (1H, s).

Production Example 198: Synthesis of (3-bromo-4-methoxyphenyl)(5-(5-cyclopropyl-1,3,4-oxadiazol-2-yl)-2,4-dimethyl-1H-pyrrol-3-yl)methanone [compound No. 198]

Production Example 198-1: Synthesis of (3-bromo-4-methoxyphenyl)(5-(5-cyclopropyl-1,3,4-oxadiazol-2-yl)-2,4-dimethyl-1H-pyrrol-3-yl)methanone [compound No. 198]

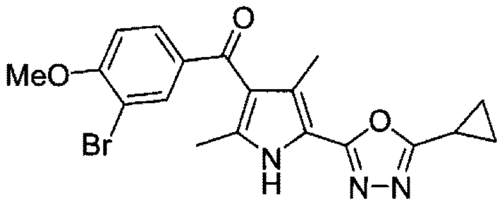

The title compound was obtained by the same procedures as in Production Example 173-2 using the compound obtained in Production Example 197-1 and cyclopropanecarbonyl chloride.
Yield: 91.3 %, yellow solid.
$^1$H-NMR (400 MHz, $CDCl_3$) δ: 1.18 (4H, d, J = 6.7 Hz), 2.20 (1H, quin, J = 6.7 Hz), 2.26 (3H, s), 2.29 (3H, s), 3.98 (3H, s), 6. 95 (1H, d, J = 8.5 Hz), 7. 74 (1H,dd, J = 8.5, 2.0 Hz), 7.98 (1H, d, J = 2.2 Hz), 9.47 (1H, s).

Production Example 199: Synthesis of (5-(5-cyclopropyl-1,3,4-oxadiazol-2-yl)-2,4-dimethyl-1H-pyrrol-3-yl)(3-(hydroxymethyl)phenyl)methanone [compound No. 199]

Production Example 199-1: Synthesis of 4-(3-(hydroxymethyl)benzoyl)-3,5-dimethyl-1H-pyrrole-2-carboxylic acid

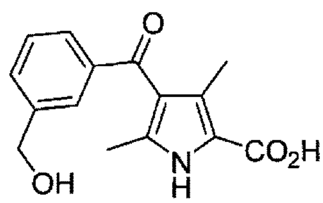

The title compound was obtained by the same procedures as in Production Example 96-1 using compound 44 as a starting material.
Yield: quant, orange solid.
$^1$H-NMR (400 MHz, DMSO-$d_6$) δ: 2.06 (3H, s), 2.15 (3H, s), 4.55 (2H, s), 5.30 (1H, s), 7.39-7.59 (4H, m), 11.83 (1H, s), 12.41 (1H, s).

Production Example 199-2: Synthesis of 4-(3-(hydroxymethyl)benzoyl)-3,5-dimethyl-1H-pyrrole-2-carbohydrazide

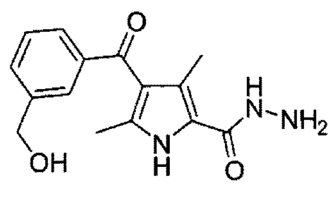

The title compound was obtained by the same procedures as in Production Example 173-1 using the compound obtained in Production Example 199-1 as a starting material. Yield: 26.6%, pale yellow solid. $^1$H-NMR (400 MHz, DMSO-$d_6$) δ: 2.13 (3H, s), 2.17 (3H, s), 4.55 (2H, d, J = 5.6 Hz), 5.29 (1H, t, J = 5.6 Hz), 7.39-7.61 (4H, m), 8.73 (1H, s), 11.48 (1H, s).

Production Example 199-3: Synthesis of (5-(5-cyclopropyl-1,3,4-oxadiazol-2-yl)-2,4-dimethyl-1H-pyrrol-3-yl)(3-(hydroxymethyl)phenyl)methanone [compound No. 199]

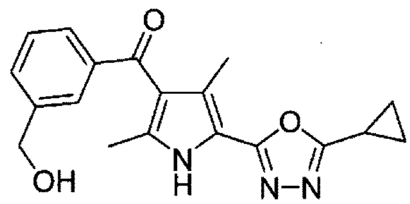

The title compound was obtained by the same procedures as in Production Example 173-2 using the compound obtained in Production Example 199-2 and cyclopropanecarbonyl chloride.
Yield: 13.5 %, white solid.
$^1$H-NMR (400 MHz, $CDCl_3$) δ: 1.18 (4H, d, J = 6.7 Hz), 2.20 (1H, quin, J = 6.7 Hz), 2.25 (3H, s), 2.25 (3H, s), 4.77 (2H, s), 7.43-7.48 (1H, m), 7.55-7.59 (1H, m), 7.64-7.68 (1H, m), 7.74-7.76 (1H, m), 9.24 (1H, s).

Production Example 200: Synthesis of (3-bromophenyl)(5-(5-cyclopropyl-1,3,4-oxadiazol-2-yl)-2-(1,3-dithian-2-yl)-4-methyl-1H-pyrrol-3-yl)methanone [compound No. 200]

Production Example 200-1: Synthesis of 4-(3-bromobenzoyl)-5-(1,3-dithian-2-yl)-3-methyl-1H-pyrrole-2-carboxylic acid

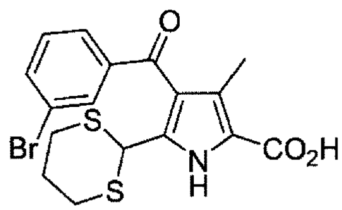

The title compound was obtained by the same procedures as in Production Example 96-1 using compound 66 as a starting material.
Yield: 83.0 %, yellow solid.
$^1$H-NMR (400 MHz, DMSO-$d_6$) δ: 1.55-1.68 (1H, m), 1.93-2.04 (1H, m), 2.06 (3H, s), 2.71-2.90 (4H, m), 5.28 (1H, s), 7.46-7.53 (1H, m), 7.64-7.70 (1H, m), 7.76-7.81 (1H, m), 7.83-7.89 (1H, m), 11.93 (1H, s), 12.74 (1H, s).

Production Example 200-2: Synthesis of 4-(3-bromobenzoyl)-5-(1,3-dithian-2-yl)-3-methyl-1H-pyrrole-2-carbohydrazide

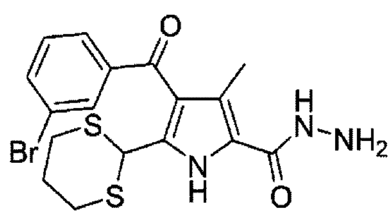

The title compound was obtained by the same procedures as in Production Example 173-1 using the compound obtained in Production Example 200-1 as a starting material.

Yield: 83.5 %, pale yellow solid.
$^1$H-NMR (400 MHz, DMSO-$d_6$) δ: 1.62-1.79 (1H, m), 1.98-2.09 (1H, m), 2.12 (3H, s), 2.72-2.95 (4H, m), 4.39 (1H, s), 5.16 (1H, s), 7.47-7.53 (1H, m), 7.65-7.69 (1H, m), 7.76-7.78 (1H, m), 7.85-7.89 (1H, m), 9.36 (1H, s), 11.74 (1H, s).

Production Example 200-3: Synthesis of (3-bromophenyl)(5-(5-cyclopropyl-1,3,4-oxadiazol-2-yl)-2-(1,3-dithian-2-yl)-4-methyl-1H-pyrrol-3-yl)methanone [compound No. 200]

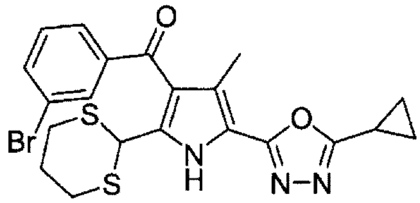

The title compound was obtained by the same procedures as in Production Example 173-2 using the compound obtained in Production Example 200-2 and cyclopropanecarbonyl chloride.
Yield: 48.2 %, yellow solid.
$^1$H-NMR (400 MHz, $CDCl_3$) δ: 1.17-1.23 (4H, m), 1.85-1.95 (1H, m), 2.07-2.15 (1H,m), 2.20-2.25 (1H, m), 2.23 (3H, s), 2.84-2.90 (4H, m), 5.27 (1H, s), 7.33-7.39(1H, m), 7.70-7.76 (2H, m), 7.93-7.95 (1H, m), 9.53 (1H, s).

Production Example 201: Synthesis of (5-bromo-2-methoxyphenyl)(5-(5-(chloromethyl)-1,3,4-oxadiazol-2-yl)-2,4-dimethyl-1H-pyrrol-3-yl)methanone [compound No. 201]

Production Example 201-1: Synthesis of 4-(5-bromo-2-methoxybenzoyl)-3,5-dimethyl-1H-pyrrole-2-carboxylic acid

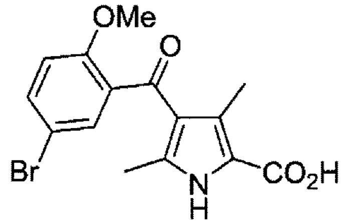

The title compound was obtained by the same procedures as in Production Example 96-1 using compound 35 as a starting material. Yield: quant., yellow solid. $^1$H-NMR (400 MHz, DMSO-$d_6$) δ: 2.02 (3H, s), 2.19 (3H, s), 3.71 (3H, s), 7.08 (1H, d, J = 8.8 Hz), 7.30 (1H, d, J = 2.4 Hz), 7.60 (1H, dd, J = 8.8, 2.4 Hz), 11.87 (1H, br s), 12.53 (1H, br s). ESI-MS m/z: 352[M+H]$^+$, 354[M+2+H]$^+$.

Production Example 201-2: Synthesis of 4-(5-bromo-2-methoxybenzoyl)-3,5-dimethyl-1H-pyrrole-2-carbohydrazide

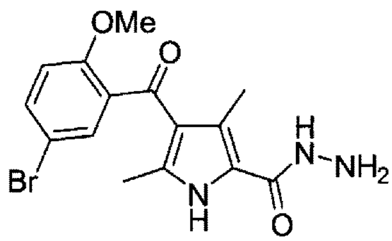

The title compound was obtained in the same way as in Production Example 173-1 using the compound obtained

in Production Example 201-1 as a starting material. Yield: 98.2%, white solid.
$^1$H-NMR (400 MHz, DMSO-$d_6$) δ: 1.96 (3H, s), 2.16 (3H, s), 3.71 (3H, s), 7.07 (1H, d, J = 8.8 Hz), 7.26 (1H, d, J = 2.7 Hz), 7.58 (1H, dd, J = 8.8, 2.7 Hz).
ESI-MS m/z: 366[M+H]$^+$, 368[M+2+H]$^+$.

Production Example 201-3: Synthesis of (5-bromo-2-methoxyphenyl)(5-(5-(chloromethyl)-1,3,4-oxadiazol-2-yl)-2,4-dimethyl-1H-pyrrol-3-yl)methanone [compound No. 201]

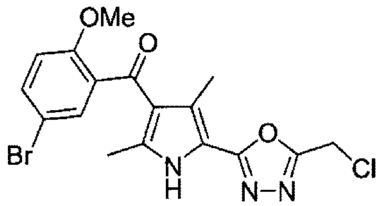

The title compound was obtained by the same procedures as in Production Example 173-2 using the compound obtained in Production Example 201-2 and 2-chloroacetyl chloride. Yield: 29.0%, yellow solid.
$^1$H-NMR (400 MHz, DMSO-$d_6$) δ: 2.07 (3H, s), 2.28 (3H, s), 3.73 (3H, s), 5.10 (2H, s), 7.10 (1H, d, J = 8.8 Hz), 7.36 (1H, d, J = 2.4 Hz), 7.63 (1H, dd, J = 8.8,2.4 Hz), 12.42 (1H, s).
ESI-MS m/z: 424[M+H]$^+$, 426[M+2+H]$^+$.

Production Example 202: Synthesis of (5-(4-(5-bromo-2-methoxybenzoyl)-3,5-dimethyl-1H-pyrrol-2-yl)-1,3,4-oxadiazol-2-yl)methyl acetate [compound No. 202]

Production Example 202-1: Synthesis of (5-(4-(5-bromo-2-methoxybenzoyl)-3,5-dimethyl-1H-pyrrol-2-yl)-1,3,4-oxadiazol-2-yl)methyl acetate [compound No. 202]

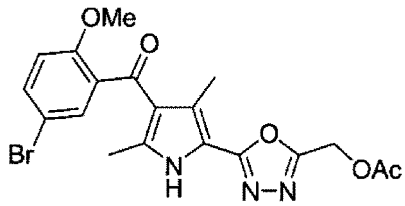

Compound 201 (160 mg, 0.38 mmol) was dissolved in N,N-dimethylformamide (3.2 ml). To the solution, potassium acetate (186 mg, 1.9 mmol) was added, and the mixture was reacted at 60°C for 4 hours. After reaction, the solvent was distilled off. The residue was purified by silica gel column chromatography to obtain the title compound (147 mg, yield: 86.3%) as a yellow oil.
$^1$H-NMR (400 MHz, DMSO-$d_6$) δ: 2.26 (3H, s), 3.72 (3H, s), 5.35 (2H, s), 7.10 (1H, d, J = 8.9 Hz), 7.35 (1H, d, J = 2.4 Hz), 7.63 (1H, dd, J = 8.9, 2.4 Hz), 12.38 (1H, br s). ESI-MS m/z: 448[M+H]$^+$, 450[M+2+H]$^+$.

Production Example 203: Synthesis of (5-bromo-2-hydroxyphenyl)(5-(5-(hydroxymethyl)-1,3,4-oxadiazol-2-yl)-2,4-dimethyl-1H-pyrrol-3-yl)methanone [compound No. 203]

Production Example 203-1: Synthesis of (5-bromo-2-hydroxyphenyl)(5-(5-(hydroxymethyl)-1,3,4-oxadiazol-2-yl)-2,4-dimethyl-1H-pyrrol-3-yl)methanone [compound No. 203]

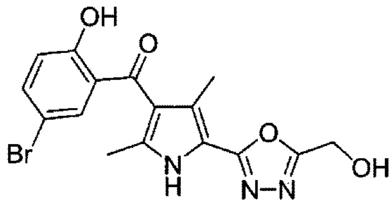

The title compound was obtained in the same way as in Production Example 91-1 using compound 202 as a starting material. Yield: 42.2%, yellow oil.
$^1$H-NMR (400 MHz, DMSO-$d_6$) δ: 2.14 (3H, s), 2.29 (3H, s), 4.69 (2H, d, J = 6.1 Hz), 5.89 (1H, t, J = 6.1 Hz), 6.90 (1H, d, J = 8.8 Hz), 7.34 (1H, d, J = 2.7 Hz), 7.50 (1H, dd, J = 8.8, 2.7 Hz), 10.35 (1H, s), 12.32 (1H, br s). ESI-MS m/z: 392[M+H]$^+$, 394[M+2+H]$^+$.

Production Example 204: Synthesis of 2-(5-(4-(3-bromobenzoyl)-3,5-dimethyl-1H-pyrrol-2-yl)-1,3,4-oxadiazol-2-yl)ethyl acetate [compound No. 204]

Production Example 204-1: Synthesis of 3-acetoxypropanoic acid

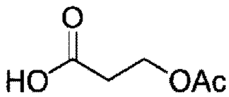

Oxetan-2-one (1.144 g, 15.9 mmol) was dissolved in acetic acid (4.5 ml). To the solution, concentrated sulfuric acid (0.017 ml) was added, and the mixture was stirred at room temperature for 4 hours. After reaction, the solvent was distilled off to obtain the title compound (2.063 g, yield: 98.2%) as a yellow oil.
$^1$H-NMR (400 MHz, DMSO-$d_6$) δ: 1.99 (3H, d, J = 1.0 Hz), 2.56 (2H, td, J = 6.3, 1.0 Hz), 4.17 (2H, td, J = 6.3, 0.7 Hz), 12.34 (1H, br s).

Production Example 204-2: Synthesis of 4-(3-bromobenzoyl)-N'-(3-hydroxypropanoyl)-3,5-dimethyl-1H-pyrrole-2-carbohydrazide

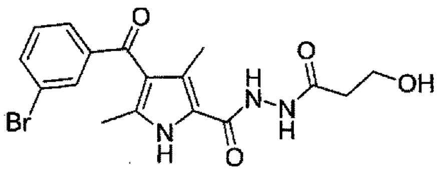

The compound (184 mg, 1.395 mmol) obtained in Production Example 204-1 was dissolved in N,N-dimethylformamide (7 ml). To the solution, HATU (530 mg, 1.395 mmol), the compound (469 mg, 1.395 mmol) obtained in Production Example 173-1, and DIPEA (0.461 ml, 2.79 mmol) were added, and the mixture was stirred overnight at room temperature. After reaction, the solvent was distilled off. After addition of chloroform, the organic layer was washed with 1 M hydrochloric acid, a 1 M aqueous sodium hydroxide solution, and saturated saline. The organic layer was dried over magnesium sulfate. The solvent was distilled off. The residue was purified by silica gel column chromatography to obtain the title compound (552, yield: 96.9%) as a white solid.
$^1$H-NMR (400 MHz, DMSO-$d_6$) δ: 2.08 (3H, s), 2.15 (3H, s), 2.35 (2H, t, J = 6.7 Hz), 3.66 (2H, dd, J = 12.0, 6.7 Hz), 4.65 (1H, t, J = 5.2 Hz), 7.46 (1H, t, J =7.8 Hz), 7.59 (1H, dt, J = 7.8, 1.3 Hz), 7.72 (1H, t, J = 1.8 Hz), 7.80 (1H, dq, J = 8.0, 1.0 Hz), 9.43 (1H, d, J = 1.5 Hz), 9.89 (1H, d, J = 1.7 Hz), 11.72 (1H, br s).
ESI-MS m/z: 408[M+H]$^+$, 410[M+2+H]$^+$.

Production Example 204-3: Synthesis of 3-(2-(4-(3-bromobenzoyl)-3,5-dimethyl-1H-pyrrole-2-carbonyl)hydrazinyl)-3-oxopropyl acetate

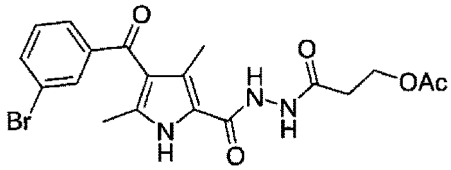

The compound (292 mg, 0.72 mmol) obtained in Production Example 204-2 was dissolved in 1,2-dichloroethane (6 ml). To the solution, acetic anhydride (0.102 ml, 1.08 mmol), pyridine (0.087 ml, 1.08 mmol), and N,N-dimethyl-4-aminopyridine (8.8 mg, 0.072 mmol) were added, and the mixture was stirred at room temperature for 2 hours. After reaction, the solvent was distilled off. The residue was purified by silica gel column chromatography to obtain the title compound (281 mg, yield: 86.7%) as a white solid.
$^1$H-NMR (400 MHz, DMSO-$d_6$) $\delta$: 2.00 (3H, s), 2.08 (3H, s), 2.15 (3H, s), 2.54 (2H, t, J = 6.2 Hz), 4.24 (2H, t, J = 6.2 Hz), 7.46 (1H, t, J = 7.9 Hz), 7.59 (1H, dt, J = 7.9, 1.3 Hz), 7.72 (1H, t, J = 1.7 Hz), 7.79 (1H, dq, J = 7.9, 1.1 Hz), 9.47 (1H, s), 10.01 (1H, br s), 11.71 (1H, br s). ESI-MS m/z: 450[M+H]$^+$, 452[M+2+H]$^+$.

Production Example 204-4: Synthesis of 2-(5-(4-(3-bromobenzoyl)-3,5-dimethyl-1H-pyrrol-2-yl)-1,3,4-oxadiazol-2-yl)ethyl acetate [compound No. 204]

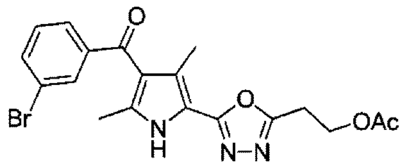

The compound (163 mg, 0.36 mmol) obtained in Production Example 204-3 was dissolved in acetonitrile (3.3 ml). To the solution, phenylphosphonic dichloride (0.051 ml, 0.36 mmol) was added, and the mixture was stirred overnight at 80°C. After reaction, the solvent was distilled off. After addition of chloroform, the organic layer was washed with a saturated aqueous solution of sodium bicarbonate. The organic layer was dried over magnesium sulfate. The solvent was distilled off. The residue was purified by silica gel column chromatography to obtain the title compound (110 mg, yield: 70.7%) as a white solid.
$^1$H-NMR (400 MHz, DMSO-$d_6$) $\delta$: 2.00 (3H, s), 2.13 (3H, s), 2.22 (3H, s), 3.27 (2H, t, J = 6.2 Hz), 4.42 (2H, t, J = 6.2 Hz), 7.48 (1H, t, J = 7.9 Hz), 7.62 (1H, dt, J = 7.9, 1.0 Hz), 7.75 (1H, t, J = 1.7 Hz), 7.81 (1H, dq, J = 7.9, 1.0 Hz), 12.33 (1H, br s).
ESI-MS m/z: 432[M+H]$^+$, 434[M+2+H]$^+$.

Production Example 205: Synthesis of (3-bromophenyl)(5-(5-(2-hydroxyethyl)-1,3,4-oxadiazol-2-yl)-2,4-dimethyl-1H-pyrrol-3-yl)methanone [compound No. 205]

Production Example 205-1: Synthesis of (3-bromophenyl)(5-(5-(2-hydroxyethyl)-1,3,4-oxadiazol-2-yl)-2,4-dimethyl-1H-pyrrol-3-yl)methanone [compound No. 205]

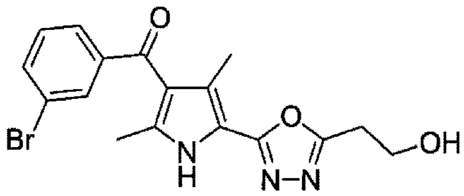

The title compound was obtained in the same way as in Production Example 117-1 using compound 204 as a starting material. Yield: 79.2 %, yellow solid.
$^1$H-NMR (400 MHz, DMSO-$d_6$) δ: 2.13 (3H, s), 2.21 (3H, s), 3.04 (2H, t, J = 6.3 Hz), 3.80-3.85 (2H, m), 4.97 (1H, t, J = 5.6 Hz), 7.48 (1H, t, J = 7.9 Hz), 7.61 (1H, dq, J = 7.9, 1.0 Hz), 7.74 (1H, t, J = 1.6 Hz), 7.81 (1H, dq, J = 7.9, 1.0 Hz), 12.32 (1H, br s).
ESI-MS m/z: 390[M+H]$^+$, 392[M+2+H]$^+$.

Production Example 206: Synthesis of (3-bromophenyl)(2,4-dimethyl-5-(5-vinyl-1,3,4-oxadiazol-2-yl)-1H-pyrrol-3-yl)methanone [compound No. 206]

Production Example 206-1: Synthesis of (3-bromophenyl)(2,4-dimethyl-5-(5-vinyl-1,3,4-oxadiazol-2-yl)-1H-pyrrol-3-yl)methanone [compound No. 206]

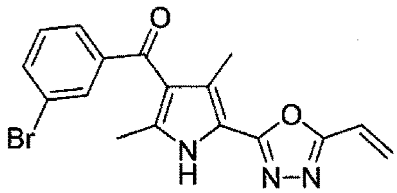

Compound 174 (22 mg, 0.054 mmol) was dissolved in N,N-dimethylformamide (0.5 ml). To the solution, potassium acetate (26 mg, 0.27 mmol) was added, and the mixture was stirred overnight at 50°C. After reaction, the solvent was distilled off. Water was added to the residue, followed by extraction with chloroform. The organic layer was dried over magnesium sulfate. The solvent was distilled off. The residue was purified by silica gel column chromatography to obtain the title compound (13 mg, yield: 64.7%) as a yellow solid.
$^1$H-NMR (400 MHz, DMSO-$d_6$) δ: 2.16 (3H, s), 2.25 (3H, s), 5.94 (1H, d, J = 11.2 Hz), 6.29 (1H, d, J = 17.6 Hz), 6.87 (1H, dd, J = 17.6, 11.2 Hz), 7.48 (1H, t, J= 7.8 Hz), 7.63 (1H, dt, J = 7.6, 1.3 Hz), 7.76 (1H, t, J = 1.6 Hz), 7.82 (1H, dq, J = 7.9, 1.0 Hz), 12.38 (1H, br s).
ESI-MS m/z: 373[M+H]$^+$, 375[M+2+H]$^+$.

Production Example 207: Synthesis of (3-bromophenyl)(5-(5-(chloromethyl)-1,3,4-oxadiazol-2-yl)-2,4-dimethyl-1H-pyrrol-3-yl)methanone [compound No. 207]

Production Example 207-1: Synthesis of (3-bromophenyl)(5-(5-(chloromethyl)-1,3,4-oxadiazol-2-yl)-2,4-dimethyl-1H-pyrrol-3-yl)methanone

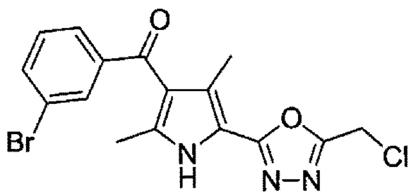

The title compound was obtained in the same way as in Production Example 173-2 using 2-chloroacetyl chloride.

Yield: 52.7 %, light brown solid.
$^1$H-NMR (400 MHz, DMSO-$d_6$) δ: 2.14 (3H, s), 2.24 (3H, s), 7.48 (1H, t, J = 7.7 Hz), 7.63 (1H, dt, J = 7.6, 1.3 Hz), 7.76 (1H, t, J = 1.7 Hz), 7.82 (1H, dq, J = 8.0, 1.1 Hz), 12.46 (1H, s).

Production Example 208: Synthesis of (3-bromophenyl)(2,4-dimethyl-5-(5-((4-methylpiperazin-1-yl)methyl)-1,3,4-oxadiazol-2-yl)-1H-pyrrol-3-yl)methanone [compound No. 208]

Production Example 208-1: Synthesis of (3-bromophenyl)(2,4-dimethyl-5-(5-((4-methylpiperazin-1-yl)methyl)-1,3,4-oxadiazol-2-yl)-1H-pyrrol-3-yl)methanone [compound No. 208]

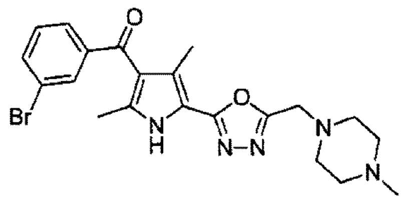

Compound 207 (70 mg, 0.18 mmol) was dissolved in methanol (1.4 ml). To the solution, N-methylpiperazine (0.04 ml, 0.36 mmol) was added, and the mixture was stirred overnight at 50°C. After reaction, the solvent was distilled off. The residue was dissolved in chloroform. The organic layer was washed with a saturated aqueous solution of sodium bicarbonate. The organic layer was dried over magnesium sulfate. The solvent was distilled off. The residue was purified by silica gel column chromatography to obtain the title compound (56 mg, yield: 67.9%) as a yellow solid. ESI-MS m/z: 458[M+H]$^+$, 460[M+2+H]$^+$.

Production Example 209: Synthesis of (3-bromophenyl)(5-(5-((dimethylamino)methyl)-1,3,4-oxadiazol-2-yl)-2,4-dimethyl-1H-pyrrol-3-yl)methanone [compound No. 209]

Production Example 209-1: Synthesis of (3-bromophenyl)(5-(5-((dimethylamino)methyl)-1,3,4-oxadiazol-2-yl)-2,4-dimethyl-1H-pyrrol-3-yl)methanone [compound No. 209]

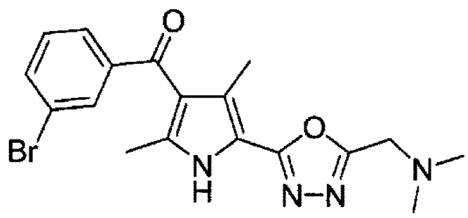

Dimethylamine hydrochloride (28 mg, 0.34 mmol) and triethylamine (0.047 ml, 0.34 mmol) were added to a solution of compound 207 (68 mg, 0.17 mmol) in methanol (1.4 ml), and the mixture was stirred overnight at 50°C. After reaction, the solvent was distilled off. After addition of chloroform, the organic layer was washed with a saturated aqueous solution of sodium bicarbonate. The organic layer was dried over magnesium sulfate. The solvent was distilled off. The residue was purified by silica gel column chromatography to obtain the title compound (47 mg, yield: 68.6%) as a yellow solid.
$^1$H-NMR (400 MHz, DMSO-$d_6$) δ: 2.13 (3H, s), 2.23 (3H, s), 2.26 (6H, s), 3.78 (2H, s), 7.48 (1H, t, J = 8.0 Hz), 7.62 (1H, dt, J = 8.0, 1.3 Hz), 7.75 (1H, t, J =1.8 Hz), 7.81 (1H, dq, J = 8.0, 1.0 Hz), 12.35 (1H, s).
ESI-MS m/z: 403[M+H]$^+$, 405[M+2+H]$^+$.

Production Example 210: Synthesis of (5-(4-(3-bromobenzoyl)-3,5-dimethyl-1H-pyrrol-2-yl)-1,3,4-oxadiazol-2-yl)methyl acetate [compound No. 210]

Production Example 210-1: Synthesis of (5-(4-(3-bromobenzoyl)-3,5-dimethyl-1H-pyrrol-2-yl)-1,3,4-oxadiazol-2-yl)methyl acetate [compound No. 210]

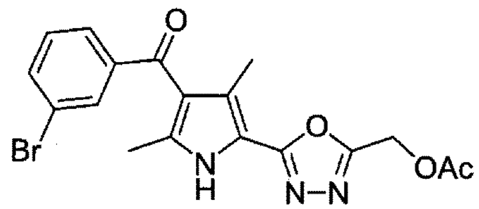

The title compound was obtained in the same way as in Production Example 202-1 using compound 207 as a starting material. Yield: 83.7 %, yellow solid.
$^1$H-NMR (400 MHz, DMSO-$d_6$) δ: 2.13 (3H, s), 2.14 (3H, s), 2.22 (3H, s), 5.36 (2H, s), 7.48 (1H, t, J = 7.8 Hz), 7.62 (1H, dt, J = 7.6, 1.2 Hz), 7.75 (1H, t, J = 1.7 Hz), 7.82 (1H, dq, J = 7.9, 1.2 Hz), 12.42 (1H, br s).

Production Example 211: Synthesis of (3-bromophenyl)(5-(5-(hydroxymethyl)-1,3,4-oxadiazol-2-yl)-2,4-dimethyl-1H-pyrrol-3-yl)methanone [compound No. 211]

Production Example 211-1: Synthesis of (3-bromophenyl)(5-(5-(hydroxymethyl)-1,3,4-oxadiazol-2-yl)-2,4-dimethyl-1H-pyrrol-3-yl)methanone [compound No. 211]

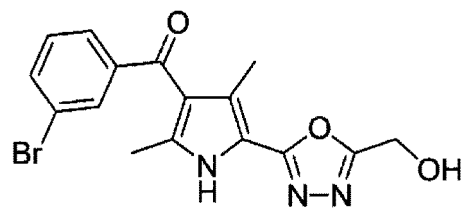

The title compound was obtained in the same way as in Production Example 117-1 using compound 210 as a starting material. Yield: 43.5 %, white solid.
$^1$H-NMR (400 MHz, DMSO-$d_6$) δ: 2.13 (3H, s), 2.23 (3H, s), 4.69 (2H, s), 5.89 (1H, br s), 7.48 (1H, t, J = 7.8 Hz), 7.62 (1H, dt, J = 7.7, 1.3 Hz), 7.75 (1H, t, J = 1.7 Hz), 7.81 (1H, dq, J = 7.9, 1.0 Hz), 12.37 (1H, br s).

Production Example 212: Synthesis of S-((5-(4-(3-bromobenzoyl)-3,5-dimethyl-1H-pyrrol-2-yl)-1,3,4-oxadiazol-2-yl)methyl)ethanethioate [compound No. 212]

Production Example 212-1: Synthesis of S-((5-(4-(3-bromobenzoyl)-3,5-dimethyl-1H-pyrrol-2-yl)-1,3,4-oxadiazol-2-yl)methyl)ethanethioate [compound No. 212]

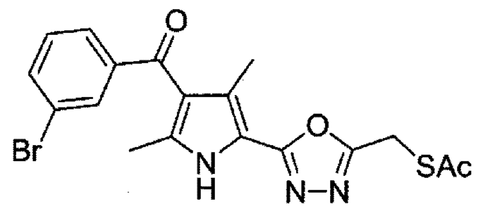

Compound 207 (124 mg, 0.31 mmol) was dissolved in N,N-dimethylformamide (2.5 ml). To the solution, S-potassium thioacetate (177 mg, 1.55 mmol) was added, and the mixture was stirred overnight at 50°C. After reaction, the solvent was distilled off. Water was added to the residue, followed by extraction with chloroform. The organic layer was washed with saturated saline. The organic layer was dried over magnesium sulfate. The solvent was distilled off. The residue was purified by silica gel column chromatography to obtain the title compound (113 mg, yield: 83.9%) as a yellow solid. $^1$H-NMR (400 MHz, DMSO-$d_6$) δ: 2.13 (3H, s), 2.19 (3H, s), 2.43 (3H, s), 4.45 (2H, s), 7.48 (1H, t, J = 7.8 Hz), 7.61 (1H, dt, J = 7.8, 1.3 Hz), 7.74 (1H, t, J =1.8 Hz), 7.81 (1H, dq, J = 8.0, 1.0 Hz), 12.37 (1H, s).
ESI-MS m/z: 434[M+H]$^+$, 436[M+2+H]$^+$.

Production Example 213: Synthesis of 2-((5-(4-(3-bromobenzoyl)-3,5-dimethyl-1H-pyrrol-2-yl)-1,3,4-oxadiazol-2-yl)methyl)isoindole-1,3-dione [compound No. 213]

Production Example 213-1: Synthesis of 2-((5-(4-(3-bromobenzoyl)-3,5-dimethyl-1H-pyrrol-2-yl)-1,3,4-oxadiazol-2-yl)methyl)isoindole-1,3-dione [compound No. 213]

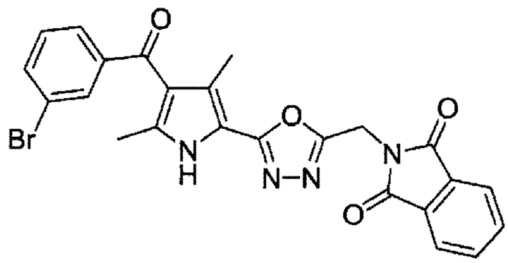

Compound 207 (49 mg, 0.124 mmol) was dissolved in N,N-dimethylformamide (1 ml). To the solution, phthalimide potassium (115 mg, 0.62 mmol) was added, and the mixture was stirred at 50°C for 3 hours. After reaction, the solvent was distilled off. Water was added to the residue, followed by extraction with chloroform. The organic layer was dried over magnesium sulfate. The solvent was distilled off. The residue was purified by silica gel column chromatography to obtain the title compound (58 mg, yield: 92.6%) as a yellow solid.
$^1$H-NMR (400 MHz, DMSO-$d_6$) δ: 2.12 (3H, s), 2.17 (3H, s), 5.13 (2H, s), 7.47 (1H, t, J = 7.8 Hz), 7.60 (1H, dt, J = 7.8, 1.3 Hz), 7.73 (1H, t, J = 1.7 Hz), 7.80(1H, dd, J = 2.1, 1.1 Hz), 7. 89-7. 93 (2H, m), 7.95-7.98 (2H, m), 12.35 (1H, br s).
ESI-MS m/z: 505[M+H]$^+$, 507[M+2+H]$^+$.

Production Example 214: Synthesis of (5-(5-(aminomethyl)-1,3,4-oxadiazol-2-yl)-2,4-dimethyl-1H-pyrrol-3-yl)(3-bromophenyl)methanone [compound No. 214]

Production Example 214-1: Synthesis of (5-(5-(aminomethyl)-1,3,4-oxadiazol-2-yl)-2,4-dimethyl-1H-pyrrol-3-yl)(3-bromophenyl)methanone [compound No. 214]

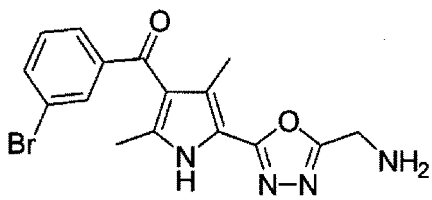

A methanol solution containing 40% methylamine (2 ml) was added to compound 213 (103 mg, 0.2 mmol). The mixture was stirred overnight at room temperature. After reaction, the solvent was distilled off. A 1 M aqueous sodium hydroxide solution was added to the residue, followed by extraction with chloroform. The organic layer was dried over magnesium sulfate. The solvent was distilled off. The residue was purified by silica gel column chromatography to obtain the title compound (59 mg, yield: 78.6%) as a yellow solid.
$^1$H-NMR (400 MHz, DMSO-$d_6$) δ: 2.13 (3H, s), 2.22 (3H, s), 3.94 (2H, s), 7.48 (1H, t, J = 7.9 Hz), 7.62 (1H, dt, J = 7.9,

1.1 Hz), 7.74 (1H, t, J = 1.8 Hz), 7.81(1H, dq, J = 7.9, 1.1 Hz), 12.34 (1H, br s).
ESI-MS m/z: 375[M+H]$^+$, 377[M+2+H]$^+$.

Production Example 215: Synthesis of N-((5-(4-(3-bromobenzoyl)-3,5-dimethyl-1H-pyrrol-2-yl)-1,3,4-oxadiazol-2-yl)methyl)acetamide [compound No. 215]

Production Example 215-1: Synthesis of N-((5-(4-(3-bromobenzoyl)-3,5-dimethyl-1H-pyrrol-2-yl)-1,3,4-oxadiazol-2-yl)methyl)acetamide [compound No. 215]

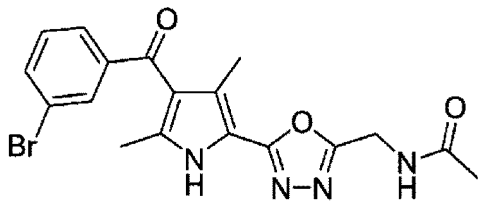

Compound 214 (35 mg, 0.09 mmol) was dissolved in dichloromethane (1 ml). To the solution, acetyl chloride (0.008 ml, 0.108 mmol) and triethylamine (0.019 ml, 0.135 mmol) were added, and the mixture was stirred at room temperature for 1.5 hours. After reaction, the solvent was distilled off. The residue was purified by silica gel column chromatography to obtain the title compound (38 mg, yield: quant.) as a yellow solid.
$^1$H-NMR (400 MHz, DMSO-d$_6$) δ: 1.89 (3H, s), 2.13 (3H, s), 2.20 (3H, s), 4.54 (2H, d, J = 5.5 Hz), 7.48 (1H, t, J = 7.8 Hz), 7.61 (1H, d, J = 7.8 Hz), 7.74 (1H, t, J = 1.7 Hz), 7.81 (1H, dt, J = 7.8, 1.0 Hz), 8.62 (1H, t, J = 5.5 Hz), 12.36 (1H, br s).
ESI-MS m/z: 417[M+H]$^+$, 419[M+2+H]$^+$.

Production Example 216: Synthesis of methyl ((5-(4-(3-bromobenzoyl)-3,5-dimethyl-1H-pyrrol-2-yl)-1,3,4-oxadiazol-2-yl)methyl)carbamate [compound No. 216]

Production Example 216-1: Synthesis of methyl ((5-(4-(3-bromobenzoyl)-3,5-dimethyl-1H-pyrrol-2-yl)-1,3,4-oxadiazol-2-yl)methyl)carbamate [compound No. 216]

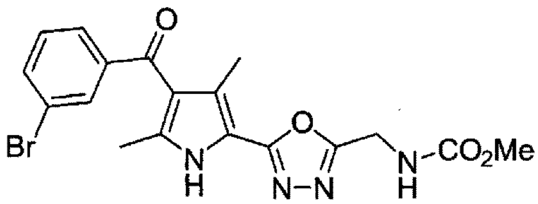

Compound 214 (39 mg, 0.10 mmol) was dissolved in dichloromethane (1 ml). To the solution, methyl chloroformate (0.009 ml, 0.12 mmol) and triethylamine (0.021 ml, 0.15 mmol) were added, and the mixture was stirred at room temperature for 2 hours. After reaction, the solvent was distilled off. The residue was purified by silica gel column chromatography to obtain the title compound (32 mg, yield: 73.9%) as a yellow solid.
$^1$H-NMR (400 MHz, DMSO-d$_6$) δ: 2.13 (3H, s), 2.20 (3H, s), 3.58 (3H, s), 4.49 (2H, d, J = 5.6 Hz), 7.48 (1H, t, J = 7.9 Hz), 7.62 (1H, dt, J = 7.9, 1.1 Hz), 7.75(1H, t, J = 1.8 Hz), 7.81 (1H, dq, J = 7.9, 1.1 Hz), 7.93 (1H, t, J = 5.6 Hz), 12.36 (1H, br s).
ESI-MS m/z: 433[M+H]$^+$, 435[M+2+H]$^+$.

Production Example 217: Synthesis of N-((5-(4-(3-bromobenzoyl)-3,5-dimethyl-1H-pyrrol-2-yl)-1,3,4-oxadiazol-2-yl)methyl)methanesulfonamide [compound No. 217]

Production Example 217-1: Synthesis of N-((5-(4-(3-bromobenzoyl)-3,5-dimethyl-1H-pyrrol-2-yl)-1,3,4-oxadiazol-2-yl)methyl)methanesulfonamide [compound No. 217]

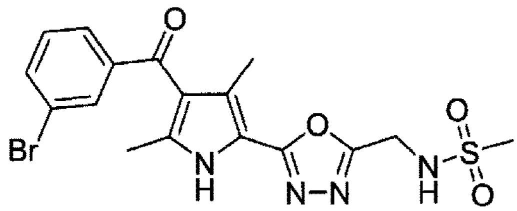

Compound 214 (40 mg, 0.11 mmol) was dissolved in dichloromethane (1 ml). To the solution, mesyl chloride (0.010 ml, 0.132 mmol) and triethylamine (0.023 ml, 0.165 mmol) were added, and the mixture was stirred overnight at room temperature. After reaction, the solvent was distilled off. The residue was purified by silica gel column chromatography to obtain the title compound (35 mg, yield: 70.2%) as a yellow solid.
$^1$H-NMR (400 MHz, DMSO-$d_6$) $\delta$: 2.14 (3H, s), 2.23 (3H, s), 3.02 (3H, s), 4.52 (2H, d, J = 5.4 Hz), 7.48 (1H, t, J = 7.9 Hz), 7.62 (1H, dd, J = 7.9, 1.0 Hz), 7.75(1H, s), 7.81 (1H, dq, J = 7.9, 1.0 Hz), 7.97 (1H, t, J = 5.4 Hz), 12.40 (1H, br s).
ESI-MS m/z: 453[M+H]$^+$, 455[M+2+H]$^+$.

Production Example 218: Synthesis of 1-((5-(4-(3-bromobenzoyl)-3,5-dimethyl-1H-pyrrol-2-yl)-1,3,4-oxadiazol-2-yl)methyl)-3-ethylurea [compound No. 218]

Production Example 218-1: Synthesis of 1-((5-(4-(3-bromobenzoyl)-3,5-dimethyl-1H-pyrrol-2-yl)-1,3,4-oxadiazol-2-yl)methyl)-3-ethylurea [compound No. 218]

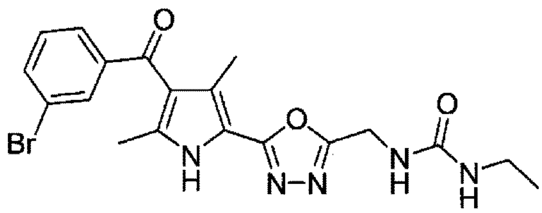

Compound 214 (37 mg, 0.099 mmol) was dissolved in dichloromethane (1 ml). To the solution, ethyl isocyanate (0.012 ml, 0.15 mmol) was added, and the mixture was stirred overnight at room temperature. After reaction, the solvent was distilled off. The residue was purified by silica gel column chromatography to obtain the title compound (42 mg, yield: 95.1%) as a yellow solid.
$^1$H-NMR (400 MHz, DMSO-$d_6$) $\delta$: 0.99 (3H, t, J = 7.1 Hz), 2.13 (3H, s), 2.20 (3H, s), 2.98-3.06 (2H, m), 4.48 (2H, d, J = 5.7 Hz), 6.16 (1H, t, J = 5.7 Hz), 6.52 (1H, t, J = 6.0 Hz), 7.48 (1H, t, J = 7.8 Hz), 7.61 (1H, dt, J = 7.8, 1.2 Hz), 7.74 (1H, t, J = 1.8 Hz), 7.81 (1H, dq, J = 7.8, 1.2 Hz), 12.36 (1H, br s).
ESI-MS m/z: 446[M+H]$^+$, 447[M+2+H]$^+$.

Production Example 219: Synthesis of 1-((5-(4-(3-bromobenzoyl)-3,5-dimethyl-1H-pyrrol-2-yl)-1,3,4-oxadiazol-2-yl)methyl)-3-ethylthiourea [compound No. 219]

Production Example 219-1: Synthesis of 1-((5-(4-(3-bromobenzoyl)-3,5-dimethyl-1H-pyrrol-2-yl)-1,3,4-oxadiazol-2-yl)methyl)-3-ethylthiourea [compound No. 219]

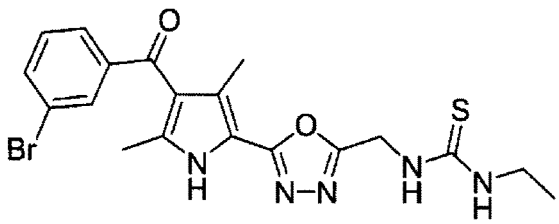

Compound 214 (37 mg, 0.099 mmol) was dissolved in dichloromethane (1 ml). To the solution, ethyl isothiocyanate (0.013 ml, 0.15 mmol) was added, and the mixture was stirred overnight at room temperature. After reaction, the solvent was distilled off. The residue was purified by silica gel column chromatography to obtain the title compound (39 mg, yield: 85.2%) as a yellow solid.
$^1$H-NMR (400 MHz, DMSO-$d_6$) δ: 1.08 (3H, t, J = 7.2 Hz), 2.13 (3H, s), 2.20 (3H, s), 3.36-3.45 (2H, m), 4.94 (2H, d, J = 5.1 Hz), 7.48 (1H, t, J = 7.8 Hz), 7.62 (1H, d, J = 7.8 Hz), 7.74 (1H, s), 7.81 (1H, dq, J = 7.8, 1.0 Hz), 7.94 (1H, s), 12.37 (1H, br s).
ESI-MS m/z: 462[M+H]$^+$, 464[M+2+H]$^+$.

Production Example 220: Synthesis of 3-((5-(4-(3-bromobenzoyl)-3,5-dimethyl-1H-pyrrol-2-yl)-1,3,4-oxadiazol-2-yl)methyl)-1,1-dimethylthiourea [compound No. 220]

Production Example 220-1: Synthesis of 3-((5-(4-(3-bromobenzoyl)-3,5-dimethyl-1H-pyrrol-2-yl)-1,3,4-oxadiazol-2-yl)methyl)-1,1-dimethylthiourea [compound No. 220]

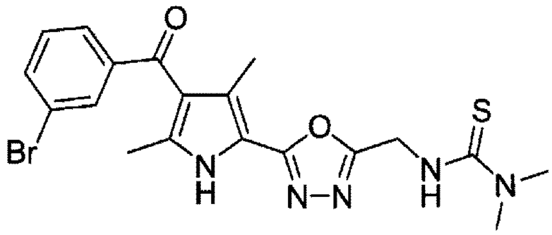

Compound 214 (34 mg, 0.09 mmol) was dissolved in dichloromethane (1 ml). To the solution, thiophosgene (0.008 ml, 0.11 mmol) and triethylamine (0.03 ml, 0.216 mmol) were added, and the mixture was stirred at room temperature for 6 hours. Dimethylamine hydrochloride (15 mg, 0.18 mmol) and triethylamine (0.025 ml, 0.18 mmol) were added thereto, and the mixture was stirred overnight at room temperature. After reaction, the solvent was distilled off. The residue was purified by silica gel column chromatography to obtain the title compound (42 mg, yield: quant.) as a yellow solid.
$^1$H-NMR (400 MHz, DMSO-$d_6$) δ: 2.13 (3H, s), 2.20 (3H, s), 3.20 (6H, s), 4.95 (2H, d, J = 5.1 Hz), 7.48 (1H, t, J = 7.8 Hz), 7.62 (1H, dt, J = 7.8, 1.3 Hz), 7.75(1H, t, J = 1.8 Hz), 7.81 (1H, dq, J = 7.8, 1.3 Hz), 8.10 (1H, t, J = 5.1 Hz), 12.34 (1H, br s).
ESI-MS m/z: 462[M+H]$^+$, 464[M+2+H]$^+$.

Production Example 221: Synthesis of 2-(((5-(4-(3-bromobenzoyl)-3,5-dimethyl-1H-pyrrol-2-yl)-1,3,4-oxadiazol-2-yl)methyl)amino)-2-oxoethyl acetate [compound No. 221]

Production Example 221-1: Synthesis of 2-(((5-(4-(3-bromobenzoyl)-3,5-dimethyl-1H-pyrrol-2-yl)-1,3,4-oxadiazol-2-yl)methyl)amino)-2-oxoethyl acetate [compound No. 221]

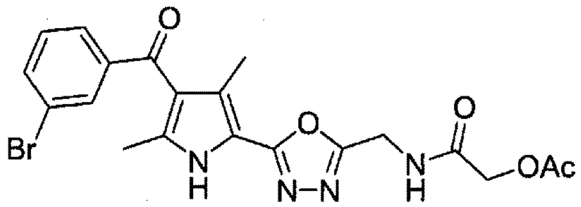

Compound 214 (61 mg, 0.16 mmol) was dissolved in dichloromethane (1.2 ml). To the solution, 2-acetoxyacetyl chloride (0.021 ml, 0.20 mmol) and triethylamine (0.033 ml, 0.24 mmol) were added, and the mixture was stirred at room temperature for 3 hours. After reaction, the solvent was distilled off. The residue was purified by silica gel column chromatography to obtain the title compound (79 mg, yield: quant.) as a yellow solid.
$^1$H-NMR (400 MHz, DMSO-$d_6$) δ: 2.09 (3H, s), 2.13 (3H, s), 2.20 (3H, s), 4.53 (2H, s), 4.60 (2H, d, J = 5.6 Hz), 7.48 (1H, t, J = 8.0 Hz), 7.62 (1H, d, J = 8.0 Hz), 7.75 (1H, t, J = 1.6 Hz), 7.81 (1H, dq, J = 8.0, 0.9 Hz), 8.81 (1H, t, J = 5.6 Hz), 12.37 (1H, br s).
ESI-MS m/z: 475$[M+H]^+$, 476$[M+2+H]^+$.

Production Example 222: Synthesis of N-((5-(4-(3-bromobenzoyl)-3,5-dimethyl-1H-pyrrol-2-yl)-1,3,4-oxadiazol-2-yl)methyl)-2-hydroxyacetamide [compound No. 222]

Production Example 222-1: Synthesis of N-((5-(4-(3-bromobenzoyl)-3,5-dimethyl-1H-pyrrol-2-yl)-1,3,4-oxadiazol-2-yl)methyl)-2-hydroxyacetamide [compound No. 222]

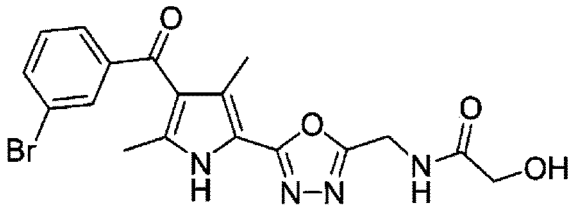

The title compound was obtained in the same way as in Production Example 117-1 using compound 221 as a starting material. Yield: 56.7 %, white solid.
$^1$H-NMR (400 MHz, DMSO-$d_6$) δ: 2.13 (3H, s), 2.20 (3H, s), 3.86 (1H, t, J = 6.6 Hz), 3.89 (2H, d, J = 5.9 Hz), 4.58 (2H, d, J = 5.9 Hz), 5.62 (1H, t, J = 5.9 Hz), 7.48 (1H, t, J = 7.8 Hz), 7.61 (1H, dt, J = 7.8, 1.1 Hz), 7.74 (1H, t, J = 1.8Hz), 7.81 (1H, dq, J = 7.8, 1.1 Hz), 8.52 (1H, t, J = 5.9 Hz), 12.37 (1H, br s).
ESI-MS m/z: 433$[M+H]^+$, 435$[M+2+H]^+$.

Production Example 223: Synthesis of (3-bromophenyl)(5-(5-ethyl-1,3,4-oxadiazol-2-yl)-1,2,4-trimethyl-1H-pyrrol-3-yl)methanone [compound No. 223]

Production Example 223-1: Synthesis of (3-bromophenyl)(5-(5-ethyl-1,3,4-oxadiazol-2-yl)-1,2,4-trimethyl-1H-pyrrol-3-yl)methanone [compound No. 223]

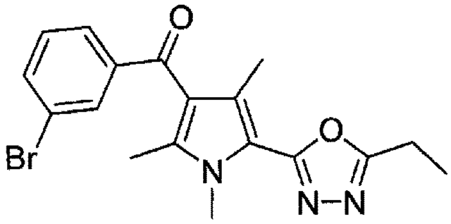

Compound 173 (68 mg, 0.18 mmol) was dissolved in N,N-dimethylformamide (1.4 ml). To the solution, 60%

sodium hydride (10 mg, 0.216 mmol) was added, and the mixture was stirred at room temperature for 30 minutes. Methyl iodide (0.017 ml, 0.27 mmol) was added thereto, and the mixture was stirred overnight at room temperature. After reaction, the solvent was distilled off. Water was added to the residue, followed by extraction with chloroform. The organic layer was dried over magnesium sulfate, and the solvent was distilled off. The residue was purified by silica gel column chromatography to obtain the title compound (48 mg, yield: 68.7%) as a yellow oil.
$^1$H-NMR (400 MHz, DMSO-$d_6$) δ: 1.31 (3H, t, J = 7.6 Hz), 2.10 (3H, s), 2.18 (3H, s), 2.93 (2H, q, J = 7.6 Hz), 3.81 (3H, s), 7.49 (1H, t, J = 7.9 Hz), 7.64-7.66 (1H, m), 7.78 (1H, t, J = 1.7 Hz), 7.83 (1H, dq, J = 7.9, 1.0 Hz). ESI-MS m/z: 388[M+H]$^+$, 390[M+2+H]$^+$.

Production Example 224: Synthesis of 1-(3-(3-bromobenzoyl)-5-(5-ethyl-1,3,4-oxadiazol-2-yl)-2,4-dimethyl-1H-pyrrol-1-yl)ethanone [compound No. 224]

Production Example 224-1: Synthesis of 1-(3-(3-bromobenzoyl)-5-(5-ethyl-1,3,4-oxadiazol-2-yl)-2,4-dimethyl-1H-pyrrol-1-yl)ethanone [compound No. 224]

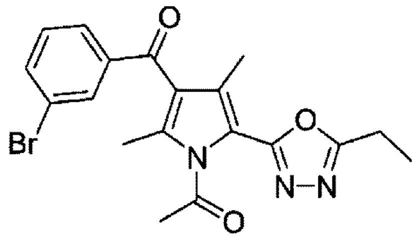

Compound 173 (68 mg, 0.18 mmol) was dissolved in N,N-dimethylformamide (1.4 ml). To the solution, 60% sodium hydride (10 mg, 0.216 mmol) was added, and the mixture was stirred at room temperature for 30 minutes. Acetyl chloride (0.019 ml, 0.27 mmol) was added thereto, and the mixture was stirred at room temperature for 3.5 hours. After reaction, the solvent was distilled off. The residue was purified by silica gel column chromatography to obtain the title compound (25 mg, yield: 33.3%) as a colorless oil.
$^1$H-NMR (400 MHz, DMSO-$d_6$) δ: 1.29 (3H, t, J = 7.7 Hz), 1.99 (3H, s), 2.28 (3H, s), 2.92 (2H, q, J = 7.7 Hz), 7.51-7.54 (1H, m), 7.74-7.76 (1H, m), 7.88-7.90 (2H, m). ESI-MS m/z: 416[M+H]$^+$, 418[M+2+H]$^+$.

Production Example 225: Synthesis of (3-bromophenyl)(5-(5-ethyl-1,3,4-oxadiazol-2-yl)-2,4-dimethyl-1-(methylsulfonyl)-1H-pyrrol-3-yl)methanone [compound No. 225]

Production Example 225-1: Synthesis of (3-bromophenyl)(5-(5-ethyl-1,3,4-oxadiazol-2-yl)-2,4-dimethyl-1-(methylsulfonyl)-1H-pyrrol-3-yl)methanone [compound No. 225]

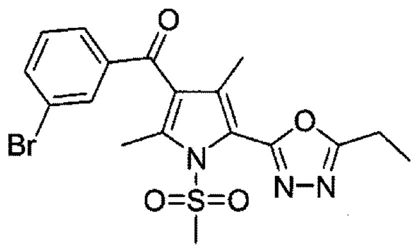

Compound 173 (68 mg, 0.18 mmol) was dissolved in N,N-dimethylformamide (1.4 ml). To the solution, 60% sodium hydride (10 mg, 0.216 mmol) was added, and the mixture was stirred at room temperature for 30 minutes. Methanesulfonyl chloride (0.021 ml, 0.27 mmol) was added thereto, and the mixture was stirred at room temperature for 2 hours. After reaction, the solvent was distilled off. The residue was purified by silica gel column chromatography to obtain the title compound (77 mg, yield: 94.6%) as a colorless oil.
$^1$H-NMR (400 MHz, DMSO-$d_6$) δ: 1.30 (3H, t, J = 7.6 Hz), 1.85 (3H, s), 2.38 (3H, s), 2.95 (2H, q, J = 7.6 Hz), 3.74 (3H, s), 7.54 (1H, t, J = 7.8 Hz), 7.77 (1H, dt, J = 7.8, 1.1 Hz), 7.89-7.92 (1H, m), 7.93-7.93 (1H, m). ESI-MS m/z: 452[M+H]$^+$,

454[M+2+H]+.

Production Example 226: Synthesis of 2-(3-(3-bromobenzoyl)-5-(5-ethyl-1,3,4-oxadiazol-2-yl)-2,4-dimethyl-1H-pyrrol-1-yl)-2-oxoethyl acetate [compound No. 226]

Production Example 226-1: Synthesis of 2-(3-(3-bromobenzoyl)-5-(5-ethyl-1,3,4-oxadiazol-2-yl)-2,4-dimethyl-1H-pyrrol-1-yl)-2-oxoethyl acetate [compound No. 226]

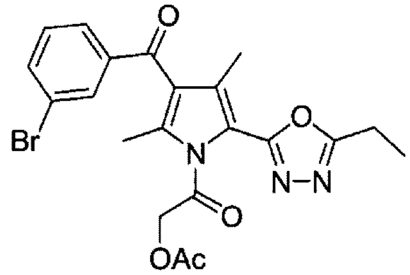

The title compound was obtained by the same procedures as in Production Example 224-1 using compound 173 and 2-chloro-2-oxoethyl acetate. Yield: 23.5 %, colorless oil.
$^1$H-NMR (400 MHz, DMSO-$d_6$) δ: 1.30 (3H, t, J = 7.4 Hz), 2.05 (3H, s), 2.09 (3H, s), 2.23 (3H, s), 2.93 (2H, q, J = 7.4 Hz), 5.08 (2H, s), 7.52-7.55 (1H, m), 7.74-7.77 (1H, m), 7.88-7.91 (2H, m).
ESI-MS m/z: 474[M+H]+, 476[M+2+H]+.

Production Example 227: Synthesis of (3-bromophenyl)(1-(cyclopropylsulfonyl)-5-(5-ethyl-1,3,4-oxadiazol-2-yl)-2,4-dimethyl-1H-pyrrol-3-yl)methanone [compound No. 227]

Production Example 227-1: Synthesis of (3-bromophenyl)(1-(cyclopropylsulfonyl)-5-(5-ethyl-1,3,4-oxadiazol-2-yl)-2,4-dimethyl-1H-pyrrol-3-yl)methanone [compound No. 227]

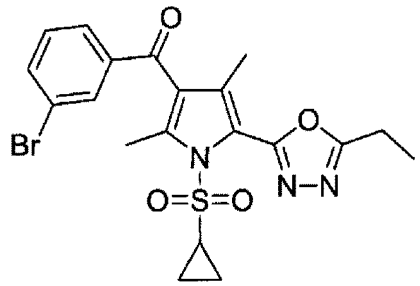

The title compound was obtained by the same procedures as in Production Example 225-1 using compound 173 and cyclopropanesulfonyl chloride. Yield: 56.8 %, colorless oil.
$^1$H-NMR (400 MHz, DMSO-$d_6$) δ: 1.26-1.31 (7H, m), 1.81 (3H, s), 2.41 (3H, s), 2.93 (2H, q, J = 7.6 Hz), 3.40-3.45 (1H, m), 7.54 (1H, t, J = 8.0 Hz), 7.75 (1H, dt, J = 7.6, 1.3 Hz), 7.89-7.92 (2H, m).
ESI-MS m/z: 478[M+H]+, 480[M+2+H]+.

Production Example 228: Synthesis of (3-bromophenyl)(1-(difluoromethyl)-5-(5-ethyl-1,3,4-oxadiazol-2-yl)-2,4-dimethyl-1H-pyrrol-3-yl)methanone [compound No. 228]

Production Example 228-1: Synthesis of (3-bromophenyl)(1-(difluoromethyl)-5-(5-ethyl-1,3,4-oxadiazol-2-yl)-2,4-dimethyl-1H-pyrrol-3-yl)methanone [compound No. 228]

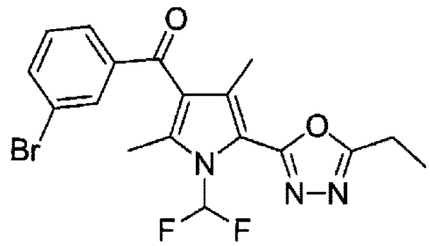

Compound 173 (95 mg, 0.25 mmol) was dissolved in N,N-dimethylformamide (1.5 ml). To the solution, water (0.4 ml), chlorodifluoroacetic acid sodium salt (76 mg, 0.5 mmol), and cesium carbonate (244 mg, 0.75 mmol) were added, and the mixture was stirred overnight at 100°C. After reaction, the solvent was distilled off. Water was added to the residue, followed by extraction with chloroform. The organic layer was dried over magnesium sulfate, and the solvent was distilled off. The residue was purified by silica gel column chromatography to obtain the title compound (1 mg, yield: 0.9%) as a white solid.
ESI-MS m/z: 424[M+H]+, 426[M+2+H]+.

Production Example 229: Synthesis of (3-bromophenyl)(5-(5-ethyl-1,3,4-oxadiazol-2-yl)-2,4-dimethyl-1-(2,2,2-trifluoroethyl)-1H-pyrrol-3-yl)methanone [compound No. 229]

Production Example 229-1: Synthesis of (3-bromophenyl)(5-(5-ethyl-1,3,4-oxadiazol-2-yl)-2,4-dimethyl-1-(2,2,2-trifluoroethyl)-1H-pyrrol-3-yl)methanone [compound No. 229]

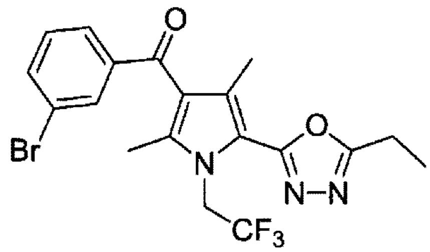

The title compound was obtained by the same procedures as in Production Example 223-1 using compound 173 and trifluoromethanesulfonic acid 2,2,2-trifluoroethyl ester. Yield: 79.2%, white solid.
$^1$H-NMR (400 MHz, DMSO-$d_6$) δ: 1.31 (3H, t, J = 7.6 Hz), 2.13 (3H, s), 2.23 (3H, s), 2.95 (2H, q, J = 7.6 Hz), 5.49 (2H, q, J = 8.8 Hz), 7.51 (1H, t, J = 7.8 Hz), 7.66 (1H, d, J = 7.8 Hz), 7.80 (1H, t, J = 1.8 Hz), 7.86 (1H, dq, J = 7.9, 0.9Hz).
ESI-MS m/z: 456[M+H]+, 458[M+2+H]+.

Production Example 230: Synthesis of (3-bromophenyl)(1-cyclopropyl-5-(5-ethyl-1,3,4-oxadiazol-2-yl)-2,4-dimethyl-1H-pyrrol-3-yl)methanone [compound No. 230]

Production Example 230-1: Synthesis of (3-bromophenyl)(1-cyclopropyl-5-(5-ethyl-1,3,4-oxadiazol-2-yl)-2,4-dimethyl-1H-pyrrol-3-yl)methanone [compound No. 230]

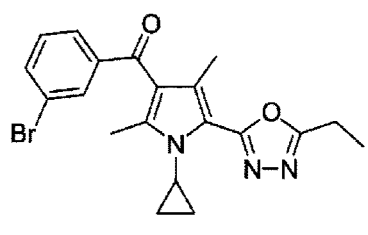

Compound 173 (77 mg, 0.21 mmol) was dissolved in 1,2-dichloroethane (4 ml). To the solution, cyclopropylboronic acid (36 mg, 0.42 mmol), sodium carbonate (45 mg, 0.42 mmol), copper acetate (38 mg, 0.21 mmol), and 2,2'-bipyridyl (33 mg, 0.21 mmol) were added, and the mixture was stirred at 70°C for 6 hours. After reaction, a saturated aqueous solution of ammonium chloride was added thereto, followed by extraction with chloroform. The organic layer was washed with saturated saline. The organic layer was dried over magnesium sulfate, and the solvent was distilled off. The residue was purified by silica gel column chromatography to obtain the title compound (35 mg, yield: 40.2%) as a yellow oil.
$^1$H-NMR (400 MHz, DMSO-$d_6$) δ: 0.67-0.71 (2H, m), 0.99-1.04 (2H, m), 1.32 (3H, t, J = 7.6 Hz), 2.01 (3H, s), 2.25 (3H, s), 2.95 (2H, q, J = 7.6 Hz), 3.36-3.40 (1H, m), 7.48 (1H, t, J = 7.8 Hz), 7.64 (1H, dt, J = 7.6, 0.7 Hz), 7.78 (1H, t, J = 1.7 Hz), 7.82 (1H, dq, J = 7.9, 1.0 Hz).
ESI-MS m/z: 414[M+H]$^+$, 416[M+2+H]$^+$.

Production Example 231: Synthesis of tert-butyl(2-(3-(3-bromobenzoyl)-5-(5-ethyl-1,3,4-oxadiazol-2-yl)-2,4-dimethyl-1H-pyrrol-1-yl)ethyl)carbamate [compound No. 231]

Production Example 231-1: Synthesis of tert-butyl(2-(3-(3-bromobenzoyl)-5-(5-ethyl-1,3,4-oxadiazol-2-yl)-2,4-dimethyl-1H-pyrrol-1-yl)ethyl)carbamate [compound No. 231]

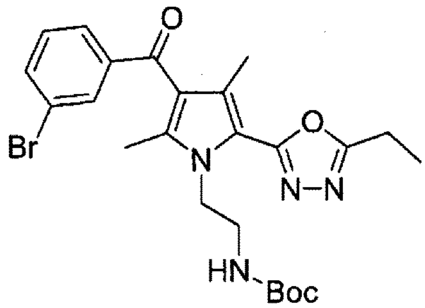

Compound 173 (73 mg, 0.195 mmol) was dissolved in N,N-dimethylformamide (1.5 ml). To the solution, 2-(tert-butoxycarbonylamino)ethyl bromide (87 mg, 0.39 mmol) and cesium carbonate (254 mg, 0.78 mmol) were added, and the mixture was stirred at 85°C for 4 hours. After reaction, the solvent was distilled off. The residue was dissolved in chloroform. The organic layer was washed with water. The organic layer was dried over magnesium sulfate, and the solvent was distilled off. The residue was purified by silica gel column chromatography to obtain the title compound (98 mg, yield: 97.1%) as a white solid.
$^1$H-NMR (400 MHz, DMSO-$d_6$) δ: 1.22-1.33 (12H, m), 2.08 (3H, s), 2.20 (3H, s), 2.93 (2H, q, J = 7.5 Hz), 3.23 (2H, q, J = 5.8 Hz), 4.35 (2H, t, J = 5.5 Hz), 7.00 (1H, t, J = 5.9 Hz), 7.48 (1H, t, J = 7.7 Hz), 7.68 (1H, d, J = 7.6 Hz), 7.81-7.85 (2H, m).
ESI-MS m/z: 517[M+H]$^+$, 519[M+2+H]$^+$.

Production Example 232: Synthesis of (3-bromophenyl)(1-ethyl-2,4-dimethyl-5-(5-ethyl-1,3,4-oxadiazol-2yl)-1H-pyrrol-3-yl)methanone [compound No. 232]

Production Example 232-1: Synthesis of (3-bromophenyl)(1-ethyl-2,4-dimethyl-5-(5-ethyl-1,3,4-oxadiazol-2yl)-1H-pyrrol-3-yl)methanone [compound No. 232]

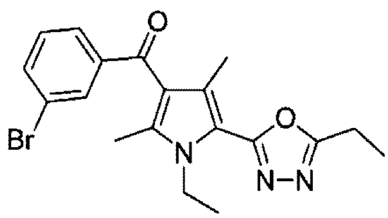

The title compound was obtained by the same procedures as in Production Example 231-1 using compound 173 and ethyl iodide. Yield: 97.9 %, yellow oil.

$^1$H-NMR (400 MHz, DMSO-$d_6$) δ: 1.27 (3H, t, J = 7.1 Hz), 1.31 (3H, t, J = 7.6 Hz), 2.09 (3H, s), 2.21 (3H, s), 2.93 (2H, q, J = 7.6 Hz), 4.34 (2H, q, J = 7.1 Hz), 7.49 (1H, t, J = 7.8 Hz), 7.64 (1H, dd, J = 6.3, 1.2 Hz), 7.78 (1H, t, J = 1.8Hz), 7.83 (1H, dq, J = 7.9, 1.0 Hz). ESI-MS m/z: 402[M+H]$^+$, 404[M+2+H]$^+$.

Production Example 233: Synthesis of (3-bromophenyl)(1-isopropyl-2,4-dimethyl-5-(5-ethyl-1,3,4-oxadiazol-2-yl)-1H-pyrrol-3-yl)methanone [compound No. 233]

Production Example 233-1: Synthesis of (3-bromophenyl)(1-isopropyl-2,4-dimethyl-5-(5-ethyl-1,3,4-oxadiazol-2-yl)-1H-pyrrol-3-yl)methanone [compound No. 233]

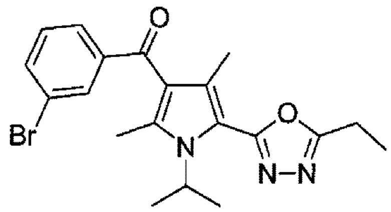

The title compound was obtained in the same way as in Production Example 231-1 using compound 173 and 2-iodopropane as a starting material. Yield: 15.5 %, yellow oil.
$^1$H-NMR (400 MHz, DMSO-$d_6$) δ: 1.31 (3H, t, J = 7.6 Hz), 1.41 (6H, d, J = 6.8 Hz), 1.89 (3H, s), 2.28 (3H, s), 2.94 (2H, q, J = 7.6 Hz), 4.89-4.98 (1H, m), 7.49 (1H, t, J = 7.8 Hz), 7.64 (1H, dt, J = 7.6, 1.3 Hz), 7.78 (1H, t, J = 1.6 Hz), 7.83 (1H, dq, J = 7.9, 1.1 Hz).
ESI-MS m/z: 416[M+H]$^+$, 418[M+2+H]$^+$.

Production Example 234: Synthesis of (1-(2-aminoethyl)-5-(5-ethyl-1,3,4-oxadiazol-2-yl)-2,4-dimethyl-1H-pyrrol-3-yl)(3-bromophenyl)methanone [compound No. 234]

Production Example 234-1: Synthesis of (1-(2-aminoethyl)-5-(5-ethyl-1,3,4-oxadiazol-2-yl)-2,4-dimethyl-1H-pyrrol-3-yl)(3-bromophenyl)methanone [compound No. 234]

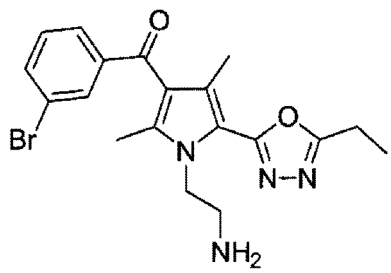

The title compound was obtained in the same way as in Production Example 48-1 using compound 231 as a starting material. Yield: quant., yellow oil.
$^1$H-NMR (400 MHz, DMSO-$d_6$) δ: 1.31 (3H, t, J = 7.6 Hz), 2.09 (3H, s), 2.23 (3H, s), 2.80 (2H, t, J = 6.8 Hz), 2.93 (2H, q, J = 7.6 Hz), 4.26 (2H, t, J = 6.8 Hz), 7.49 (1H, t, J = 7.8 Hz), 7.64-7.67 (1H, m), 7.79 (1H, dd, J = 2.0, 1.5 Hz), 7.83 (1H, dq, J = 7.9, 1.0 Hz).
ESI-MS m/z: 417[M+H]$^+$, 419[M+2+H]$^+$.

Production Example 235: Synthesis of 2-(3-(3-bromobenzoyl)-2,4-dimethyl-5-(5-ethyl-1,3,4-oxadiazol-2-yl)-1H-pyrrol-1-yl)ethyl acetate [compound No. 235]

Production Example 235-1: Synthesis of 2-iodoethyl acetate

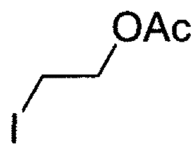

2-Iodoethanol (1.541 g, 8.96 mmol) was dissolved in dichloromethane (30 ml). To the solution, pyridine (1.54 ml, 17.92 mmol), N,N-dimethyl-4-aminopyridine (109 mg, 0.896 mmol), and acetic anhydride (2.54 ml, 26.88 mmol) were added, and the mixture was stirred at room temperature for 3 hours. After reaction, the organic layer was washed with 0.1 M hydrochloric acid and a saturated aqueous solution of sodium bicarbonate. The organic layer was dried over magnesium sulfate, and the solvent was distilled off to obtain the title compound (1.108 g, yield: 57.8%) as a yellow oil.
$^1$H-NMR (400 MHz, DMSO-$d_6$) δ: 2.10 (3H, s), 3.30 (2H, t, J = 6.8 Hz), 4.33 (2H, t, J = 6.8 Hz).

Production Example 235-2: Synthesis of 2-(3-(3-bromobenzoyl)-2,4-dimethyl-5-(5-ethyl-1,3,4-oxadiazol-2-yl)-1H-pyrrol-1-yl)ethyl acetate [compound No. 235]

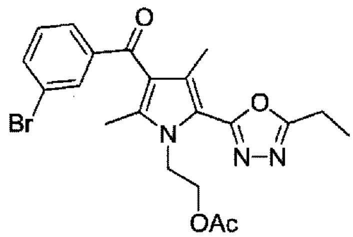

The title compound was obtained by the same procedures as in Production Example 231-1 using compound 173 and the compound obtained in Production Example 235-1. Yield: 47.3%, yellow oil.
$^1$H-NMR (400 MHz, DMSO-$d_6$) δ: 1.31 (3H, t, J = 7.6 Hz), 1.92 (3H, s), 2.12 (3H, s), 2.20 (3H, s), 2.93 (2H, q, J = 7.6 Hz), 4.32 (2H, t, J = 5.2 Hz), 4.59 (2H, t, J = 5.2 Hz), 7.50 (1H, t, J = 7.8 Hz), 7.65 (1H, dt, J = 7.8, 1.3 Hz), 7.78 (1H, t, J = 1.7 Hz), 7.84 (1H, dq, J = 7.9, 1.0 Hz).
ESI-MS m/z: 460[M+H]$^+$, 462[M+2+H]$^+$.

Production Example 236: Synthesis of (3-bromophenyl)(1-(2-hydroxyethyl)-2,4-dimethyl-5-(5-ethyl-1,3,4-oxadiazol-2-yl)-1H-pyrrol-3-yl)methanone [compound No. 236]

Production Example 236-1: Synthesis of (3-bromophenyl)(1-(2-hydroxyethyl)-2,4-dimethyl-5-(5-ethyl-1,3,4-oxadiazol-2-yl)-1H-pyrrol-3-yl)methanone [compound No. 236]

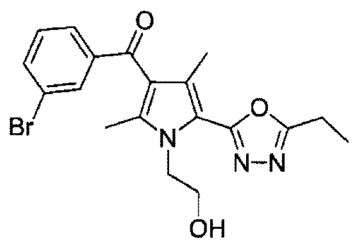

The compound of interest was also obtained by the procedures of Production Example 235-2. Yield: 35.2%, yellow solid.
$^1$H-NMR (400 MHz, DMSO-$d_6$) δ: 1.30 (3H, t, J = 7.6 Hz), 2.11 (3H, s), 2.22 (3H, s), 2.92 (2H, q, J = 7.6 Hz), 3.64 (2H, q, J = 5.5 Hz), 4.38 (2H, t, J = 8.4 Hz), 4.92 (1H, t, J = 5.5 Hz), 7.49 (1H, t, J = 7.8 Hz), 7.65 (1H, dt, J = 7.7, 1.3Hz), 7.79 (1H, t, J = 1.8 Hz), 7.83 (1H, dq, J = 7.9, 1.0 Hz).
ESI-MS m/z: 418[M+H]$^+$, 420[M+2+H]$^+$.

Production Example 237: Synthesis of (1-benzoyl-5-(5-ethyl-1,3,4-oxadiazol-2-yl)-2,4-dimethyl-1H-pyrrol-3-yl)(3-bromophenyl)methanone [compound No. 237]

Production Example 237-1: Synthesis of (1-benzoyl-5-(5-ethyl-1,3,4-oxadiazol-2-yl)-2,4-dimethyl-1H-pyrrol-3-yl)(3-bromophenyl)methanone [compound No. 237]

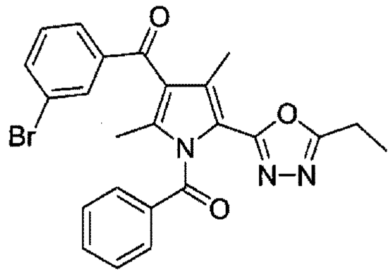

The title compound was obtained by the same procedures as in Production Example 224-1 using compound 173 and benzoyl chloride. Yield: 74.2 %, yellow oil.
$^1$H-NMR (400 MHz, DMSO-$d_6$) $\delta$: 1.07 (3H, t, J = 7.6 Hz), 2.14 (3H, s), 2.15 (3H, s), 2.67 (2H, q, J = 7.6 Hz), 7.51-7.56 (3H, m), 7.67-7.72 (3H, m), 7.80-7.83 (1H, m), 7.89 (1H, dq, J = 7.9, 1.0 Hz), 7.96 (1H, t, J = 1.8 Hz).
ESI-MS m/z: 478[M+H]$^+$, 480[M+2+H]$^+$.

Production Example 238: Synthesis of tert-butyl (2-(4-bromo-2-(5-(5-(hydroxymethyl)-1,3,4-oxadiazol-2-yl)-2,4-dimethyl-1H-pyrrole-3-carbonyl)phenoxy)ethyl)carbamate [compound No. 238]

Production Example 238-1: Synthesis of tert-butyl (2-(4-bromo-2-(5-(5-(hydroxymethyl)-1,3,4-oxadiazol-2-yl)-2,4-dimethyl-1H-pyrrole-3-carbonyl)phenoxy)ethyl)carbamate [compound No. 238]

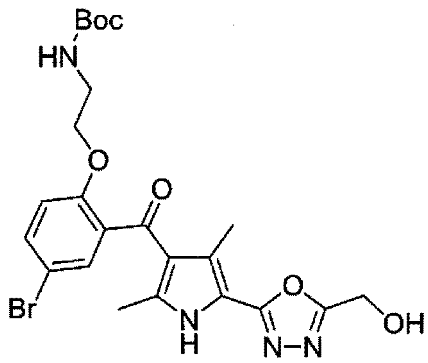

The title compound was obtained in the same way as in Production Example 50-1 using compound 203 as a starting material. Yield: 62.8 %, yellow solid.
$^1$H-NMR (400 MHz, DMSO-$d_6$) $\delta$: 1.31 (9H, s), 2.07 (3H, s), 2.27 (3H, s), 3.08 (2H, q, J = 6.0 Hz), 3.95 (2H, t, J = 6.2 Hz), 4.68 (2H, d, J = 6.1 Hz), 5.90 (1H, t, J = 6.1 Hz), 6.65 (1H, t, J = 5.4 Hz), 7.11 (1H, d, J = 8.9 Hz), 7.36 (1H, d,J = 2.6 Hz), 7.61 (1H, dd, J = 8.9, 2.6 Hz), 12.31 (1H, br s).

Production Example 239: Synthesis of (2-(2-aminoethoxy)-5-bromophenyl)(5-(5-(hydroxymethyl)-1,3,4-oxadiazol-2-yl)-2,4-dimethyl-1H-pyrrol-3-yl)methanone [compound No. 239]

Production Example 239-1: Synthesis of (2-(2-aminoethoxy)-5-bromophenyl)(5-(5-(hydroxymethyl)-1,3,4-oxadiazol-2-yl)-2,4-dimethyl-1H-pyrrol-3-yl)methanone [compound No. 239]

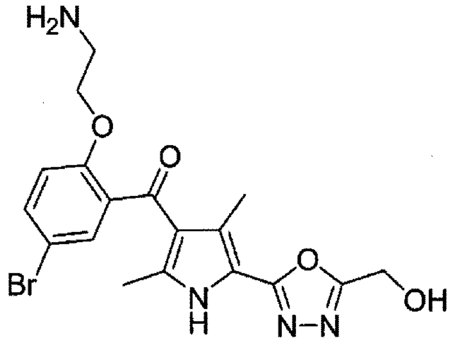

Compound 238 (35 mg, 0.065 mmol) was dissolved in dichloromethane (0.7 ml). To the solution, trifluoroacetic acid (0.2 ml) was added, and the mixture was stirred at room temperature for 1 hour. The solvent was distilled off. A 1 M aqueous sodium hydroxide solution was added to the residue, followed by extraction with chloroform. The organic layer was dried over magnesium sulfate. After addition of 1 M hydrochloric acid (0.07 ml), the solvent was distilled off. The residue was purified using a preparative LC system with MS triggers manufactured by Waters Corp. to obtain the title compound as hydrochloride (6 mg, yield: 21.2%).
$^1$H-NMR (400 MHz, DMSO-$d_6$) δ: 2.09 (3H, s), 2.28 (3H, s), 3.04 (2H, d, J = 5.5 Hz), 4.17 (2H, t, J = 5.5 Hz), 4.69 (2H, s), 5.92 (1H, br s), 7.20 (1H, d, J = 8.8 Hz), 7.43 (1H, d, J = 2.7 Hz), 7.68 (1H, dd, J = 8.8, 2.7 Hz), 7.81 (3H, br s), 12.38 (1H, br s).
ESI-MS m/z: 435$[M+H]^+$, 437$[M+2+H]^+$.

Production Example 240: Synthesis of (5-bromo-2-methoxyphenyl)(5-(5-cyclopropyl-1,3,4-oxadiazol-2-yl)-2,4-dimethyl-1H-pyrrol-3-yl)methanone [compound No. 240]

Production Example 240-1: Synthesis of (5-bromo-2-methoxyphenyl)(5-(5-cyclopropyl-1,3,4-oxadiazol-2-yl)-2,4-dimethyl-1H-pyrrol-3-yl)methanone [compound No. 240]

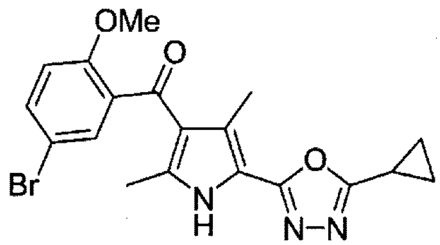

The title compound was obtained by the same procedures as in Production Example 173-2 using the compound obtained in Production Example 201-2 and cyclopropanecarbonyl chloride. Yield: 19.4%, white solid.
$^1$H-NMR (400 MHz, DMSO-$d_6$) δ: 1.05-1.09 (2H, m), 1.11-1.18 (2H, m), 2.06 (3H, s), 2.22 (3H, s), 2.24-2.29 (1H, m), 3.72 (3H, s), 7.10 (1H, d, J = 8.8 Hz), 7.33 (1H, d, J = 2.4 Hz), 7.62 (1H, dd, J = 8.8, 2.4 Hz), 12.19 (1H, s). ESI-MS m/z: 416$[M+H]^+$, 418$[M+2+H]^+$.

Production Example 241: Synthesis of (5-bromo-2-hydroxyphenyl)(5-(5-cyclopropyl-1,3,4-oxadiazol-2-yl)-2,4-dimethyl-1H-pyrrol-3-yl)methanone [compound No. 241]

Production Example 241-1: Synthesis of (5-bromo-2-hydroxyphenyl)(5-(5-cyclopropyl-1,3,4-oxadiazol-2-yl)-2,4-dimethyl-1H-pyrrol-3-yl)methanone [compound No. 241]

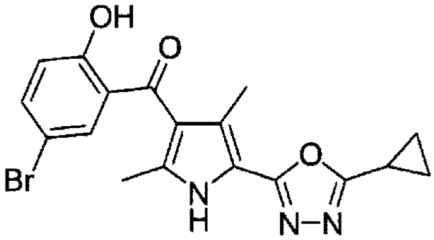

The title compound was obtained in the same way as in Production Example 91-1 using compound 240 as a starting material. Yield: 95.3 %, yellow solid.
$^1$H-NMR (400 MHz, DMSO-$d_6$) δ: 1.05-1.09 (2H, m), 1.13-1.19 (2H, m), 2.13 (3H, s), 2.24 (3H, s), 2.26-2.30 (1H, m), 6.89 (1H, d, J = 8.8 Hz), 7.32 (1H, d, J = 2.4 Hz), 7.50 (1H, dd, J = 8.8, 2.4 Hz), 10.34 (1H, s), 12.18 (1H, br s).
ESI-MS m/z: 402$[M+H]^+$, 404$[M+2+H]^+$.

Production Example 242: Synthesis of tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate [compound No. 242]

Production Example 242-1: Synthesis of tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate [compound No. 242]

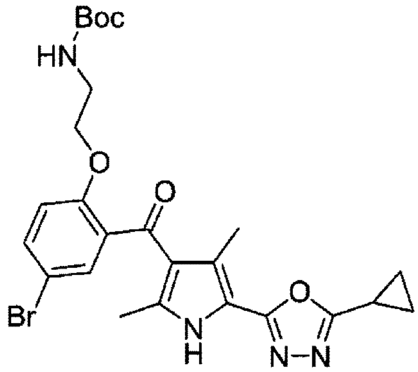

The title compound was obtained in the same way as in Production Example 50-1 using compound 241 as a starting material. Yield: 15.6 %, white solid.
$^1$H-NMR (400 MHz, DMSO-$d_6$) δ: 1.04-1.08 (2H, m), 1.13-1.18 (2H, m), 1.31 (9H, s), 2.08 (3H, s), 2.21 (3H, s), 2.23-2.29 (1H, m), 3.08 (2H, q, J = 5.9 Hz), 3.94 (2H, t, J = 6.3 Hz), 6.62 (1H, t, J = 5.2 Hz), 7.11 (1H, d, J = 8.9 Hz), 7.35 (1H, d, J = 2.7 Hz), 7.60 (1H, dd, J = 8.9, 2.7 Hz), 12.16 (1H, br s).
ESI-MS m/z: 545$[M+H]^+$, 547$[M+2+H]^+$.

Production Example 243: Synthesis of tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate [compound No. 243]

Production Example 243-1: Synthesis of tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate [compound No. 243]

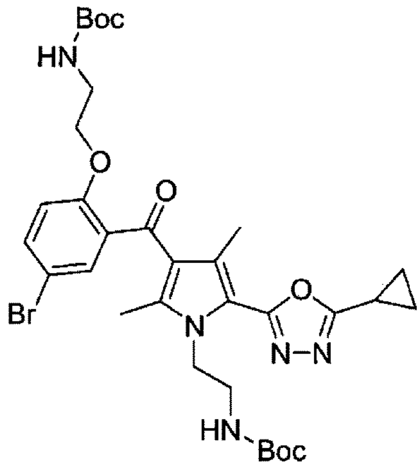

The title compound was obtained in the same way as in Production Example 50-1 using compound 241 as a starting material. Yield: 44.6 %, white solid.
$^1$H-NMR (400 MHz, DMSO-$d_6$) δ: 1.03-1.07 (2H, m), 1.13-1.18 (2H, m), 1.32 (9H, s), 1.34 (9H, s), 2.03 (3H, s), 2.21 (3H, s), 2.26-2.33 (1H, m), 3.07 (2H, q, J = 6.1 Hz), 3.17 (2H, q, J = 6.1 Hz), 3.94 (2H, t, J = 6.1 Hz), 4.28 (2H, t, J = 6.1 Hz), 6.67 (1H, t, J = 5.2 Hz), 6.97 (1H, t, J = 6.0 Hz), 7.12 (1H, d, J = 8.9 Hz), 7.35 (1H, d, J = 2.6 Hz), 7.63 (1H, dd, J = 8.9, 2.6 Hz).
ESI-MS m/z: 688[M+H]$^+$, 690[M+2+H]$^+$.

Production Example 244: Synthesis of (2-(2-aminoethoxy)-5-bromophenyl)(5-(5-cyclopropyl-1,3,4-oxadiazol-2-yl)-2,4-dimethyl-1H-pyrrol-3-yl)methanone [compound No. 244]

Production Example 244-1: Synthesis of (2-(2-aminoethoxy)-5-bromophenyl)(5-(5-cyclopropyl-1,3,4-oxadiazol-2-yl)-2,4-dimethyl-1H-pyrrol-3-yl)methanone [compound No. 244]

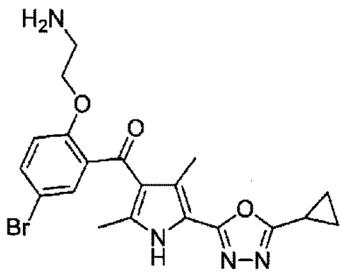

The title compound was obtained by the same procedures as in Production Example 48-1 using compound 242 as a starting material. Yield: 89.8 %, white solid. $^1$H-NMR (400 MHz, DMSO-$d_6$) δ: 0.81-1.32 (4H, m), 2.08 (3H, s), 2.22 (3H, s), 2.23-2.30 (1H, m), 2.64 (2H, t, J = 5.5 Hz), 3.90 (2H, t, J = 5.5 Hz), 7.09 (1H, d,J = 8.8 Hz), 7.37 (1H, d, J = 2.7 Hz), 7.61 (1H, dd, J = 8.7, 2.3 Hz).

Production Example 245: Synthesis of (2-(2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone [compound No. 245]

Production Example 245-1: Synthesis of (2-(2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone [compound No. 245]

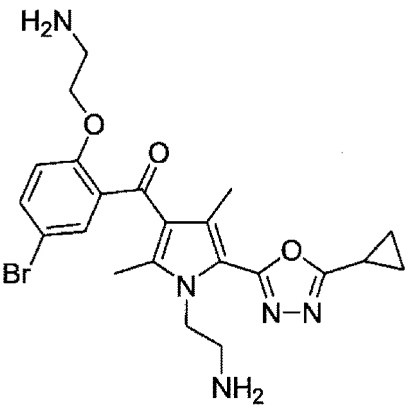

The title compound was obtained in the same way as in Production Example 48-1 using compound 243 as a starting material. Yield: 79.9 %, yellow oil.
$^1$H-NMR (400 MHz, DMSO-$d_6$) δ: 1.03-1.07 (2H, m), 1.13-1.18 (2H, m), 1.42-1.61 (2H, m), 2.04 (3H, s), 2.23 (3H, s), 2.28-2.31 (1H, br m), 2.61 (2H, t, J = 5.4 Hz), 2.73 (2H, t, J = 6.7 Hz), 3.88 (2H, t, J = 5.4 Hz), 4.18 (2H, t, J = 6.7 Hz), 7.08 (1H, d, J = 8.8 Hz), 7.41 (1H, d, J = 2.4 Hz), 7.63 (1H, dd, J = 8.8, 2.4 Hz).
ESI-MS m/z: 488$[M+H]^+$, 490$[M+2+H]^+$.

Production Example 246: Synthesis of (5-(5-ethyl-1,3,4-oxadiazol-2-yl)-2,4-dimethyl-1H-pyrrol-3-yl)(3-hydroxyphenyl)methanone [compound No. 246]

Production Example 246-1: Synthesis of (5-(5-ethyl-1,3,4-oxadiazol-2-yl)-2,4-dimethyl-1H-pyrrol-3-yl)(3-hydroxyphenyl)methanone [compound No. 246]

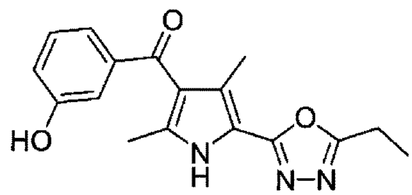

The title compound was obtaine in the same way as in Production Example 91-1 using compound 193 as a starting material. Yield: 83.6 %, brown solid.
$^1$H-NMR (400 MHz, $CDCl_3$) δ: 1.43 (3H, t, J = 7.6 Hz), 2.26 (3H, s), 2.30 (3H, s), 2.94 (2H, q, J = 7.6 Hz), 7.03-7.08 (1H, m), 7.26-7.36 (3H, m), 9.66 (1H, s).

Production Example 247: Synthesis of tert-butyl (2-(3-(5-(5-ethyl-1,3,4-oxadiazol-2-yl)-2,4-dimethyl-1H-pyrrole-3-carbonyl)phenoxy)ethyl)carbamate [compound No. 247]

Production Example 247-1: Synthesis of tert-butyl (2-(3-(5-(5-ethyl-1,3,4-oxadiazol-2-yl)-2,4-dimethyl-1H-pyrrole-3-carbonyl)phenoxy)ethyl)carbamate [compound No. 247]

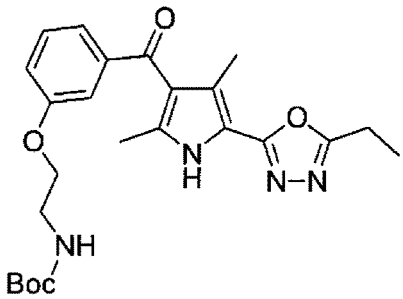

The title compound was obtaine in the same way as in Production Example 50-1 using compound 246 as a starting material. Yield: 56.6 %, white solid.
$^1$H-NMR (400 MHz, $CDCl_3$) δ: 1.43 (3H, t, J = 7.7 Hz), 1.45 (9H, s), 2.27 (3H, s), 2.29 (3H, s), 2.94 (2H, q, J = 7.6 Hz), 3.49-3.60 (2H, m), 4.02-4.11 (2H, m), 4.92-5.04 (1H, m), 7.06-7.12 (1H, m), 7.26-7.40 (3H, m), 9.37 (1H, s).

Production Example 248: Synthesis of (3-(2-aminoethoxy)phenyl)(5-(5-ethyl-1,3,4-oxadiazol-2-yl)-2,4-dimethyl-1H-pyrrol-3-yl)methanone [compound No. 248]

Production Example 248-1: Synthesis of (3-(2-aminoethoxy)phenyl)(5-(5-ethyl-1,3,4-oxadiazol-2-yl)-2,4-dimethyl-1H-pyrrol-3-yl)methanone [compound No. 248]

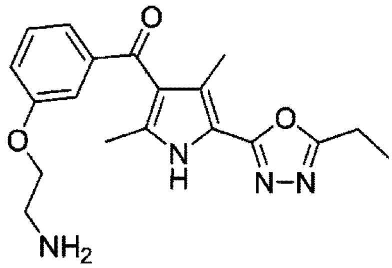

The title compound was obtained in the same way as in Production Example 48-1 using compound 247 as a starting material. Yield: 58.4 %, yellow solid.
$^1$H-NMR (400 MHz, DMSO-$d_6$) δ: 1.32 (3H, t, J = 7.6 Hz), 2.13 (3H, s), 2.22 (3H, s), 2.91 (2H, q, J = 7.5 Hz), 3.19-3.26 (2H, m), 4.19-4.25 (2H, m), 7.19-7.27 (3H, m), 7.44-7.49 (1H, m), 12.26 (1H, s).

Production Example 249: Synthesis of (3-bromo-4-hydroxyphenyl)(5-(5-ethyl-1,3,4-oxadiazol-2-yl)-2,4-dimethyl-1H-pyrrol-3-yl)methanone [compound No. 249]

Production Example 249-1: Synthesis of (3-bromo-4-hydroxyphenyl)(5-(5-ethyl-1,3,4-oxadiazol-2-yl)-2,4-dimethyl-1H-pyrrol-3-yl)methanone [compound No. 249]

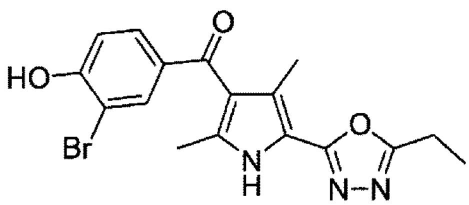

The title compound was obtained in the same way as in Production Example 91-1 using compound 197 as a starting material. Yield: 95.8 %, orange oil.
$^1$H-NMR (400 MHz, DMSO-$d_6$) δ: 1.32 (3H, t, J = 7.6 Hz), 2.15 (3H, s), 2.21 (3H, s), 2.91 (2H, q, J = 7.6 Hz), 7.04 (1H,

d, J = 8.3 Hz), 7.54 (1H, dd, J = 8.3, 2.0 Hz), 7.76 (1H, d, J = 2.0 Hz), 11.16 (1H, s), 12.18 (1H, s).

Production Example 250: Synthesis of tert-butyl (2-(2-bromo-4-(5-(5-ethyl-1,3,4-oxadiazol-2-yl)-2,4-dimethyl-1H-pyrrole-3-carbonyl)phenoxy)ethyl)carbamate [compound No. 250]

Production Example 250-1: Synthesis of tert-butyl (2-(2-bromo-4-(5-(5-ethyl-1,3,4-oxadiazol-2-yl)-2,4-dimethyl-1H-pyrrole-3-carbonyl)phenoxy)ethyl)carbamate [compound No. 250]

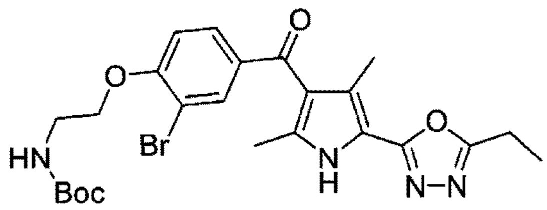

The title compound was obtained in the same way as in Production Example 50-1 using compound 249 as a starting material. Yield: 62.5 %, white solid.
$^1$H-NMR (400 MHz, $CDCl_3$) δ: 1.43 (3H, t, J = 7.7 Hz), 1.46 (9H, s), 2.29 (3H, s), 2.29 (3H, s), 2.94 (2H, q, J = 7.7 Hz), 3.56-3.76 (2H, m), 4.10-4.26 (2H, m), 5.07 (1H, s), 6.93 (1H, d, J = 8.5 Hz), 7.72 (1H, dd, J = 8.5, 2.2 Hz), 7.99 (1H, d, J = 2.0 Hz), 9.55 (1H, s).

Production Example 251: Synthesis of (4-(2-aminoethoxy)-3-bromophenyl)(5-(5-ethyl-1,3,4-oxadiazol-2-yl)-2,4-dimethyl-1H-pyrrol-3-yl)methanone [compound No. 251]

Production Example 251-1: Synthesis of (4-(2-aminoethoxy)-3-bromophenyl)(5-(5-ethyl-1,3,4-oxadiazol-2-yl)-2,4-dimethyl-1H-pyrrol-3-yl)methanone [compound No. 251]

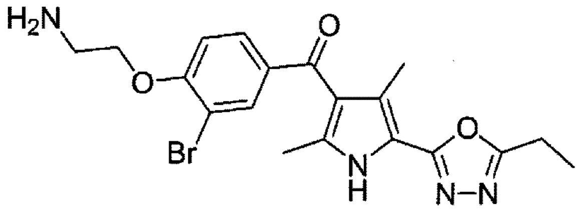

The title compound was obtained in the same way as in Production Example 48-1 using compound 250 as a starting material. Yield: 67.7 %, white solid.
$^1$H-NMR (400 MHz, DMSO-$d_6$) δ: 1.32 (3H, t, J = 7.6 Hz), 2.15 (3H, s), 2.21 (3H, s), 2.92 (2H, q, J = 7.6 Hz), 2.97-3.04 (2H, m), 4.11-4.19 (2H, m), 7.22 (1H, d,J = 8.8 Hz), 7.66 (1H, dd, J = 8.8, 2.0 Hz), 7.83 (1H, d, J = 2.2 Hz).

Production Example 252: Synthesis of (5-(5-cyclopropyl-1,3,4-oxadiazol-2-yl)-2,4-dimethyl-1H-pyrrol-3-yl)(3-hydroxyphenyl)methanone [compound No. 252]

Production Example 252-1: Synthesis of (5-(5-cyclopropyl-1,3,4-oxadiazol-2-yl)-2,4-dimethyl-1H-pyrrol-3-yl)(3-hydroxyphenyl)methanone [compound No. 252]

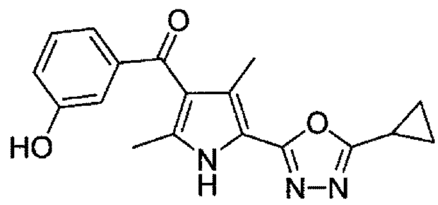

The title compound was obtained in the same way as in Production Example 91-1 using compound 193 as a starting material. Yield: 62.3 %, yellow solid.
$^1$H-NMR (400 MHz, $CDCl_3$) δ: 1.14-1.21 (4H, m), 2.15-2.22 (1H, m), 2.26 (3H, s), 2.30 (3H, s), 7.03-7.08 (1H, m), 7.26-7.36 (3H, m), 9.66 (1H, s).

Production Example 253: Synthesis of (3-(2-aminoethoxy)phenyl)(5-(5-cyclopropyl-1,3,4-oxadiazol-2-yl)-2,4-dimethyl-1H-pyrrol-3-yl)methanone [compound No. 253]

Production Example 253-1: Synthesis of (3-(2-aminoethoxy)phenyl)(5-(5-cyclopropyl-1,3,4-oxadiazol-2-yl)-2,4-dimethyl-1H-pyrrol-3-yl)methanone [compound No. 253]

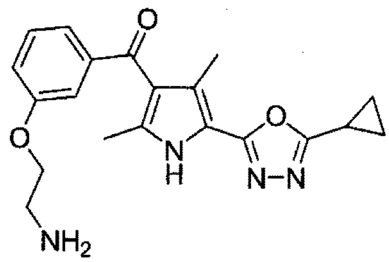

The title compound was obtained in the same way as in Production Example 50-1 and Production Example 48-1 using compound 252 as a starting material.
Yield: 31.8 %, white solid.
$^1$H-NMR (400 MHz, DMSO-$d_6$) δ: 1.02-1.31 (4H, m), 2.12 (3H, s), 2.19 (3H, s), 2.23-2.32 (1H, m), 2.94-3.07 (2H, m), 3.98-4.11 (2H, m), 7.07-7.28 (3H, m), 7.36-7.49 (1H, m).

Production Example 254: Synthesis of (3-bromo-4-hydroxyphenyl)(5-(5-cyclopropyl-1,3,4-oxadiazol-2-yl)-2,4-dimethyl-1H-pyrrol-3-yl)methanone [compound No. 254]

Production Example 254-1: Synthesis of (3-bromo-4-hydroxyphenyl)(5-(5-cyclopropyl-1,3,4-oxadiazol-2-yl)-2,4-dimethyl-1H-pyrrol-3-yl)methanone [compound No. 254]

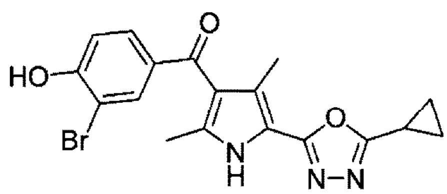

The title compound was obtained in the same way as in Production Example 91-1 using compound 198 as a starting material.
Yield: 80.4 %, light orange solid.
$^1$H-NMR (400 MHz, $CDCl_3$) δ: 1.19 (4H, d, J = 6.7 Hz), 2.21 (1H, quin, J = 6.7 Hz), 2.26 (3H, s), 2.29 (3H, s), 6.02 (1H, s), 7.08 (1H, d, J = 8.5 Hz), 7.67 (1H, dd, J = 8.5, 2.0 Hz), 7.95 (1H, d, J = 1.5 Hz), 9.38 (1H, s).

Production Example 255: Synthesis of tert-butyl (2-(2-bromo-4-(5-(5-cyclopropyl-1,3,4-oxadiazol-2-yl)-2,4-dimethyl-1H-pyrrole-3-carbonyl)phenoxy)ethyl)carbamate [compound No. 255]

Production Example 255-1: Synthesis of tert-butyl (2-(2-bromo-4-(5-(5-cyclopropyl-1,3,4-oxadiazol-2-yl)-2,4-dimethyl-1H-pyrrole-3-carbonyl)phenoxy)ethyl)carbamate [compound No. 255]

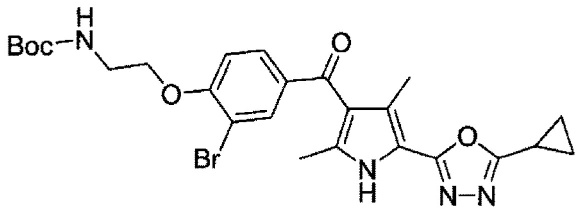

The title compound was obtained in the same way as in Production Example 50-1 using compound 254 as a starting material. Yield: 58.3 %, white solid.
$^1$H-NMR (400 MHz, $CDCl_3$) δ: 1.19 (4H, d, J = 6.8 Hz), 1.46 (9H, s), 2.20 (1H, quin, J = 6.8 Hz), 2.25 (3H, s), 2.28 (3H, s), 3.59-3.69 (2H, m), 4.13-4.23 (2H, m), 5.07 (1H, s), 6.93 (1H, d, J = 8.5 Hz), 7.71 (1H, dd, J = 8.7, 2.1 Hz), 7.99 (1H, d, J = 2.0 Hz), 9.32 (1H, s).

Production Example 256: Synthesis of (4-(2-aminoethoxy)-3-bromophenyl)(5-(5-cyclopropyl-1,3,4-oxadiazol-2-yl)-2,4-dimethyl-1H-pyrrol-3-yl)methanone [compound No. 256]

Production Example 256-1: Synthesis of (4-(2-aminoethoxy)-3-bromophenyl)(5-(5-cyclopropyl-1,3,4-oxadiazol-2-yl)-2,4-dimethyl-1H-pyrrol-3-yl)methanone [compound No. 256]

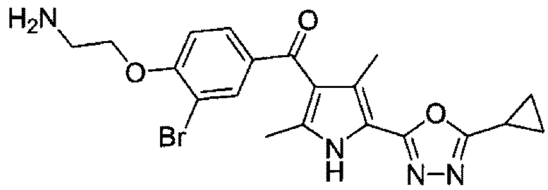

The title compound was obtained in the same way as in Production Example 48-1 using compound 255 as a starting material.
Yield: quant., white solid.
$^1$H-NMR (400 MHz, DMSO-$d_6$) δ: 1.04-1.11 (2H, m), 1.13-1.20 (2H, m), 2.15 (3H, s), 2.18 (3H, s), 2.23-2.31 (1H, m), 2.95 (2H, t, J = 5.5 Hz), 4.11 (2H, t, J = 5.7 Hz), 7.21 (1H, d, J = 8.8 Hz), 7.65 (1H, dd, J = 8.5, 2.2 Hz), 7.82 (1H, d, J = 2.0 Hz).

Production Example 257: Synthesis of (3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone [compound No. 257]

Production Example 257-1: Synthesis of (3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone [compound No. 257]

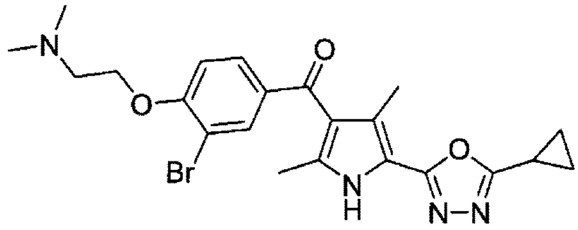

The title compound was obtained in the same way as in Production Example 94-1 using compound 256 as a starting material.
Yield: 15.7 %, white solid.
$^1$H-NMR (400 MHz, DMSO-$d_6$) δ: 1.04-1.20 (4H, m), 2.15 (3H, s), 2.19 (3H, s), 2.23-2.31 (1H, m), 2.26 (6H, s), 2.71 (2H, t, J = 5.6 Hz), 4.23 (2H, t, J = 5.6 Hz), 7.23 (1H, d, J = 8.5 Hz), 7.65 (1H, dd, J = 8.5, 2.2 Hz), 7.82 (1H, d, J = 2.2Hz), 12.16 (1H, s).

Production Example 258: Synthesis of 3-(5-bromo-2-methoxybenzoyl)-5-(5-cyclopropyl-1,3,4-oxadiazol-2-yl)-4-methyl-1H-pyrrole-2-carboaldehyde [compound No. 258]

Production Example 258-1: Synthesis of 3-(5-bromo-2-methoxybenzoyl)-5-(5-cyclopropyl-1,3,4-oxadiazol-2-yl)-4-methyl-1H-pyrrole-2-carboaldehyde [compound No. 258]

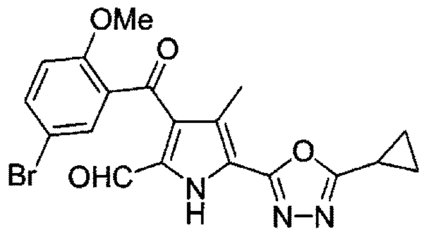

Compound 240 (92 mg, 0.22 mmol) was dissolved in carbon tetrachloride (5 ml), and the solution was cooled in ice. Tert-butyl hypochlorite (0.125 ml, 1.10 mmol) was added thereto, and the mixture was stirred for 4 hours. After reaction, the organic layer was washed with a saturated aqueous solution of sodium bicarbonate. The organic layer was dried over magnesium sulfate, and the solvent was distilled off. The residue was purified by silica gel column chromatography to obtain the title compound (19 mg, yield: 20.1%) as a white solid.
$^1$H-NMR (400 MHz, DMSO-$d_6$) δ: 1.17-1.19 (4H, m), 2.15 (3H, s), 2.32-2.34 (1H, m), 3.69 (3H, s), 7.15 (1H, d, J = 8.8 Hz), 7.59 (1H, d, J = 2.7 Hz), 7.74 (1H, dd, J = 8.8, 2.7 Hz), 9.54 (1H, s), 13.40 (1H, br s).
ESI-MS m/z: 430[M+H]$^+$, 432[M+2+H]$^+$.

Production Example 259: Synthesis of (5-bromo-2-methoxyphenyl)(5-(5-cyclopropyl-1,3,4-oxadiazol-2-yl)-2-(hydroxymethyl)-4-methyl-1H-pyrrol-3-yl)methanone [compound No. 259]

Production Example 259-1: Synthesis of (5-bromo-2-methoxyphenyl)(5-(5-cyclopropyl-1,3,4-oxadiazol-2-yl)-2-(hydroxymethyl)-4-methyl-1H-pyrrol-3-yl)methanone [compound No. 259]

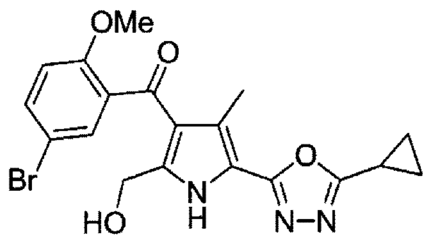

The title compound was obtained by the same procedures as in Production Example 58-1 using compound 258 as a starting material. Yield: 44.2 %, yellow solid.
$^1$H-NMR (400 MHz, DMSO-$d_6$) δ: 1.10-1.16 (4H, m), 2.10 (3H, s), 2.23-2.30 (1H, m), 3.71 (3H, s), 4.38 (2H, s), 5.31 (1H, s), 7.10 (1H, d, J = 8.8 Hz), 7.35 (1H, d, J = 2.6 Hz), 7.63 (1H, dd, J = 8.8, 2.6 Hz), 12.15 (1H, br s).
ESI-MS m/z: 432[M+H]$^+$, 434[M+2+H]$^+$.

Production Example 260: Synthesis of (3-bromophenyl)(5-(5-cyclopropyl-1,3,4-oxadiazol-2-yl)-4-methyl-2-vinyl-1H-pyrrol-3-yl)methanone [compound No. 260]

Production Example 260-1: Synthesis of 4-(3-bromobenzoyl)-3-methyl-5-vinyl-1H-pyrrole-2-carboxylic acid

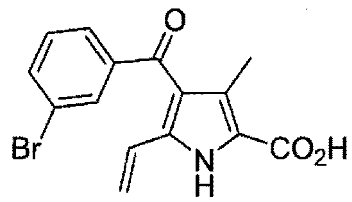

The title compound was obtained in the same way as in Production Example 96-1 using compound 69 as a starting material. Yield: quant., yellow solid.
$^1$H-NMR (400 MHz, DMSO-$d_6$) δ: 2.11 (3H, s), 5.22 (1H, d, J = 11.6 Hz), 6.03 (1H, d, J = 17.9 Hz), 6.36 (1H, dd, J = 17.9, 11.6 Hz), 7.47 (1H, t, J = 7.9 Hz), 7.63 (1H, dt, J = 7.9, 1.2 Hz), 7.76 (1H, t, J = 1.8 Hz), 7.83 (1H, dq, J = 7.9, 1.2 Hz), 12.12 (1H, br s), 12.82 (1H, br s).
ESI-MS m/z: 334[M+H]$^+$, 336[M+2+H]$^+$.

Production Example 260-2: Synthesis of 4-(3-bromobenzoyl)-3-methyl-5-vinyl-1H-pyrrole-2-carbohydrazide

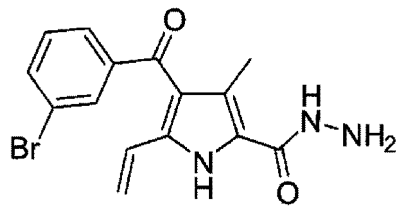

The title compound was obtained in the same way as in Production Example 173-1 using the compound obtained in Production Example 260-1 as a starting material. Yield: 68.6%, yellow solid.
$^1$H-NMR (400 MHz, DMSO-$d_6$) δ: 2.09 (3H, s), 4.40 (2H, br s), 5.15 (1H, d, J = 11.0 Hz), 5.83 (1H, d, J = 17.7 Hz), 6.35 (1H, dd, J = 17.7, 11.0 Hz), 7.46 (1H, t, J = 7.8 Hz), 7.60 (1H, d, J = 7.6 Hz), 7.73 (1H, t, J = 1.6 Hz), 7.82-7.79 (1H, m).
ESI-MS m/z: 348[M+H]$^+$, 350[M+2+H]$^+$.

Production Example 260-3: Synthesis of 4-(3-bromobenzoyl)-N'-(cyclopropanecarbonyl)-3-methyl-5-vinyl-1H-pyrrole-2-carbohydrazide

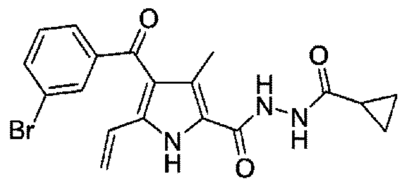

The title compound was obtained in the same way as in Production Example 204-2 using the compound obtained in Production Example 260-2 as a starting material. Yield: 45.1%, yellow solid.
$^1$H-NMR (400 MHz, DMSO-$d_6$) δ: 0.72-0.81 (4H, m), 1.67-1.74 (1H, m), 2.12 (3H, s), 5.24 (1H, d, J = 11.5 Hz), 5.88 (1H, d, J = 17.8 Hz), 6.36 (1H, dd, J = 17.8, 11.5 Hz), 7.47 (1H, t, J = 7.8 Hz), 7.64 (1H, dt, J = 7.8, 1.3 Hz), 7.77 (1H, t, J = 1.7 Hz), 7.83 (1H, dq, J = 7.8, 1.3 Hz), 9.73 (1H, br s), 10.18 (1H, d, J =1.5 Hz), 11.84 (1H, br s) .
ESI-MS m/z: 416[M+H]$^+$, 418[M+2+H]$^+$.

Production Example 260-4: Synthesis of (3-bromophenyl)(5-(5-cyclopropyl-1,3,4-oxadiazol-2-yl)-4-methyl-2-vinyl-1H-pyrrol-3-yl)methanone [compound No. 260]

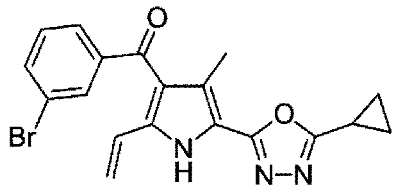

The compound (51 mg, 0.12 mmol) obtained in Production Example 260-3 was suspended in dichloromethane (1 ml). To the suspension, tosyl chloride (49 mg, 0.257 mmol) and triethylamine (0.097 ml, 0.696 mmol) were added, and the mixture was stirred at room temperature for 3 hours. After reaction, the solvent was distilled off.
The residue was purified by silica gel column chromatography to obtain the title compound (33 mg, 69.1%) as a yellow solid.
$^1$H-NMR (400 MHz, DMSO-$d_6$) δ: 1.09-1.20 (4H, m), 2.15 (3H, s), 2.27-2.33 (1H, m), 5.27 (1H, d, J = 11.7 Hz), 6.01 (1H, d, J = 17.8 Hz), 6.39 (1H, dd, J = 17.8, 11.7 Hz), 7.49 (1H, t, J = 7.8 Hz), 7.66 (1H, dt, J = 7.8, 1.2 Hz), 7.79 (1H, t, J = 1.8 Hz), 7.85 (1H, dq, J = 7.8, 1.2 Hz), 12.44 (1H, br s).
ESI-MS m/z: 398[M+H]$^+$, 400[M+2+H]$^+$.

Production Example 261: Synthesis of 3-(3-bromobenzoyl)-5-(5-cyclopropyl-1,3,4-oxadiazol-2-yl)-4-methyl-1H-pyrrole-2-carboaldehyde [compound No. 261]

Production Example 261-1: Synthesis of 3-(3-bromobenzoyl)-5-(5-cyclopropyl-1,3,4-oxadiazol-2-yl)-4-methyl-1H-pyrrole-2-carboaldehyde [compound No. 261]

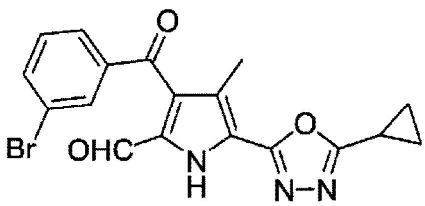

The title compound was obtained in the same way as in Production Example 87-1 using compound 260 as a starting material. Yield: 66.3 %, yellow solid.
$^1$H-NMR (400 MHz, DMSO-$d_6$) δ: 1.17-1.22 (4H, m), 2.20 (3H, s), 2.32-2.34 (1H, m), 7.51 (1H, td, J = 7.8, 0.8 Hz), 7.76 (1H, dt, J = 7.8, 1.3 Hz), 7.88-7.91 (2H,m), 9.50 (1H, s), 13.56 (1H, br s).
ESI-MS m/z: 400[M+H]$^+$, 402[M+2+H]$^+$.

Production Example 262: Synthesis of (3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone [compound No. 262]

Production Example 262-1: Synthesis of 4-(3-bromo-4-methoxybenzoyl)-5-(1,3-dithian-2-yl)-3-methyl-1H-pyrrole-2-carboxylic acid

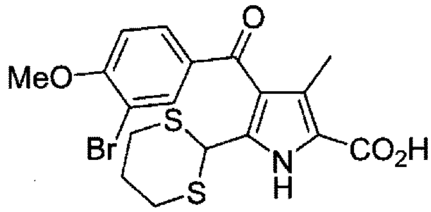

The title compound was obtained in the same way as in Production Example 96-1 using compound 66 as a starting material. Yield: 77.3 %, brown solid.
$^1$H-NMR (400 MHz, DMSO-$d_6$) δ: 1.60-1.72 (1H, m), 1.87 (3H, d, J = 0.7 Hz), 2.00-2.05 (1H, m), 2.77-2.89 (4H, m), 3.95 (3H, s), 5.22 (1H, s), 6.59 (1H, dd, J = 2.4, 1.0 Hz), 7.24 (1H, d, J = 8.5 Hz), 7.70 (1H, dd, J = 8.5, 2.2 Hz), 7.84 (1H, d, J = 2.2 Hz), 11.45 (1H, br s).
ESI-MS m/z: 456[M+H]$^+$, 458[M+2+H]$^+$.

Production Example 262-2: Synthesis of 4-(3-bromo-4-methoxybenzoyl)-5-(1,3-dithian-2-yl)-3-methyl-1H-pyrrole-2-carbohydrazide

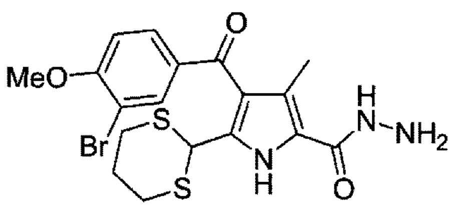

The title compound was obtained in the same way as in Production Example 173-1 using the compound obtained in Production Example 262-1 as a starting material. Yield: 47.0 %, yellow solid.
$^1$H-NMR (400 MHz, DMSO-$d_6$) δ: 1.66-1.75 (1H, m), 2.01-2.08 (1H, m), 2.11 (3H, s), 2.85-2.89 (4H, m), 3.95 (3H, s), 4.38 (2H, br s), 5.25 (1H, s), 7.25 (1H, d, J= 8.5 Hz), 7.73 (1H, dd, J = 8.5, 2.2 Hz), 7.87 (1H, d, J = 2.2 Hz), 9.34 (1H, br s), 11.65 (1H, br s).
ESI-MS m/z: 470[M+H]$^+$, 472[M+2+H]$^+$.

Production Example 262-3: Synthesis of (3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone [compound No. 262]

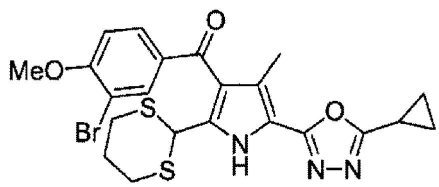

The compound (1.319 g, 2.80 mmol) obtained in Production Example 262-2 was suspended in acetonitrile (26 ml). To the suspension, a 1 M aqueous sodium hydroxide solution (3.08 ml, 3.08 mmol) and cyclopropanecarbonyl chloride (0.28 ml) were added, and the mixture was stirred at room temperature for 4 hours. After reaction, the solvent

was distilled off. Water was added to the residue, followed by extraction with chloroform. The organic layer was dried over magnesium sulfate, and the solvent was distilled off. This residue was suspended in dichloromethane (30 ml). To the suspension, tosyl chloride (1.121 g, 5.88 mmol) and triethylamine (2.26 ml, 16.24 mmol) were added, and the mixture was stirred overnight at room temperature. After reaction, chloroform was added thereto. The organic layer was washed with a saturated aqueous solution of ammonium chloride. The organic layer was dried over magnesium sulfate, and the solvent was distilled off. The residue was purified by silica gel column chromatography to obtain the title compound (674 mg, yield: 46.3%) as a brown solid.
$^1$H-NMR (400 MHz, DMSO-$d_6$) δ: 1.17 (4H, d, J = 6.8 Hz), 1.66-1.76 (1H, m), 2.01-2.06 (1H, m), 2.10 (3H, s), 2.29 (1H, t, J = 6.8 Hz), 2.86 (2H, d, J = 3.2 Hz), 2.88 (2H, d, J = 3.2 Hz), 3.96 (3H, s), 5.33 (1H, s), 7.26 (1H, d, J = 8.8 Hz), 7.75 (1H, dd, J = 8.8, 2.2 Hz), 7.90 (1H, d, J = 2.2 Hz), 12.55 (1H, br s).
ESI-MS m/z: 520[M+H]$^+$, 522[M+2+H]$^+$.

Production Example 263: Synthesis of 3-(3-bromo-4-methoxybenzoyl)-5-(5-cyclopropyl-1,3,4-oxadiazol-2-yl)-4-methyl-1H-pyrrole-2-carboaldehyde [compound No. 263]

Production Example 263-1: Synthesis of 3-(3-bromo-4-methoxybenzoyl)-5-(5-cyclopropyl-1,3,4-oxadiazol-2-yl)-4-methyl-1H-pyrrole-2-carboaldehyde [compound No. 263]

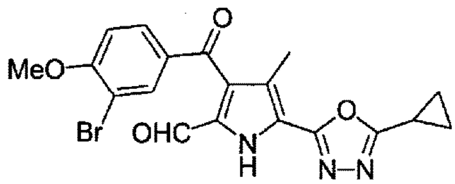

N-Chlorosuccinimide (690 mg, 5.16 mmol) and silver nitrate (986 mg, 5.81 mmol) were suspended in an 80% aqueous acetonitrile solution (67 ml). To the suspension, compound 262 (672 mg, 1.29 mmol), 2,6-lutidine (0.18 ml, 1.55 mmol), and an 80% aqueous acetonitrile solution (67 ml) were added, and the mixture was stirred at room temperature for 1.5 hours. After reaction, the solvent was distilled off. The residue was dissolved in chloroform. The organic layer was washed with water.
The organic layer was dried over magnesium sulfate, and the solvent was distilled off. The residue was purified by silica gel column chromatography to obtain the title compound (379 mg, yield: 68.3%) as a yellow solid.
$^1$H-NMR (400 MHz, DMSO-$d_6$) δ: 1.17-1.22 (4H, m), 2.20 (3H, s), 2.32-2.34 (1H, m), 3.96 (3H, s), 7.24 (1H, d, J = 8.5 Hz), 7.78 (1H, dd, J = 8.5, 2.2 Hz), 7.97 (1H, d, J = 2.2 Hz), 9.50 (1H, s), 13.48 (1H, br s).
ESI-MS m/z: 430[M+H]$^+$, 432[M+2+H]$^+$.

Production Example 264: Synthesis of (3-bromophenyl)(5-(5-cyclopropyl-1,3,4-oxadiazol-2-yl)-2-(hydroxymethyl)-4-methyl-1H-pyrrol-3-yl)methanone [compound No. 264]

Production Example 264-1: Synthesis of (3-bromophenyl)(5-(5-cyclopropyl-1,3,4-oxadiazol-2-yl)-2-(hydroxymethyl)-4-methyl-1H-pyrrol-3-yl)methanone [compound No. 264]

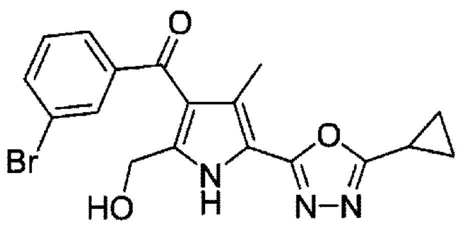

The title compound was obtained in the same way as in Production Example 58-1 using compound 261 as a starting material. Yield: 55.9 %, white solid.
$^1$H-NMR (400 MHz, DMSO-$d_6$) δ: 1.10-1.17 (4H, m), 2.18 (3H, s), 2.25-2.30 (1H, m), 4.33 (2H, d, J = 5.4 Hz), 5.09 (1H,

t, J = 5.4 Hz), 7.47 (1H, t, J = 7.8 Hz), 7.65 (1H, dt, J = 7.8, 1.3 Hz), 7.77 (1H, t, J = 1.7 Hz), 7.82 (1H, dq, J = 7.8,1.3 Hz), 12.25 (1H, br s).
ESI-MS m/z: 402$[M+H]^+$, 404$[M+2+H]^+$.

Production Example 265: Synthesis of (3-bromophenyl)(5-(5-cyclopropyl-1,3,4-oxadiazol-2-yl)-2-(difluoromethyl)-4-methyl-1H-pyrrol-3-yl)methanone [compound No. 265]

Production Example 265-1: Synthesis of (3-bromophenyl)(5-(5-cyclopropyl-1,3,4-oxadiazol-2-yl)-2-(difluoromethyl)-4-methyl-1H-pyrrol-3-yl)methanone [compound No. 265]

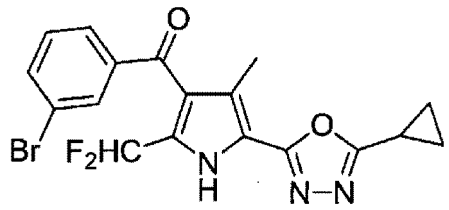

The title compound was obtained in the same way as in Production Example 57-1 using compound 261 as a starting material. Yield: 19.2 %, yellow solid.
$^1$H-NMR (400 MHz, DMSO-$d_6$) δ: 1.12-1.15 (4H, m), 2.11 (3H, s), 2.28-2.34 (1H, m), 6.90 (1H, t, J = 53.2 Hz), 7.51 (1H, t, J = 7.9 Hz), 7.70 (1H, dd, J = 7.0, 1.0 Hz), 7.83 (1H, s), 7.88 (1H, dt, J = 7.9, 1.0 Hz), 13.23 (1H, br s). ESI-MS m/z: 422$[M+H]^+$, 424$[M+2+H]^+$.

Production Example 266: Synthesis of (3-bromo-4-methoxyphenyl)(5-(5-cyclopropyl-1,3,4-oxadiazol-2-yl)-2-(hydroxymethyl)-4-methyl-1H-pyrrol-3-yl)methanone [compound No. 266]

Production Example 266-1: Synthesis of (3-bromo-4-methoxyphenyl)(5-(5-cyclopropyl-1,3,4-oxadiazol-2-yl)-2-(hydroxymethyl)-4-methyl-1H-pyrrol-3-yl)methanone [compound No. 266]

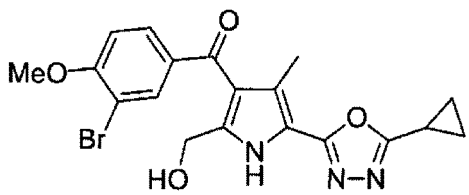

The title compound was obtained in the same way as in Production Example 58-1 using compound 263 as a starting material. Yield: 60.0 %, white solid.
$^1$H-NMR (400 MHz, DMSO-$d_6$) δ: 1.10-1.17 (4H, m), 2.18 (3H, s), 2.25-2.30 (1H, m), 3.95 (3H, s), 4.35 (2H, s), 5.08 (1H, br s), 7.22 (1H, d, J = 8.5 Hz), 7.71 (1H, dd, J = 8.5, 2.2 Hz), 7.86 (1H, d, J = 2.2 Hz), 12.22 (1H, br s). ESI-MS m/z: 432$[M+H]^+$, 434$[M+2+H]^+$.

Production Example 267: Synthesis of (3-bromo-4-methoxyphenyl)(5-(5-cyclopropyl-1,3,4-oxadiazol-2-yl)-2-(difluoromethyl)-4-methyl-1H-pyrrol-3-yl)methanone [compound No. 267]

Production Example 267-1: Synthesis of (3-bromo-4-methoxyphenyl)(5-(5-cyclopropyl-1,3,4-oxadiazol-2-yl)-2-(difluoromethyl)-4-methyl-1H-pyrrol-3-yl)methanone [compound No. 267]

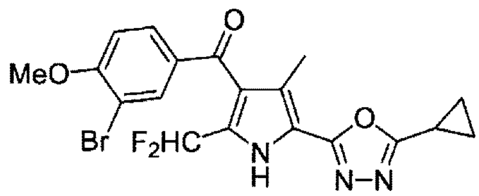

The title compound was obtained in the same way as in Production Example 57-1 using compound 263 as a starting material. Yield: 10.3 %, brown solid.
$^1$H-NMR (400 MHz, DMSO-$d_6$) δ: 1.13-1.16 (2H, m), 1.16-1.20 (2H, m), 2.14 (3H, s), 2.29-2.34 (1H, m), 3.96 (3H, s), 6.88 (1H, t, J = 53.3 Hz), 7.26 (1H, d, J = 8.5 Hz), 7.74 (1H, dd, J = 8.5, 2.2 Hz), 7.90 (1H, d, J = 2.2 Hz), 13.20 (1H, br s).
ESI-MS m/z: 452[M+H]$^+$, 454[M+2+H]$^+$.

Production Example 268: Synthesis of (3-bromo-4-methoxyphenyl)(5-(5-cyclopropyl-1,3,4-oxadiazol-2-yl)-2-(1-hydroxyethyl)-4-methyl-1H-pyrrol-3-yl)methanone [compound No. 268]

Production Example 268-1: Synthesis of (3-bromo-4-methoxyphenyl)(5-(5-cyclopropyl-1,3,4-oxadiazol-2-yl)-2-(1-hydroxyethyl)-4-methyl-1H-pyrrol-3-yl)methanone [compound No. 268]

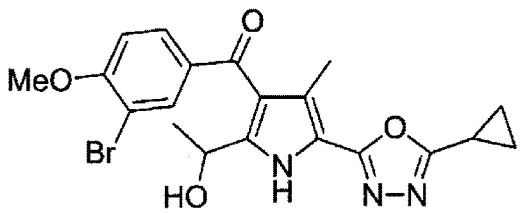

The title compound was obtained in the same way as in Production Example 61-1 using compound 263 as a starting material. Yield: 45.1 %, yellow solid.
$^1$H-NMR (400 MHz, DMSO-$d_6$) δ: 1.12-1.17 (4H, m), 1.36 (3H, d, J = 6.6 Hz), 2.11 (3H, s), 2.30-2.26 (1H, m), 3.95 (3H, s), 4.72 (1H, dd, J = 15.0, 6.6 Hz), 5.29 (1H, br s), 7.24 (1H, d, J = 8.5 Hz), 7.71 (1H, dd, J = 8.5, 2.2 Hz), 7.85 (1H, d, J = 2.2 Hz), 12.05 (1H, br s).
ESI-MS m/z: 446[M+H]$^+$, 448[M+2+H]$^+$.

Production Example 269: Synthesis of (3-(3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate [compound No. 269]

Production Example 269-1: Synthesis of (3-(3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate [compound No. 269]

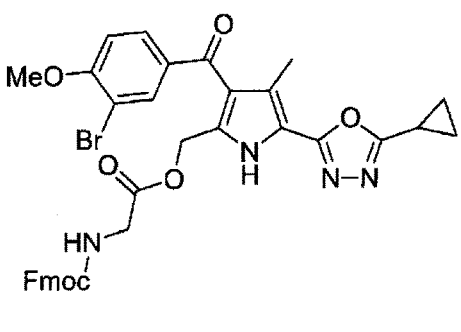

The title compound was obtained in the same way as in Production Example 151-1 using compound 266 as a starting material. Yield: 60.9 %, yellow solid.

ESI-MS m/z: 711[M+H]+, 713[M+2+H]+.

Production Example 270: Synthesis of (3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone [compound No. 270]

Production Example 270-1: Synthesis of (3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone [compound No. 270]

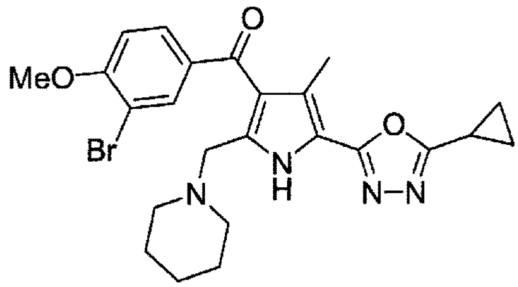

Compound 269 (39 mg, 0.055 mmol) was dissolved in N,N-dimethylformamide (0.4 ml). To the solution, piperidine (6.1 ml) was added, and the mixture was stirred at room temperature for 1 hour. After reaction, the solvent was distilled off. The residue was purified by silica gel column chromatography to obtain the title compound (39 mg, yield: 15.5%) as a white solid.
$^1$H-NMR (400 MHz, DMSO-$d_6$) δ: 1.09-1.21 (4H, m), 1.32-1.43 (1H, m), 1.63-1.82 (7H, m), 2.08 (3H, s), 2.28-2.34 (1H, m), 2.56 (11H, s), 2.91-3.00 (3H, m), 3.97 (4H, s), 4.31 (2H, d, J = 4.6 Hz), 7.26 (1H, d, J = 8.5 Hz), 7.76 (1H, dd, J = 8.5, 2.2 Hz), 7.93 (1H, d, J = 2.2 Hz), 9.66 (1H, br s), 11.05 (2H, br s), 12.83 (1H, br s).
ESI-MS m/z: 499[M+H]+, 501[M+2+H]+.

Production Example 271: Synthesis of (3-bromophenyl)(2,4-dimethyl-5-(3-methyl-1,2,4-oxadiazol-5-yl)-1H-pyrrol-3-yl)methanone [compound No. 271]

Production Example 271-1: Synthesis of (E)-N'-((4-(3-bromobenzoyl)-3,5-dimethyl-1H-pyrrole-2-carbonyl)oxy)acetamide

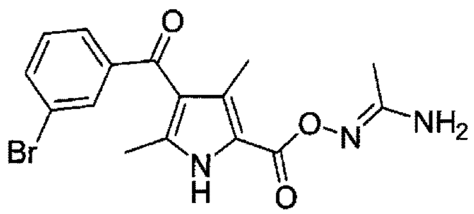

To a solution of the compound (322 mg, 1 mmol) obtained in Production Example 96-1 in N,N-dimethylformamide (5 ml), HATU (380 mg, 1 mmol) and diisopropylethylamine (349 μl, 2 mmol) were added in the argon atmosphere, and subsequently acetamidoxime (74 mg, 1 mmol) was added thereto. After 12 hours, the reaction solution was quenched by the addition of water. The aqueous layer was extracted with chloroform. The obtained organic layer was dried over anhydrous magnesium sulfate. After filtering off of magnesium sulfate, the solvent was distilled off. The residue was purified by column chromatography to obtain the title compound (213 mg, yield: 56.3%, light orange solid).
$^1$H-NMR (400 MHz, DMSO-$d_6$) δ: 1.81 (3H, s), 2.13 (3H, s), 2.18 (3H, s), 6.55 (2H, s), 7.44-7.50 (1H, m), 7.58-7.62 (1H, m), 7.71-7.74 (1H, m), 7.78-7.82 (1H, m), 11.84 (1H, s).

Production Example 271-2: Synthesis of (3-bromophenyl)(2,4-dimethyl-5-(3-methyl-1,2,4-oxadiazol-5-yl)-1H-pyrrol-3-yl)methanone [compound No. 271]

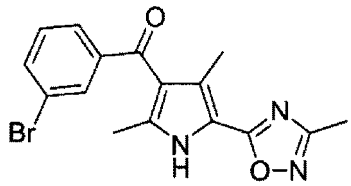

A solution of the compound (213 mg, 0.56 mmol) obtained in Production Example 271-1 in N,N-dimethylformamide (10 ml) was heated with stirring at 140°C in the argon atmosphere. After 3 hours, the reaction solution was allowed to cool and quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the title compound (14 mg, yield: 6.9%, yellow oil).
$^1$H-NMR (400 MHz, $CDCl_3$) δ: 2.31 (3H, s), 2.32 (3H, s), 2.43 (3H, s), 7.32-7.38 (1H, m), 7.64-7.71 (2H, m), 7.85-7.88 (1H, m), 9.04 (1H, s).

Production Example 272: Synthesis of (3-bromophenyl)(5-(5-ethyloxazol-2-yl)-2,4-dimethyl-1H-pyrrol-3-yl)methanone [compound No. 272]

Production Example 272-1: Synthesis of 4-(3-bromobenzoyl)-N-(2-hydroxybutyl)-3,5-dimethyl-1H-pyrrole-2-carboxamide

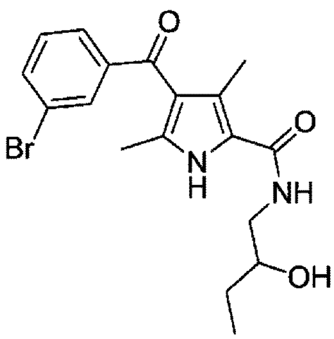

To a solution of the compound (322 mg, 1 mmol) obtained in Production Example 96-1 in N,N-dimethylformamide (5 ml), 1-ethyl-3-(3-dimethylaminopropyl)carbodiimide hydrochloride (231 mg, 1.2 mmol) and 1-hydroxybenzotriazole (163 mg, 1.2 mmol) were added in the argon atmosphere. After 1 hour, 1-amino-2-butanol (107 μl, 1.2 mmol) was added to the mixture. After 12 hours, the reaction solution was quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the title compound (194 mg, yield: 49.3%, white solid).
$^1$H-NMR (400 MHz, DMSO-$d_6$) δ: 0.89 (3H, t, J = 7.3 Hz), 1.27-1.52 (2H, m), 2.06 (3H, s), 2.15 (3H, s), 3.09-3.18 (1H, m), 3.27-3.36 (1H, m), 3.45-3.53 (1H, m), 4.74 (1H, d, J = 5.1 Hz), 7.34-7.40 (1H, m), 7.43-7.49 (1H, m), 7.55-7.60 (1H, m), 7.69-7.71 (1H, m), 7.77-7.81 (1H, m), 11.66 (1H, s).

Production Example 272-2: Synthesis of 4-(3-bromobenzoyl)-3,5-dimethyl-N-(2-oxobutyl)-1H-pyrrole-2-carboxamide

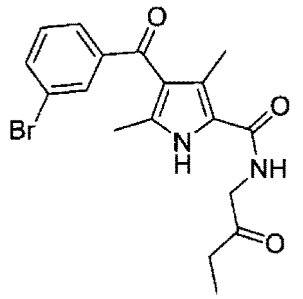

To a suspension of the compound (100 mg, 0.25 mmol) obtained in Production Example 272-1 in dichloromethane (5 ml), Dess-Martin periodinane (216 mg, 0.51 mmol) was added in the argon atmosphere. After 2 hours, the reaction solution was quenched by the addition of a saturated aqueous solution of sodium bicarbonate. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the title compound (65 mg, yield: 65.4%, orange solid).
$^1$H-NMR (400 MHz, DMSO-$d_6$) $\delta$: 0.96 (3H, t, J = 7.3 Hz), 2.07 (3H, s), 2.16 (3H, s), 2.48-2.52 (2H, m), 4.09 (2H, d, J = 5.4 Hz), 7.44-7.49 (1H, m), 7.57-7.61 (1H, m), 7.66-7.73 (2H, m), 7.77-7.81 (1H, m), 11.73 (1H, s).

Production Example 272-3: Synthesis of (3-bromophenyl)(5-(5-ethyloxazol-2-yl)-2,4-dimethyl-1H-pyrrol-3-yl)methanone [compound No. 272]

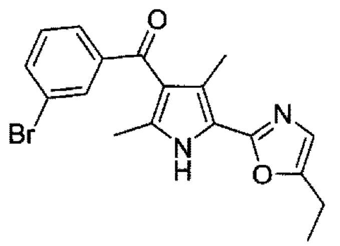

To a solution of the compound (65 mg, 0.17 mmol) obtained in Production Example 272-2 in acetonitrile (10 ml), phenylphosphonic dichloride (24 μl, 0.17 mmol) was added in the argon atmosphere, and the mixture was heated with stirring at 80°C. After 12 hours, the reaction solution was allowed to cool and quenched by the addition of water. The aqueous layer was extracted with chloroform. The obtained organic layer was dried over anhydrous magnesium sulfate. Magnesium sulfate was filtered off, and the solvent was distilled off under reduced pressure. Then, the residue was purified by column chromatography to obtain the title compound (50 mg, yield: 80.8%, yellow solid).
$^1$H-NMR (400 MHz, DMSO-$d_6$) $\delta$: 1.24 (3H, t, J = 7.4 Hz), 2.11 (3H, s), 2.21 (3H, s), 2.72 (2H, q, J = 7.6 Hz), 6.92-6.94 (1H, m), 7.44-7.49 (1H, m), 7.58-7.62 (1H, m), 7.71-7.73 (1H, m), 7.77-7.81 (1H, m), 12.00 (1H, s).

Production Example 273: Synthesis of (5-bromo-2-methoxyphenyl)(5-(5-(1-bromocyclopropyl)-1,3,4-oxadiazol-2-yl)-2,4-dimethyl-1H-pyrrol-3-yl)methanone [compound No. 273]

Production Example 273-1: Synthesis of (5-bromo-2-methoxyphenyl)(5-(5-(1-bromocyclopropyl)-1,3,4-oxadiazol-2-yl)-2,4-dimethyl-1H-pyrrol-3-yl)methanone [compound No. 273]

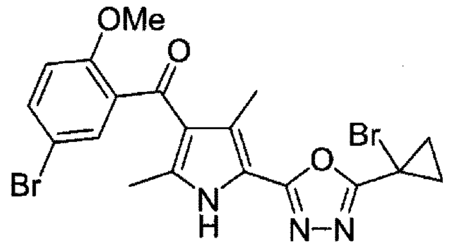

Compound 240 (156 mg, 0.37 mmol) was dissolved in carbon tetrachloride (4.7 ml). To the solution, N-bromosuccinimide (73 mg, 0.41 mmol) and AIBN (6 mg, 0.037 mmol) were added, and the mixture was heated to reflux for 7 hours. After reaction, the solvent was distilled off under reduced pressure. The residue was purified using a preparative LC system with MS triggers manufactured by Waters Corp. to obtain the compound of interest (5 mg, yield: 2.7%, yellow solid).
$^1$H-NMR (400 MHz, DMSO-$d_6$) δ: 2.09 (3H, s), 2.27 (3H, s), 2.76-2.90 (2H, m), 3.62-3.69 (1H, m), 3.73 (3H, s), 3.79-3.75 (1H, m), 5.58 (1H, dd, J = 8.3, 6.1 Hz), 7.11 (1H, d, J = 9.0 Hz), 7.36 (1H, d, J = 2.7 Hz), 7.64 (1H, dd, J = 9.0, 2.7 Hz), 12.39 (1H, br s).
ESI-MS m/z: 493[M+H]$^+$, 495[M+2+H]$^+$, 497[M+4+H]$^+$.

Production Example 274: Synthesis of (5-bromo-2-methoxyphenyl)(1-bromo-5-(5-(1-bromocyclopropyl)-1,3,4-oxadiazol-2-yl)-2,4-dimethyl-1H-pyrrol-3-yl)methanone [compound No. 274]

Production Example 274-1: Synthesis of (5-bromo-2-methoxyphenyl)(1-bromo-5-(5-(1-bromocyclopropyl)-1,3,4-oxadiazol-2-yl)-2,4-dimethyl-1H-pyrrol-3-yl)methanone [compound No. 274]

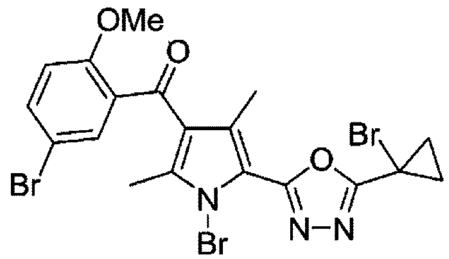

This compound was obtained as a by-product through the reaction of Production Example 273-1. Yield: 4.2%, yellow solid.
ESI-MS m/z: 571[M+H]$^+$,573[M+2+H]$^+$,575[M+4+H]$^+$.

Production Example 275: Synthesis of (3-bromophenyl)(2,4-dimethyl-5-(1,3,4-oxadiazol-2-yl)-1H-pyrrol-3-yl)methanone [compound No. 275]

Production Example 275-1: Synthesis of (3-bromophenyl)(2,4-dimethyl-5-(1,3,4-oxadiazol-2-yl)-1H-pyrrol-3-yl)methanone [compound No. 275]

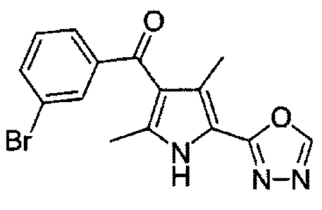

The title compound was obtained by the same procedures as in Production Example 173-2 using dimethylcarbamyl chloride. Yield: 27.9%, ocher solid. ESI-MS m/z: 346[M+H]+,348[M+2+H]+.

Production Example 276: Synthesis of (5-(5-(2-aminoethyl)-1,3,4-oxadiazol-2-yl)-2,4-dimethyl-1H-pyrrol-3-yl)(3-bromophenyl)methanone [compound No. 276]

Production Example 276-1: Synthesis of (5-(5-(2-aminoethyl)-1,3,4-oxadiazol-2-yl)-2,4-dimethyl-1H-pyrrol-3-yl)(3-bromophenyl)methanone [compound No. 276]

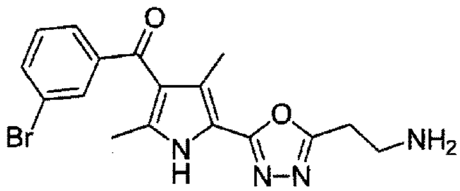

The title compound was obtained by the same procedures as in Production Examples 204-3, 204-4, and 48-1 using 3-((tert-butoxycarbonyl)amino)propionic acid. Yield: 18.1%, pale yellow solid.
$^1$H-NMR (400 MHz, DMSO-$d_6$) δ: 2.14 (3H, s), 2.21 (3H, s), 3.01-3.14 (2H, m), 7.44-7.52 (1H, m), 7.57-7.64 (1H, m), 7.71-7.76 (1H, m), 7.77-7.85 (1H, m).

Production Example 277: Synthesis of 2-(2-(5-(4-(3-bromobenzoyl)-3,5-dimethyl-1H-pyrrol-2-yl)-1,3,4-oxadiazol-2-yl)ethyl)isoindoline-1,3-dione [compound No. 277]

Production Example 277-1: Synthesis of 2-(2-(5-(4-(3-bromobenzoyl)-3,5-dimethyl-1H-pyrrol-2-yl)-1,3,4-oxadiazol-2-yl)ethyl)isoindoline-1,3-dione [compound No. 277]

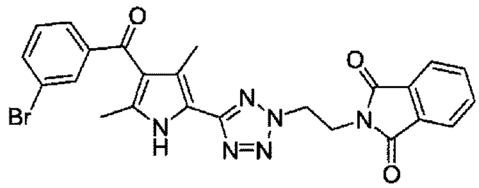

The compound of interest was obtained by the same procedures as in Production Example 97-1 using N-(2-bromoethyl)phthalimide. Yield: 65.1 %, pale yellow solid. $^1$H-NMR (400 MHz, $CDCl_3$) δ: 2.09 (3H, s), 2.26 (3H, s), 4.27 (2H, t, J = 5.5 Hz), 4.97 (2H, t, J = 5.6 Hz), 7.30-7.37 (1H, m), 7.63-7.89 (7H, m), 8.98 (1H, s).

Production Example 278: Synthesis of 3-(3-bromobenzoyl)-5-(2-(2-(1,3-dioxoisoindolin-2-yl)ethyl)-2H-tetrazol-5-yl)-4-methyl-1H-pyrrole-2-carboaldehyde [compound No. 278]

Production Example 278-1: Synthesis of 3-(3-bromobenzoyl)-5-(2-(2-(1,3-dioxoisoindolin-2-yl)ethyl)-2H-tetrazol-5-yl)-4-methyl-1H-pyrrole-2-carboaldehyde [compound No. 278]

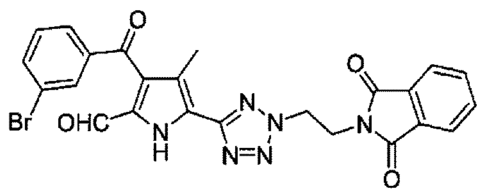

The compound of interest was obtained by the same procedures as in Production Example 116-1 using compound 277 as a starting material. Yield: 62.9 %, white solid. $^1H$-NMR (400 MHz, $CDCl_3$) δ: 2.21 (3H, s), 4.30 (2H, t, J = 5.5 Hz), 5.02 (2H, t, J = 5.5 Hz), 7.35-7.42 (1H, m), 7.71-7.79 (4H, m), 7.81-7.87 (2H, m), 7.99-8.03(1H, m), 9.49 (1H, s), 10.10 (1H, s).

Production Example 279: Synthesis of 2-(2-(5-(4-(3-bromobenzoyl)-5-(hydroxymethyl)-3-methyl-1H-pyrrol-2-yl)-2H-tetrazol-2-yl)ethyl)isoindoline-1,3-dione [compound No. 279]

Production Example 279-1: Synthesis of 2-(2-(5-(4-(3-bromobenzoyl)-5-(hydroxymethyl)-3-methyl-1H-pyrrol-2-yl)-2H-tetrazol-2-yl)ethyl)isoindoline-1,3-dione [compound No. 279]

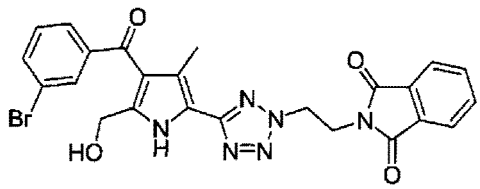

The compound of interest was obtained by the same procedures as in Production Example 58-1 using compound 278 as a starting material. Yield: 37.4 %, white solid.
$^1H$-NMR (400 MHz, $CDCl_3$) δ: 2.00 (3H, s), 4.28 (2H, t, J = 5.4 Hz), 4.68 (2H, s), 4.98 (2H, t, J = 5.6 Hz), 7.32-7.38 (1H, m), 7.70-7.89 (7H, m), 9.59 (1H, s).

Production Example 280: Synthesis of (5-(2-(2-aminoethyl)-2H-tetrazol-5-yl)-2-(hydroxymethyl)-4-methyl-1H-pyrrol-3-yl)(3-bromophenyl)methanone [compound No. 280]

Production Example 280-1: Synthesis of (5-(2-(2-aminoethyl)-2H-tetrazol-5-yl)-2-(hydroxymethyl)-4-methyl-1H-pyrrol-3-yl)(3-bromophenyl)methanone [compound No. 280]

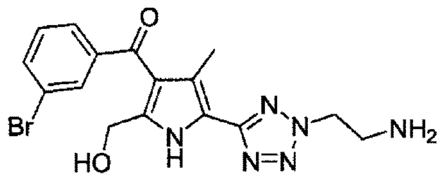

Hydrazine monohydrate (32 μl, 0.66 mmol) was added to a solution of compound 279 (58 mg, 0.11 mmol) in methanol (2 ml). After 12 hours, saturated saline was added to the reaction solution, followed by extraction with chloroform. The obtained organic layer was dried over anhydrous magnesium sulfate. Then, magnesium sulfate was filtered off, and the solvent was distilled off. The residue was purified by column chromatography to obtain the compound of interest (26 mg, yield: 58.3%, pale yellow solid).
$^1H$-NMR (400 MHz, $CDCl_3$) δ: 2.00 (3H, s), 4.28 (2H, t, J = 5.4 Hz), 4.68 (2H, s), 4.98 (2H, t, J = 5.6 Hz), 7.32-7.38 (1H, m), 7.70-7.89 (7H, m), 9.59 (1H, s).

Production Example 281: Synthesis of 3-(3-bromobenzoyl)-5-(2-(2-(1,3-dioxoisoindolin-2-yl)ethyl)-2H-tetrazol-5-yl)-N,4-dimethyl-1H-pyrrole-2-carboxamide [compound No. 281]

Production Example 281-1: Synthesis of 3-(3-bromobenzoyl)-5-(2-(2-(1,3-dioxoisoindolin-2-yl)ethyl)-2H-tetrazol-5-yl)-N,4-dimethyl-1H-pyrrole-2-carboxamide [compound No. 281]

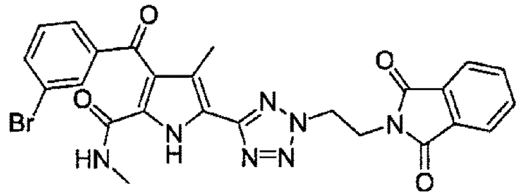

To a suspension of compound 142 (174 mg, 0.40 mmol), tributylphosphine (302 μl, 1.21 mmol), and phthalimide (178 mg, 1.21 mmol) in benzene (6 ml), N,N,N',N'-tetramethylazodicarboxamide (208 mg, 1.21 mmol) was added under ice cooling in the argon atmosphere. After 10 minutes, the reaction temperature was increased to 60°C. After 1 hour, the reaction solution was allowed to cool. Saturated saline was added thereto, followed by extraction with chloroform. The obtained organic layer was dried over anhydrous magnesium sulfate. Then, magnesium sulfate was filtered off, and the solvent was distilled off. The residue was purified by column chromatography to obtain the compound of interest (126 mg, yield: 55.7%, white solid).
$^1$H-NMR (400 MHz, $CDCl_3$) δ: 1.95 (3H, s), 2.98 (3H, d, J = 4.9 Hz), 4.26-4.31 (2H, m), 4.96-5.03 (2H, m), 7.34-7.40 (1H, m), 7.64-7.69 (1H, m), 7.71-7.77 (3H, m), 7.81-7.86 (2H, m), 7.89-7.92 (1H, m), 8.87 (1H, s).

Production Example 282: Synthesis of 5-(2-(2-aminoethyl)-2H-tetrazol-5-yl)-3-(3-bromobenzoyl)-N,4-dimethyl-1H-pyrrole-2-carboxamide [compound No. 282]

Production Example 282-1: Synthesis of 5-(2-(2-aminoethyl)-2H-tetrazol-5-yl)-3-(3-bromobenzoyl)-N,4-dimethyl-1H-pyrrole-2-carboxamide [compound No. 282]

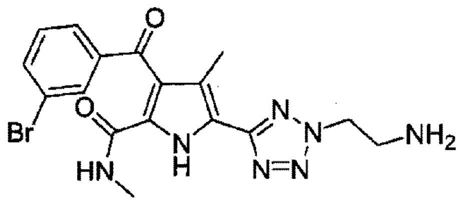

Hydrazine monohydrate (64 μl, 1.32 mmol) was added to a suspension of compound 281 (126 mg, 0.22 mmol) in ethanol (4 ml), and the mixture was heated to reflux. After 2 hours, the reaction solution was allowed to cool. Saturated saline was added thereto, followed by extraction with chloroform. The obtained organic layer was dried over anhydrous magnesium sulfate. Then, magnesium sulfate was filtered off, and the solvent was distilled off under reduced pressure. The residue was purified by column chromatography to obtain the compound of interest (48 mg, yield: 49.6%, pale yellow solid).
$^1$H-NMR (400 MHz, DMSO-$d_6$) δ: 2.24 (3H, s), 2.56 (3H, d, J = 4.6 Hz), 3.15 (2H, t, J = 6.1 Hz), 4.71 (2H, t, J = 6.0 Hz), 7.39-7.48 (1H, m), 7.61-7.68 (1H, m), 7.75-7.82 (2H, m), 8.26-8.34 (1H, m).

Production Example 283: Synthesis of 3-(3-bromobenzoyl)-5-(2-(2-(1,3-dioxoisoindolin-2-yl)ethyl)-2H-tetrazol-5-yl)-4-methyl-1H-pyrrole-2-carbonitrile [compound No. 283]

Production Example 283-1: Synthesis of 3-(3-bromobenzoyl)-5-(2-(2-(1,3-dioxoisoindolin-2-yl)ethyl)-2H-tetrazol-5-yl)-4-methyl-1H-pyrrole-2-carbonitrile [compound No. 283]

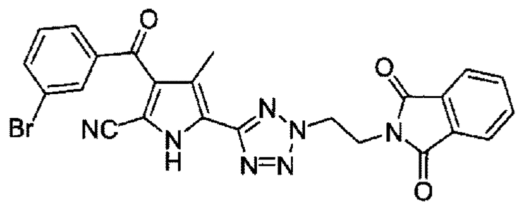

The compound of interest was obtained by the same procedures as in Production Example 132-1 using compound 278 as a starting material. Yield: 42.7%, yellow solid. $^1$H-NMR (400 MHz, $CDCl_3$) δ: 2.31 (3H, s), 4.30 (2H, t, J = 5.4 Hz), 5.03 (2H, t, J = 5.4 Hz), 7.36-7.43 (1H, m), 7.72-7.79 (4H, m), 7.81-7.87 (2H, m), 7.94-7.97(1H, m), 10.07 (1H, s).

Production Example 284: Synthesis of 5-(2-(2-aminoethyl)-2H-tetrazol-5-yl)-3-(3-bromobenzoyl)-4-methyl-1H-pyrrole-2-carbonitrile [compound No. 284]

Production Example 284-1: Synthesis of 5-(2-(2-aminoethyl)-2H-tetrazol-5-yl)-3-(3-bromobenzoyl)-4-methyl-1H-pyrrole-2-carbonitrile [compound No. 284]

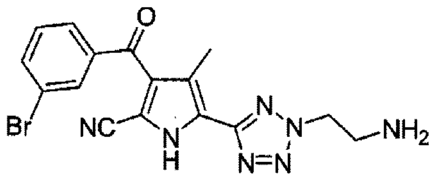

The compound of interest was obtained by the same procedures as in Production Example 280-1 using compound 283 as a starting material. Yield: 51.8 %, white solid. ESI-MS m/z: 400[M+H]+, 402[M+2+H]+.

Production Example 285: Synthesis of (3-bromophenyl)(5-(2-(2-hydroxyethyl)-2H-tetrazol-5-yl)-2-(2-hydroxypropan-2-yl)-4-methyl-1H-pyrrol-3-yl)methanone [compound No. 285]

Production Example 285-1: Synthesis of (3-bromophenyl)(5-(2-(2-hydroxyethyl)-2H-tetrazol-5-yl)-2-(2-hydroxypropan-2-yl)-4-methyl-1H-pyrrol-3-yl)methanone [compound No. 285]

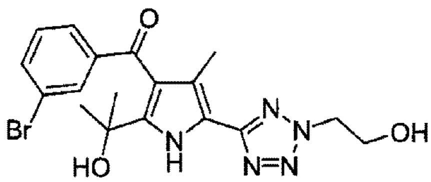

To a solution of compound 137 (117 mg, 0.27 mmol) in tetrahydrofuran (6 ml), methyl magnesium bromide (1.4 M solution in toluene and tetrahydrofuran, 770 μl, 1.08 mmol) was added in the argon atmosphere, and the mixture was heated with stirring. After 12 hours, the reaction solution was allowed to cool, and 1 M hydrochloric acid was added thereto. The aqueous layer was extracted with chloroform. The obtained organic layer was dried over anhydrous magnesium sulfate. After filtering off of magnesium sulfate, the solvent was distilled off under reduced pressure. The residue was purified by column chromatography to obtain the compound of interest (27 mg, yield: 23.0%, yellow solid).
$^1$H-NMR (400 MHz, DMSO-$d_6$) δ: 1.46 (6H, s), 2.01 (3H, s), 3.90-4.00 (2H, m), 4.73 (2H, t, J = 5.2 Hz), 5.07 (1H, t, J = 5.6 Hz), 5.25 (1H, s), 7.45-7.50 (1H, m), 7.67-7.72 (1H, m), 7.78-7.83 (2H, m), 11.13 (1H, s).

Production Example 286: Synthesis of (4-bromo-2-methyl-5-(1H-tetrazol-5-yl)-1H-pyrrol-3-yl)(3-bromophenyl)methanone [compound No. 286]

Production Example 286-1: Synthesis of ethyl 3-bromo-4-(3-bromobenzoyl)-5-methyl-1H-pyrrole-2-carboxylate

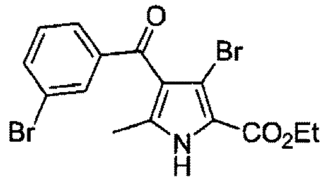

The compound of interest was obtained by the same procedures as in Production Example 85-1 using compound 41 as a starting material. Yield: 60.6 %, white solid. $^1$H-NMR (400 MHz, $CDCl_3$) δ: 1.40 (3H, t, J = 7.1 Hz), 2.39 (3H, s), 4.38 (2H, q, J = 7.1 Hz), 7.31-7.36 (1H, m), 7.67-7.72 (2H, m), 7.89-7.92 (1H, m), 9.24 (1H, s).

Production Example 286-2: Synthesis of 3-bromo-4-(3-bromobenzoyl)-5-methyl-1H-pyrrole-2-carboxylic acid

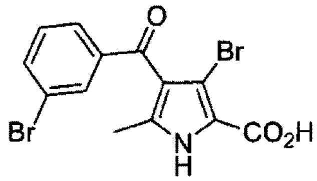

The compound of interest was obtained by the same procedures as in Production Example 96-1 using the compound obtained in Production Example 286-1 as a starting material. Yield: quant., white solid.
$^1$H-NMR (400 MHz, DMSO-$d_6$) δ: 2.21 (3H, s), 7.45-7.50 (1H, m), 7.63-7.67 (1H, m), 7.76-7.78 (1H, m), 7.80-7.84 (1H, m), 12.54 (1H, s), 12.97 (1H, s).

Production Example 286-3: Synthesis of 3-bromo-4-(3-bromobenzoyl)-5-methyl-1H-pyrrole-2-carboxamide

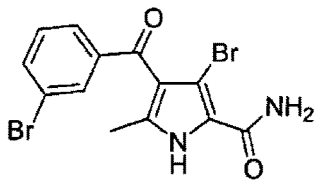

To a suspension of the compound (300 mg, 0.78 mmol) obtained in Production Example 286-2 in dichloromethane (5 ml), oxalyl chloride (67 μl, 0.78 mmol) and N,N-dimethylformamide (one drop) were added in the argon atmosphere. After 1 hour, 28% ammonia water was added to the mixture. After 30 minutes, saturated saline was added to the reaction solution. The aqueous layer was extracted with chloroform. The obtained organic layer was dried over anhydrous magnesium sulfate. Magnesium sulfate was filtered off, and the solvent was distilled off under reduced pressure to obtain the compound of interest (171 mg, yield: 56.6%, orange solid).
$^1$H-NMR (400 MHz, $CDCl_3$) δ: 2.34 (3H, s), 7.32-7.38 (1H, m), 7.67-7.73 (2H, m), 7.87-7.91 (1H, m), 10.00 (1H, s).

Production Example 286-4: Synthesis of 3-bromo-4-(3-bromobenzoyl)-5-methyl-1H-pyrrole-2-carbonitrile

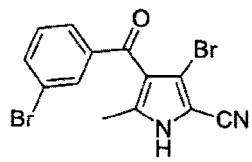

To a solution of the compound (171 mg, 0.44 mmol) obtained in Production Example 286-3 in dichloromethane

(6 ml), phosphorus oxychloride (116 μl, 1.26 mmol) was added in the argon atmosphere, and the mixture was heated to reflux. After 2 hours, ice water was added to the reaction solution. The aqueous layer was extracted with chloroform. The obtained organic layer was dried over anhydrous magnesium sulfate. Magnesium sulfate was filtered off, and the solvent was distilled off under reduced pressure. The residue was purified by column chromatography to obtain the compound of interest (111 mg, yield: 68.1%, pink solid).
$^1$H-NMR (400 MHz, $CDCl_3$) δ: 2.43 (3H, s), 7.33-7.39 (1H, m), 7.65-7.75 (2H, m), 7.86-7.89 (1H, m), 9.05 (1H, s).

Production Example 286-5: Synthesis of (4-bromo-2-methyl-5-(1H-tetrazol-5-yl)-1H-pyrrol-3-yl)(3-bromophenyl)methanone [compound No. 286]

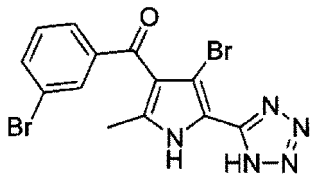

The compound of interest was obtained by the same procedures as in Production Example 96-4 using the compound obtained in Production Example 286-4 as a starting material. Yield: 35.9%, black solid.
$^1$H-NMR (400 MHz, DMSO-$d_6$) δ: 2.28 (3H, s), 7.46-7.52 (1H, m), 7.67-7.72 (1H, m), 7.80-7.87 (2H, m), 12.85 (1H, s).

Production Example 287: Synthesis of 2-(5-(3-bromo-4-(3-bromobenzoyl)-5-methyl-1H-pyrrol-2-yl)-2H-tetrazol-2-yl)ethyl acetate [compound No. 287]

Production Example 287-1: Synthesis of 2-(5-(3-bromo-4-(3-bromobenzoyl)-5-methyl-1H-pyrrol-2-yl)-2H-tetrazol-2-yl)ethyl acetate [compound No. 287]

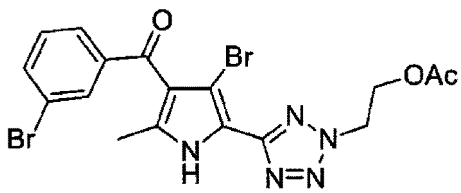

The compound of interest was obtained by the same procedures as in Production Example 97-1 using compound 286 and 2-bromoethyl acetate. Yield: 13.4 %, white solid. $^1$H-NMR (400 MHz, $CDCl_3$) δ: 2.28 (3H, s), 2.45 (3H, s), 4.22-4.27 (2H, m), 4.81-4.86 (2H, m), 7.32-7.38 (1H, m), 7.66-7.76 (2H, m), 7.92-7.95 (1H, m), 9.54 (1H, s).

Production Example 288: Synthesis of (4-bromo-5-(2-(2-hydroxyethyl)-2H-tetrazol-5-yl)-2-methyl-1H-pyrrol-3-yl)(3-bromophenyl)methanone [compound No. 288]

Production Example 288-1: Synthesis of (4-bromo-5-(2-(2-hydroxyethyl)-2H-tetrazol-5-yl)-2-methyl-1H-pyrrol-3-yl)(3-bromophenyl)methanone [compound No. 288]

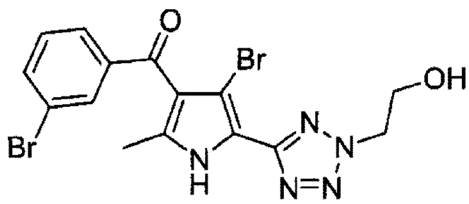

The compound of interest was obtained by the same procedures as in Production Example 117-1 using compound 287 as a starting material. Yield: trace, white solid. $^1$H-NMR (400 MHz, $CDCl_3$) δ: 2.45 (3H, s), 4.24 (2H, t, J = 5.0 Hz), 4.83 (2H, t, J = 5.0 Hz), 7.32-7.38 (1H, m), 7.67-7.76 (2H, m), 7.92-7.95 (1H, m), 9.43 (1H, s).

Production Example 289: Synthesis of tert-butyl (2-(5-(3-bromo-4-(3-bromobenzoyl)-5-methyl-1H-pyrrol-2-yl)-2H-tetrazol-2-yl)ethyl)carbamate [compound No. 289]

Production Example 289-1: Synthesis of tert-butyl (2-(5-(3-bromo-4-(3-bromobenzoyl)-5-methyl-1H-pyrrol-2-yl)-2H-tetrazol-2-yl)ethyl)carbamate [compound No. 289]

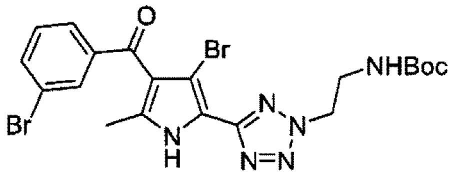

The compound of interest was obtained by the same procedures as in Production Example 97-1 using compound 286 and tert-butyl (2-bromoethyl)carbamate. Yield: 19.5 %, white solid.
$^1$H-NMR (400 MHz, $CDCl_3$) δ: 1.43 (9H, s), 2.44 (3H, s), 3.75-3.82 (2H, m), 4.77-4.83 (2H, m), 7.31-7.38 (1H, m), 7.66-7.77 (2H, m), 7.92-7.96 (1H, m), 9.39 (1H, s).

Production Example 290: Synthesis of (5-(2-(2-aminoethyl)-2H-tetrazol-5-yl)-4-bromo-2-methyl-1H-pyrrol-3-yl)(3-bromophenyl)methanone [compound No. 290]

Production Example 290-1: Synthesis of (5-(2-(2-aminoethyl)-2H-tetrazol-5-yl)-4-bromo-2-methyl-1H-pyrrol-3-yl)(3-bromophenyl)methanone [compound No. 290]

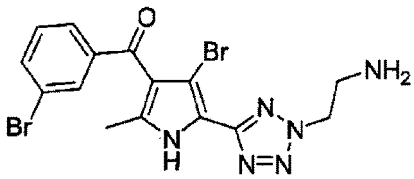

The compound of interest was obtained by the same procedures as in Production Example 48-1 using compound 289 as a starting material. Yield: 56.3 %, white solid. $^1$H-NMR (400 MHz, DMSO-$d_6$) δ: 2.28 (3H, s), 3.13 (2H, t, J = 5.9 Hz), 4.68 (2H, t, J = 6.1 Hz), 7.45-7.53 (1H, m), 7.65-7.72 (1H, m), 7.75-7.86 (2H, m).

Production Example 291: Synthesis of tert-butyl (2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate [compound No. 291]

Production Example 291-1: Synthesis of tert-butyl (2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate [compound No. 291]

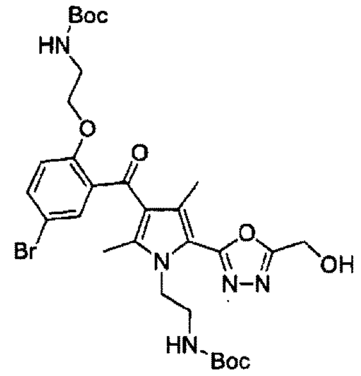

The title compound was obtained by the same procedures as in Production Example 50-1 using compound 203 as a starting material.
Yield: 13.4 %, yellow solid.
$^1$H-NMR (400 MHz, DMSO-$d_6$) δ: 1.32 (9H, s), 1.33 (9H, s), 2.10 (3H, s), 2.21 (3H, s), 3.08 (2H, q, J = 5.9 Hz), 3.19 (2H, dd, J = 12.3, 6.1 Hz), 3.95 (2H, t, J = 6.1 Hz), 4.32 (2H, t, J = 5.9 Hz), 4.69 (2H, d, J = 6.3 Hz), 5.89 (1H, t, J = 6.2 Hz), 6.68 (1H, t, J = 6.0 Hz), 6.98 (1H, t, J = 6.0 Hz), 7.13 (1H, d, J = 8.8 Hz), 7.36 (1H, d, J = 2.4 Hz), 7.63 (1H, dd, J = 8.8, 2.4 Hz).
ESI-MS m/z: 678[M+H]$^+$, 680[M+2+H]$^+$.

Production Example 292: Synthesis of ethyl 5-(2-(2-acetoxyethyl)-2H-tetrazol-5-yl)-3-(3-bromo-4-methoxybenzoyl)-4-methyl-1H-pyrrole-2-carboxylate [compound No. 292]

Production Example 292-1: Synthesis of ethyl 5-(2-(2-acetoxyethyl)-2H-tetrazol-5-yl)-3-(3-bromo-4-methoxybenzoyl)-4-methyl-1H-pyrrole-2-carboxylate [compound No. 292]

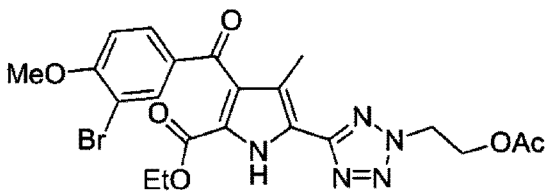

Compound 134 (600 mg, 1.22 mmol) was dissolved in chloroform (12 ml). To the solution, thionyl chloride (0.329 ml, 4.51 mmol) and N,N-dimethylformamide (one drop) were added, and the mixture was heated to reflux for 1 hour. After reaction, the reaction solution was poured into cold ammonia water (10 ml). The mixture was extracted with chloroform. The obtained organic layer was dried over anhydrous magnesium sulfate, and magnesium sulfate was filtered off. The solvent was distilled off under reduced pressure. The residue was purified by column chromatography to obtain the compound of interest (83 mg, yield: 13.4%, yellow solid).
$^1$H-NMR (400 MHz, DMSO-$d_6$) δ: 0.92 (3H, t, J = 7.1 Hz), 1.97 (3H, s), 2.19 (3H, s), 3.94 (3H, s), 3.99 (2H, q, J = 7.1 Hz), 4.59 (2H, t, J = 5.0 Hz), 5.05 (2H, t, J = 5.0 Hz), 7.22 (1H, d, J = 8.8 Hz), 7.73 (1H, dd, J = 8.7, 2.1 Hz), 7.93 (1H, d, J = 2.2 Hz), 12.93 (1H, br s).
ESI-MS m/z: 506[M+H]$^+$, 508[M+2+H]$^+$.

Production Example 293: Synthesis of ethyl 3-(3-bromo-4-methoxybenzoyl)-5-(2-(2-hydroxyethyl)-2H-tetrazol-5-yl)-4-methyl-1H-pyrrole-2-carboxylate [compound No. 293]

Production Example 293-1: Synthesis of ethyl 3-(3-bromo-4-methoxybenzoyl)-5-(2-(2-hydroxyethyl)-2H-tetrazol-5-yl)-4-methyl-1H-pyrrole-2-carboxylate [compound No. 293]

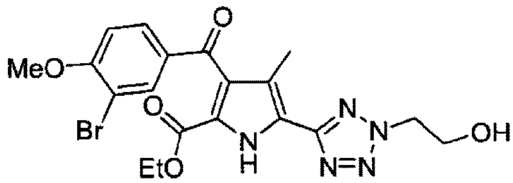

The compound of interest was obtained by the same procedures as in Production Example 117-1 using compound 292 as a starting material. Yield: 99.7 %, white solid.
$^1$H-NMR (400 MHz, DMSO-$d_6$) δ: 0.92 (3H, t, J = 7.1 Hz), 2.20 (3H, s), 3.94 (3H, s), 3.95-4.01 (4H, m), 4.79 (2H, t, J = 5.1 Hz), 5.11 (1H, t, J = 5.6 Hz), 7.72 (1H, dd, J = 8.7, 2.2 Hz), 7.92 (1H, d, J = 2.2 Hz), 12.94 (1H, br s).
ESI-MS m/z: 464[M+H]$^+$, 466[M+2+H]$^+$.

Production Example 294: Synthesis of (5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone) [compound No. 294]

Production Example 294-1: Synthesis of (5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone) [compound No. 294]

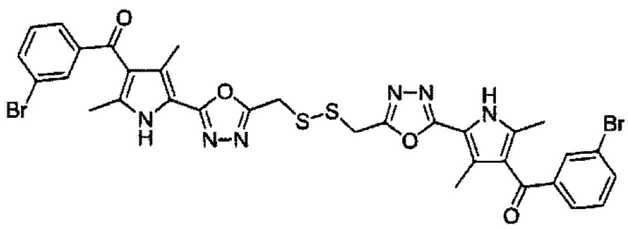

The compound of interest was obtained by the same procedures as in Production Example 117-1 using compound 212 as a starting material. Yield: 42.4 %, yellow solid.
$^1$H-NMR (400 MHz, DMSO-$d_6$) δ: 2.13 (3H, s), 2.22 (3H, s), 4.38 (2H, s), 7.47 (1H, t, J = 7.8 Hz), 7.61 (1H, dt, J = 7.8, 1.3 Hz), 7.75 (1H, t, J = 1.8 Hz), 7.81(1H, dq, J = 8.0, 1.0 Hz), 12.40 (1H, br s).
ESI-MS m/z: 780[M+H]$^+$, 782[M+2+H]$^+$.

Production Example 295: Synthesis of 2-(5-(4-(3-bromo-4-methoxybenzoyl)-3-methyl-5-(methylcarbamoyl)-1H-pyrrol-2-yl)-2H-tetrazol-2-yl)ethyl acetate [compound No. 295]

Production Example 295-1: Synthesis of 2-(5-(4-(3-bromo-4-methoxybenzoyl)-3-methyl-5-(methylcarbamoyl)-1H-pyrrol-2-yl)-2H-tetrazol-2-yl)ethyl acetate [compound No. 295]

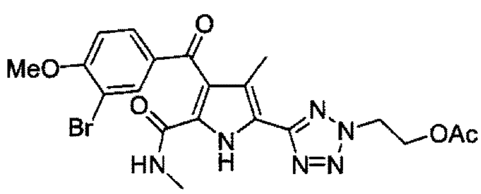

Compound 140 (65 mg, 0.13 mmol) was dissolved in N,N-dimethylformamide (1.3 ml). To the solution, 1-ethyl-3-(3-dimethylaminopropyl)carbodiimide hydrochloride (30 mg, 0.156 mmol) and 1-hydroxybenzotriazole (21 mg, 0.156 mmol) were added, and the mixture was stirred at room temperature for 30 minutes. Methylamine hydrochloride (11 mg, 0.156 mmol) and triethylamine (0.022 ml, 0.156 mmol) were added thereto, and the mixture was stirred overnight at room temperature. After reaction, the solvent was distilled off. The residue was dissolved in chloroform. The organic layer was washed with 1 M hydrochloric acid, a 1 M aqueous sodium hydroxide solution, and saturated saline. The organic layer was dried over magnesium sulfate, and the solvent was distilled off. The residue was purified by silica gel

column chromatography to obtain the title compound (54 mg, yield: 82.2%) as a yellow solid.
$^1$H-NMR (400 MHz, DMSO-$d_6$) δ: 1.97 (3H, s), 2.20 (3H, s), 2.61 (3H, d, J = 4.6 Hz), 3.93 (3H, s), 4.58 (2H, t, J = 5.1 Hz), 5.04 (2H, t, J = 5.1 Hz), 7.17 (1H, d, J = 8.8 Hz), 7.68 (1H, dd, J = 8.8, 2.1 Hz), 7.88 (1H, d, J = 2.1 Hz), 8.28 (1H, q, J = 4.6 Hz), 12.36 (1H, br s).
ESI-MS m/z: 505[M+H]$^+$, 507[M+2+H]$^+$.

Production Example 296: Synthesis of (3-(2-aminoethoxy)phenyl)(5-(2-ethyl-2H-tetrazol-5-yl)-2,4-dimethyl-1H-pyrrol-3-yl)methanone hydrochloride [compound No. 296]

Production Example 296-1: Synthesis of (3-(2-aminoethoxy)phenyl)(5-(2-ethyl-2H-tetrazol-5-yl)-2,4-dimethyl-1H-pyrrol-3-yl)methanone hydrochloride [compound No. 296]

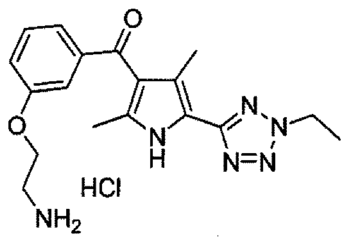

1 M hydrochloric acid (158 μl, 0.16 mmol) and purified water (10 ml) were added to compound 165 (51 mg, 0.144 mmol). The obtained aqueous solution was filtered. The filtrate was freeze-dried to obtain the compound of interest (47 mg, yield: 83.7%, white solid).
$^1$H-NMR (400 MHz, $D_2O$) δ: 1.44 (3H, t, J = 7.3 Hz), 1.98 (3H, s), 2.02 (3H, s), 3.24-3.32 (2H, m), 4.12-4.18 (2H, m), 4.55 (2H, q, J = 7.3 Hz), 7.07-7.18 (3H, m), 7.27-7.36 (1H, m).

Production Example 297: Synthesis of 2-(5-(4-(3-bromobenzoyl)-3,5-dimethyl-1H-pyrrol-2-yl)-2H-tetrazol-2-yl)ethyl 2-aminoacetate hydrochloride [compound No. 297]

Production Example 297-1: Synthesis of 2-(5-(4-(3-bromobenzoyl)-3,5-dimethyl-1H-pyrrol-2-yl)-2H-tetrazol-2-yl)ethyl 2-aminoacetate hydrochloride [compound No. 297]

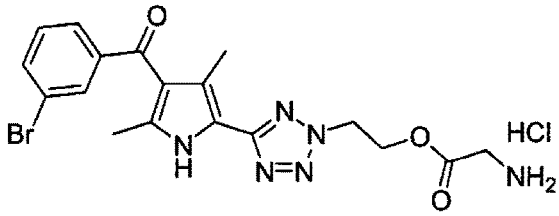

The compound of interest was obtained by the same procedures as in Production Example 296-1 using compound 152 as a starting material. Yield: quant., yellow solid.
$^1$H-NMR (400 MHz, DMSO-$d_6$) δ: 2.13 (3H, s), 2.26 (3H, s), 3.79 (2H, s), 4.75 (2H, t, J = 4.9 Hz), 5.05 (2H, t, J = 4.9 Hz), 7.45-7.51 (1H, m), 7.58-7.63 (1H, m), 7.71-7.75 (1H, m), 7.78-7.83 (1H, m), 8.19 (2H, s), 12.15 (1H, s).

Production Example 298: Synthesis of (3-bromophenyl)(2,4-dimethyl-5-(5-((4-methylpiperazin-1-yl)methyl)-1,3,4-oxadiazol-2-yl)-1H-pyrrol-3-yl)methanone hydrochloride [compound No. 298]

Production Example 298-1: Synthesis of (3-bromophenyl)(2,4-dimethyl-5-(5-((4-methylpiperazin-1-yl)methyl)-1,3,4-oxadiazol-2-yl)-1H-pyrrol-3-yl)methanone hydrochloride [compound No. 298]

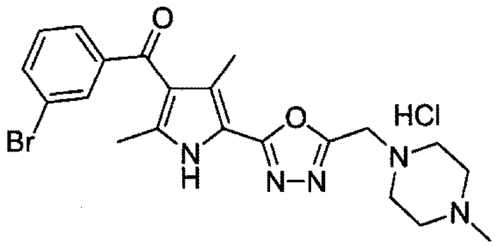

Compound 208 (50 mg, 0.11 mmol) was dissolved in methanol (5 ml). To the solution, a 4 M solution of hydrogen chloride in dioxane (0.083 ml) was added, and the mixture was stirred. The solvent was distilled off to obtain the title compound (49 mg, yield: 83.8%) as a yellow solid.
$^1$H-NMR (400 MHz, DMSO-$d_6$) δ: 2.14 (3H, s), 2.23 (3H, s), 2.68 (2H, t, J = 11.2 Hz), 2.75 (3H, d, J = 3.2 Hz), 3.01-3.08 (4H, m), 3.39 (2H, d, J = 12.2 Hz), 4.03 (2H, s), 7.48 (1H, t, J = 7.8 Hz), 7.62 (1H, d, J = 7.8 Hz), 7.74 (1H, t, J = 1.7 Hz), 7.82 (1H, dq, J = 7.8, 1.0 Hz), 10.41 (1H, br s), 12.42 (1H, s).
ESI-MS m/z: 458[M+H]$^+$, 460[M+2+H]$^+$.

Production Example 299: Synthesis of (3-bromophenyl)(5-(5-((dimethylamino)methyl)-1,3,4-oxadiazol-2-yl)-2,4-dimethyl-1H-pyrrol-3-yl)methanone hydrochloride [compound No. 299]

Production Example 299-1: Synthesis of (3-bromophenyl)(5-(5-((dimethylamino)methyl)-1,3,4-oxadiazol-2-yl)-2,4-dimethyl-1H-pyrrol-3-yl)methanone hydrochloride [compound No. 299]

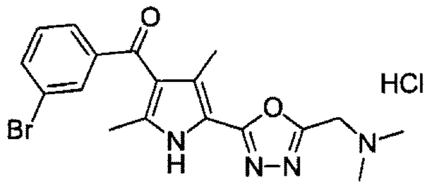

The compound of interest was obtained by the same procedures as in Production Example 298-1 using compound 209 as a starting material. Yield: 86.8 %, yellow solid. $^1$H-NMR (400 MHz, DMSO-$d_6$) δ: 2.16 (3H, s), 2.25 (3H, s), 2.91 (6H, s), 4.74 (2H, s), 7.49 (1H, t, J = 7.8 Hz), 7.62 (1H, dt, J = 7.8, 1.0 Hz), 7.75 (1H, t, J =1.8 Hz), 7.83 (1H, dq, J = 8.1, 1.0 Hz), 11.08 (1H, br s), 12.49 (1H, s).

Production Example 300: Synthesis of (2-(2-aminoethoxy)-5-bromophenyl)(5-(5-cyclopropyl-1,3,4-oxadiazol-2-yl)-2,4-dimethyl-1H-pyrrol-3-yl)methanone hydrochloride [compound No. 300]

Production Example 300-1: Synthesis of (2-(2-aminoethoxy)-5-bromophenyl)(5-(5-cyclopropyl-1,3,4-oxadiazol-2-yl)-2,4-dimethyl-1H-pyrrol-3-yl)methanone hydrochloride [compound No. 300]

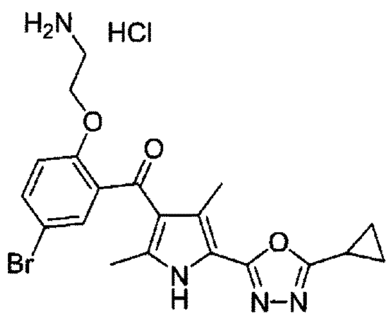

The compound of interest was obtained by the same procedures as in Production Example 298-1 using compound 244 as a starting material. Yield: 44.9 %, white solid.

$^1$H-NMR (400 MHz, DMSO-$d_6$) δ: 1.05-1.09 (2H, m), 1.14-1.18 (2H, m), 2.09 (3H, s), 2.22 (3H, s), 2.24-2.30 (1H, m), 3.02 (2H, dd, J = 11.1, 5.6 Hz), 4.18 (2H, t, J = 5.6 Hz), 7.20 (1H, d, J = 8.8 Hz), 7.41 (1H, d, J = 2.4 Hz), 7.67 (1H, dd, J = 8.8, 2.4 Hz), 7.87 (3H, br s), 12.23 (1H, br s).
ESI-MS m/z: 445[M+H]$^+$, 447[M+2+H]$^+$.

Production Example 301: Synthesis of (2-(2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone hydrochloride [compound No. 301]

Production Example 301-1: Synthesis of (2-(2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone hydrochloride [compound No. 301]

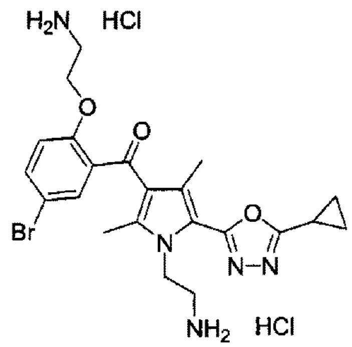

Compound 245 (31 mg, 0.063 mmol) was dissolved in methanol (1 ml). To the solution, 1 M hydrochloric acid (0.126 ml) was added, and the mixture was stirred. The solvent was distilled off to obtain the title compound. Yield: 96.1%, yellow solid.
$^1$H-NMR (400 MHz, DMSO-$d_6$) δ: 1.05-1.09 (2H, m), 1.16-1.21 (2H, m), 2.06 (3H, s), 2.26 (3H, s), 2.30-2.36 (1H, m), 2.97-3.02 (2H, m), 3.08-3.14 (2H, m), 4.20 (2H, t, J = 5.5 Hz), 4.50 (2H, t, J = 7.4 Hz), 7.22 (1H, d, J = 8.8 Hz), 7.44 (1H,d, J = 2.7 Hz), 7.71 (1H, dd, J = 8.8, 2.7 Hz), 8.03 (3H, br s), 8.26 (3H, br s).

Production Example 302: Synthesis of (4-(2-aminoethoxy)-3-bromophenyl)(5-(2-ethyl-2H-tetrazol-5-yl)-2,4-dimethyl-1H-pyrrol-3-yl)methanone hydrochloride [compound No. 302]

Production Example 302-1: Synthesis of (4-(2-aminoethoxy)-3-bromophenyl)(5-(2-ethyl-2H-tetrazol-5-yl)-2,4-dimethyl-1H-pyrrol-3-yl)methanone hydrochloride [compound No. 302]

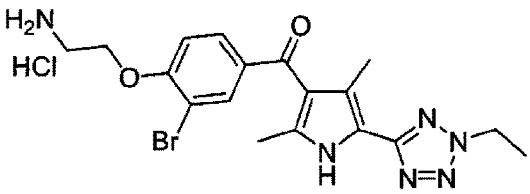

The compound of interest was obtained by the same procedures as in Production Example 296-1 using compound 167 as a starting material. Yield: quant., white solid. $^1$H-NMR (400 MHz, DMSO-$d_6$) δ: 1.57 (3H, t, J = 7.1 Hz), 2.16 (3H, s), 2.24 (3H, s), 4.36 (1H, s), 4.74 (2H, q, J = 7.1 Hz), 7.22-7.31 (1H, m), 7.65-7.72 (1H, m), 7.83-7.87 (1H, m), 8.01 (2H, s), 12.07 (1H, s).

Production Example 303: Synthesis of 2-(5-(4-(3-bromobenzoyl)-3-methyl-5-(methylcarbamoyl)-1H-pyrrol-2-yl)-2H-tetrazol-2-yl)ethyl dihydrogen phosphate [compound No. 303]

Production Example 303-1: Synthesis of 2-(5-(4-(3-bromobenzoyl)-3-methyl-5-(methylcarbamoyl)-1H-pyrrol-2-yl)-2H-tetrazol-2-yl)ethyl dihydrogen phosphate [compound No. 303]

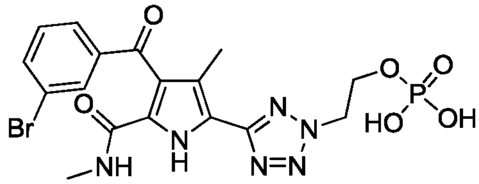

To a solution of phosphorus oxychloride (45 μl, 0.45 mmol) in dichloromethane (2 ml), tert-butanol (36 mg, 0.45 mmol) and triethylamine (63 μl, 0.45 mmol) were added with stirring under ice cooling in the argon atmosphere. After 30 minutes, compound 142 (62 mg, 0.15 mmol) and triethylamine (23 μl, 0.17 mmol) were added to the mixture. The reaction temperature was increased to room temperature. After 1 hour, the solvent was distilled off under reduced pressure. A 1 M aqueous sodium hydroxide solution was added to the residue. The aqueous layer was washed with chloroform. Then, the aqueous layer was rendered acidic by the addition of hydrochloric acid, followed by extraction with chloroform to obtain the compound of interest (41 mg, yield: 53.3%, white solid).
$^1$H-NMR (400 MHz, DMSO-$d_6$) δ: 2.25 (3H, s), 2.56 (3H, d, J = 3.7 Hz), 4.34-4.42 (2H, m), 4.94-5.02 (2H, m), 7.39-7.46 (1H, m), 7.62-7.67 (1H, m), 7.75-7.83 (2H, m), 8.32 (1H, s), 12.44 (1H, s).

Production Example 304: Synthesis of sodium 2-(5-(4-(3-bromobenzoyl)-3-methyl-5-(methylcarbamoyl)-1H-pyrrol-2-yl)-2H-tetrazol-2-yl)ethyl phosphate [compound No. 304]

Production Example 304-1: Synthesis of sodium 2-(5-(4-(3-bromobenzoyl)-3-methyl-5-(methylcarbamoyl)-1H-pyrrol-2-yl)-2H-tetrazol-2-yl)ethyl phosphate [compound No. 304]

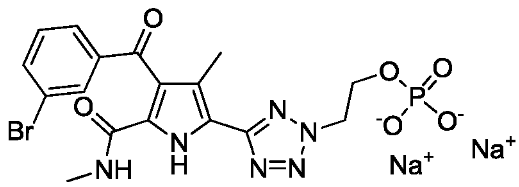

A 1 M aqueous sodium hydroxide solution (180 μl, 0.18 mmol) was added to compound 303 (41 mg, 0.08 mmol) to prepare a solution. Then, acetonitrile was added thereto to precipitate a solid. The precipitated solid was collected by filtration, washed with acetonitrile, and then dried to obtain the compound of interest (60 mg, yield: quant., yellow solid).
$^1$H-NMR (400 MHz, $D_2O$) δ: 2.28 (3H, s), 2.45 (3H, s), 4.14-4.22 (2H, m), 4.81-4.87 (2H, m), 7.28-7.34 (1H, m), 7.57-7.62 (1H, m), 7.66-7.71 (1H, m), 7.75-7.79 (1H, m).

## Claims

**1.** A compound represented by the general formula (1) or a salt thereof:

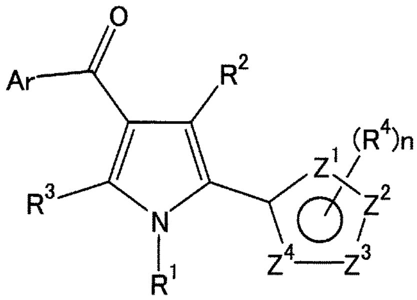

(1)

wherein Ar represents an optionally substituted aryl group or an optionally substituted heteroaryl group;
$Z^1$, $Z^2$, $Z^3$, and $Z^4$ each independently represent C, CH, a nitrogen atom, an oxygen atom, or a sulfur atom;
$R^1$ represents a hydrogen atom, a halogen atom, an optionally substituted alkyl group, an optionally substituted cycloalkyl group, an optionally substituted alkylcarbonyl group, an optionally substituted alkylsulfonyl group, an optionally substituted cycloalkylsulfonyl group, an optionally substituted aryl group, an optionally substituted aralkyl group, or an optionally substituted arylcarbonyl group;
$R^2$ and $R^3$ are the same or different and each represent a hydrogen atom, a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, an optionally substituted arylcarbonyl group, or an optionally substituted dithianyl group;
n units of $R^4$ are the same or different and each represent a hydrogen atom, an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group; and
n represents a number of 0 to 4.

2. The compound according to claim 1 or a salt thereof, wherein in the general formula (1), $R^2$ is a halogen atom, a formyl group, or an optionally substituted alkyl group having 1 to 8 carbon atoms.

3. The compound according to claim 1 or 2 or a salt thereof, wherein in the general formula (1), at least one of $Z^1$, $Z^2$, $Z^3$, and $Z^4$ is a nitrogen atom.

4. The compound according to any one of claims 1 to 3 or a salt thereof, wherein in the general formula (1), the ring constituted by $Z^1$, $Z^2$, $Z^3$, and $Z^4$ has a structure represented by any of the following formulas (2a) to (2e) :

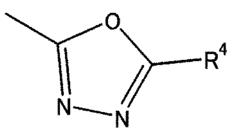

(2a)

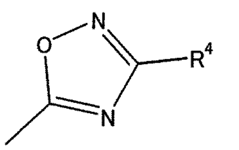

(2b)

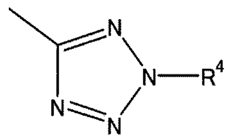

(2c)

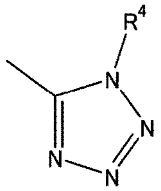

(2d)

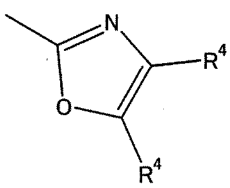

(2e)

wherein $R^4$ is as defined above.

5. The compound according to any one of claims 1 to 4 or a salt thereof, wherein Ar is an optionally substituted aryl group having 6 to 14 carbon atoms or an optionally substituted 5- to 10-membered heteroaryl group.

6. The compound according to any one of claims 1 to 5 or a salt thereof, wherein Ar is an optionally substituted aryl group having 6 to 14 carbon atoms.

7. A compound selected from the following or a salt thereof:

(3-bromophenyl)(2,4-dimethyl-5-(1H-tetrazol-5-yl)-1H-pyrrol-3-yl)methanone
(3-bromophenyl)(5-(2-ethyl-2H-tetrazol-5-yl)-2,4-dimethyl-1H-pyrrol-3-yl)methanone
(3-bromophenyl)(5-(1-ethyl-1H-tetrazol-5-yl)-2,4-dimethyl-1H-pyrrol-3-yl)methanone
(3-bromophenyl)(2,4-dimethyl-5-(2-(2,2,2-trifluoroethyl)-2H-tetrazol-5-yl)-1H-pyrrol-3-yl)methanone
(3-bromophenyl)(2,4-dimethyl-5-(1-(2,2,2-trifluoroethyl)-1H-tetrazol-5-yl)-1H-pyrrol-3-yl)methanone
(3-bromophenyl)(5-(2-(2-(dimethylamino)ethyl)-2H-tetrazol-5-yl)-2,4-dimethyl-1H-pyrrol-3-yl)methanone
(3-bromophenyl)(5-(2-(2-hydroxyethyl)-2H-tetrazol-5-yl)-2,4-dimethyl-1H-pyrrol-3-yl)methanone
(5-((2-(2-aminoethyl)-2H-tetrazol-5-yl)-2,4-dimethyl-1H-pyrrol-3-yl)(3-bromophenyl)methanone
2-((5-(4-(3-bromobenzoyl)-3,5-dimethyl-1H-pyrrol-2-yl)-2H-tetrazol-2-yl)ethyl acetate
(2,4-dimethyl-5-(1H-tetrazol-5-yl)-1H-pyrrol-3-yl)(3-methoxyphenyl)methanone
(3-methoxyphenyl)(5-(2-ethyl-2H-tetrazol-5-yl)-2,4-dimethyl-1H-pyrrol-3-yl)methanone
(5-((2-ethyl-2H-tetrazol-5-yl)-2,4-dimethyl-1H-pyrrol-3-yl)(3-methoxyphenyl)methanone
(3-bromo-4-methoxyphenyl)(2,4-dimethyl-5-(1H-tetrazol-5-yl)-1H-pyrrol-3-yl)methanone
(3-bromo-4-methoxyphenyl)(5-(2-ethyl-2H-tetrazol-5-yl)-2,4-dimethyl-1H-pyrrol-3-yl)methanone
(3-bromo-4-methoxyphenyl)(5-(2-(2-hydroxyethyl)-2H-tetrazol-5-yl)-2,4-dimethyl-1H-pyrrol-3-yl)methanone
2-((5-(4-(3-bromo-4-methoxybenzoyl)-3,5-dimethyl-1H-pyrrol-2-yl)-2H-tetrazol-2-yl)ethyl acetate
(3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone
(3-cyclopropylphenyl)(2,4-dimethyl-5-(1H-tetrazol-5-yl)-1H-pyrrol-3-yl)methanone
2-((5-(4-(3-cyclopropylbenzoyl)-3,5-dimethyl-1H-pyrrol-2-yl)ethyl acetate
(3-cyclopropylphenyl)(5-(2-(2-hydroxyethyl)-2H-tetrazol-5-yl)-2,4-dimethyl-1H-pyrrol-3-yl)methanone
2-((5-(4-(3-bromobenzoyl)-5-formyl-3-methyl-1H-pyrrol-2-yl)-2H-tetrazol-2-yl)ethyl acetate
3-((3-bromobenzoyl)-5-(2-(2-hydroxyethyl)-2H-tetrazol-5-yl)-4-methyl-1H-pyrrole-2-carboaldehyde
2-((5-(4-(3-bromobenzoyl)-5-(difluoromethyl)-3-methyl-1H-pyrrol-2-yl)-2H-tetrazol-2-yl)ethyl acetate
(3-bromophenyl)(2-(difluoromethyl)-5-(2-(2-hydroxyethyl)-2H-tetrazol-5-yl)-4-methyl-1H-pyrrol-3-yl)methanone
2-((5-(4-(3-bromobenzoyl)-5-(hydroxymethyl)-3-methyl-1H-pyrrol-2-yl)-2H-tetrazol-2-yl)ethyl acetate
(3-bromophenyl)(5-(2-(2-hydroxyethyl)-2H-tetrazol-5-yl)-2-(hydroxymethyl)-4-methyl-1H-pyrrol-3-yl)methanone
2-((5-(4-(3-bromobenzoyl)-5-(1-hydroxyethyl)-3-methyl-1H-pyrrol-2-yl)-2H-tetrazol-2-yl)ethyl acetate
(3-bromophenyl)(2-(1-hydroxyethyl)-5-(2-(2-hydroxyethyl)-2H-tetrazol-5-yl)-4-methyl-1H-pyrrol-3-yl)methanone
2-((5-(4-(3-bromo-4-methoxybenzoyl)-5-formyl-3-methyl-1H-pyrrol-2-yl)-2H-tetrazol-2-yl)ethyl acetate
3-((3-bromo-4-methoxybenzoyl)-5-(2-(2-hydroxyethyl)-2H-tetrazol-5-yl)-4-methyl-1H-pyrrole-2-carboaldehyde
2-((5-(4-(3-bromo-4-methoxybenzoyl)-5-(difluoromethyl)-3-methyl-1H-pyrrol-2-yl)-2H-tetrazol-2-yl)ethyl acetate
(3-bromo-4-methoxyphenyl)(2-(difluoromethyl)-5-(2-(2-hydroxyethyl)-2H-tetrazol-5-yl)-4-methyl-1H-pyrrol-3-yl)methanone
2-((5-(4-(3-bromo-4-methoxybenzoyl)-5-(hydroxymethyl)-3-methyl-1H-pyrrol-2-yl)-2H-tetrazol-2-yl)ethyl acetate
(3-bromo-4-methoxyphenyl)(5-(2-(2-hydroxyethyl)-2H-tetrazol-5-yl)-2-(hydroxymethyl)-4-methyl-1H-pyrrol-3-yl)methanone
2-((5-(4-(3-bromo-4-methoxybenzoyl)-5-(1-hydroxyethyl)-3-methyl-1H-pyrrol-2-yl)-2H-tetrazol-2-yl)ethyl acetate
(3-bromo-4-methoxyphenyl)(2-(1-hydroxyethyl)-5-(2-(2-hydroxyethyl)-2H-tetrazol-5-yl)-4-methyl-1H-pyrrol-3-yl)methanone
2-((5-(4-(3-bromobenzoyl)-5-cyano-3-methyl-1H-pyrrol-2-yl)-2H-tetrazol-2-yl)ethyl acetate
3-((3-bromobenzoyl)-5-(2-(2-hydroxyethyl)-2H-tetrazol-5-yl)-4-methyl-1H-pyrrole-2-carbonitrile

5-((2-(2-acetoxyethyl)-2H-tetrazol-5-yl)-3-(3-bromobenzoyl)-4-methyl-1H-pyrrole-2-carboxylic acid
3-((3-bromobenzoyl)-5-(2-(2-hydroxyethyl)-2H-tetrazol-5-yl)-4-methyl-1H-pyrrole-2-carboxylic acid
methyl 5-(2-(2-acetoxyethyl)-2H-tetrazol-5-yl)-3-(3-bromobenzoyl)-4-methyl-1H-pyrrole-2-carboxylate
methyl 3-(3-bromobenzoyl)-5-(2-(2-hydroxyethyl)-2H-tetrazol-5-yl)-4-methyl-1H-pyrrole-2-carboxylate
2-((5-(4-(3-bromo-4-methoxybenzoyl)-5-cyano-3-methyl-1H-pyrrol-2-yl)-2H-tetrazol-2-yl)ethyl acetate
3-((3-bromo-4-methoxybenzoyl)-5-(2-(2-hydroxyethyl)-2H-tetrazol-5-yl)-4-methyl-1H-pyrrole-2-carbonitrile
5-((2-(2-acetoxyethyl)-2H-tetrazol-5-yl)-3-(3-bromo-4-methoxybenzoyl)-4-methyl-1H-pyrrole-2-carboxylic acid
3-((3-bromo-4-methoxybenzoyl)-5-(2-(2-hydroxyethyl)-2H-tetrazol-5-yl)-4-methyl-1H-pyrrole-2-carboxylic acid
3-((3-bromobenzoyl)-5-(2-(2-hydroxyethyl)-2H-tetrazol-5-yl)-N,4-dimethyl-1H-pyrrole-2-carboxamide
3-((3-bromobenzoyl)-5-(2-(2-hydroxyethyl)-2H-tetrazol-5-yl)-N,N,4-trimethyl-1H-pyrrole-2-carboxamide
N-(2-aminoethyl)-3-(3-bromobenzoyl)-5-(2-(2-hydroxyethyl)-2H-tetrazol-5-yl)-4-methyl-1H-pyrrole-2-carboxamide
3-((3-bromobenzoyl)-N-ethyl-5-(2-(2-hydroxyethyl)-2H-tetrazol-5-yl)-4-methyl-1H-pyrrole-2-carboxamide
3-((3-bromobenzoyl)-5-(2-(2-hydroxyethyl)-2H-tetrazol-5-yl)-N-isopropyl-4-methyl-1H-pyrrole-2-carboxamide
(3-((3-bromobenzoyl)-5-(2-(2-hydroxyethyl)-2H-tetrazol-5-yl)-4-methyl-1H-pyrrol-2-yl)(4-methylpiperazin-1-yl)methanone
(3-((3-bromobenzoyl)-5-(2-(2-hydroxyethyl)-2H-tetrazol-5-yl)-4-methyl-1H-pyrrol-2-yl)(piperazin-1-yl)methanone
3-((3-bromo-4-methoxybenzoyl)-5-(2-(2-hydroxyethyl)-2H-tetrazol-5-yl)-N,4-dimethyl-1H-pyrrole-2-carboxamide
3-((3-bromo-4-methoxybenzoyl)-5-(2-(2-hydroxyethyl)-2H-tetrazol-5-yl)-4-methyl-1H-pyrrole-2-carboxamide
2-((5-(4-(3-bromobenzoyl)-3,5-dimethyl-1H-pyrrol-2-yl)-2H-tetrazol-2-yl)ethyl 2-((tert-butoxycarbonyl)amino)acetate
2-((5-(4-(3-bromobenzoyl)-3,5-dimethyl-1H-pyrrol-2-yl)-2H-tetrazol-2-yl)ethyl 2-aminoacetate
2-((5-(4-(3-bromobenzoyl)-5-formyl-3-methyl-1H-pyrrol-2-yl)-2H-tetrazol-2-yl)ethyl 2-((tert-butoxycarbonyl)amino)acetate
2-((5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate
2-((5-(4-(3-bromobenzoyl)-5-(hydroxymethyl)-3-methyl-1H-pyrrol-2-yl)-2H-tetrazol-2-yl)ethyl 2-aminoacetate
methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate
methyl 5-(2-(2-(2-aminoacetoxy)ethyl)-2H-tetrazol-5-yl)-3-(3-bromobenzoyl)-4-methyl-1H-pyrrole-2-carboxylate
2-((5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate
2-((5-(4-(3-bromobenzoyl)-3-methyl-5-(methylcarbamoyl)-1H-pyrrol-2-yl)-2H-tetrazol-2-yl)ethyl 2-aminoacetate
2-((5-(3,5-dimethyl-4-(3-vinylbenzoyl)-1H-pyrrol-2-yl)-2H-tetrazol-2-yl)ethyl acetate
2-((5-(4-(3-formylbenzoyl)-3,5-dimethyl-1H-pyrrol-2-yl)-2H-tetrazol-2-yl)ethyl acetate
2-((5-(4-(3-(hydroxymethyl)benzoyl)-3,5-dimethyl-1H-pyrrol-2-yl)-2H-tetrazol-2-yl)ethyl acetate
(5-((2-(2-hydroxyethyl)-2H-tetrazol-5-yl)-2,4-dimethyl-1H-pyrrol-3-yl)(3-(hydroxymethyl)phenyl)methanone
(5-((2-ethyl-2H-tetrazol-5-yl)-2,4-dimethyl-1H-pyrrol-3-yl)(3-hydroxyphenyl)methanone
(3-((2-aminoethoxy)phenyl)(5-(2-ethyl-2H-tetrazol-5-yl)-2,4-dimethyl-1H-pyrrol-3-yl)methanone
(3-bromo-4-hydroxyphenyl)(5-(2-ethyl-2H-tetrazol-5-yl)-2,4-dimethyl-1H-pyrrol-3-yl)methanone
(4-((2-aminoethoxy)-3-bromophenyl)(5-(2-ethyl-2H-tetrazol-5-yl)-2,4-dimethyl-1H-pyrrol-3-yl)methanone
(4-((2-aminoethoxy)-3-bromophenyl)(5-(2-(2-hydroxyethyl)-2H-tetrazol-5-yl)-2,4-dimethyl-1H-pyrrol-3-yl)methanone
(E)-2-(5-(4-(3-bromo-4-methoxybenzoyl)-5-((hydroxyimino)methyl)-3-methyl-1H-pyrrol-2-yl)-2H-tetrazol-2-yl)ethyl acetate
(Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate
2-((5-(4-(3-bromobenzoyl)-5-(chloromethyl)-3-methyl-1H-pyrrol-2-yl)-2H-tetrazol-2-yl)ethyl acetate
2-((5-(4-(3-bromo-4-methoxybenzoyl)-5-(chloromethyl)-3-methyl-1H-pyrrol-2-yl)-2H-tetrazol-2-yl)ethyl acetate
(3-bromophenyl)(5-(5-ethyl-1,3,4-oxadiazol-2-yl)-2,4-dimethyl-1H-pyrrol-3-yl)methanone
(3-bromophenyl)(5-(5-(2-chloroethyl)-1,3,4-oxadiazol-2-yl)-2,4-dimethyl-1H-pyrrol-3-yl)methanone
(3-bromophenyl)(2,4-dimethyl-5-(5-phenyl-1,3,4-oxadiazol-2-yl)-1H-pyrrol-3-yl)methanone
(5-((5-benzyl-1,3,4-oxadiazol-2-yl)-2,4-dimethyl-1H-pyrrol-3-yl)(3-bromophenyl)methanone
(3-bromophenyl)(5-(5-cyclopropyl-1,3,4-oxadiazol-2-yl)-2,4-dimethyl-1H-pyrrol-3-yl)methanone

(3-bromophenyl)(5-(5-isopropyl-1,3,4-oxadiazol-2-yl)-2,4-dimethyl-1H-pyrrol-3-yl)methanone
(3-bromophenyl)(5-(5-cyclobutyl-1,3,4-oxadiazol-2-yl)-2,4-dimethyl-1H-pyrrol-3-yl)methanone
(3-bromophenyl)(5-(5-cyclopentyl-1,3,4-oxadiazol-2-yl)-2,4-dimethyl-1H-pyrrol-3-yl)methanone
(3-bromophenyl)(5-(5-cyclohexyl-1,3,4-oxadiazol-2-yl)-2,4-dimethyl-1H-pyrrol-3-yl)methanone
methyl 3-(5-(4-(3-bromobenzoyl)-3,5-dimethyl-1H-pyrrol-2-yl)-1,3,4-oxadiazol-2-yl)propionate
ethyl 2-(5-(4-(3-bromobenzoyl)-3,5-dimethyl-1H-pyrrol-2-yl)-1,3,4-oxadiazol-2-yl)acetate
(3-bromophenyl)(5-(5-tert-butyl)-1,3,4-oxadiazol-2-yl)-2,4-dimethyl-1H-pyrrol-3-yl)methanone
(3-bromophenyl)(2,4-dimethyl-5-(5-methyl-1,3,4-oxadiazol-2-yl)-1H-pyrrol-3-yl)methanone
(3-bromophenyl)(5-(5-(difluoromethyl)-1,3,4-oxadiazol-2-yl)-2,4-dimethyl-1H-pyrrol-3-yl)methanone
(3-bromophenyl)(2,4-dimethyl-5-(5-(2,2,2-trifluoroethyl)-1,3,4-oxadiazol-2-yl)-1H-pyrrol-3-yl)methanone
(3-bromophenyl)(5-(5-(1-hydroxycyclopropyl)-1,3,4-oxadiazol-2-yl)-2,4-dimethyl-1H-pyrrol-3-yl)methanone
(3-bromophenyl)(2,4-dimethyl-5-(5-(1-methylcyclopropyl)-1,3,4-oxadiazol-2-yl)-1H-pyrrol-3-yl)methanone
(3-bromophenyl)(2,4-dimethyl-5-(5-(2-methylcyclopropyl)-1,3,4-oxadiazol-2-yl)-1H-pyrrol-3-yl)methanone
(3-bromophenyl)(5-(5-(2,2-dimethylcyclopropyl)-1,3,4-oxadiazol-2-yl)-2,4-dimethyl-1H-pyrrol-3-yl)methanone
(3-bromophenyl)(2,4-dimethyl-5-(5-(2,2,3,3-tetramethylcyclopropyl)-1,3,4-oxadiazol-2-yl)-1H-pyrrol-3-yl)methanone
(5-((5-ethyl-1,3,4-oxadiazol-2-yl)-2,4-dimethyl-1H-pyrrol-3-yl)(3-methoxyphenyl)methanone
(5-((5-cyclopropyl-1,3,4-oxadiazol-2-yl)-2,4-dimethyl-1H-pyrrol-3-yl)(3-methoxyphenyl)methanone
(5-((5-ethyl-1,3,4-oxadiazol-2-yl)-2,4-dimethyl-1H-pyrrol-3-yl)(3-nitrophenyl)methanone
(5-((5-cyclopropyl-1,3,4-oxadiazol-2-yl)-2,4-dimethyl-1H-pyrrol-3-yl)(3-nitrophenyl)methanone
(3-bromo-4-methoxyphenyl)(5-(5-ethyl-1,3,4-oxadiazol-2-yl)-2,4-dimethyl-1H-pyrrol-3-yl)methanone
(3-bromo-4-methoxyphenyl)(5-(5-cyclopropyl-1,3,4-oxadiazol-2-yl)-2,4-dimethyl-1H-pyrrol-3-yl)methanone
(5-((5-cyclopropyl-1,3,4-oxadiazol-2-yl)-2,4-dimethyl-1H-pyrrol-3-yl)(3-(hydroxymethyl)phenyl)methanone
(3-bromophenyl)(5-(5-cyclopropyl-1,3,4-oxadiazol-2-yl)-2-(1,3-dithian-2-yl)-4-methyl-1H-pyrrol-3-yl)methanone
(5-bromo-2-methoxyphenyl)(5-(5-(chloromethyl)-1,3,4-oxadiazol-2-yl)-2,4-dimethyl-1H-pyrrol-3-yl)methanone
(5-((4-(5-bromo-2-methoxybenzoyl)-3,5-dimethyl-1H-pyrrol-2-yl)-1,3,4-oxadiazol-2-yl)methyl acetate
(5-bromo-2-hydroxyphenyl)(5-(5-(hydroxymethyl)-1,3,4-oxadiazol-2-yl)-2,4-dimethyl-1H-pyrrol-3-yl)methanone
2-((5-(4-(3-bromobenzoyl)-3,5-dimethyl-1H-pyrrol-2-yl)-1,3,4-oxadiazol-2-yl)ethyl acetate
(3-bromophenyl)(5-(5-(2-hydroxyethyl)-1,3,4-oxadiazol-2-yl)-2,4-dimethyl-1H-pyrrol-3-yl)methanone
(3-bromophenyl)(2,4-dimethyl-5-(5-vinyl-1,3,4-oxadiazol-2-yl)-1H-pyrrol-3-yl)methanone
(3-bromophenyl)(5-(5-(chloromethyl)-1,3,4-oxadiazol-2-yl)-2,4-dimethyl-1H-pyrrol-3-yl)methanone
(3-bromophenyl)(2,4-dimethyl-5-(5-((4-methylpiperazin-1-yl)methyl)-1,3,4-oxadiazol-2-yl)-1H-pyrrol-3-yl)methanone
(3-bromophenyl)(5-(5-((dimethylamino)methyl)-1,3,4-oxadiazol-2-yl)-2,4-dimethyl-1H-pyrrol-3-yl)methanone
(5-((4-(3-bromobenzoyl)-3,5-dimethyl-1H-pyrrol-2-yl)-1,3,4-oxadiazol-2-yl)methyl acetate
(3-bromophenyl)(5-(5-(hydroxymethyl)-1,3,4-oxadiazol-2-yl)-2,4-dimethyl-1H-pyrrol-3-yl)methanone
S-((5-(4-(3-bromobenzoyl)-3,5-dimethyl-1H-pyrrol-2-yl)-1,3,4-oxadiazol-2-yl)methyl)ethanethioate
2-(((5-(4-(3-bromobenzoyl)-3,5-dimethyl-1H-pyrrol-2-yl)-1,3,4-oxadiazol-2-yl)methyl)isoindole-1,3-dione
(5-((5-(aminomethyl)-1,3,4-oxadiazol-2-yl)-2,4-dimethyl-1H-pyrrol-3-yl)(3-bromophenyl)methanone
N-((5-(4-(3-bromobenzoyl)-3,5-dimethyl-1H-pyrrol-2-yl)-1,3,4-oxadiazol-2-yl)methyl)acetamide
methyl((5-(4-(3-bromobenzoyl)-3,5-dimethyl-1H-pyrrol-2-yl)-1,3,4-oxadiazol-2-yl)methyl)carbamate
N-((5-(4-(3-bromobenzoyl)-3,5-dimethyl-1H-pyrrol-2-yl)-1,3,4-oxadiazol-2-yl)methyl)methanesulfonamide
1-(((5-(4-(3-bromobenzoyl)-3,5-dimethyl-1H-pyrrol-2-yl)-1,3,4-oxadiazol-2-yl)methyl)-3-ethylurea
1-(((5-(4-(3-bromobenzoyl)-3,5-dimethyl-1H-pyrrol-2-yl)-1,3,4-oxadiazol-2-yl)methyl)-3-ethylthiourea
3-(((5-(4-(3-bromobenzoyl)-3,5-dimethyl-1H-pyrrol-2-yl)-1,3,4-oxadiazol-2-yl)methyl)-1,1-dimethylthiourea
2-((((5-(4-(3-bromobenzoyl)-3,5-dimethyl-1H-pyrrol-2-yl)-1,3,4-oxadiazol-2-yl)methyl)amino)-2-oxoethyl acetate
N-((5-(4-(3-bromobenzoyl)-3,5-dimethyl-1H-pyrrol-2-yl)-1,3,4-oxadiazol-2-yl)methyl)-2-hydroxyacetamide
(3-bromophenyl)(5-(5-ethyl-1,3,4-oxadiazol-2-yl)-1,2,4-trimethyl-1H-pyrrol-3-yl)methanone
1-((3-(3-bromobenzoyl)-5-(5-ethyl-1,3,4-oxadiazol-2-yl)-2,4-dimethyl-1H-pyrrol-1-yl)ethanone
(3-bromophenyl)(5-(5-ethyl-1,3,4-oxadiazol-2-yl)-2,4-dimethyl-1-(methylsulfonyl)-1H-pyrrol-3-yl)methanone
2-((3-(3-bromobenzoyl)-5-(5-ethyl-1,3,4-oxadiazol-2-yl)-2,4-dimethyl-1H-pyrrol-1-yl)-2-oxoethyl acetate
(3-bromophenyl)(1-(cyclopropylsulfonyl)-5-(5-ethyl-1,3,4-oxadiazol-2-yl)-2,4-dimethyl-1H-pyrrol-3-yl)methanone
(3-bromophenyl)(1-(difluoromethyl)-5-(5-ethyl-1,3,4-oxadiazol-2-yl)-2,4-dimethyl-1H-pyrrol-3-yl)methanone
(3-bromophenyl)(5-(5-ethyl-1,3,4-oxadiazol-2-yl)-2,4-dimethyl-1-(2,2,2-trifluoroethyl)-1H-pyrrol-3-yl)methanone
(3-bromophenyl)(1-cyclopropyl-5-(5-ethyl-1,3,4-oxadiazol-2-yl)-2,4-dimethyl-1H-pyrrol-3-yl)methanone
tert-butyl(2-(3-(3-bromobenzoyl)-5-(5-ethyl-1,3,4-oxadiazol-2-yl)-2,4-dimethyl-1H-pyrrol-1-yl)ethyl)carbamate
(3-bromophenyl)(1-ethyl-2,4-dimethyl-5-(5-ethyl-1,3,4-oxadiazol-2yl)-1H-pyrrol-3-yl)methanone
(3-bromophenyl)(1-isopropyl-2,4-dimethyl-5-(5-ethyl-1,3,4-oxadiazol-2-yl)-1H-pyrrol-3-yl)methanone
(1-((2-aminoethyl)-5-(5-ethyl-1,3,4-oxadiazol-2-yl)-2,4-dimethyl-1H-pyrrol-3-yl)(3-bromophenyl)methanone
2-((3-(3-bromobenzoyl)-2,4-dimethyl-5-(5-ethyl-1,3,4-oxadiazol-2-yl)-1H-pyrrol-1-yl)ethyl acetate
(3-bromophenyl)(1-(2-hydroxyethyl)-2,4-dimethyl-5-(5-ethyl-1,3,4-oxadiazol-2-yl)-1H-pyrrol-3-yl)methanone
(1-benzoyl-5-(5-ethyl-1,3,4-oxadiazol-2-yl)-2,4-dimethyl-1H-pyrrol-3-yl)(3-bromophenyl)methanone
tert-butyl(2-(4-bromo-2-(5-(5-(hydroxymethyl)-1,3,4-oxadiazol-2-yl)-2,4-dimethyl-1H-pyrrole-3-carbonyl)phenoxy)ethyl)carbamate
(2-((2-aminoethoxy)-5-bromophenyl)(5-(5-(hydroxymethyl)-1,3,4-oxadiazol-2-yl)-2,4-dimethyl-1H-pyrrol-3-yl)methanone
(5-bromo-2-methoxyphenyl)(5-(5-cyclopropyl-1,3,4-oxadiazol-2-yl)-2,4-dimethyl-1H-pyrrol-3-yl)methanone
(5-bromo-2-hydroxyphenyl)(5-(5-cyclopropyl-1,3,4-oxadiazol-2-yl)-2,4-dimethyl-1H-pyrrol-3-yl)methanone
tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate
tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate
(2-((2-aminoethoxy)-5-bromophenyl)(5-(5-cyclopropyl-1,3,4-oxadiazol-2-yl)-2,4-dimethyl-1H-pyrrol-3-yl)methanone
(2-((2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone
(5-((5-ethyl-1,3,4-oxadiazol-2-yl)-2,4-dimethyl-1H-pyrrol-3-yl)(3-hydroxyphenyl)methanone
tert-butyl(2-(3-(5-(5-ethyl-1,3,4-oxadiazol-2-yl)-2,4-dimethyl-1H-pyrrole-3-carbonyl)phenoxy)ethyl)carbamate
(3-((2-aminoethoxy)phenyl)(5-(5-ethyl-1,3,4-oxadiazol-2-yl)-2,4-dimethyl-1H-pyrrol-3-yl)methanone
(3-bromo-4-hydroxyphenyl)(5-(5-ethyl-1,3,4-oxadiazol-2-yl)-2,4-dimethyl-1H-pyrrol-3-yl)methanone
tert-butyl(2-(2-bromo-4-(5-(5-ethyl-1,3,4-oxadiazol-2-yl)-2,4-dimethyl-1H-pyrrole-3-carbonyl)phenoxy)ethyl)carbamate
(4-((2-aminoethoxy)-3-bromophenyl)(5-(5-ethyl-1,3,4-oxadiazol-2-yl)-2,4-dimethyl-1H-pyrrol-3-yl)methanone
(5-((5-cyclopropyl-1,3,4-oxadiazol-2-yl)-2,4-dimethyl-1H-pyrrol-3-yl)(3-hydroxyphenyl)methanone
(3-((2-aminoethoxy)phenyl)(5-(5-cyclopropyl-1,3,4-oxadiazol-2-yl)-2,4-dimethyl-1H-pyrrol-3-yl)methanone
(3-bromo-4-hydroxyphenyl)(5-(5-cyclopropyl-1,3,4-oxadiazol-2-yl)-2,4-dimethyl-1H-pyrrol-3-yl)methanone
tert-butyl(2-(2-bromo-4-(5-(5-cyclopropyl-1,3,4-oxadiazol-2-yl)-2,4-dimethyl-1H-pyrrole-3-carbonyl)phenoxy)ethyl)carbamate
(4-((2-aminoethoxy)-3-bromophenyl)(5-(5-cyclopropyl-1,3,4-oxadiazol-2-yl)-2,4-dimethyl-1H-pyrrol-3-yl)methanone
(3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone
3-((5-bromo-2-methoxybenzoyl)-5-(5-cyclopropyl-1,3,4-oxadiazol-2-yl)-4-methyl-1H-pyrrole-2-carboaldehyde
(5-bromo-2-methoxyphenyl)(5-(5-cyclopropyl-1,3,4-oxadiazol-2-yl)-2-(hydroxymethyl)-4-methyl-1H-pyrrol-3-yl)methanone
(3-bromophenyl)(5-(5-cyclopropyl-1,3,4-oxadiazol-2-yl)-4-methyl-2-vinyl-1H-pyrrol-3-yl)methanone
3-((3-bromobenzoyl)-5-(5-cyclopropyl-1,3,4-oxadiazol-2-yl)-4-methyl-1H-pyrrole-2-carboaldehyde
(3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone
3-((3-bromo-4-methoxybenzoyl)-5-(5-cyclopropyl-1,3,4-oxadiazol-2-yl)-4-methyl-1H-pyrrole-2-carboaldehyde
(3-bromophenyl)(5-(5-cyclopropyl-1,3,4-oxadiazol-2-yl)-2-(hydroxymethyl)-4-methyl-1H-pyrrol-3-yl)methanone
(3-bromophenyl)(5-(5-cyclopropyl-1,3,4-oxadiazol-2-yl)-2-(difluoromethyl)-4-methyl-1H-pyrrol-3-yl)methanone
(3-bromo-4-methoxyphenyl)(5-(5-cyclopropyl-1,3,4-oxadiazol-2-yl)-2-(hydroxymethyl)-4-methyl-1H-pyrrol-3-yl)methanone
(3-bromo-4-methoxyphenyl)(5-(5-cyclopropyl-1,3,4-oxadiazol-2-yl)-2-(difluoromethyl)-4-methyl-1H-pyrrol-3-yl)methanone
(3-bromo-4-methoxyphenyl)(5-(5-cyclopropyl-1,3,4-oxadiazol-2-yl)-2-(1-hydroxyethyl)-4-methyl-1H-pyrrol-3-yl)methanone
(3-((3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate

(3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone
(3-bromophenyl)(2,4-dimethyl-5-(3-methyl-1,2,4-oxadiazol-5-yl)-1H-pyrrol-3-yl)methanone
(3-bromophenyl)(5-(5-ethyloxazol-2-yl)-2,4-dimethyl-1H-pyrrol-3-yl)methanone
(5-bromo-2-methoxyphenyl)(5-(5-(1-bromocyclopropyl)-1,3,4-oxadiazol-2-yl)-2,4-dimethyl-1H-pyrrol-3-yl)methanone
(5-bromo-2-methoxyphenyl)(1-bromo-5-(5-(1-bromocyclopropyl)-1,3,4-oxadiazol-2-yl)-2,4-dimethyl-1H-pyrrol-3-yl)methanone
(3-bromophenyl)(2,4-dimethyl-5-(1,3,4-oxadiazol-2-yl)-1H-pyrrol-3-yl)methanone
(5-((5-(2-aminoethyl)-1,3,4-oxadiazol-2-yl)-2,4-dimethyl-1H-pyrrol-3-yl)(3-bromophenyl)methanone
2-((2-(5-(4-(3-bromobenzoyl)-3,5-dimethyl-1H-pyrrol-2-yl)-1,3,4-oxadiazol-2-yl)ethyl)isoindoline-1,3-dione
3-((3-bromobenzoyl)-5-(2-(2-(1,3-dioxoisoindolin-2-yl)ethyl)-2H-tetrazol-5-yl)-4-methyl-1H-pyrrole-2-carboaldehyde
2-((2-(5-(4-(3-bromobenzoyl)-5-(hydroxymethyl)-3-methyl-1H-pyrrol-2-yl)-2H-tetrazol-2-yl)ethyl)isoindoline-1,3-dione
(5-((2-(2-aminoethyl)-2H-tetrazol-5-yl)-2-(hydroxymethyl)-4-methyl-1H-pyrrol-3-yl)(3-bromophenyl)methanone
3-((3-bromobenzoyl)-5-(2-(2-(1,3-dioxoisoindolin-2-yl)ethyl)-2H-tetrazol-5-yl)-N,4-dimethyl-1H-pyrrole-2-carboxamide
5-((2-(2-aminoethyl)-2H-tetrazol-5-yl)-3-(3-bromobenzoyl)-N,4-dimethyl-1H-pyrrole-2-carboxamide
3-((3-bromobenzoyl)-5-(2-(2-(1,3-dioxoisoindolin-2-yl)ethyl)-2H-tetrazol-5-yl)-4-methyl-1H-pyrrole-2-carbonitrile
5-((2-(2-aminoethyl)-2H-tetrazol-5-yl)-3-(3-bromobenzoyl)-4-methyl-1H-pyrrole-2-carbonitrile
(3-bromophenyl)(5-(2-(2-hydroxyethyl)-2H-tetrazol-5-yl)-2-(2-hydroxypropan-2-yl)-4-methyl-1H-pyrrol-3-yl)methanone
(4-bromo-2-methyl-5-(1H-tetrazol-5-yl)-1H-pyrrol-3-yl)(3-bromophenyl)methanone
2-((5-(3-bromo-4-(3-bromobenzoyl)-5-methyl-1H-pyrrol-2-yl)-2H-tetrazol-2-yl)ethyl acetate
(4-bromo-5-(2-(2-hydroxyethyl)-2H-tetrazol-5-yl)-2-methyl-1H-pyrrol-3-yl)(3-bromophenyl)methanone
tert-butyl(2-(5-(3-bromo-4-(3-bromobenzoyl)-5-methyl-1H-pyrrol-2-yl)-2H-tetrazol-2-yl)ethyl)carbamate
(5-((2-(2-aminoethyl)-2H-tetrazol-5-yl)-4-bromo-2-methyl-1H-pyrrol-3-yl)(3-bromophenyl)methanone
tert-butyl(2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate
ethyl 5-(2-(2-acetoxyethyl)-2H-tetrazol-5-yl)-3-(3-bromo-4-methoxybenzoyl)-4-methyl-1H-pyrrole-2-carboxylate
ethyl 3-(3-bromo-4-methoxybenzoyl)-5-(2-(2-hydroxyethyl)-2H-tetrazol-5-yl)-4-methyl-1H-pyrrole-2-carboxylate
(5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone)
2-((5-(4-(3-bromo-4-methoxybenzoyl)-3-methyl-5-(methylcarbamoyl)-1H-pyrrol-2-yl)-2H-tetrazol-2-yl)ethyl acetate and
2-((5-(4-(3-bromobenzoyl)-3-methyl-5-(methylcarbamoyl)-1H-pyrrol-2-yl)-2H-tetrazol-2-yl)ethyl dihydrogen phosphate.

**8.** A pharmaceutical composition comprising a compound according to any one of claims 1 to 7 or a salt thereof.

**9.** An anticancer agent comprising a compound according to any one of claims 1 to 7 or a salt thereof as an active ingredient.

**10.** The anticancer agent according to claim 9, wherein the anticancer agent is an anticancer agent against a cancer having the potential to acquire drug resistance due to the involvement of P-glycoprotein and/or cancer already having the resistance.

**11.** The anticancer agent according to claim 9, wherein the anticancer agent is an anticancer agent against a cancer having the potential to acquire drug resistance due to the involvement of TUBB3 and/or a cancer already having the resistance.

**12.** A tubulin polymerization inhibitor comprising a compound according to any one of claims 1 to 7 or a salt thereof as an active ingredient.

**13.** An apoptosis inducer comprising a compound according to any one of claims 1 to 7 or a salt thereof as an active ingredient.

**14.** The compound according to any one of claims 1 to 7 or a salt thereof for use in the treatment of cancer.

**15.** The compound according to claim 14 or a salt thereof, wherein the cancer is a cancer having the potential to acquire drug resistance due to the involvement of P-glycoprotein and/or a cancer already having the resistance.

**16.** The compound according to claim 14 or a salt thereof, wherein the cancer is cancer having the potential to acquire drug resistance due to the involvement of TUBB3 and/or cancer already having the resistance.

**17.** The compound according to any one of claims 1 to 7 or a salt thereof for use in the inhibition of tubulin polymerization.

**18.** The compound according to any one of claims 1 to 7 or a salt thereof for use in the induction of apoptosis.

**19.** Use of a compound according to any one of claims 1 to 7 or a salt thereof for the production of an anticancer agent.

**20.** The use of according to claim 19, wherein the anticancer agent is an anticancer agent against a cancer having the potential to acquire drug resistance due to the involvement of P-glycoprotein and/or a cancer already having the resistance.

**21.** The use of according to claim 19, wherein the anticancer agent is an anticancer agent against a cancer having the potential to acquire drug resistance due to the involvement of TUBB3 and/or a cancer already having the resistance.

**22.** Use of a compound according to any one of claims 1 to 7 or a salt thereof for the production of a tubulin polymerization inhibitor.

**23.** Use of a compound according to any one of claims 1 to 7 or a salt thereof for the production of an apoptosis inducer.

**24.** A method for treating cancer, comprising administering an effective amount of a compound according to any one of claims 1 to 7 or a salt thereof.

**25.** The method according to claim 24, wherein the cancer is a cancer having the potential to acquire drug resistance due to the involvement of P-glycoprotein and/or a cancer already having the resistance.

**26.** The method according to claim 24, wherein the cancer is a cancer having the potential to acquire drug resistance due to the involvement of TUBB3 and/or a cancer already having the resistance.

**27.** A method for inhibiting tubulin polymerization, comprising administering an effective amount of a compound according to any one of claims 1 to 7 or a salt thereof.

**28.** A method for inducing apoptosis, comprising administering an effective amount of a compound according to any one of claims 1 to 7 or a salt thereof.

**Amended claims under Art. 19.1 PCT**

**1.** (Amended) A compound represented by the general formula (1) or a salt thereof:

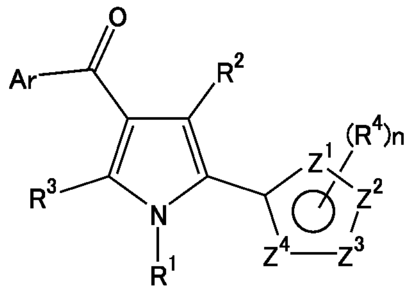

(1)

wherein Ar represents an optionally substituted aryl group or an optionally substituted heteroaryl group; $Z^1$, $Z^2$, $Z^3$, and $Z^4$ each independently represent C, CH, a nitrogen atom, an oxygen atom, or a sulfur atom; $R^1$ represents a hydrogen atom, a halogen atom, an optionally substituted alkyl group, an optionally substituted cycloalkyl group, an optionally substituted alkylcarbonyl group, an optionally substituted alkylsulfonyl group, an optionally substituted cycloalkylsulfonyl group, an optionally substituted aryl group, an optionally substituted aralkyl group, or an optionally substituted arylcarbonyl group;

$R^2$ and $R^3$ are the same or different and each represent a hydrogen atom, a halogen atom, a cyano group, a carboxyl group, an optionally substituted alkoxycarbonyl group, an optionally substituted aminocarbonyl group, an optionally substituted alkylaminocarbonyl group, an optionally substituted cyclic aminocarbonyl group, an optionally substituted imino group, a formyl group, a sulfo group, an optionally substituted alkylsulfonyl group, an optionally substituted alkyl group, an optionally substituted alkenyl group, a sulfonamide group, an optionally substituted alkylsulfonylamino group, an optionally substituted aryl group, an optionally substituted arylcarbonyl group, or an optionally substituted dithianyl group;

n units of $R^4$ are the same or different and each represent a hydrogen atom, an optionally substituted alkyl group, an optionally substituted alkenyl group, an optionally substituted cycloalkyl group, an optionally substituted aryl group, an optionally substituted aralkyl group, a carboxyl group, or an alkoxycarbonyl group; and n represents a number of 0 to 4 (except for the case where Ar is a 2-hydroxyphenyl group, $Z^1$ is a nitrogen atom, $Z^2$ is C, $Z^3$ is CH, $Z^4$ is a sulfur atom, $R^1$ is a hydrogen atom, $R^2$ is a hydrogen atom, $R^3$ is a hydrogen atom, $R^4$ is a methyl group, and n is 1;

the case where Ar is a 2-hydroxyphenyl group, $Z^1$ is a nitrogen atom, $Z^2$ is a nitrogen atom, $Z^3$ is CH, $Z^4$ is a nitrogen atom, $R^1$ is a hydrogen atom, $R^2$ is a hydrogen atom, $R^3$ is a hydrogen atom, $R^4$ is a methyl group, and n is 1;

the case where Ar is a 2-hydroxyphenyl group, $Z^1$ is a nitrogen atom, $Z^2$ is C, $Z^3$ is a nitrogen atom, $Z^4$ is an oxygen atom, $R^1$ is a hydrogen atom, $R^2$ is a hydrogen atom, $R^3$ is a hydrogen atom, $R^4$ is a phenyl group, and n is 1;

the case where Ar is a 2-hydroxyphenyl group, $Z^1$ is CH, $Z^2$ is C, $Z^3$ is a nitrogen atom, $Z^4$ is an oxygen atom, $R^1$ is a methyl group, $R^2$ is a hydrogen atom, $R^3$ is a hydrogen atom, $R^4$ is a 4-methoxyphenyl group, and n is 1;

the case where Ar is a 2-hydroxyphenyl group, $Z^1$ is a nitrogen atom, $Z^2$ is a nitrogen atom, $Z^3$ is C, $Z^4$ is an oxygen atom, $R^1$ is an isopropyl group, $R^2$ is a hydrogen atom, $R^3$ is a hydrogen atom, $R^4$ is a phenyl group, and n is 1;

the case where Ar is a phenyl group, $Z^1$ is a nitrogen atom, $Z^2$ is a nitrogen atom, $Z^3$ is C, $Z^4$ is an oxygen atom, $R^1$ is a methyl group, $R^2$ is a hydrogen atom, $R^3$ is a methyl group, $R^4$ is a 4-bromophenyl group, and n is 1;

the case where Ar is a phenyl group, $Z^1$ is a nitrogen atom, $Z^2$ is a nitrogen atom, $Z^3$ is C, $Z^4$ is an oxygen atom, $R^1$ is a methyl group, $R^2$ is a hydrogen atom, $R^3$ is a methyl group, $R^4$ is a 4-methylphenyl group, and n is 1;

the case where Ar is a phenyl group, $Z^1$ is a nitrogen atom, $Z^2$ is a nitrogen atom, $Z^3$ is C, $Z^4$ is an oxygen atom, $R^1$ is a methyl group, $R^2$ is a hydrogen atom, $R^3$ is a methyl group, $R^4$ is a 4-nitrophenyl group, and n is 1;

the case where Ar is a phenyl group, $Z^1$ is CH, $Z^2$ is CH, $Z^3$ is CH, $Z^4$ is a nitrogen atom, $R^1$ is a hydrogen atom, $R^2$ is a hydrogen atom, $R^3$ is a hydrogen atom, $R^4$ is a hydrogen atom, and n is 1; and

the case where Ar is a phenyl group, $Z^1$ is CH, $Z^2$ is CH, $Z^3$ is CH, $Z^4$ is an oxygen atom, $R^1$ is an ethyl group, $R^2$ is a benzoyl group, $R^3$ is a 4-methylsulfanylphenyl group, and n is 0).

**2.** The compound according to claim 1 or a salt thereof, wherein in the general formula (1), $R^2$ is a halogen atom, a formyl group, or an optionally substituted alkyl group having 1 to 8 carbon atoms.

**3.** The compound according to claim 1 or 2 or a salt thereof, wherein in the general formula (1), at least one of $Z^1$, $Z^2$, $Z^3$, and $Z^4$ is a nitrogen atom.

**4.** The compound according to any one of claims 1 to 3 or a salt thereof, wherein in the general formula (1), the ring constituted by $Z^1$, $Z^2$, $Z^3$, and $Z^4$ has a structure represented by any of the following formulas (2a) to (2e) :

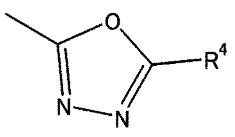

(2a)

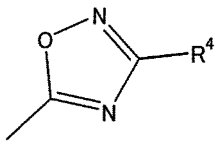

(2b)

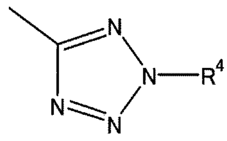

(2c)

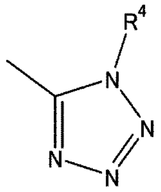

(2d)

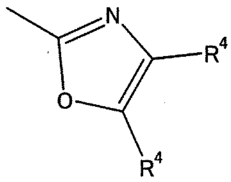

(2e)

wherein $R^4$ is as defined above.

**5.** The compound according to any one of claims 1 to 4 or a salt thereof, wherein Ar is an optionally substituted aryl group having 6 to 14 carbon atoms or an optionally substituted 5- to 10-membered heteroaryl group.

**6.** The compound according to any one of claims 1 to 5 or a salt thereof, wherein Ar is an optionally substituted aryl group having 6 to 14 carbon atoms.

**7.** A compound selected from the following or a salt thereof:

(3-bromophenyl)(2,4-dimethyl-5-(1H-tetrazol-5-yl)-1H-pyrrol-3-yl)methanone
(3-bromophenyl)(5-(2-ethyl-2H-tetrazol-5-yl)-2,4-dimethyl-1H-pyrrol-3-yl)methanone
(3-bromophenyl)(5-(1-ethyl-1H-tetrazol-5-yl)-2,4-dimethyl-1H-pyrrol-3-yl)methanone
(3-bromophenyl)(2,4-dimethyl-5-(2-(2,2,2-trifluoroethyl)-2H-tetrazol-5-yl)-1H-pyrrol-3-yl)methanone
(3-bromophenyl)(2,4-dimethyl-5-(1-(2,2,2-trifluoroethyl)-1H-tetrazol-5-yl)-1H-pyrrol-3-yl)methanone
(3-bromophenyl)(5-(2-(2-(dimethylamino)ethyl)-2H-tetrazol-5-yl)-2,4-dimethyl-1H-pyrrol-3-yl)methanone
(3-bromophenyl)(5-(2-(2-hydroxyethyl)-2H-tetrazol-5-yl)-2,4-dimethyl-1H-pyrrol-3-yl)methanone
(5-((2-(2-aminoethyl)-2H-tetrazol-5-yl)-2,4-dimethyl-1H-pyrrol-3-yl)(3-bromophenyl)methanone
2-((5-(4-(3-bromobenzoyl)-3,5-dimethyl-1H-pyrrol-2-yl)-2H-tetrazol-2-yl)ethyl acetate
(2,4-dimethyl-5-(1H-tetrazol-5-yl)-1H-pyrrol-3-yl)(3-methoxyphenyl)methanone
(3-methoxyphenyl)(5-(2-ethyl-2H-tetrazol-5-yl)-2,4-dimethyl-1H-pyrrol-3-yl)methanone
(5-((2-ethyl-2H-tetrazol-5-yl)-2,4-dimethyl-1H-pyrrol-3-yl)(3-methoxyphenyl)methanone
(3-bromo-4-methoxyphenyl)(2,4-dimethyl-5-(1H-tetrazol-5-yl)-1H-pyrrol-3-yl)methanone
(3-bromo-4-methoxyphenyl)(5-(2-ethyl-2H-tetrazol-5-yl)-2,4-dimethyl-1H-pyrrol-3-yl)methanone
(3-bromo-4-methoxyphenyl)(5-(2-(2-hydroxyethyl)-2H-tetrazol-5-yl)-2,4-dimethyl-1H-pyrrol-3-yl)methanone
2-((5-(4-(3-bromo-4-methoxybenzoyl)-3,5-dimethyl-1H-pyrrol-2-yl)-2H-tetrazol-2-yl)ethyl acetate
(3-bromo-4-methoxyphenyl)(5-(2-(2-((tertbutyldimethylsilyl)oxy)ethyl)-2H-tetrazol-5-yl)-2,4-dimethyl-1H-pyrrol-3-yl)methanone
(3-cyclopropylphenyl)(2,4-dimethyl-5-(1H-tetrazol-5-yl)-1H-pyrrol-3-yl)methanone
2-((5-(4-(3-cyclopropylbenzoyl)-3,5-dimethyl-1H-pyrrol-2-yl)ethyl acetate
(3-cyclopropylphenyl)(5-(2-(2-hydroxyethyl)-2H-tetrazol-5-yl)-2,4-dimethyl-1H-pyrrol-3-yl)methanone
2-((5-(4-(3-bromobenzoyl)-5-formyl-3-methyl-1H-pyrrol-2-yl)-2H-tetrazol-2-yl)ethyl acetate
3-((3-bromobenzoyl)-5-(2-(2-hydroxyethyl)-2H-tetrazol-5-yl)-4-methyl-1H-pyrrole-2-carboaldehyde
2-((5-(4-(3-bromobenzoyl)-5-(difluoromethyl)-3-methyl-1H-pyrrol-2-yl)-2H-tetrazol-2-yl)ethyl acetate
(3-bromophenyl)(2-(difluoromethyl)-5-(2-(2-hydroxyethyl)-2H-tetrazol-5-yl)-4-methyl-1H-pyrrol-3-yl)methanone
2-((5-(4-(3-bromobenzoyl)-5-(hydroxymethyl)-3-methyl-1H-pyrrol-2-yl)-2H-tetrazol-2-yl)ethyl acetate
(3-bromophenyl)(5-(2-(2-hydroxyethyl)-2H-tetrazol-5-yl)-2-(hydroxymethyl)-4-methyl-1H-pyrrol-3-yl)methanone
2-((5-(4-(3-bromobenzoyl)-5-(1-hydroxyethyl)-3-methyl-1H-pyrrol-2-yl)-2H-tetrazol-2-yl)ethyl acetate
(3-bromophenyl)(2-(1-hydroxyethyl)-5-(2-(2-hydroxyethyl)-2H-tetrazol-5-yl)-4-methyl-1H-pyrrol-3-yl)methanone
2-((5-(4-(3-bromo-4-methoxybenzoyl)-5-formyl-3-methyl-1H-pyrrol-2-yl)-2H-tetrazol-2-yl)ethyl acetate
3-((3-bromo-4-methoxybenzoyl)-5-(2-(2-hydroxyethyl)-2H-tetrazol-5-yl)-4-methyl-1H-pyrrole-2-carboaldehyde
2-((5-(4-(3-bromo-4-methoxybenzoyl)-5-(difluoromethyl)-3-methyl-1H-pyrrol-2-yl)-2H-tetrazol-2-yl)ethyl acetate
(3-bromo-4-methoxyphenyl)(2-(difluoromethyl)-5-(2-(2-hydroxyethyl)-2H-tetrazol-5-yl)-4-methyl-1H-pyrrol-3-yl)methanone
2-((5-(4-(3-bromo-4-methoxybenzoyl)-5-(hydroxymethyl)-3-methyl-1H-pyrrol-2-yl)-2H-tetrazol-2-yl)ethyl acetate
(3-bromo-4-methoxyphenyl)(5-(2-(2-hydroxyethyl)-2H-tetrazol-5-yl)-2-(hydroxymethyl)-4-methyl-1H-pyrrol-3-yl)methanone
2-((5-(4-(3-bromo-4-methoxybenzoyl)-5-(1-hydroxyethyl)-3-methyl-1H-pyrrol-2-yl)-2H-tetrazol-2-yl)ethyl acetate
(3-bromo-4-methoxyphenyl)(2-(1-hydroxyethyl)-5-(2-(2-hydroxyethyl)-2H-tetrazol-5-yl)-4-methyl-1H-pyrrol-3-yl)methanone
2-((5-(4-(3-bromobenzoyl)-5-cyano-3-methyl-1H-pyrrol-2-yl)-2H-tetrazol-2-yl)ethyl acetate
3-((3-bromobenzoyl)-5-(2-(2-hydroxyethyl)-2H-tetrazol-5-yl)-4-methyl-1H-pyrrole-2-carbonitrile
5-((2-(2-acetoxyethyl)-2H-tetrazol-5-yl)-3-(3-bromobenzoyl)-4-methyl-1H-pyrrole-2-carboxylic acid
3-((3-bromobenzoyl)-5-(2-(2-hydroxyethyl)-2H-tetrazol-5-yl)-4-methyl-1H-pyrrole-2-carboxylic acid
methyl 5-(2-(2-acetoxyethyl)-2H-tetrazol-5-yl)-3-(3-bromobenzoyl)-4-methyl-1H-pyrrole-2-carboxylate
methyl 3-(3-bromobenzoyl)-5-(2-(2-hydroxyethyl)-2H-tetrazol-5-yl)-4-methyl-1H-pyrrole-2-carboxylate
2-((5-(4-(3-bromo-4-methoxybenzoyl)-5-cyano-3-methyl-1H-pyrrol-2-yl)-2H-tetrazol-2-yl)ethyl acetate
3-((3-bromo-4-methoxybenzoyl)-5-(2-(2-hydroxyethyl)-2H-tetrazol-5-yl)-4-methyl-1H-pyrrole-2-carbonitrile
5-((2-(2-acetoxyethyl)-2H-tetrazol-5-yl)-3-(3-bromo-4-methoxybenzoyl)-4-methyl-1H-pyrrole-2-carboxylic acid
3-((3-bromo-4-methoxybenzoyl)-5-(2-(2-hydroxyethyl)-2H-tetrazol-5-yl)-4-methyl-1H-pyrrole-2-carboxylic acid
3-((3-bromobenzoyl)-5-(2-(2-hydroxyethyl)-2H-tetrazol-5-yl)-N,4-dimethyl-1H-pyrrole-2-carboxamide
3-((3-bromobenzoyl)-5-(2-(2-hydroxyethyl)-2H-tetrazol-5-yl)-N,N,4-trimethyl-1H-pyrrole-2-carboxamide
N-(2-aminoethyl)-3-(3-bromobenzoyl)-5-(2-(2-hydroxyethyl)-2H-tetrazol-5-yl)-4-methyl-1H-pyrrole-2-carboxamide
3-((3-bromobenzoyl)-N-ethyl-5-(2-(2-hydroxyethyl)-2H-tetrazol-5-yl)-4-methyl-1H-pyrrole-2-carboxamide
3-((3-bromobenzoyl)-5-(2-(2-hydroxyethyl)-2H-tetrazol-5-yl)-N-isopropyl-4-methyl-1H-pyrrole-2-carboxamide
(3-((3-bromobenzoyl)-5-(2-(2-hydroxyethyl)-2H-tetrazol-5-yl)-4-methyl-1H-pyrrol-2-yl)(4-methylpiperazin-1-yl)methanone
(3-((3-bromobenzoyl)-5-(2-(2-hydroxyethyl)-2H-tetrazol-5-yl)-4-methyl-1H-pyrrol-2-yl)(piperazin-1-yl)methanone
3-((3-bromo-4-methoxybenzoyl)-5-(2-(2-hydroxyethyl)-2H-tetrazol-5-yl)-N,4-dimethyl-1H-pyrrole-2-carboxamide
3-((3-bromo-4-methoxybenzoyl)-5-(2-(2-hydroxyethyl)-2H-tetrazol-5-yl)-4-methyl-1H-pyrrole-2-carboxamide
2-((5-(4-(3-bromobenzoyl)-3,5-dimethyl-1H-pyrrol-2-yl)-2H-tetrazol-2-yl)ethyl 2-((tert-butoxycarbonyl)amino)acetate
2-((5-(4-(3-bromobenzoyl)-3,5-dimethyl-1H-pyrrol-2-yl)-2H-tetrazol-2-yl)ethyl 2-aminoacetate
2-((5-(4-(3-bromobenzoyl)-5-formyl-3-methyl-1H-pyrrol-2-yl)-2H-tetrazol-2-yl)ethyl 2-((tert-butoxycarbonyl)amino)acetate
2-((5-(4-(3-bromobenzoyl)-5-(hydroxymethyl)-3-methyl-1H-pyrrol-2-yl)-2H-tetrazol-2-yl)ethyl 2-((tert-butoxycarbonyl)amino)acetate
2-((5-(4-(3-bromobenzoyl)-5-(hydroxymethyl)-3-methyl-1H-pyrrol-2-yl)-2H-tetrazol-2-yl)ethyl 2-aminoacetate
methyl 3-(3-bromobenzoyl)-5-(2-(2-(2-((tert-butoxycarbonyl)amino)acetoxy)ethyl)-2H-tetrazol-5-yl)-4-methyl-1H-pyrrole-2-carboxylate
methyl 5-(2-(2-(2-aminoacetoxy)ethyl)-2H-tetrazol-5-yl)-3-(3-bromobenzoyl)-4-methyl-1H-pyrrole-2-carboxylate
2-((5-(4-(3-bromobenzoyl)-3-methyl-5-(methylcarbamoyl)-1H-pyrrol-2-yl)-2H-tetrazol-2-yl)ethyl 2-((tert-butoxycarbonyl)amino)acetate
2-((5-(4-(3-bromobenzoyl)-3-methyl-5-(methylcarbamoyl)-1H-pyrrol-2-yl)-2H-tetrazol-2-yl)ethyl 2-aminoacetate 2-(5-(3,5-dimethyl-4-(3-vinylbenzoyl)-1H-pyrrol-2-yl)-2H-tetrazol-2-yl)ethyl acetate
2-((5-(4-(3-formylbenzoyl)-3,5-dimethyl-1H-pyrrol-2-yl)-2H-tetrazol-2-yl)ethyl acetate

2-((5-(4-(3-(hydroxymethyl)benzoyl)-3,5-dimethyl-1H-pyrrol-2-yl)-2H-tetrazol-2-yl)ethyl acetate
(5-((2-(2-hydroxyethyl)-2H-tetrazol-5-yl)-2,4-dimethyl-1H-pyrrol-3-yl)(3-(hydroxymethyl)phenyl)methanone
(5-((2-ethyl-2H-tetrazol-5-yl)-2,4-dimethyl-1H-pyrrol-3-yl)(3-hydroxyphenyl)methanone
(3-((2-aminoethoxy)phenyl)(5-(2-ethyl-2H-tetrazol-5-yl)-2,4-dimethyl-1H-pyrrol-3-yl)methanone
(3-bromo-4-hydroxyphenyl)(5-(2-ethyl-2H-tetrazol-5-yl)-2,4-dimethyl-1H-pyrrol-3-yl)methanone
(4-((2-aminoethoxy)-3-bromophenyl)(5-(2-ethyl-2H-tetrazol-5-yl)-2,4-dimethyl-1H-pyrrol-3-yl)methanone
(4-((2-aminoethoxy)-3-bromophenyl)(5-(2-(2-hydroxyethyl)-2H-tetrazol-5-yl)-2,4-dimethyl-1H-pyrrol-3-yl)methanone
(E)-2-(5-(4-(3-bromo-4-methoxybenzoyl)-5-((hydroxyimino)methyl)-3-methyl-1H-pyrrol-2-yl)-2H-tetrazol-2-yl)ethyl acetate
(Z)-2-(5-(4-(3-bromo-4-methoxybenzoyl)-5-((hydroxyimino)methyl)-3-methyl-1H-pyrrol-2-yl)-2H-tetrazol-2-yl)ethyl acetate
2-((5-(4-(3-bromobenzoyl)-5-(chloromethyl)-3-methyl-1H-pyrrol-2-yl)-2H-tetrazol-2-yl)ethyl acetate
2-((5-(4-(3-bromo-4-methoxybenzoyl)-5-(chloromethyl)-3-methyl-1H-pyrrol-2-yl)-2H-tetrazol-2-yl)ethyl acetate
(3-bromophenyl)(5-(5-ethyl-1,3,4-oxadiazol-2-yl)-2,4-dimethyl-1H-pyrrol-3-yl)methanone
(3-bromophenyl)(5-(5-(2-chloroethyl)-1,3,4-oxadiazol-2-yl)-2,4-dimethyl-1H-pyrrol-3-yl)methanone
(3-bromophenyl)(2,4-dimethyl-5-(5-phenyl-1,3,4-oxadiazol-2-yl)-1H-pyrrol-3-yl)methanone
(5-((5-benzyl-1,3,4-oxadiazol-2-yl)-2,4-dimethyl-1H-pyrrol-3-yl)(3-bromophenyl)methanone
(3-bromophenyl)(5-(5-cyclopropyl-1,3,4-oxadiazol-2-yl)-2,4-dimethyl-1H-pyrrol-3-yl)methanone
(3-bromophenyl)(5-(5-isopropyl-1,3,4-oxadiazol-2-yl)-2,4-dimethyl-1H-pyrrol-3-yl)methanone
(3-bromophenyl)(5-(5-cyclobutyl-1,3,4-oxadiazol-2-yl)-2,4-dimethyl-1H-pyrrol-3-yl)methanone
(3-bromophenyl)(5-(5-cyclopentyl-1,3,4-oxadiazol-2-yl)-2,4-dimethyl-1H-pyrrol-3-yl)methanone
(3-bromophenyl)(5-(5-cyclohexyl-1,3,4-oxadiazol-2-yl)-2,4-dimethyl-1H-pyrrol-3-yl)methanone
methyl 3-(5-(4-(3-bromobenzoyl)-3,5-dimethyl-1H-pyrrol-2-yl)-1,3,4-oxadiazol-2-yl)propionate
ethyl 2-(5-(4-(3-bromobenzoyl)-3,5-dimethyl-1H-pyrrol-2-yl)-1,3,4-oxadiazol-2-yl)acetate
(3-bromophenyl)(5-(5-tert-butyl)-1,3,4-oxadiazol-2-yl)-2,4-dimethyl-1H-pyrrol-3-yl)methanone
(3-bromophenyl)(2,4-dimethyl-5-(5-methyl-1,3,4-oxadiazol-2-yl)-1H-pyrrol-3-yl)methanone
(3-bromophenyl)(5-(5-(difluoromethyl)-1,3,4-oxadiazol-2-yl)-2,4-dimethyl-1H-pyrrol-3-yl)methanone
(3-bromophenyl)(2,4-dimethyl-5-(5-(2,2,2-trifluoroethyl)-1,3,4-oxadiazol-2-yl)-1H-pyrrol-3-yl)methanone
(3-bromophenyl)(5-(5-(1-hydroxycyclopropyl)-1,3,4-oxadiazol-2-yl)-2,4-dimethyl-1H-pyrrol-3-yl)methanone
(3-bromophenyl)(2,4-dimethyl-5-(5-(1-methylcyclopropyl)-1,3,4-oxadiazol-2-yl)-1H-pyrrol-3-yl)methanone
(3-bromophenyl)(2,4-dimethyl-5-(5-(2-methylcyclopropyl)-1,3,4-oxadiazol-2-yl)-1H-pyrrol-3-yl)methanone
(3-bromophenyl)(5-(5-(2,2-dimethylcyclopropyl)-1,3,4-oxadiazol-2-yl)-2,4-dimethyl-1H-pyrrol-3-yl)methanone
(3-bromophenyl)(2,4-dimethyl-5-(5-(2,2,3,3-tetramethylcyclopropyl)-1,3,4-oxadiazol-2-yl)-1H-pyrrol-3-yl)methanone
(5-((5-ethyl-1,3,4-oxadiazol-2-yl)-2,4-dimethyl-1H-pyrrol-3-yl)(3-methoxyphenyl)methanone
(5-((5-cyclopropyl-1,3,4-oxadiazol-2-yl)-2,4-dimethyl-1H-pyrrol-3-yl)(3-methoxyphenyl)methanone
(5-((5-ethyl-1,3,4-oxadiazol-2-yl)-2,4-dimethyl-1H-pyrrol-3-yl)(3-nitrophenyl)methanone
(5-((5-cyclopropyl-1,3,4-oxadiazol-2-yl)-2,4-dimethyl-1H-pyrrol-3-yl)(3-nitrophenyl)methanone
(3-bromo-4-methoxyphenyl)(5-(5-ethyl-1,3,4-oxadiazol-2-yl)-2,4-dimethyl-1H-pyrrol-3-yl)methanone
(3-bromo-4-methoxyphenyl)(5-(5-cyclopropyl-1,3,4-oxadiazol-2-yl)-2,4-dimethyl-1H-pyrrol-3-yl)methanone
(5-((5-cyclopropyl-1,3,4-oxadiazol-2-yl)-2,4-dimethyl-1H-pyrrol-3-yl)(3-(hydroxymethyl)phenyl)methanone
(3-bromophenyl)(5-(5-cyclopropyl-1,3,4-oxadiazol-2-yl)-2-(1,3-dithian-2-yl)-4-methyl-1H-pyrrol-3-yl)methanone
(5-bromo-2-methoxyphenyl)(5-(5-(chloromethyl)-1,3,4-oxadiazol-2-yl)-2,4-dimethyl-1H-pyrrol-3-yl)methanone
(5-((4-(5-bromo-2-methoxybenzoyl)-3,5-dimethyl-1H-pyrrol-2-yl)-1,3,4-oxadiazol-2-yl)methyl acetate
(5-bromo-2-hydroxyphenyl)(5-(5-(hydroxymethyl)-1,3,4-oxadiazol-2-yl)-2,4-dimethyl-1H-pyrrol-3-yl)methanone
2-((5-(4-(3-bromobenzoyl)-3,5-dimethyl-1H-pyrrol-2-yl)-1,3,4-oxadiazol-2-yl)ethyl acetate
(3-bromophenyl)(5-(5-(2-hydroxyethyl)-1,3,4-oxadiazol-2-yl)-2,4-dimethyl-1H-pyrrol-3-yl)methanone
(3-bromophenyl)(2,4-dimethyl-5-(5-vinyl-1,3,4-oxadiazol-2-yl)-1H-pyrrol-3-yl)methanone
(3-bromophenyl)(5-(5-(chloromethyl)-1,3,4-oxadiazol-2-yl)-2,4-dimethyl-1H-pyrrol-3-yl)methanone
(3-bromophenyl)(2,4-dimethyl-5-(5-((4-methylpiperazin-1-yl)methyl)-1,3,4-oxadiazol-2-yl)-1H-pyrrol-3-yl)methanone
(3-bromophenyl)(5-(5-((dimethylamino)methyl)-1,3,4-oxadiazol-2-yl)-2,4-dimethyl-1H-pyrrol-3-yl)methanone
(5-((4-(3-bromobenzoyl)-3,5-dimethyl-1H-pyrrol-2-yl)-1,3,4-oxadiazol-2-yl)methyl acetate
(3-bromophenyl)(5-(5-(hydroxymethyl)-1,3,4-oxadiazol-2-yl)-2,4-dimethyl-1H-pyrrol-3-yl)methanone
S-((5-(4-(3-bromobenzoyl)-3,5-dimethyl-1H-pyrrol-2-yl)-1,3,4-oxadiazol-2-yl)methyl)ethanethioate

2-(((5-(4-(3-bromobenzoyl)-3,5-dimethyl-1H-pyrrol-2-yl)-1,3,4-oxadiazol-2-yl)methyl)isoindole-1,3-dione
(5-((5-(aminomethyl)-1,3,4-oxadiazol-2-yl)-2,4-dimethyl-1H-pyrrol-3-yl)(3-bromophenyl)methanone
N-((5-(4-(3-bromobenzoyl)-3,5-dimethyl-1H-pyrrol-2-yl)-1,3,4-oxadiazol-2-yl)methyl)acetamide
methyl((5-(4-(3-bromobenzoyl)-3,5-dimethyl-1H-pyrrol-2-yl)-1,3,4-oxadiazol-2-yl)methyl)carbamate
N-((5-(4-(3-bromobenzoyl)-3,5-dimethyl-1H-pyrrol-2-yl)-1,3,4-oxadiazol-2-yl)methyl)methanesulfonamide
1-(((5-(4-(3-bromobenzoyl)-3,5-dimethyl-1H-pyrrol-2-yl)-1,3,4-oxadiazol-2-yl)methyl)-3-ethylurea
1-(((5-(4-(3-bromobenzoyl)-3,5-dimethyl-1H-pyrrol-2-yl)-1,3,4-oxadiazol-2-yl)methyl)-3-ethylthiourea
3-(((5-(4-(3-bromobenzoyl)-3,5-dimethyl-1H-pyrrol-2-yl)-1,3,4-oxadiazol-2-yl)methyl)-1,1-dimethylthiourea
2-((((5-(4-(3-bromobenzoyl)-3,5-dimethyl-1H-pyrrol-2-yl)-1,3,4-oxadiazol-2-yl)methyl)amino)-2-oxoethyl acetate
N-((5-(4-(3-bromobenzoyl)-3,5-dimethyl-1H-pyrrol-2-yl)-1,3,4-oxadiazol-2-yl)methyl)-2-hydroxyacetamide
(3-bromophenyl)(5-(5-ethyl-1,3,4-oxadiazol-2-yl)-1,2,4-trimethyl-1H-pyrrol-3-yl)methanone
1-((3-(3-bromobenzoyl)-5-(5-ethyl-1,3,4-oxadiazol-2-yl)-2,4-dimethyl-1H-pyrrol-1-yl)ethanone
(3-bromophenyl)(5-(5-ethyl-1,3,4-oxadiazol-2-yl)-2,4-dimethyl-1-(methylsulfonyl)-1H-pyrrol-3-yl)methanone
2-((3-(3-bromobenzoyl)-5-(5-ethyl-1,3,4-oxadiazol-2-yl)-2,4-dimethyl-1H-pyrrol-1-yl)-2-oxoethyl acetate
(3-bromophenyl)(1-(cyclopropylsulfonyl)-5-(5-ethyl-1,3,4-oxadiazol-2-yl)-2,4-dimethyl-1H-pyrrol-3-yl)methanone
(3-bromophenyl)(1-(difluoromethyl)-5-(5-ethyl-1,3,4-oxadiazol-2-yl)-2,4-dimethyl-1H-pyrrol-3-yl)methanone
(3-bromophenyl)(5-(5-ethyl-1,3,4-oxadiazol-2-yl)-2,4-dimethyl-1-(2,2,2-trifluoroethyl)-1H-pyrrol-3-yl)methanone
(3-bromophenyl)(1-cyclopropyl-5-(5-ethyl-1,3,4-oxadiazol-2-yl)-2,4-dimethyl-1H-pyrrol-3-yl)methanone
tert-butyl(2-(3-(3-bromobenzoyl)-5-(5-ethyl-1,3,4-oxadiazol-2-yl)-2,4-dimethyl-1H-pyrrol-1-yl)ethyl)carbamate
(3-bromophenyl)(1-ethyl-2,4-dimethyl-5-(5-ethyl-1,3,4-oxadiazol-2yl)-1H-pyrrol-3-yl)methanone
(3-bromophenyl)(1-isopropyl-2,4-dimethyl-5-(5-ethyl-1,3,4-oxadiazol-2-yl)-1H-pyrrol-3-yl)methanone
(1-((2-aminoethyl)-5-(5-ethyl-1,3,4-oxadiazol-2-yl)-2,4-dimethyl-1H-pyrrol-3-yl)(3-bromophenyl)methanone
2-((3-(3-bromobenzoyl)-2,4-dimethyl-5-(5-ethyl-1,3,4-oxadiazol-2-yl)-1H-pyrrol-1-yl)ethyl acetate
(3-bromophenyl)(1-(2-hydroxyethyl)-2,4-dimethyl-5-(5-ethyl-1,3,4-oxadiazol-2-yl)-1H-pyrrol-3-yl)methanone
(1-benzoyl-5-(5-ethyl-1,3,4-oxadiazol-2-yl)-2,4-dimethyl-1H-pyrrol-3-yl)(3-bromophenyl)methanone
tert-butyl(2-(4-bromo-2-(5-(5-(hydroxymethyl)-1,3,4-oxadiazol-2-yl)-2,4-dimethyl-1H-pyrrole-3-carbonyl)phenoxy)ethyl)carbamate
(2-((2-aminoethoxy)-5-bromophenyl)(5-(5-(hydroxymethyl)-1,3,4-oxadiazol-2-yl)-2,4-dimethyl-1H-pyrrol-3-yl)methanone
(5-bromo-2-methoxyphenyl)(5-(5-cyclopropyl-1,3,4-oxadiazol-2-yl)-2,4-dimethyl-1H-pyrrol-3-yl)methanone
(5-bromo-2-hydroxyphenyl)(5-(5-cyclopropyl-1,3,4-oxadiazol-2-yl)-2,4-dimethyl-1H-pyrrol-3-yl)methanone
tert-butyl(2-(4-bromo-2-(5-(5-cyclopropyl-1,3,4-oxadiazol-2-yl)-2,4-dimethyl-1H-pyrrole-3-carbonyl)phenoxy)ethyl)carbamate
tert-butyl(2-(4-bromo-2-(5-(5-cyclopropyl-1,3,4-oxadiazol-2-yl)-2,4-dimethyl-1-tert-butoxycarbonylaminoethyl-pyrrole-3-carbonyl)phenoxy)ethyl)carbamate
(2-((2-aminoethoxy)-5-bromophenyl)(5-(5-cyclopropyl-1,3,4-oxadiazol-2-yl)-2,4-dimethyl-1H-pyrrol-3-yl)methanone
(2-((2-aminoethoxy)-5-bromophenyl)(1-(2-aminoethyl)-5-(5-cyclopropyl-1,3,4-oxadiazol-2-yl)-2,4-dimethyl-1H-pyrrol-3-yl)methanone
(5-((5-ethyl-1,3,4-oxadiazol-2-yl)-2,4-dimethyl-1H-pyrrol-3-yl)(3-hydroxyphenyl)methanone
tert-butyl(2-(3-(5-(5-ethyl-1,3,4-oxadiazol-2-yl)-2,4-dimethyl-1H-pyrrole-3-carbonyl)phenoxy)ethyl)carbamate
(3-((2-aminoethoxy)phenyl)(5-(5-ethyl-1,3,4-oxadiazol-2-yl)-2,4-dimethyl-1H-pyrrol-3-yl)methanone
(3-bromo-4-hydroxyphenyl)(5-(5-ethyl-1,3,4-oxadiazol-2-yl)-2,4-dimethyl-1H-pyrrol-3-yl)methanone
tert-butyl(2-(2-bromo-4-(5-(5-ethyl-1,3,4-oxadiazol-2-yl)-2,4-dimethyl-1H-pyrrole-3-carbonyl)phenoxy)ethyl)carbamate
(4-((2-aminoethoxy)-3-bromophenyl)(5-(5-ethyl-1,3,4-oxadiazol-2-yl)-2,4-dimethyl-1H-pyrrol-3-yl)methanone
(5-((5-cyclopropyl-1,3,4-oxadiazol-2-yl)-2,4-dimethyl-1H-pyrrol-3-yl)(3-hydroxyphenyl)methanone
(3-((2-aminoethoxy)phenyl)(5-(5-cyclopropyl-1,3,4-oxadiazol-2-yl)-2,4-dimethyl-1H-pyrrol-3-yl)methanone
(3-bromo-4-hydroxyphenyl)(5-(5-cyclopropyl-1,3,4-oxadiazol-2-yl)-2,4-dimethyl-1H-pyrrol-3-yl)methanone
tert-butyl(2-(2-bromo-4-(5-(5-cyclopropyl-1,3,4-oxadiazol-2-yl)-2,4-dimethyl-1H-pyrrole-3-carbonyl)phenoxy)ethyl)carbamate
(4-((2-aminoethoxy)-3-bromophenyl)(5-(5-cyclopropyl-1,3,4-oxadiazol-2-yl)-2,4-dimethyl-1H-pyrrol-3-yl)methanone
(3-bromo-4-(2-(dimethylamino)ethoxy)phenyl)(5-(5-cyclopropyl-1,3,4-oxadiazol-2-yl)-2,4-dimethyl-1H-pyrrol-3-yl)methanone

3-((5-bromo-2-methoxybenzoyl)-5-(5-cyclopropyl-1,3,4-oxadiazol-2-yl)-4-methyl-1H-pyrrole-2-carboaldehyde
(5-bromo-2-methoxyphenyl)(5-(5-cyclopropyl-1,3,4-oxadiazol-2-yl)-2-(hydroxymethyl)-4-methyl-1H-pyrrol-3-yl)methanone
(3-bromophenyl)(5-(5-cyclopropyl-1,3,4-oxadiazol-2-yl)-4-methyl-2-vinyl-1H-pyrrol-3-yl)methanone
3-((3-bromobenzoyl)-5-(5-cyclopropyl-1,3,4-oxadiazol-2-yl)-4-methyl-1H-pyrrole-2-carboaldehyde
(3-bromo-4-methoxyphenyl)(5-(5-cyclopropyl-1,3,4-oxadiazol-2-yl)-2-(1,3-dithian-2-yl)-4-methyl-1H-pyrrol-3-yl)methanone
3-((3-bromo-4-methoxybenzoyl)-5-(5-cyclopropyl-1,3,4-oxadiazol-2-yl)-4-methyl-1H-pyrrole-2-carboaldehyde
(3-bromophenyl)(5-(5-cyclopropyl-1,3,4-oxadiazol-2-yl)-2-(hydroxymethyl)-4-methyl-1H-pyrrol-3-yl)methanone
(3-bromophenyl)(5-(5-cyclopropyl-1,3,4-oxadiazol-2-yl)-2-(difluoromethyl)-4-methyl-1H-pyrrol-3-yl)methanone
(3-bromo-4-methoxyphenyl)(5-(5-cyclopropyl-1,3,4-oxadiazol-2-yl)-2-(hydroxymethyl)-4-methyl-1H-pyrrol-3-yl)methanone
(3-bromo-4-methoxyphenyl)(5-(5-cyclopropyl-1,3,4-oxadiazol-2-yl)-2-(difluoromethyl)-4-methyl-1H-pyrrol-3-yl)methanone
(3-bromo-4-methoxyphenyl)(5-(5-cyclopropyl-1,3,4-oxadiazol-2-yl)-2-(1-hydroxyethyl)-4-methyl-1H-pyrrol-3-yl)methanone
(3-((3-bromo-4-methoxybenzoyl)-5-(5-cyclopropyl-1,3,4-oxadiazol-2-yl)-4-methyl-1H-pyrrol-2-yl)methyl 2-((((9H-fluoren-9-yl)methoxy)carbonyl)amino)acetate
(3-bromo-4-methoxyphenyl)(5-(5-cyclopropyl-1,3,4-oxadiazol-2-yl)-4-methyl-2-(piperidin-1-ylmethyl)-1H-pyrrol-3-yl)methanone
(3-bromophenyl)(2,4-dimethyl-5-(3-methyl-1,2,4-oxadiazol-5-yl)-1H-pyrrol-3-yl)methanone
(3-bromophenyl)(5-(5-ethyloxazol-2-yl)-2,4-dimethyl-1H-pyrrol-3-yl)methanone
(5-bromo-2-methoxyphenyl)(5-(5-(1-bromocyclopropyl)-1,3,4-oxadiazol-2-yl)-2,4-dimethyl-1H-pyrrol-3-yl)methanone
(5-bromo-2-methoxyphenyl)(1-bromo-5-(5-(1-bromocyclopropyl)-1,3,4-oxadiazol-2-yl)-2,4-dimethyl-1H-pyrrol-3-yl)methanone
(3-bromophenyl)(2,4-dimethyl-5-(1,3,4-oxadiazol-2-yl)-1H-pyrrol-3-yl)methanone
(5-((5-(2-aminoethyl)-1,3,4-oxadiazol-2-yl)-2,4-dimethyl-1H-pyrrol-3-yl)(3-bromophenyl)methanone
2-((2-(5-(4-(3-bromobenzoyl)-3,5-dimethyl-1H-pyrrol-2-yl)-1,3,4-oxadiazol-2-yl)ethyl)isoindoline-1,3-dione
3-((3-bromobenzoyl)-5-(2-(2-(1,3-dioxoisoindolin-2-yl)ethyl)-2H-tetrazol-5-yl)-4-methyl-1H-pyrrole-2-carboaldehyde
2-((2-(5-(4-(3-bromobenzoyl)-5-(hydroxymethyl)-3-methyl-1H-pyrrol-2-yl)-2H-tetrazol-2-yl)ethyl)isoindoline-1,3-dione
(5-((2-(2-aminoethyl)-2H-tetrazol-5-yl)-2-(hydroxymethyl)-4-methyl-1H-pyrrol-3-yl)(3-bromophenyl)methanone
3-((3-bromobenzoyl)-5-(2-(2-(1,3-dioxoisoindolin-2-yl)ethyl)-2H-tetrazol-5-yl)-N,4-dimethyl-1H-pyrrole-2-carboxamide
5-((2-(2-aminoethyl)-2H-tetrazol-5-yl)-3-(3-bromobenzoyl)-N,4-dimethyl-1H-pyrrole-2-carboxamide
3-((3-bromobenzoyl)-5-(2-(2-(1,3-dioxoisoindolin-2-yl)ethyl)-2H-tetrazol-5-yl)-4-methyl-1H-pyrrole-2-carbonitrile
5-((2-(2-aminoethyl)-2H-tetrazol-5-yl)-3-(3-bromobenzoyl)-4-methyl-1H-pyrrole-2-carbonitrile
(3-bromophenyl)(5-(2-(2-hydroxyethyl)-2H-tetrazol-5-yl)-2-(2-hydroxypropan-2-yl)-4-methyl-1H-pyrrol-3-yl)methanone
(4-bromo-2-methyl-5-(1H-tetrazol-5-yl)-1H-pyrrol-3-yl)(3-bromophenyl)methanone
2-((5-(3-bromo-4-(3-bromobenzoyl)-5-methyl-1H-pyrrol-2-yl)-2H-tetrazol-2-yl)ethyl acetate
(4-bromo-5-(2-(2-hydroxyethyl)-2H-tetrazol-5-yl)-2-methyl-1H-pyrrol-3-yl)(3-bromophenyl)methanone
tert-butyl(2-(5-(3-bromo-4-(3-bromobenzoyl)-5-methyl-1H-pyrrol-2-yl)-2H-tetrazol-2-yl)ethyl)carbamate
(5-((2-(2-aminoethyl)-2H-tetrazol-5-yl)-4-bromo-2-methyl-1H-pyrrol-3-yl)(3-bromophenyl)methanone
tert-butyl(2-(2-(1-(2-(tert-butoxycarbonylaminoethyl)-5-(5-(hydroxymethyl)-1,3,4-oxazol-2-yl)-2,4-dimethyl-1H-pyrrole-3-carbonyl)-4-bromophenoxy)ethyl)carbamate
ethyl 5-(2-(2-acetoxyethyl)-2H-tetrazol-5-yl)-3-(3-bromo-4-methoxybenzoyl)-4-methyl-1H-pyrrole-2-carboxylate
ethyl 3-(3-bromo-4-methoxybenzoyl)-5-(2-(2-hydroxyethyl)-2H-tetrazol-5-yl)-4-methyl-1H-pyrrole-2-carboxylate
(5,5'-(5,5'-(disulfanediylbis(methylene))bis(1,3,4-oxadiazole-5,2-diyl))bis(2,4-dimethyl-1H-pyrrole-5,3-diyl))bis((3-bromophenyl)methanone)
2-((5-(4-(3-bromo-4-methoxybenzoyl)-3-methyl-5-(methylcarbamoyl)-1H-pyrrol-2-yl)-2H-tetrazol-2-yl)ethyl acetate and

2-((5-(4-(3-bromobenzoyl)-3-methyl-5-(methylcarbamoyl)-1H-pyrrol-2-yl)-2H-tetrazol-2-yl)ethyl dihydrogen phosphate.

**8.** A pharmaceutical composition comprising a compound according to any one of claims 1 to 7 or a salt thereof.

**9.** An anticancer agent comprising a compound according to any one of claims 1 to 7 or a salt thereof as an active ingredient.

**10.** The anticancer agent according to claim 9, wherein the anticancer agent is an anticancer agent against a cancer having the potential to acquire drug resistance due to the involvement of P-glycoprotein and/or cancer already having the resistance.

**11.** The anticancer agent according to claim 9, wherein the anticancer agent is an anticancer agent against a cancer having the potential to acquire drug resistance due to the involvement of TUBB3 and/or a cancer already having the resistance.

**12.** A tubulin polymerization inhibitor comprising a compound according to any one of claims 1 to 7 or a salt thereof as an active ingredient.

**13.** An apoptosis inducer comprising a compound according to any one of claims 1 to 7 or a salt thereof as an active ingredient.

**14.** The compound according to any one of claims 1 to 7 or a salt thereof for use in the treatment of cancer.

**15.** The compound according to claim 14 or a salt thereof, wherein the cancer is a cancer having the potential to acquire drug resistance due to the involvement of P-glycoprotein and/or a cancer already having the resistance.

**16.** The compound according to claim 14 or a salt thereof, wherein the cancer is cancer having the potential to acquire drug resistance due to the involvement of TUBB3 and/or cancer already having the resistance.

**17.** The compound according to any one of claims 1 to 7 or a salt thereof for use in the inhibition of tubulin polymerization.

**18.** The compound according to any one of claims 1 to 7 or a salt thereof for use in the induction of apoptosis.

**19.** Use of a compound according to any one of claims 1 to 7 or a salt thereof for the production of an anticancer agent.

**20.** The use of according to claim 19, wherein the anticancer agent is an anticancer agent against a cancer having the potential to acquire drug resistance due to the involvement of P-glycoprotein and/or a cancer already having the resistance.

**21.** The use of according to claim 19, wherein the anticancer agent is an anticancer agent against a cancer having the potential to acquire drug resistance due to the involvement of TUBB3 and/or a cancer already having the resistance.

**22.** Use of a compound according to any one of claims 1 to 7 or a salt thereof for the production of a tubulin polymerization inhibitor.

**23.** Use of a compound according to any one of claims 1 to 7 or a salt thereof for the production of an apoptosis inducer.

**24.** A method for treating cancer, comprising administering an effective amount of a compound according to any one of claims 1 to 7 or a salt thereof.

**25.** The method according to claim 24, wherein the cancer is a cancer having the potential to acquire drug resistance due to the involvement of P-glycoprotein and/or a cancer already having the resistance.

**26.** The method according to claim 24, wherein the cancer is a cancer having the potential to acquire drug resistance due to the involvement of TUBB3 and/or a cancer already having the resistance.

**27.** A method for inhibiting tubulin polymerization, comprising administering an effective amount of a compound according to any one of claims 1 to 7 or a salt thereof.

**28.** A method for inducing apoptosis, comprising administering an effective amount of a compound according to any one of claims 1 to 7 or a salt thereof.

Figure 1

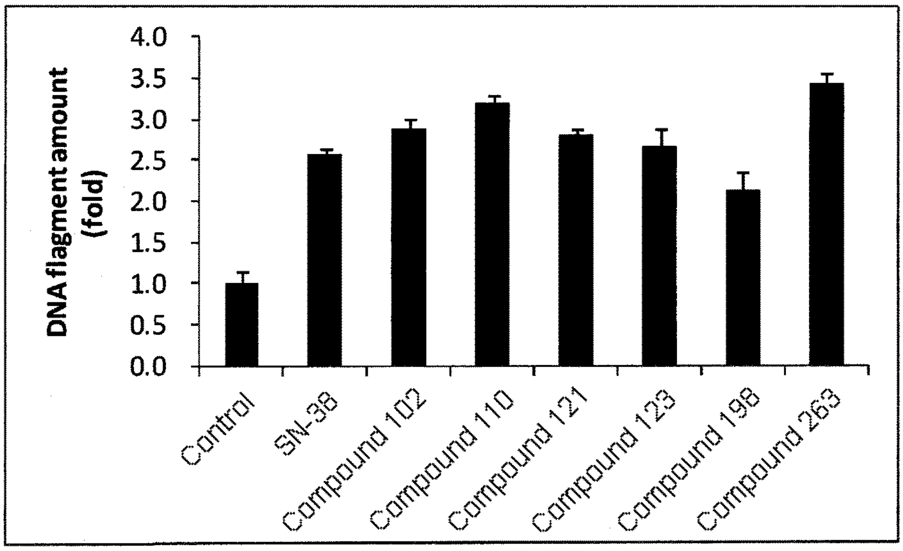


Figure 2

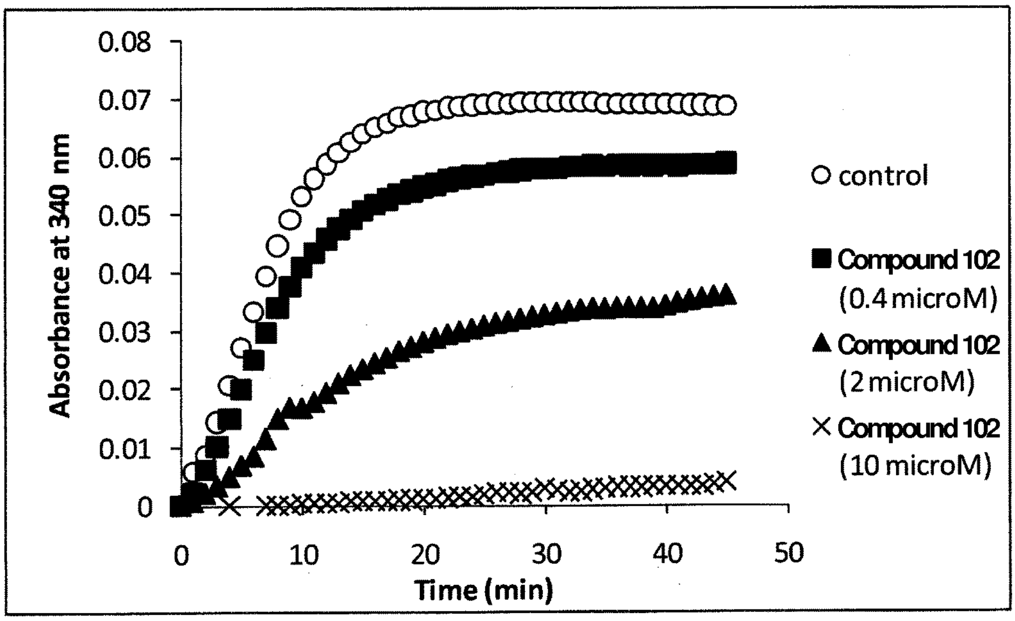

Figure 3

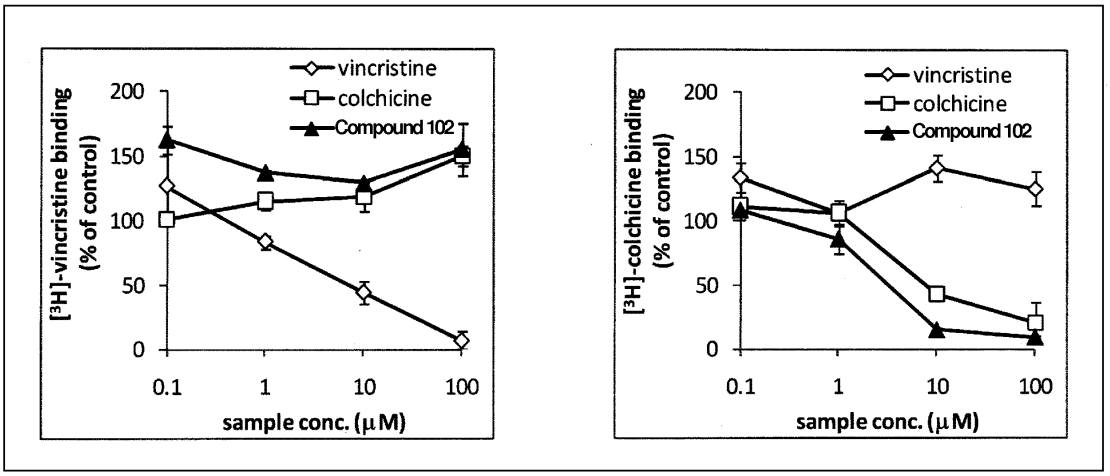
vincristine
colchicine
Compound 102
[³H]-vincristine binding (% of control)
200
150
100
50
0
0.1
1
10
100
sample conc. (μM)
vincristine
colchicine
Compound 102
[³H]-colchicine binding (% of control)
200
150
100
50
0
0.1
1
10
100
sample conc. (μM)

Figure 4

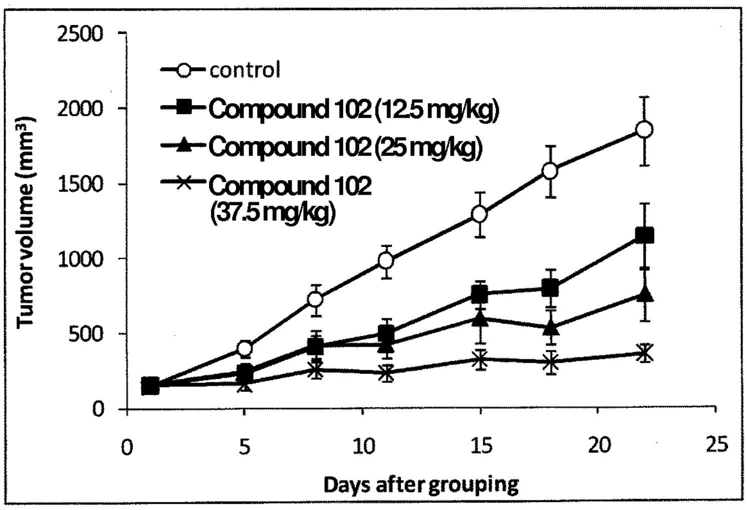


Figure 5

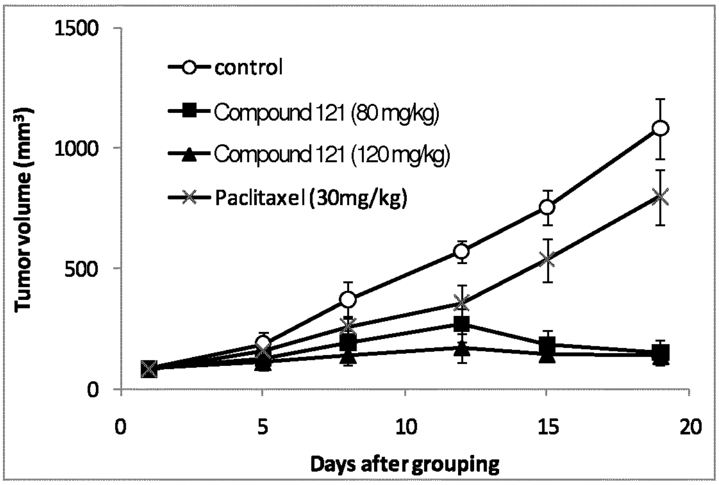

**INTERNATIONAL SEARCH REPORT**

International application No. PCT/JP2013/051497

A. CLASSIFICATION OF SUBJECT MATTER
*C07D403/04*(2006.01)i, *A61K31/41*(2006.01)i, *A61K31/422*(2006.01)i, *A61K31/4245*(2006.01)i, *A61K31/454*(2006.01)i, *A61K31/496*(2006.01)i, *A61P35/00*(2006.01)i, *C07D413/04*(2006.01)i, *C07D413/14*(2006.01)i

According to International Patent Classification (IPC) or to both national classification and IPC

B. FIELDS SEARCHED

Minimum documentation searched (classification system followed by classification symbols)
C07D403/04, A61K31/41, A61K31/422, A61K31/4245, A61K31/454, A61K31/496, A61P35/00, C07D413/04, C07D413/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CA/REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>A | PLASKON,A.S. et al, A synthesis of 5-hetaryl-3-(2-hydroxybenzoyl) pyrroles, Tetrahedron, 2008, Vol.64, No.25, p.5933-5943, Compound 3g,3i,3l, 6f,6i | 1,3-6,14-18<br>2,7-13,19-23 |
| X<br>A | EL-SADEK,M.M. et al, Synthesis and spectral studies of 3-benzoyl-1,2-dimethyl-5-(5-aryl-1,3,4-oxadiazol-2-yl) pyrrole, Egyptian Journal of Chemistry, 1995, Vol.38, No.4, p.435-441, Compound 8-10 | 1,3-6,14-18<br>2,7-13,19-23 |
| X<br><br>A | ODA,K. et al, Photochemistry of nitrogen-thiocarbonyl systems. 33. Photoreaction of arenecarbothioamides with furan. Facile synthesis of pentagonal di- and tri-heterocyclic compounds, Heterocycles, 1999, Vol.50, No.1, p.277-282, Compound 3d | 1,3,5,6,<br>14-18<br>2,4,7-13,<br>19-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
11 April, 2013 (11.04.13)

Date of mailing of the international search report
23 April, 2013 (23.04.13)

Name and mailing address of the ISA/
Japanese Patent Office

Facsimile No.

Authorized officer

Telephone No.

Form PCT/ISA/210 (second sheet) (July 2009)

INTERNATIONAL SEARCH REPORT

International application No.
PCT/JP2013/051497

C (Continuation). DOCUMENTS CONSIDERED TO BE RELEVANT

| Category* | Citation of document, with indication, where appropriate, of the relevant passages | Relevant to claim No. |
|---|---|---|
| X<br>A | DHAWAN,R. et al, Palladium-Catalyzed Multicomponent Coupling of Alkynes, Imines, and Acid Chlorides: A Direct and Modular Approach to Pyrrole Synthesis, Journal of the American Chemical Society, 2004, Vol.126, No.2, p.468-469, Compound 2e | 1,5,6,14-18<br>2-4,7-13, 19-23 |
| A | STAPLES,O.D. et al, Characterization, chemical optimization and anti-tumor activity of a tubulin poison identified by a p53-based phenotypic screen, Cell Cycle, 2008, Vol.7, No.21, p.3417-3427, Fig.2 | 1-23 |
| A | WO 2004/108685 A1 (AVENTIS PHARMA S.A.), 16 December 2004 (16.12.2004), Revendications; examples<br>& JP 2006-526596 A & US 2005/0014765 A1<br>& EP 1641765 A1 & KR 10-2006-0006850 A<br>& CN 1829697 A | 1-23 |
| A | WO 2003/011833 A1 (AVENTIS PHARMA S.A.), 13 February 2003 (13.02.2003), Revendications; example 5-4<br>& JP 2005-503376 A & US 2004/0162276 A1<br>& EP 1414805 A1 | 1-23 |

Form PCT/ISA/210 (continuation of second sheet) (July 2009)

**INTERNATIONAL SEARCH REPORT**

International application No.
PCT/JP2013/051497

**Box No. II Observations where certain claims were found unsearchable (Continuation of item 2 of first sheet)**

This international search report has not been established in respect of certain claims under Article 17(2)(a) for the following reasons:

1. [x] Claims Nos.: 24-28
because they relate to subject matter not required to be searched by this Authority, namely:
Claims 24-28 involve "methods for treatment of the human body or animal body by surgery or therapy".

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Expert. Opin. Investig. Drugs,* 2008, vol. 17 (5), 707-722
- *Oncogene,* 2003, vol. 22, 7280-7295
- *Cancer Investigation,* 2005, vol. 23, 264-273